(19) Europäisches Patentamt
European Patent Office
Office européen des brevets

(11) EP 3 533 420 A1

(12) # EUROPEAN PATENT APPLICATION
published in accordance with Art. 153(4) EPC

(43) Date of publication:
**04.09.2019 Bulletin 2019/36**

(21) Application number: **17865214.5**

(22) Date of filing: **30.10.2017**

(51) Int Cl.:
***A61F 2/91*** *(2013.01)*
***A61L 31/02*** *(2006.01)*
***A61L 31/04*** *(2006.01)*
***A61L 31/14*** *(2006.01)*
***A61F 2/915*** *(2013.01)*

(86) International application number:
**PCT/KR2017/012057**

(87) International publication number:
**WO 2018/080255 (03.05.2018 Gazette 2018/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **28.10.2016 KR 20160142423**

(71) Applicant: **Nextbiomedical Co., Ltd.**
**Incheon 21990 (KR)**

(72) Inventors:
- **LEE, Don Haeng**
  **Seoul 04322 (KR)**
- **PARK, Jong Chae**
  **Sejong-si 30101 (KR)**
- **KIM, Dong Gon**
  **Incheon 21954 (KR)**

(74) Representative: **V.O.**
**P.O. Box 87930**
**2508 DH Den Haag (NL)**

(54) **METHOD FOR CALCULATING DIAMETER OF STENT AFTER COMPRESSION, METHOD FOR MANUFACTURING WIRE-MESH STENT, AND METHOD FOR PATTERNING POLYMORPHIC STRUCTURE THEREOF**

(57) The present disclosure relates to a wire-mesh stent having a radius of the outmost boundary defined by the following Formula 1 and consisting of a combination of a hook and a cross, a method for manufacturing the same and a method for patterning a multi-alterable structure thereof:

## Formula 1

Formula 1

$$R_{ob} = R_{tb}/(\cos(180/N_{xo}))$$

The present disclosure microscopically analyzes and quantifies a basic structure of a wire-mesh stent from a viewpoint of integrating, rather than separating, a stent and a delivery device which are main components of a stent system, especially in order to ensure the loadability that can be applied to the delivery device, which is a practical necessity. Moreover, the present disclosure forms a multi-alterable structure pattern that is transformable to various application structures in order to realize a required specific performance, instead of one specific stent structure.

FIG. 43

schematic view of the structural patterning configuration of wire-mesh stent

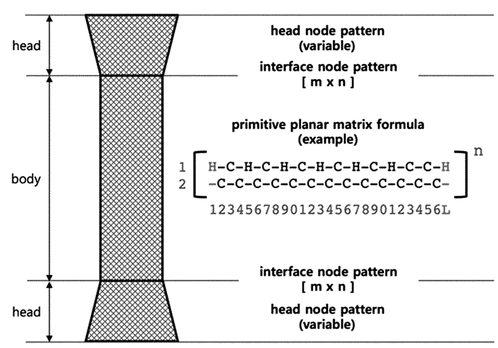




Printed by Jouve, 75001 PARIS (FR)

## Description

[Technical Field]

The present disclosure relates to a method for calculating a diameter of a stent after compression, method for manufacturing a wire-mesh stent, and a method for patterning a multi-alterable structure thereof, more particularly to a wire-mesh stent having a radius of the outmost boundary defined by Formula 1 and consisting of a combination of a hook and a cross, a method for manufacturing the wire-mesh stent and a method for patterning a multi-alterable structure of the wire-mesh stent.

[Background Art]

A stent, which is a cylindrical medical device inserted into the lumen of the human body (FIG. 1), may be classified depending on the organs to which it is applied. However, more broadly, it can be largely classified into a plastic stent, a tubular stent or a wire stent depending on the material or shape of the stent (FIG. 2).

Because the stent is a medical device used in the field of interventional medical operation based on the concept of minimally invasive approach, it should be used together with a delivery device which delivers (moves) the stent to a target site (FIG. 3). Therefore, in addition to the performance of the stent itself, the structure of the stent considering loadability for easy loading into the delivery device is very important.

Particularly, because the delivery device should move through the lumen of the human body, development of a stent structure considering the loadability of the delivery device is expected to be the major focus of in the development of a stent, particularly a wire stent, because smaller thickness, i.e., smaller diameter, is necessary to minimize patient's pain and improve operation convenience.

The loadability of a stent can be understood more easily if the way how the stent is loaded is known. In the method commonly used at present, the stent is loaded by compressing into the delivery device (push type) or, conversely, by pulling the stent (pull type) (FIG. 4).

Therefore, for easy loading into the delivery device, it is desired that the stent has a smaller diameter such that the compressed volume is smaller when compressed in the radial direction.

This is a technical factor reflecting the performance of the stent and may be defined as a compression ratio. That is to say, because it means how much the stent can be compressed, it may be said that a stent with a higher compression ratio can be loaded into a smaller (thinner) delivery device.

In order to consider the compression ratio related with the loadability of a delivery device having a small diameter, it is necessary to microscopically analyze the morphological structure of a wire stent occurring during meshing so as to provide a sufficiently stable compressed structure.

In addition, the present disclosure has been designed due to the necessity of a method for forming or patterning a multi-alterable stent structure, which is capable of customizing performance depending on the characteristics of the stent.

The existing art has the following problems. First, it describes one specific structure and a method for meshing the structure. Second, it merely describes the order of meshing without technical description of the major features of the meshing method. Third, the meshing method is described very difficult to understand and applicability to altered structure (pattern) is very limited. Fourth, because it is focused on embodiment of a specific function of the stent itself from a macroscopic point of view, the analytical approach from a microscopic point of view for considering the loadability (loading accessibility) into a delivery device, which moves a stent loaded therein to a target site, is insufficient.

Microscopically, a wire-mesh stent has a pattern of a multi-layer structure unlike a tubular stent which forms a single-layer pattern through laser processing, etc.

The core of the tubular stent structure lies in the shape and arrangement of a strut and a cell. The strut serves as a frame/axis of the structure and the (open or closed) state of the cell is determined depending on how the strut is connected (FIG. 5).

In addition, because the structure of a tubular stent consisting of a strut and a cell has the cross section of the cylindrical tube as a basic layer (thickness) and all patterns are formed from various struts and cells on the single cross section, it can be seen as a stent having a single layer (FIG. 6).

In contrast, a wire-mesh stent has simple and basic structural units called a hook and a cross because a wire is crossed or bent to form a specific structure (shape or pattern). The hook is a structural form formed when two wires are meshed with each other and the cross is a structural form formed when two wires cross each other (FIG. 7).

Due to this structural feature of the wire meshing, the wire-mesh stent can be seen to have a multi-layer structure, rather than a single-layer structure. Because multiple layers are formed, investigation from a microscopic point of view is necessary for satisfactory loading into a delivery device.

Although the basic form of the hook and cross is very simple, patterns of a wire-mesh stent having various

mechanical performance are formed depending on the arrangement of the hook and the cross.

A specific structure (shape) of the wire-mesh stent may be formed through manual labor or machinery work. When machinery work is employed, a stent structure consisting only of a cross is formed because the meshing work such as bending, etc. is difficult. A typical stent of this type is the wall stent (U.S. Pat. No. 4,655,771) (FIG. 8).

There is also a stent structure consisting only of a hook formed through manual labor. A typical example is shown in FIG. 9 (KR Pat. No. 267019).

Although the basic form of the hook and cross is very simple, patterns of a wire-mesh stent having various mechanical performance are formed depending on the arrangement of the hook and the cross.

Accordingly, it can be said that the core of the wire-mesh stent structure lies in the shape and arrangement (deployed pattern, hereinafter referred to as structure pattern) of hooks and crosses. The stent may exhibit different performance characteristics depending on the structure pattern.

To describe the two stents briefly, a stent consisting only of crosses exhibits good loadability into a delivery device but shows disadvantages in terms of shortening, axial force and anti-migration performance.

For reference, a smaller degree of shortening is advantageous because, when a stent having a large diameter is loaded into a delivery device having a small diameter after being compressed it is elongated as compared to the original length of the stent, it is difficult to accurately load the stent at the target site during medical operation.

And, the axial force refers to the stretching out force of a stent along the axial direction. If the axial force is large, the stent has a higher tendency of maintaining a straight state and causes pain when inserted into the intestine by forcibly stretching the intestine. Therefore, a smaller axial force is advantageous.

The anti-migration property refers to prevention of the movement of the inserted stent away from the target site due to intestinal motility. Therefore, a higher anti-migration property is advantageous.

A stent consisting only of hooks is superior in the shortening, axial force and anti-migration performance as compared to a stent consisting only of crosses. However, because of volume increase due to the hook and additional volume increase due to the formation of twisted lines (FIG. 10), the loadability into a delivery device is negatively affected greatly. For this reason, a delivery device with a larger diameter has to be used as compared to when a stent consisting only of crosses is used. The increased diameter leads to decreased flexibility, causing trouble for both patients and operators due to the difficulty in bending when the stent is inserted into the human body and moved inside the intestinal canal.

In addition, it has disadvantages in terms of fatigue fracture of the stent caused by long-term peristaltic motion of the esophagus, large intestine, etc. as compared to the cross structure.

Because a stent consisting only of hooks or crosses has the extreme advantages and disadvantages of the wire-mesh stent as described above, an appropriate combination of hooks and crosses, which can basically ensure stable loadability into a delivery device, will be more effective for the wire-mesh stent structure.

In addition, because organs such as the upper esophagus, lower esophagus, duodenum, biliary tract, hepatobiliary tract, pancreas, ascending colon, transverse colon, descending colon, sigmoid colon, rectum, etc. have intrinsic structures, functions and motion characteristics, a method for multi-alterable structure patterning of a wire-mesh stent, capable of forming a stent structure appropriate for each organ rather than a single structure with limited multi-alterability, is necessary.

A number of literatures and patent documents are cited throughout the specification. The disclosures of the cited literatures and patent documents are incorporated herein by reference so that the level of the technical art to which the present disclosure belongs and the content of the present disclosure can be described more clearly.

## [Disclosure]

[Technical Problem]

The inventors of the present disclosure have made efforts to develop a method for forming a stent structure considering loadability for each loading into a delivery device as well as the performance of the stent itself. As a result, they have fabricated a wire-mesh stent having a basic structure of the wire-mesh stent defined by a compression ratio ($r_{comp}$) by microscopically analyzing and quantifying the basic structure of the wire-mesh stent from a viewpoint of integrating, rather than separating, especially in order to ensure the loadability that can be applied to the delivery device, which is a practical necessity, and have developed a method for describing the structure pattern and change of the wire-mesh stent easily through an easily understandable symbolic expression. In addition, they have developed various technical embodiments of hooks and crosses, which are basic components of the wire-mesh stent that can change the function, performance and embodiment of the wire-mesh stent variously, and have developed a method for forming a multi-alterable structure pattern that can be applied to various applications, rather than one specific stent structure.

Accordingly, the present disclosure is directed to providing a wire-mesh stent having a radius of the outmost boundary defined by Formula 1 and consisting of a combination of a hook and a cross.

The present disclosure is also directed to providing a method for manufacturing the wire-mesh stent of the present disclosure described above.

The present disclosure is also directed to providing a method for patterning a multi-alterable structure of the wire-mesh stent of the present disclosure described above.

Other features and advantages of the present disclosure will become more apparent from the following detailed description, the drawings and claims.

[Technical Solution]

In an aspect of the present disclosure, the present disclosure provides a wire-mesh stent having a compression ratio ($r_{comp}$) defined by the following Formula 1 and consisting of a combination of a hook and a cross.

**Formula 1**

$$r_{comp} = (D_{stent} - D_{ob}) / D_{stent}$$

In Formula 1, the $D_{stent}$ represents a diameter of a stent before compression, the $D_{ob}$ represents a diameter of the stent after compression, $D_{ob} = 2R_{ob}$, and the $R_{ob}$ represents a radius of the outmost boundary.

In an exemplary embodiment of the present disclosure, the radius of the outmost boundary ($R_{ob}$) is defined by the following Formula 2.

**Formula 2**

$$R_{ob} = \frac{R_{tb}}{\cos(180/N_{xo})}$$

In Formula 2, the $R_{tb}$ represents an inradius of the outmost boundary and is defined by the following Formula 3, and the $N_{xo}$ represents the number of virtual hook nodes tangential on the outmost boundary and is defined by the following Formula 4.

**Formula 3**

$$R_{tb} = R_{ib} + H_{hook}$$

**Formula 4**

$$N_{xo} = \left\| \frac{180}{\tan^{-1}(W_{hook}/(2R_{tb}))} \right\|$$

In Formula 4, the $W_{hook}$ represents a nominal width of a hook node and is defined by the following Formula 4-1.

**Formula 4-1**

$$W_{hook} = \Phi_w \times SF_{wh}$$

In another exemplary embodiment of the present disclosure, in Formula 4-1, the $\Phi_w$ represents a diameter of a wire, the $SF_{wh}$ represents a width scale factor of a hook node and has a value of 3.3, and in Formula 4, the $R_{tb}$ represents an inradius of the outmost boundary and is defined by the following Formula 5, and the number of virtual hook nodes tangential on the outmost boundary ($N_{xo}$) is a round to the nearest integer satisfying the boundary condition of the following Formula 6.

**Formula 5**

$$R_{tb} = R_{ib} + H_{hook}$$

**Formula 6**

$$\left\| \frac{2\pi R_{tb}}{W_{hook}} \right\| \leq N_{xo} \leq \left\| \frac{2\pi R_{ob}}{W_{hook}} \right\|, R_{ib} < R_{tb} < R_{ob}$$

In Formula 5, the $R_{ib}$ represents a radius of the inmost boundary, and the $H_{hook}$ represents a nominal height of a hook node.

In another exemplary embodiment of the present disclosure, in Formula 4, if **($R_{ob}$ - $R_{tb}$) << $H_{hook}$**, the number of virtual hook nodes tangential on the outmost boundary ($N_{xo}$) is an integer satisfying the boundary condition of the following Formula 7:

**Formula 7**

$$\frac{180}{\tan^{-1}(W_{hook}/(2R_{tb}))} \approx \frac{2\pi R_{tb}}{W_{hook}}$$

In another exemplary embodiment of the present disclosure, in Formula 4, the radius of the inmost boundary ($R_{ib}$) is defined by the following Formula 8 and the nominal height of a hook node ($H_{hook}$) is defined by the following Formula 9:

**Formula 8**

$$R_{ib} = \frac{W_{hook}}{2 \cdot \tan(180/N_{xi})}$$

**Formula 9**

$$H_{hook} = \Phi_w \times SF_{hh}$$

In Formula 8, the $N_{xi}$ represents the number of virtual hook nodes tangential on the inmost boundary and, in Formula 9, the $SF_{hh}$ represents a height scale factor of a hook node and has a value of 3.

More specifically, the number of virtual hook nodes tangential on the inmost boundary ($N_{xi}$) is the largest round to the nearest integer satisfying the boundary condition of the following Formula 10:

**Formula 10**

$$N_h < \mathrm{Max}\ \{ N_{xi} \} \leq \| (W_{avg} \times N_t) / W_{hook} \|$$

More specifically, the $W_{avg}$ represents the average nominal width of node per section of the stent and is defined by the following Formula 11, the $N_t$ represents the total number of node per section of the stent and is defined by the following Formula 12, and the $N_h$ represents the number of hook node per section of the stent:

**Formula 11**

$$W_{avg} = W_{total} / N_t$$

**Formula 12**

$$N_t = N_h + N_c$$

In Formula 12, the $N_c$ represents the number of cross node per section of the stent.

More specifically, the $W_{total}$ represents the sum total of nominal width of all nodes per section of the stent and is defined by the following Formula 13:

**Formula 13**

$$W_{total} = W_{hook} \times N_h + W_{cross} \times N_c$$

In Formula 13, the $W_{cross}$ represents the nominal width of a cross node and is defined by $\mathbf{W_{cross}} = \Phi_w \times \mathbf{SF_{wc}}$ (Formula 14), and the $SF_{wc}$ represents the width scale factor of a cross node and has a value of 2.

In another exemplary embodiment of the present disclosure, the number of hooks satisfying the compression ratio defined by the Formula 1 is an integer from 3 to 8 per radial section of the stent.

In another exemplary embodiment of the present disclosure, the stent is made of a material selected from a group consisting of a metal, a synthetic polymer and a natural polymer.

In another exemplary embodiment of the present disclosure, the metal is selected from a group consisting of a nickel-titanium (Ni-Ti) shape memory alloy, a martensitic nickel-titanium (Ni-Ti) shape memory alloy, stainless steel, tantalum, tungsten (W), gold (Au), platinum, silver (Ag), nickel, titanium (Ti), chromium (Cr), a cobalt-chromium (Co-Cr) alloy, a platinum-chromium (Pt-Cr) alloy, a platinum-iridium (Pt-Ir) alloy and a magnesium alloy.

In another exemplary embodiment of the present disclosure, the synthetic polymer is a degradable or non-degradable polymer or a combination thereof.

More specifically, the degradable polymer is selected from a group consisting of poly(lactic acid) and a copolymer thereof, poly(glycolic acid) and a copolymer, poly(hydroxy butyrate), poly(ε-caprolactone) and a copolymer thereof, poly(alkylene succinate), polyanhydride and poly(ortho ester).

More specifically, the non-degradable polymer is selected from a group consisting of polyamide (nylon), polycyanoacrylate, polyphosphazene, thermoplastic polyurethane, low-density polyethylene, poly(vinyl alcohol), poly(ethylene oxide), poly(hydroxyethyl methacrylate), poly(methyl methacrylate), poly(tetrafluoroethylene) (PTFE), polydimethylsiloxane, poly(ethylene oxide-b-propylene oxide)), poly(vinyl methyl ether), poly(N-alkyl acrylamide), polyethylene terephthalate and polypropylene.

In another exemplary embodiment of the present disclosure, the natural polymer is selected from a group consisting of collagen, albumin, silk protein, poly(L-lysine), poly(L-glutamic acid), poly(aspartic acid), polysaccharide and a derivative thereof, carboxymethyl cellulose, cellulose sulfate, agarose, alginate, carrageenan, hyaluronic acid, heparin, glycosaminoglycan, dextran and a derivative thereof, and chitosan and a derivative thereof.

In another aspect of the present disclosure, the present disclosure provides a method for manufacturing a wire-mesh stent, including: (a) a phase of confirming a planar matrix wherein a plurality of nodes formed by horizontal node lines and vertical node lines crossing each other are represented by coordinates (i, j), as shown in FIG. 35; (b) a phase of selecting a movement type of wire meshing from sandglass-type movement, as shown in FIG. 44; (c) a phase of selecting a start node position (S#(i, j)) by identifying the number of the horizontal node lines, the number of the vertical node lines, node position information, node shape information and figure-cutting line information; (d) a phase of selecting one basic movement direction (D.mv) from the start node position (S#(i, j)) of the phase (c); (e) a phase of moving to a neighbor node along the basic movement direction (D.mv) of the phase (d); and (f) a phase of completing wire meshing by selecting one basic movement direction (D.mv) at the neighbor node of the phase (e) and selecting the start node position (S#(i, j)) as an end node position (E#(i, j)).

In an exemplary embodiment of the present disclosure, if sandglass-type movement is selected in the phase (b), the basic movement direction of the phase (d) is selected from a group consisting of horizontal to left ($H_L$), horizontal to right ($H_R$), diagonal to left up ($D_{LU}$), diagonal to left down ($D_{LD}$), diagonal to right up ($D_{RU}$) and diagonal to right down ($D_{RD}$).

In another exemplary embodiment of the present disclosure, if radial movement is selected in the phase (b),

the basic movement direction of the phase (d) is selected from a group consisting of horizontal to left ($H_L$), horizontal to right ($H_R$), diagonal to left up ($D_{LU}$), diagonal to left down ($D_{LD}$), diagonal to right up ($D_{RU}$), diagonal to right down ($D_{RD}$), vertical to up ($V_U$) and vertical to down ($V_D$).

In another exemplary embodiment of the present disclosure, the method further includes, after the phase (e), (e-1) a phase of repeating moving from the neighbor node of the phase (e) to another neighbor node along the basic movement direction (D.mv) of the phase (d).

In another exemplary embodiment of the present disclosure, the neighbor node of the phase (e) has a node shape of a hook or a cross selected from Table 3, FIGS. 45-51 and FIG. 53.

In another exemplary embodiment of the present disclosure, the method further includes, after the phase (f), (g) a phase of expressing a fabrication expression and verifying a planar mesh.

In another exemplary embodiment of the present disclosure, the method is conducted according to a flow chart of FIG. 64a.

In another aspect of the present disclosure, the present disclosure provides a multi-alterable structure patterning method of a wire-mesh stent, including: (a) a patterning policy phase of selecting at least one of a primitive n-gon, rotation angle ($\theta_{rot}$), rotation direction, number of crosses per side and section layer, number of primitive section layers, skip and loop type; (b) a parameter design phase of setting up design parameters according to the selection of the phase (a) and verifying the design parameters, compression ratio, symmetry and circularity; (c) a patterning configuration phase of deciding the exponent of a primitive node pattern, configuring a primitive planar matrix formula, setting up a balancing node line, selecting technical hooks and crosses, configuring an interface node pattern and configuring a planar matrix; and (d) a fabrication expression phase of selecting a movement type of meshing, configuring a fabrication expression and configuring a primitive planar mesh.

In an exemplary embodiment of the present disclosure, the primitive n-gon of the phase (a) is a regular or irregular polygon, and the n is at least one integer from 3 to 8.

In another exemplary embodiment of the present disclosure, the predetermined rotation angle ($\theta_{rot}$) is within a range defined by the following Formula 15, and the predetermined rotation direction is a clockwise direction (forward direction) or a counterclockwise direction (reverse direction):

## Formula 15

$$0 \leq \theta_{rot} < 360°$$

wherein, if the primitive n-gon is a regular polygon, $\theta_{rot}$ is not a rotation angle corresponding to an integer multiple of $\|360/vertex(n)\|$.

In another exemplary embodiment of the present disclosure, the number of the node radial sections is an integer which is equal to or greater than the number of the selected primitive n-gon if the predetermined rotation angle ($\theta_{rot}$) is 0° and is equal to or smaller than the number of angle division ($N_{ang}$) and is equal to or greater than the number of the selected primitive n-gon if the predetermined rotation angle ($\theta_{rot}$) > 0, and the number of angle division satisfies the following Formula 16:

## Formula 16

$$N_{ang} = \| 360°/\theta_{rot} \| .$$

In another exemplary embodiment of the present disclosure, the skip of the phase (a) is selected from missing skip and pushing skip. For missing skip, the number of primitive section layers is identical before and after the skip. But, for pushing skip, the number of section layers satisfies the following Formula 17:

## Formula 17

$$N_{lay.after} = N_{lay.before} + N_{skip.push}$$

In Formula 17, the $N_{lay.after}$ represents the number of primitive section layers after the skip, the $N_{lay.before}$ represents the number of primitive section layers before the skip, and the $N_{skip.push}$ represents the number of pushed section layers.

In another exemplary embodiment of the present disclosure, the loop type of the phase (a) is open loop type

or close loop type.

In another exemplary embodiment of the present disclosure, the method further includes, after the phase (a), (a-1) a phase of selecting combination or outburst.

More specifically, the combination of the phase (a-1) is parameter combination or pattern combination.

More specifically, the parameter combination combines at least one selected from a group consisting of rotation angle, rotation direction, primitive n-gon and crosses.

More specifically, the pattern combination is forward pattern combination or reverse pattern combination.

In another exemplary embodiment of the present disclosure, the compression ratio of the phase (b) is defined by the following Formula 1:

## Formula 1

$$r_{comp} = (D_{stent} - D_{ob}) / D_{stent}$$

In Formula 1, the $D_{stent}$ represents the diameter of the stent before compression, the $D_{ob}$ represents the diameter of the stent after compression, $D_{ob} = 2R_{ob}$, and the $R_{ob}$ represents the radius of the outmost boundary.

In the multi-alterable structure patterning method of a wire-mesh stent of the present disclosure, the description of the compression ratio is omitted to avoid unnecessary complexity of the present disclosure because it is the same as that mentioned above with reference to the wire-mesh stent of the present disclosure.

In another exemplary embodiment of the present disclosure, the symmetry ($Q_{symm}$) of the phase (b) is defined by the following Formula 18:

## Formula 18

$$Q_{symm} = 1 - (CV_{N.h.horiz} + CV_{N.h.vert} + CV_{\Delta.h.horiz})/3$$

wherein $CV_{N.h.horiz} = (SD/MV)_{N.h.horiz}$ (Formula 19), $CV_{N.h.vert} = (SD/MV)_{N.h.vert}$ (Formula 20), $CV_{\Delta.h.horiz} = (SD/MV)_{\Delta.h.horiz} = \Sigma(CV_{\Delta.h.horiz.i\text{-}th})_{i,\ i=1\ to\ N.lay} / N_{lay}$ (Formula 21) and $\Delta$.h.horiz = $N_{H\text{-}H.vert}$ (Formula 22), wherein the CV is a coefficient of variation, the SD is a standard deviation, the MV is a mean value, the N.h.horiz is the number of hooks per horizontal node line, the N.h.vert is the number of hooks per vertical node line, the $\Delta$.h.horiz is the distance between neighbor hooks per horizontal node line, the $\Delta$.h.horiz.i-th is the distance between neighbor hooks per i-th horizontal node line, the N.lay (or $N_{lay}$) is the number of four or more primitive section layers except a skip node line, the $N_{H\text{-}H.vert}$ is the number of vertical node lines between neighbor hooks per horizontal node line, a vertical node line in which the whole vertical node line is composed of null node is excluded from calculation, and the symmetry ($Q_{symm}$) is 0.5 or greater.

It is preferred that the symmetry ($Q_{symm}$) is higher. Most specifically, the symmetry ($Q_{symm}$) is 1.

In another exemplary embodiment of the present disclosure, the $N_{H\text{-}H.vert}$ of Formula 22 is defined by the following Formula 23:

## Formula 23

$$N_{H\text{-}H.vert} = \theta_{H\text{-}H} / \theta_{div}$$

wherein $\theta_{H\text{-}H} = N_{H\text{-}H.vert} \times \theta_{div} = N_{H\text{-}H.vert} \times W_{n.line}$ (Formula 24), the $\theta_{H\text{-}H}$ is the angle contained between neighbor hooks, the $\theta_{div}$ represents the division angle between nodes, and the $W_{n.line}$ is a node line width.

In another exemplary embodiment of the present disclosure, the circularity ($Q_{circ}$) of the phase (b) is defined by the following Formula 25:

## Formula 25

$$Q_{circ} = Q_{circ.simple} \times (1 - CV_{avg})$$

wherein $Q_{circ.simple} = n \times \sin(180°/n)/\pi$ (Formula 26), $CV_{avg} = (CV_{\Delta h.radial} + CV_{N.h.vert})/2$ (Formula 27), $CV_{.h.radial} = (SD/MV)_{\Delta.h.radial}$ (Formula 28), $CV_{N.h.vert} = (SD/MV)_{N.h.vert}$ (Formula 29) and n = N.h.radial (or $N_{h.radial}$) (Formula 30), wherein the $Q_{circ.simple}$ represents simple circularity assuming a regular n-gon, the $CV_{avg}$ represents an average coefficient

of variation, the CV is a coefficient of variation, the SD is a standard deviation, the MV is a mean value, the n is the number of sides of the regular n-gon (= total number of hooks on the axial view), the $N_{.h.radial}$ is the total number of hooks on the axial view, the $\Delta_{.h.radial}$ is the perpendicular distance of neighbor hooks on the axial view, the $N_{.h.vert}$ is the number of hooks per vertical node line, a vertical node line in which the whole vertical node line is composed of null node is excluded from calculation, and the circularity ($Q_{circ}$) is 0.4 or greater. It is preferred that the circularity ($Q_{circ}$) is higher. Most specifically, the circularity ($Q_{circ}$) is 1.

In another exemplary embodiment of the present disclosure, the exponent of a primitive node pattern of the phase (c) is an integer which is 1 or greater. More specifically, it is an integer from 1 to 60.

In another exemplary embodiment of the present disclosure, the technical hook and cross of the phase (c) is at least one hook and cross selected from Table 3, FIGS. 45-51 and FIG. 53.

In another exemplary embodiment of the present disclosure, the movement type of meshing of the phase (d) is sandglass-type movement (6-way) or radial movement (8-way) shown in FIG. 44.

In another exemplary embodiment of the present disclosure, the multi-alterable structure of the wire-mesh stent is selected from FIG. 84c, FIG. 85c, FIGS. 88c-91c, FIG. 92d, FIG. 93c, FIG. 94c and FIG. 95d.

[Advantageous Effects]

The features and advantages of the present disclosure may be summarized as follows:

(a) The present disclosure provides a wire-mesh stent having a compression ratio ($r_{comp}$) defined by Formula 1 and consisting of a combination of a hook and a cross, a method for manufacturing the same and a method for patterning a multi-alterable structure thereof.
(b) The present disclosure primarily considers from a viewpoint of integrating, rather than separating, a stent and a delivery device which are main components of a stent system, especially in order to ensure the loadability that can be applied to the delivery device, which is a practical necessity.
(c) In addition, the present disclosure microscopically analyzes and quantifies the basic structure of the wire-mesh stent in order to ensure practicable loadability.
(d) And, the present disclosure describes the structure pattern and change of the wire-mesh stent easily through an easily understandable symbolic expression.
(e) The present disclosure also provides various technical embodiments of hooks and crosses, which are the basic elements of the wire-mesh stent, capable of varying the function, performance and fabrication method of the wire-mesh stent.
(f) And, the present disclosure forms a multi-alterable structure pattern that is transformable to various application structures in order to realize a required specific performance, instead of one specific stent structure.

[Brief Description of Drawings]

FIG. 1 shows a stent, which is a cylindrical medical device inserted into the lumen of the human body.
FIG. 2 shows a plastic stent, a tube stent and a wire stent, which are different in terms of materials or shape.
FIG. 3 shows a delivery device which moves a stent to a target site.
FIG. 4 shows a process of loading a stent either by compressing the stent into a delivery device (push type) or, conversely, by pulling the stent (pull type).
FIG. 5 shows the structure of a tubular stent depending on the arrangement of a strut and a cell.
FIG. 6 shows a tubular stent having a single layer.
FIG. 7 shows a hook and a cross of a wire-mesh stent.
FIG. 8 shows a wall stent consisting only of crosses (U.S. Pat. No. 4,655,771).
FIG. 9 shows a stent structure consisting only of hook (KR Pat. No. 267019) fabricated through manual labor.
FIG. 10 shows a twisted line formed in a stent consisting only of hooks.
FIG. 11 shows 3D scanning images of a stent having hooks, crosses and twisted lines.
FIG. 12 shows a model of a hook, a cross and a twisted line of a stent in three directions (top, side and section).
FIG. 13 shows twisted lines formed on a hook and a cross.
FIG. 14 shows section views Model A consisting only of 13 hooks and Model B wherein 6 hooks and 7 crosses are arranged alternatingly with predetermined angles along the radial section of the stent.
FIG. 15 schematically shows compression modeling with no interference for Models A and B of FIG. 14.
FIG. 16 shows model interference and physical interference in a hook-and-cross node model.
FIG. 17 shows determination of $R_{ob}$ (radius of outmost boundary), which is an important factor in evaluating compression ratio, using a hook-and-cross node model described in the present disclosure.

FIG. 18 shows calculation of the inradius of a regular n-gon.

FIG. 19 shows calculation of $R_{ib}$ using the number of hook nodes, $N_{xi}$, by forming a regular n-gon with sides of the same length by introducing a nominal width ($W_{hook}$) of a hook node.

FIG. 20 shows setting of a boundary condition for $N_{xi}$.

FIG. 21 shows that, in calculation of the radius of the outmost boundary $R_{ob}$, the circumcenter of $R_{ob}$ is identical to the incenter of $R_{ib}$.

FIG. 22 shows calculation of the radius $R_{ob}$ of a circumcircle through the number of sides ($N_{xo}$) formed by a hook node with a length tangential to the circumcircle being $W_{hook}$.

FIG. 23 shows that an incircle of a hook node exists in addition to a circumcircle.

FIG. 24 shows twisted lines in a hook-and-cross node model.

FIG. 25 shows modeling of the twisted lines of FIG. 24.

FIG. 26 shows a result of evaluating compression ratio for twisted line node models, Model A and Model B.

FIG. 27 shows the structure of a stent consisting of a body and heads.

FIG. 28 shows the heads of a stent with the shape of a flare type, dumbbell type and jar type.

FIG. 29 shows the section of a stent.

FIG. 30 shows the section of a stent consisting of a hook, a cross, or a combination of a hook and a cross.

FIG. 31 shows node position, etc. when a plurality of sections, rather than a single section, are superposed continuously.

FIG. 32 shows a plan view for understanding the structure pattern of a stent widely, clearly and systematically.

FIG. 33 shows a top view of a stent having a cylindrical shape.

FIG. 34 shows a planar figure of a stent having a cylindrical shape.

FIG. 35 shows a result of expressing the planar figure or planar mesh of a stent planar grid and a matrix coordinate expression.

FIG. 36 describes a meshing method using 12 planar matrices and shows the finally superposed single structure pattern (KR Patent Application No. 10-2008-0076586).

FIG. 37 shows planar matrix formulas expressed by the symbolic expression of the present disclosure for Model A consisting only of hooks and Model B consisting of a combination of hooks and crosses.

FIG. 38 shows a primitive planar matrix formula according to an exemplary embodiment of the present disclosure.

FIG. 39 shows a primitive node pattern consisting of node pattern elements.

FIG. 40 shows primitive planar matrix formulas for open loop type and closed loop type.

FIG. 41 shows a primitive planar matrix formula for closed loop type.

FIG. 42 shows a node pattern element, i.e., interface node pattern, at the portion connecting a body portion and a head portion of a stent.

FIG. 43 schematically shows the structure pattern of a wire-mesh stent.

FIG. 44 shows sandglass-type movement (6-way, ⧗) and radial movement (8-way, ✳) according to an exemplary embodiment of the present disclosure, with regard to a method for manufacturing (meshing) a wire-mesh stent.

FIG. 45 shows a horizontal hook as an example of a technical hook of the present disclosure.

FIG. 46 shows a vertical hook as an example of a technical hook of the present disclosure.

FIG. 47 shows a horizontal open hook as an example of a technical hook of the present disclosure.

FIG. 48 shows a vertical open hook as an example of a technical hook of the present disclosure.

FIG. 49 shows a horizontal half hook as an example of a technical hook of the present disclosure.

FIG. 50 shows a vertical half hook as an example of a technical hook of the present disclosure.

FIG. 51 shows additional forms of a technical hook.

FIG. 52 shows a formatted type of fabrication expression for a (meshing) method of completing a structure pattern using 3 wires.

FIG. 53 shows notations of a half cross passing a cross node.

FIG. 54 shows the primitive planar matrix formula of structure patterns of Model A consisting only of hooks and Model B consisting of a combination of hooks and crosses.

FIG. 55 shows an upper interface node pattern and a lower interface node pattern of Model A.

FIG. 56 shows a fabrication expression with no twisted line, which expresses the interface node pattern of FIG. 55 sequentially from a start point to an end point.

FIG. 57 shows a planar mesh the wire meshing of which has been completed according to the fabrication expression of FIG. 56.

FIG. 58 shows a planar matrix including an upper interface node pattern and a lower interface node pattern of Model B.

FIG. 59 shows a fabrication expression with no twisted line, which expresses the interface node pattern of FIG. 55 sequentially from a start point to an end point.

FIG. 60 shows a planar mesh the wire meshing of which has been completed according to the fabrication expression of FIG. 59.
FIG. 61 shows a primitive planar matrix formula and a planar matrix for Model B when the exponent of a primitive node pattern n is 2.
FIG. 62 shows a fabrication expression for FIG. 61.
FIG. 63 shows a planar mesh the wire meshing of which has been completed according to FIG. 61 and FIG. 62.
FIGS. 64a-64c show flow charts for systemically determining a fabrication expression for a fabrication (meshing) method according to an exemplary embodiment of the present disclosure.
FIG. 65 shows primitive shapes from a triangle to an octagon based on the analysis result that an appropriate number of hooks per radial section, i.e., horizontal node line, of wire-mesh stent is 3-8.
FIG. 66 shows the radial section of a stent seen from the axial direction.
FIG. 67 shows a result of arranging node radial sections sequentially in the form of an isometric view with reference to the axis.
FIG. 68 shows a case wherein triangles inscribed to node radial sections are arranged for each node radial section.
FIG. 69 shows a result of arranging hook nodes on vertices of a 'primitive n-gon' by applying the primitive n-gon to FIG. 67.
FIG. 70 shows an isometric view of sequentially arranged node radial sections of FIGS. 68 and 69 seen from the axial direction.
FIG. 71 shows that the phase of hook nodes located at the vertices of a primitive regular triangle can be changed by applying 'rotation', which is the second concept of multi-alterability.
FIG. 72 shows a result of comparing symmetry and circularity by combining the concept of 'rotation' and 'section layer'.
FIG. 73 shows a result of missing and pushing 'skip' of a 'section layer' with a rotation angle of 180° from the accumulated rotation angles 0°, 90°, 180°, 270° and 360° (= 0°) when the rotation angle ($\theta_{rot}$) is 90°.
FIG. 74 shows a result of expressing FIG. 73 with a primitive planar matrix formula.
FIG. 75 shows a primitive planar matrix formula wherein missing and pushing of 'skip' are applied for a combination of two, i.e., k = 2, wherein the first 'skip' occurs at an accumulated rotation angle ($\theta_{accu}$) of 180° and the second 'skip' occurs at an accumulated rotation angle ($\theta_{accu}$) of 90°.
FIG. 76 shows a pattern combination showing that an interface node pattern may be necessary.
FIG. 77a shows a symmetry combination as a pattern combination.
FIG. 77b shows a reverse symmetry combination as a pattern combination.
FIG. 78 shows a parameter combination wherein a primitive regular triangle and a primitive regular tetragon are applied simultaneously and a rotation angle ($\theta_{rot}$) of 90° is applied.
FIG. 79 shows a method of arranging cross nodes according to an exemplary embodiment of the present disclosure. It shows a primitive planar matrix formula wherein cross nodes are arranged on 3 sides of a primitive regular triangle and a rotation angle of 90° is applied.
FIG. 80 shows that the number of hooks present on the same phase may be different in the symmetry evaluation of a multi-alterable structure pattern.
FIG. 81 shows the basic concept of evaluating the circularity of a multi-alterable structure pattern.
FIGS. 82a-82e show a simple flow chart of multi-alterable structure patterning according to an exemplary embodiment of the present disclosure.
FIGS. 83a-83d show a detailed flow chart of the flow chart of FIG. 82a.
FIG. 84a, FIG. 84b and FIG. 84c show Example 1 (REG-TRIA-90-L-12) of multi-alterable structure patterning for a regular triangle ('primitive n-gon') with the exponent of a primitive node pattern being 2, i.e., n = 2.
FIG. 85a, FIG. 85b and FIG. 85c show Example 2 (REG-TRIA-180-L-12) of multi-alterable structure patterning for a regular triangle ('primitive n-gon') with the exponent of a primitive node pattern being 4, i.e., n = 4.
FIG. 86a and FIG. 86b show a process of applying an equation of symmetry evaluation for Example 1 (REG-TRIA-90-L-12) and Example 2 (REG-TRIA-180-L-12) of the present disclosure.
FIG. 87a and FIG. 87b show a process of applying an equation of circularity evaluation for Example 1 (REG-TRIA-90-L-12) and Example 2 (REG-TRIA-180-L-12) of the present disclosure.
FIG. 88a, FIG. 88b and FIG. 88c show Example 3 (REG-TRIA-15-R-COMB-12) of multi-alterable structure patterning for a regular triangle ('primitive n-gon') with the exponent of a primitive node pattern being 2, i.e., n = 2.
FIG. 89a, FIG. 89b and FIG. 89c show Example 4 (REG-TETR-18-R-SKIP-20) of multi-alterable structure patterning for a regular tetragon ('primitive n-gon') with the exponent of a primitive node pattern being 2, i.e., n = 2.
FIG. 90a, FIG. 90b and FIG. 90c show Example 5 (REG-TETR-45-RL-SKIP-20) of multi-alterable structure patterning for a regular tetragon ('primitive n-gon') with the exponent of a primitive node pattern being 3, i.e., n = 3.
FIG. 91a, FIG. 91b and FIG. 91c show Example 6 (REG-PENT-24-RL-COMB-20) of multi-alterable structure patterning for a regular pentagon ('primitive n-gon') with the exponent of a primitive node pattern being 1, i.e., n = 1.
FIG. 92a, FIG. 92b, FIG. 92c and FIG. 92d show Example 7 (REG-TRPH-0-L-COMB-12) of multi-alterable structure

patterning for a 'combination' of a regular triangle, a regular tetragon, a regular pentagon and a regular hexagon ('primitive n-gon') with the exponent of a primitive node pattern being 3, i.e., n = 3.
FIG. 93a, FIG. 93b and FIG. 93c show Example 8 (REG-TRPH-0-L-COMB-SYMM-12) of multi-alterable structure patterning for a symmetry combination of a regular triangle, a regular tetragon, a regular pentagon and a regular hexagon ('primitive n-gon') with the exponent of a primitive node pattern being 1, i.e., n = 1.
FIG. 94a, FIG. 94b and FIG. 94c show Example 9 (REG-HXTR-30/90-LR/L-COMB-PATN-18/12) of compound multi-alterable structure patterning with a half hook applied.
FIG. 95a, FIG. 95b, FIG. 95c and FIG. 95d show Example 10 (IRR-TRPH-20-L-COMB-14) of multi-alterable structure patterning for an irregular 'primitive n-gon' with a half cross applied.
FIG. 96a, FIG. 96b and FIG. 96c show Example 11 (IRR-TETR-NA-L-COMB-REVS-8/10/14) of a reverse symmetry combination of a compound structure pattern consisting of 8-node, 10-node and 14-node using an irregular tetragon.
FIG. 97 shows a top view, a side view and a sectional view of a hook-and-cross model of a stent.
FIG. 98 shows a top view, a side view and a sectional view of a twisted line formed on a hook and a cross of a stent.

[Best Mode]

Hereinafter, a wire-mesh stent, a method for manufacturing the same and a method for patterning a multi-alterable structure of the present disclosure are described in detail referring to the attached drawings. The exemplary embodiments described below are only for illustrating the present disclosure more specifically and the scope of the present disclosure is not limited by the exemplary embodiments.

Microscopic structural analysis of wire-mesh stent

For application of a method for patterning a multi-alterable structure, microscopic structural analysis of a wire-mesh stent is necessary.
Microscopic structural analysis is a process that has to be considered essentially for application of the multi-alterable structure patterning technology ensuring the loadability into a delivery device, which is required basically and commonly for a wire-mesh stent structure.
For microscopic structural analysis, a process of modeling a hook and a cross, which are the basic units of a wire-mesh stent, is necessary. It is also necessary to model a twisted line that may occur during the fabrication of a wire-mesh stent structure including a hook for comparative analysis.
In order to help understanding the modeling process, description will be given referring to a 3D scanning image of a stent having all of the hook, cross and twisted line (FIG. 11).
The hook, cross and twisted line of a stent can be modeled in along viewing directions (top, side and section) (FIG. 12, FIG. 97).
Because a twisted line can be formed either on a hook or on a cross (FIG. 13), both cases are modeled separately (FIG. 98).
Many hooks and crosses are formed in a stent meshed from a wire and each of them acts as it were a node in a network. Therefore, it will be referred to as a node hereinafter.
If a wire with a diameter of $\Phi$ is used, the physical height, width and length of each node are determined in consideration of scale factors at each node as sort of marginal factors in order to avoid interference with neighboring hook and cross nodes. They are referred to as nominal height, nominal width and nominal length, respectively.
For example, when a wire with $\Phi$ = 0.1 mm is used, the nominal height, nominal width and nominal length of the hook node are 0.3 mm, 0.33 mm and 0.4 mm, respectively.
The interference will be described further later.
Based on the modeling, a virtual stent structure is established as follows for comparative analysis of the loadability of the stent into a delivery device.
When seen from the radial section of the stent, Model A consists only of 13 hooks and Model B consists 6 hooks and 7 crosses which are arranged alternatingly with predetermined angles (FIG. 14).
The loadability can be evaluated as superior if the stent can be compressed along the radial direction as much as possible without interference with neighboring hooks or crosses.
FIG. 15 schematically shows compression modeling with no interference for the two virtual stent structures.
It can be said that the compression ratio is higher as the radius of the outmost boundary $R_{ob}$ is smaller after the compression of the stent.
The formula for calculating the $R_{ob}$ will be described later and, here, the interference is reviewed first.
The interference can be classified into model interference and physical interference.
The model interference means a kind of logical interference, occurring between cubic blocks when cubic blocks considering a scale factor are applied to a hook-and-cross node model proposed by the present disclosure, and the

physical interference means an actual interference in which mutual physical contact occurs actually in hook and cross nodes formed by an actual wire (FIG. 16).

Accordingly, the evaluation of the compression ratio of the stent structure for comparing loadability into a delivery device is conducted under a condition with no model or physical interference, i.e., under the tangent-on-boundary condition.

The compression ratio ($r_{comp}$) of the stent may be defined by the following Formula 1.

Formula 1

$$r_{comp} = (D_{stent} - D_{ob}) / D_{stent}$$

wherein $D_{stent}$ represents the diameter of the stent before compression, $D_{ob}$ represents the diameter of the after compression, and $D_{ob} = 2R_{ob}$.

However, because the comparison of compression ratio depending on the structure pattern is possible simply by comparing the radius ($R_{ob}$) of the outmost boundary when the stent is compressed under a condition with no mutual interference, $R_{ob}$ will be used in the present disclosure.

In order to achieve a structure pattern with improved loadability using the hook-and-cross node model described in the present disclosure, a quantitative analysis of the compression ratio is necessary. Therefore, a method for determining $R_{ob}$ (radius of outmost boundary), which is an important factor in evaluating the compression ratio, will be described.

$R_{ob}$ may be calculated from the inradius and circumradius formulas of a regular n-gon and the boundary condition.

Of the stent structures described above, for Model A consisting only of hooks, the circumradius $R_{ib}$ can be calculated easily because it has the same base ($W_{hook}$). However, for Model B consisting of a combination of hooks and crosses, $R_{ib}$ cannot be calculated directly and a boundary condition is necessary.

Accordingly, as a method that can be applied to a structure such as Model B consisting of a combination of hooks and crosses, a method for calculating the radius of the inmost boundary ($R_{ib}$) and the radius of the outmost boundary ($R_{ob}$) will be described.

This method can also be applied to a structure consisting only of hooks or crosses.

If a structure consisting of a combination of hooks and crosses is compressed under the tangent-on-boundary condition, all the hook and cross nodes are present inside the radius of the outmost boundary ($R_{ob}$) (FIG. 17).

Because the nominal height of the hook ($H_{hook}$) is larger than the nominal height of the cross ($H_{cross}$) (i.e., $H_{hook} > H_{cross}$), the boundary tangent on the hook node becomes the inmost boundary and $R_{ib}$ can be calculated from the formula of the incircle tangent on the base of the hook node.

However, as seen from FIG. 17, because the base of the hook node tangent on the inmost boundary does not form a regular n-gon as in FIG. 18, the formula for the inradius of a regular n-gon cannot be applied.

But, to examine FIG. 17 in detail, it can be seen that the radius $R_{ib}$ of the inmost boundary is inscribed to the bases of all the hook nodes at the incenter. Therefore, a regular n-gon having sides with the same length as the nominal width of the hook node ($W_{hook}$) can be formed (FIG. 19). Accordingly, $R_{ib}$ can be calculated easily if the number of the hook nodes $N_{xi}$ is known (FIG. 19).

However, the length of the circumference configured by the radius of the incircle $R_{ib}$, $S_{ib}$, should be equal to or smaller than the sum of total nominal widths of the nodes, $W_{hook} \times N_{xi}$. That is, $S_{ib} = 2\pi H_{ib} \leq W_{hook} \times N_{xi}$.

This equation can be derived from $\pi/(N_{xi} \tan(180/N_{xi})) \leq 1$ and is always established at $N_{xi} \geq 3$, which is the minimum polygon condition. Therefore, the solution cannot be obtained unless the boundary condition for $N_{xi}$ (or $R_{ib}$) is given.

The boundary condition for $N_{xi}$ for a combination hook and cross nodes can be set as follows. The following boundary condition is unnecessary for the case consisting only of hook nodes (because $N_{xi} = N_t = N_h$) or the case consisting only of cross nodes (because $N_{xi} = N_t = N_c$).

$N_{xi}$ has the largest integer value (max $\{N_{xi}\}$) from among the integers which are greater than the number of the hook nodes ($N_h$) and smaller than the sum of the nominal widths of ($W_{total}$) the hooks and crosses (FIG. 20).

This condition is defined by the following formulas.

$$2\pi R_{ib} \leq W_{hook} \times N_{xi} < 2\pi R_{ib.max} \leq W_{avg} \times N_t$$

$$W_{hook} \times N_{xi} < W_{avg} \times N_t$$

$$N_{xi} < (W_{avg} \times N_t) / W_{hook}$$

$$N_h < N_{xi} < (W_{avg} \times N_t) / W_{hook}$$

Since $N_{xi}$ is an integer,

$$N_h < \mathrm{Max}\,\{ N_{xi} \} \leq \|\, (W_{avg} \times N_t) / W_{hook} \,\|.$$

wherein $N_t = N_h + N_c$ and $W_{avg} = (W_{hook} \times N_h + W_{cross} \times N_c) / (N_h + N_c)$, wherein the $W_{hook}$ represents the nominal width of the hook, the $W_{cross}$ represents the nominal width of the cross, the $N_h$ is the number of the hooks, and the $N_c$ is the number of the crosses.

The radius of the inmost boundary, $R_{ib}$, can be calculated as follows.

$$R_{ib} = \frac{W_{hook}}{2 \cdot \tan(180/N_{xi})}$$

**here,**

**$N_{xi}$ is the largest integer in the boundary of**

$$N_h < \mathrm{Max}\,\{ N_{xi} \} \leq \|\, (W_{avg} \times N_t) / W_{hook} \,\|$$

The radius of the outmost boundary, $R_{ob}$, is calculated as follows.

Referring to FIG. 21, the distances ($R_{ob}$) from the center to the respective vertices of the hook node are equal. Thus, there exists a circumcircle which passes through the respective vertices and the center of the circumcircle, i.e., the circumcenter of $R_{ob}$, is identical to the incenter of $R_{ib}$.

Therefore, the radius $R_{ob}$ of the circumcircle for a regular $N_x$-polygon can be obtained if only the number of sides formed by the hook nodes with a length of $W_{hook}$ tangential on the circumcircle ($N_{xo}$) and the inradius of the incircle ($R_{tb}$) or the number of the sides ($N_x$) are given (FIG. 22).

To describe FIG. 22 in more detail, because the incircle also exists for the hook nodes as well as the circumcircle (FIG. 23), the number of the sides tangential on the hook nodes, $N_{xo}$, can be obtained from the radius of the incircle (inradius, $R_{tb}$) as follows.

$$R_{tb} = \frac{W_{hook}}{2 \cdot \tan(180/N_{xo})}$$

$$N_{xo} = \left\| \frac{180}{\tan^{-1}(W_{hook}/(2R_{tb}))} \right\|$$

where, $R_{tb} = R_{ib} + H_{hook}$

Since $N_{xo}$ is an integer, the round to the nearest integer number can be chosen.

The radius of the circumcircle, $R_{ob}$, can be obtained from the $N_{xo}$ as follows.

$$R_{ob} = \frac{R_{tb}}{\cos(180/N_{xo})}$$

Here, the following conditions are satisfied for the radius of the circumcircle ($R_{ob}$) and the incircle($R_{tb}$) tangential

on the hook nodes: a) The length of the circumference, $S_{ob}$, configured by the radius of the circumcircle, $R_{ob}$, should be greater than or equal to the sum total of the nominal width of the hook nodes, $W_{hook}$ x $N_{xo}$. That is, $S_{ob} = 2\pi R_{ob} \geq W_{hook}$ x $N_{xo}$. b) The length of the circumference, $R_{tb}$, configured by the radius of the incircle should be smaller than or equal to the sum total of the nominal width of the hook nodes, $W_{hook}$ x $N_{xo}$. That is, $S_{tb} = 2\pi R_{tb} \leq W_{hook}$ X $N_{xo}$.

$N_{xo}$ should satisfy the following conditional expression from these two conditions.

$$\left\| \frac{2\pi R_{tb}}{W_{hook}} \right\| \leq N_{xo} \leq \left\| \frac{2\pi R_{ob}}{W_{hook}} \right\| \quad \text{(here, } R_{tb} = R_{ib} + H_{hook}\text{)}$$

When $(R_{ob} - R_{tb})$ **<<** $H_{hook}$, the following condition is established, and thus, the value can be obtained from $N_{xo} = \| 2\pi R_{tb}/W_{hook} \|$.

$$\frac{180}{\tan^{-1}(W_{hook}/(2R_{tb}))} \approx \frac{2\pi R_{tb}}{W_{hook}}$$

A formula for calculating the compressed outmost radius, $R_{ob}$, by applying a hook-and-cross node model for determining compression ratio ($r_{comp}$), which is important in evaluating the compressing loadability of the wire-mesh stent consisting of a combination of hooks and crosses, is summarized as follows.

$$\mathbf{R_{ob} = \frac{R_{tb}}{\cos(180/N_{xo})}}$$

where,

$$N_{xo} = \left\| \frac{180}{\tan^{-1}(W_{hook}/(2R_{tb}))} \right\|$$

$$R_{tb} = R_{ib} + H_{hook}$$

here, $N_{xo}$ should be satisfied the following condition

$$\left\| \frac{2\pi R_{tb}}{W_{hook}} \right\| \leq N_{xo} \leq \left\| \frac{2\pi R_{ob}}{W_{hook}} \right\| \quad \text{and} \quad R_{ib} < R_{tb} < R_{ob}$$

And, in the case of $(R_{ob} - R_{tb}) << H_{hook}$ , the following condition may be applied

$$\frac{180}{\tan^{-1}(W_{hook}/(2R_{tb}))} \approx \frac{2\pi R_{tb}}{W_{hook}}$$

$$R_{ib} = \frac{W_{hook}}{2 \bullet \tan(180/N_{xi})}$$

here,
$N_{xi}$ is the largest integer in the boundary of

$$N_h < \text{Max}\{ N_{xi} \} \leq \| (W_{avg} \times N_t) / W_{hook} \|$$

But if for hook or cross only

$$N_{xi} = N_t = \begin{cases} N_h & : \text{for hook only} \\ N_c & : \text{for cross only} \end{cases}$$

$$W_{avg} = W_{total} / N_t$$

$$N_t = N_h + N_c$$

$$W_{total} = W_{hook} \times N_h + W_{cross} \times N_c$$

$$H_{hook} = \Phi_w \times SF_{hh}$$

$$W_{hook} = \Phi_w \times SF_{wh}$$

$$W_{cross} = \Phi_w \times SF_{wc}$$

here, the designated values of scale factor for each are follows:

$$SF_{hh} = 3$$

$$SF_{wh} = 3.3$$

$$SF_{wc} = 2$$

In the present disclosure, $R_{ob}$ represents the radius of the outmost boundary, $R_{tb}$ represents the inradius of the outmost boundary, $R_{ib}$ represents the radius of the inmost boundary, $N_{xi}$ represents the number of virtual hook nodes tangential on the inmost boundary, $N_{xo}$ represents the number of virtual hook nodes tangential on the outmost boundary, $N_t$ represents the total number of node per section of the stent, $N_h$ represents the number of hook node per section of the stent, $N_c$ represents the number of cross node per section of the stent, $\Phi_w$ represents the diameter of a wire, $W_{total}$ represents the sum total of nominal width of all nodes per section of the stent, $W_{avg}$ represents the average nominal width of node per section of the stent, $W_{hook}$ represents the nominal width of a hook node, $W_{cross}$ represents the nominal width of a cross node, $H_{hook}$ represents the nominal height of a hook node, $SF_{hh}$ represents the height scale factor of a hook node, $SF_{wh}$ represents the width scale factor of a hook node and $SF_{wc}$ represents the width scale factor of a cross node.

Using this formula for the compressed outmost radius, $R_{ob}(\Phi_w, N_h, N_c)$, the $R_{ob}$ values of Model A consisting only of 13 hooks and Model B consisting of a combination of 6 hooks and 7 crosses using the same wire with a diameter of 0.1 mm are calculated as follows.

$\Phi_w = 0.1$, $W_{hook} = 0.33$, $W_{cross} = 0.2$, $H_{hook} = 0.3$ : common parameter
$N_h = 13$, $N_{xi} = N_h = 13$, $R_{ib} = 0.669$, $R_{tb} = 0.969$, $N_{xo} = 19$ : for MODEL A
$N_h = 6$, $N_c = 7$, $N_{xi} = 10$, $R_{ib} = 0.508$, $R_{tb} = 0.808$, $N_{xo} = 16$ : for MODEL B

(continued)

$R_{ob}(0.1, 13, 0)|_{MODEL\ A}$ = 0.982
$R_{ob}(0.1, 6, 7)|_{MODEL\ B}$ = 0.824

To compare the $R_{ob}$ of the two models, Model B shows about 19% better result in the compression ratio than Model A.

To give a more practical explanation for the difference, manufacturers of a delivery device of a stent, which is commonly called a catheter, promote that they provide thinner delivery devices, marking in units of 0.1 fr (1 fr ≒ 0.33 mm).

Considering the two models, the compressed diameter is 1.964 mm for Model A and 1.684 mm for Model B. The difference is 0.28 mm, which corresponds to 0.85 fr. Accordingly, it can be said that Model B is better by about 9 times.

While establishing the hook-and-cross node model for evaluating the loadability of the stent into a delivery device, modeling was also conducted for the twisted line (FIG. 24, FIG. 25). A result of applying the twisted line node model to Model A and Model B for comparative evaluation of compression ratio is shown in FIG. 98 and FIG. 26. Usually, two twisted lines are formed symmetrically. In extreme cases, 6 twisted lines can be formed. For the sake of convenience, let's assume that the twisted lines are formed symmetrically at two hook nodes only.

The outmost radius $R_{ob.tw}$ of the two models having twisted line is as follows.

$\phi_w = 0.1, W_{hook} = 0.33, W_{hook.tw} = 0.33, W_{cross} = 0.2, H_{hook} = 0.3, H_{hook.tw} = 0.4$ : common parameter
$N_h = 13, N_{xi} = N_h = 13, R_{ib} = 0.669, R_{tb.tw} = 1.069, N_{xo} = 21$ : for MODEL A with twisted line
$N_h = 6, N_c = 7, N_{xi} = 10, R_{ib} = 0.508, R_{tb,tw} = 0.908, N_{xo} = 17$ : for MODEL B with twisted line

$R_{ob.tw}(0.1, 13, 0)|_{MODEL\ A.}$ = 1.081
$R_{ob.tw}(0.1, 6, 7)|_{MODEL\ B}$ = 0.924

To compare the compression ratio of the two models by comparing $R_{ob.tw}$ with $R_{ob}$ with no twisted line, Model A shows about 10% of decrease and Model B shows about 12% of decrease. They correspond to increase in the inner diameter of the delivery device by 0.6 fr for Model A and 0.6 fr for Model B. Therefore, a delivery device with a larger inner diameter should be used.

From the microscopic structural analysis result using the hook-and-cross node model, it can be seen that the formation of twisted lines during the fabrication of the wire-mesh stent should be avoided because the twisted lines negatively affect the loadability into a delivery device greatly.

At present, a nickel-titanium shape memory alloy (nitinol) wire with $\Phi$ = 0.11-0.22 mm is commonly used in the fabrication of a wire-mesh stent. However, some manufacturers suffer from deployment problem due to the formation of twisted lines.

Accordingly, for the wire-mesh stent structure, it is very important to adopt a fabrication (meshing) method capable of avoiding the formation of twisted lines.

Structure patterning expression of multi-alterable wire-mesh stent

Because the 'method for patterning a multi-alterable structure of a wire-mesh stent with superior loading compressibility into a delivery device' presented by the present disclosure can result in from tens to hundreds of structure patterning depending on variation, the configuration of the stent, structure patterning expression and structural fabrication expression will be described briefly before explaining the structure patterning method in detail.

Structure patterning expression and structural fabrication expression cannot be found in other patents related to a wire-mesh stent and are uniquely proposed by the present disclosure. For the single structure pattern proposed by other patents, the structure patterning expression presented by the present disclosure may be unnecessary because it is required to consider one structure only.

Because other patents describe the fabrication method (procedure) very complicatedly, it is difficult to understand and very long time is required for operators to be accustomed to the fabrication method of the structure pattern. It may be because the systematic concept and theory about the structure patterning are insufficient and the description is focused on a single structure pattern without considering the possibility of multi-alteration.

According to the present disclosure, multi-alteration of structure pattern is possible. Therefore, a method capable of expressing the structure pattern and fabrication sequence for various structures in a simple and systematic form is necessary.

First, a stent simply consists of a body and heads (FIG. 27). The main function of the heads located on both sides of the body is anti-migration. In general, the head has a shape of flare type, dumbbell type, jar type, etc. (FIG. 28).

The body and head may be designed to have a fine or sparse structure depending on the function and performance of the stent. A fine structure means one having many hook or cross nodes and a sparse structure means the opposite case. Although the term 'band' is often used in the related art, 'node' will be used in the present disclosure for the sake of clarity.

Since the structure pattern of the head portion is designed to be identical to the body structure pattern or is slightly modified depending on the shape of the head and since the body actually occupies the most part of the stent, the structure pattern of the stent body is of greater importance.

Accordingly, the method and sequence of forming the multi-alterable structure pattern will be described focusing on the structure pattern of the body.

The following description is given to help the understanding of the stent structure pattern of the present disclosure. The section of a stent (FIG. 29) may consist of hooks, crosses or a combination of hooks and crosses depending on the structure pattern (FIG. 30).

For a single section, the position and arrangement of hook and cross nodes are identified easily.

However, if a plurality of sections are superposed continuously, it is difficult to identify the node position and arrangement information, i.e., the structure pattern (FIG. 31).

Therefore, in order to analyze the structure pattern widely, clearly and systematically, the analysis has to be conducted on a plan view (or top view) (FIG. 32). It is also possible to conduct analysis using a 3D CAD file on a computer. However, the 2D plan view is better to clearly identify the structure pattern at a glance.

But, it is still difficult to identify the structure pattern of the stent having a cylindrical shape even on a top view due to superposition (FIG. 33).

For this reason, the structure pattern on the plan view is developed into a planar figure for easier understanding (FIG. 34).

Then, a planar mesh wherein the wire is spread like a net is obtained. In the planar mesh, the connection points (marked by ©) are hook and cross nodes.

And, the connection points (Ⓢ) located on the figure-cutting line (line L in FIG. 34) for cutting open the cylinder are not two different nodes but the same one node.

If all the connection points, i.e., nodes, in the planar mesh are connected horizontally and vertically, a planar grid looking like a checkerboard or a graph paper is obtained. The node positions can be marked accurately by expressing the cross points on the grid by matrix coordinate expression, i.e., (x, y) or (i, j) (FIG. 35). This matrix expression of the node positions will be referred to as a planar matrix in the present disclosure.

The horizontal (row) and vertical (column) lines in the planar grid and the planar matrix are referred to as horizontal node lines and vertical node lines, respectively. The distance between the horizontal node lines is called a node line width and the distance between the vertical node lines is called a node line height. The node line width ($W_{n.line,\ i\text{-}th}$) and the node line height ($H_{n.line,\ j\text{-}th}$) are not constant but may be changed depending on the required specific function or performance.

The planar matrix expression has been attempted previously for the wire-mesh stent structure pattern. However, as described above, because it is focused on the complicated fabrication (meshing) method or fabrication (meshing) sequence of a single structure pattern, the single structure pattern is described superposing a plurality of planar matrices. For example, KR Patent Application No. 10-2008-0076586 describes a meshing method of using as many as 12 planar matrices and presents the finally superposed single structure pattern (FIG. 36).

For a single structure pattern, description can be made by using several planar matrices although it is rather complicated and time-consuming. But, for a multi-alterable structure pattern which can be varied into from tens to hundreds of forms, this method is very ineffective and inefficient. Therefore, the present disclosure is directed to providing a structure patterning expression adequate for the multi-alterable structure pattern.

At present, the planar matrix expressed on a 2D plane is the most simple and clearly recognizable expression of the structure pattern of a wire-mesh stent. Accordingly, if the position and arrangement of hook and cross nodes can be accurately expressed on the planar matrix, it will be a sufficiently effective and useful expression.

The expression can be either graphical symbolic expression or textual symbolic expression. In the present disclosure, textual symbolic expression is applied for a multi-alterable structure pattern using a planar matrix, and graphical symbolic expression is applied for a structure pattern completed by meshing wires. A similar example is the expression of a chemical structural formula.

Therefore, in the present disclosure, a hook, a cross and a grid cross point with no hook or cross (null) are called nodes. The symbolic expression for the 3 nodes is as follows (Table 3). The hook node can be classified largely into a horizontal hook, a vertical hook, an open hook and a half hook, and is specifically classified into a horizontal open hook, a vertical open hook, a horizontal half hook and a vertical half hook. The cross node, which includes a cross and a half cross, will be described later because it is related with the required performance of the stent and the fabrication (meshing) method thereof.

[Table 3]

| category | node name | textual symbolic expression | graphical symbolic expression |
|---|---|---|---|
| | horizontal hook | H | ● |
| hook | vertical hook | $H_v$ (V) | ▲ |
| | horizontal open hook | $H_{ho}$ (O) | ○ |
| | vertical open hook | $H_{vo}$ (o) | Δ |
| | horizontal half hook | $H_{hh}$ (h) | ◒ |
| | vertical half hook | $H_{vh}$ (v) | ◣ |
| | cross | C | X |
| cross | half cross | $C_h$ (c) | / |
| null | null | - | |

The planar matrix formulas expressed by the symbolic expression designed by the present disclosure for Model A consisting only of hooks and Model B consisting of a combination of hooks and crosses, which have been described above as the examples for explaining the compression ratio relating to loadability into a delivery device, are as follows (FIG. 37). The expression is based on the assumption that the respective hooks and crosses arranged on the section in both models are arranged on the same axial line or on the same phase repeatedly and alternately.

The planar matrix formula is embraced by [ ]. The numbers on the left side (1, 2, 3, ..., i) indicate the number of the horizontal node line and the numbers at the bottom (1, 2, 3, ..., L) indicate the number of the vertical node line. And, the nodes located on the vertical node lines on the left and right sides, marked in red color, in the planar matrix are the same node because they are located on the figure-cutting line. The same node on the figure-cutting line is marked with L in the vertical node line.

The numbers of the horizontal and vertical node lines are repeated in the form of 1, 2, 3, ..., 8, 9, 0, 1, 2, ..., where 0 means 10. Therefore, the first 0 means 10 and the second 0 means 20. It is expressed such because, as the planar matrix forms a well-formatted grid, the arrangement of the node positions and vertical node line numbers in the planar matrix formula are dislocated if the numbers are expressed with two digits.

Referring to FIG. 37, it can be seen that the node pattern on the horizontal node line is repeated in the planar matrix formulas for Model A and Model B. Accordingly, an abbreviated version of planar matrix expression is necessary for simplification of the expression.

In the present disclosure, this is called a primitive planar matrix formula and the abbreviated node pattern is called a primitive node pattern. The primitive planar matrix formula is expressed as follows (FIG. 38).

The exponent n in the upper right corner is the value representing the minimum repetition allowing the formation of a completed structure pattern from the primitive node pattern through wire meshing, i.e., allowing the planar mesh expression. It is called the exponent of a primitive node pattern and n has a value of 1 or greater. That is, $n \geq 1$.

The primitive node pattern is composed of node pattern element(s) (FIG. 39), which may be plural.

There are two types of the primitive planar matrix formula: open loop type and closed loop type.

The open loop type refers to a type wherein the start node pattern element and the end node pattern element are different from each other, and the closed loop type refers to a type wherein the start node pattern element and the end node pattern element are identical. That is to say, in the open loop type, the primitive node pattern is maintained identical after repetition by the exponent of a primitive node pattern, n. In contrast, the closed loop type ends, after repetition by n times, by adding the start node pattern element (+1) (FIG. 40).

The closed loop type is marked by the symbol c (closed) at the lower right corner of the primitive planar matrix formula, i.e., as $[]_c$ (FIG. 41).

Because the primitive planar matrix formula expresses the structure pattern of the stent body, which plays a key role of the stent structure pattern, the determination of open loop type or closed loop type is made by the node pattern element connecting the body portion and head portion of the stent, i.e., the interface node pattern. Because the stent head portion can be designed into various forms to provide the anti-migration function, the determination of open loop type or closed loop type may be greatly variable (FIG. 42).

The interface node pattern is also used in the compound structure pattern wherein different primitive planar matrix formulas are combined to form a new structure pattern. Here, the interface node pattern serves to ensure the connectivity of different structure patterns and the retainability of the inherent structure patterns included in the connected different structure patterns, i.e., in the primitive planar matrix formula.

The biggest problem occurring when forming the compound structure pattern is the discrepancy of connection points, or the discontinuity of connection. Because it was difficult to solve this problem with the existing technology and method, it was difficult to realize a wire-mesh stent with a compound structure, capable of providing various function

and performance.

For example, a wire-mesh stent using hooks requires a process of returning to the start point to complete the meshing (fabrication) process. This problem is similar to the "Koenigsberg's bridge problem". Suppose that a certain number of hooks are present below and above a certain horizontal node line. For them to be connected without repetition, the numbers of the hooks on both sides should be equal. A virtual horizontal node line that splits such that the numbers of the hooks on both sides are equal is called a balancing node line. The location of the balancing node line is variable depending on the structure pattern. If the numbers of the hooks are different, it is mathematically impossible to pass through the horizontal node line and then return to the start point without repetition.

Accordingly, with the existing technology and method, connection cannot be achieved if there is no balancing node line when forming a compound structure consisting of a combination of different structure patterns. For this reason, focus is given on a single structure pattern that can be meshed (fabricated) regardless of the balancing node line, rather than on the compound structure capable of realizing various function and performance of the wire-mesh stent.

However, the present disclosure solves the connectivity problem by introducing the concept of interface node pattern.

The expression of the present disclosure solves the problem of discrepancy of connection points or discontinuity of connection by freely adjusting the number of hook nodes such that perfect connection of the compound structure in the interface node pattern and the multi-alterable structure pattern can be ensured mathematically, i.e., such that the balancing node line can exist. The adjusted hook is called a balancing hook.

When seen from the aspect of the hook and cross, the hook is a crossing point of connection where two wires are connected with each other, whereas the cross is a crossing point of passing where two wires pass each other. Because the existing technology and method are fixed on the idea that all the hooks constituting a stent body should be crossing points of connection, i.e., that two wires should be connected with each other, the possibility of various function and performance that can be realized by a wire-mesh stent has been limited.

If all the hooks constituting a stent body need not be crossings point of connection, i.e., if only one wire may pass without being connected with another wire, the corresponding hook serves as a turning point. This hook is called a half hook. The function and performance of the wire-mesh stent may be varied by changing the form of the hook. Details about the half hook will be described referring to the fabrication method.

Also, in the interface node pattern, not only the number of the balancing hooks but also the position thereof can be adjusted. Therefore, the shape of the hooks and crosses can be conserved well such that the function and performance of the structure pattern can be maintained when forming the compound structure pattern by combining different structure patterns.

FIG. 43 schematically shows the structure pattern of a wire-mesh stent.

Then, the fabrication expression used to describe (express) the fabrication (meshing) method of actually meshing the node structure pattern expressed by the primitive planar matrix formula using a wire is described.

The existing art describes the method of fabricating (meshing) the single structure pattern very lengthily and complicatedly. However, to describe the tens or hundreds of structure patterns that can be achieved by the multi-alterable structure pattern proposed by the present disclosure is practically very ineffective. Therefore, a fabrication expression capable of describing the fabrication (meshing) method plainly and clearly is necessary for effective application and description of the present disclosure.

At present, a stent having hook nodes is mostly fabricated through manual labor using a jig. There may be slight difference in the type of the jig or the application of machinery work depending on manufacturers. In general, a small hole of about 0.5-1.0 mm, capable of forming a structure pattern, is made on a cylindrical metal rod and a thin metal pin is stuck into the hole. A shape memory alloy (mostly, NiTi shape memory alloy) wire is wound (or bent) around the metal pin serving as a support and then is wound around another metal pin. A desired structure pattern is formed by repeating this procedure and, finally, the wire is fixed by knotting, welding, tube pressing, etc. The stent structure pattern completed at the jig of the metal rod is finished through heat treatment for shape memory setting together with the metal rod.

Both ends of the stent head mostly form hooks as long as they are for connection between nodes without special design purposes. Accordingly, a desired structure pattern may be formed by sequentially marking the movement path of the wire from the start point to the end point, with the hook node position at the center. Also, when a plurality of wires, rather than a single wire, are used, the fabrication (meshing) method can be explained very easily by the fabrication expression simply by distinguishing the movement path of each wire from the start point to the end point.

For faster understanding, imagine a very long snake (i.e., wire), which starts from a specific start point, preys on rats (i.e., hook) at several places, and then finally returns to the start point (i.e., end point) and bites its own tail (i.e., wire fixing) at the start point.

[Mode for Invention]

Hereinafter, the fabrication (meshing) method described briefly above will be described referring to drawings.

All the movement of a specific structure pattern, i.e., a node structure pattern expressed by a primitive planar matrix formula, occurs inside the planar matrix (FIG. 44, except the movement for connection between the stent head node pattern and the interface node pattern). All the movement in the planar matrix may be described by horizontal movement, vertical movement and diagonal movement. Because the horizontal movement can be leftward or rightward, the vertical movement can be upward or downward and the diagonal movement can be upward or downward, all the movement occurring in the planar matrix, i.e., horizontal to left ($H_L$), horizontal to right ($H_R$), vertical to up ($V_U$), vertical to down ($V_D$), diagonal to left up ($D_{LU}$), diagonal to left down ($D_{LD}$), diagonal to right up ($D_{RU}$) and diagonal to right down ($D_{RD}$) movement may be summarized briefly by sandglass-type movement (6-way, ⧖), more accurately by radial movement (8-way, ✳) (FIG. 44). The radial movement is named so because movement is possible in 8 directions from the current position.

The radial movement has a higher degree of freedom (DOF) of movement than zigzag (Z) movement, more accurately W movement, commonly used in the related art. Therefore, it is advantageous over the existing fabrication (meshing) method in that the start point can be selected freely and the quantity of wire can be adjusted freely.

Accordingly, a desired structure pattern, i.e., a node structure pattern of a primitive planar matrix formula, may be completed by simply by sequentially marking the movement of the hook node position $(i, j)_{hook}$ in the planar matrix and then carrying out the movement of the wire.

The textual symbolic expression used in the fabrication expression proposed by the present disclosure is as follows (Table 4).

[Table 4]

| **node name** | **textual symbolic expression** |
|---|---|
| start point | S#(i, j) |
| end point | E#(i, j) |
| horizontal hook | (i, j) |
| vertical hook | $(i, j)_v$ |
| horizontal open hook | $(i, j)_{ho}$ |
| vertical open hook | $(i,j)_{vo}$ |
| horizontal half hook | $(i, j)_{hh}$ |
| vertical half hook | $(i, j)_{vh}$ |

Hereinafter, the symbolic expression of Table 4 is described briefly.

In the symbol S#(i, j) representing the start point, # is for distinguishing wires when a plurality of wires are used and numbers such as 1, 2, 3, ... etc. are used.

In the E#(i, j) representing the end point, # is the same as described in the start point. The start point and the end point are used as a set. For example, S1(i, j), ..., E1(i, j) means the case where the start and end points are wire number 1.

The horizontal hook is a hook formed when the wire is bent horizontally at the hook node (FIG. 45).

The vertical hook is a hook formed when the wire is bent vertically at the hook node (FIG. 46).

The horizontal open hook is an open hook with no hook formed when the wire is bent horizontally at the hook node (FIG. 47).

The vertical open hook is an open hook with no hook formed when the wire is bent vertically at the hook node (FIG. 48).

The horizontal half hook is an open single hook with no hook formed when the wire is bent horizontally at the hook node (FIG. 49).

The vertical half hook is an open single hook with no hook formed when the wire is bent vertically at the hook node (FIG. 50).

The horizontal open hook, the vertical open hook, the horizontal half hook and the vertical half hook are technical hooks used to realize specific function and performance required for the wire-mesh stent.

The technical hooks are the hook types related with the function, performance and fabrication method of the stent and are mainly half hooks. The basic forms include an in-coil half hook, an out-coil half hook, a band half hook, an in-band half hook, an out-band half hook, a fork half hook, an in-track half hook, an out-track half hook, a close-turning

half hook and a gap-turning half hook (FIG. 51). The turning half hook is a half hook used to maintain the movement direction of a horizontal hook without using a vertical hook. Although the technical hook is basically a half hook, an open hook or other hooks may also be used as long as the compressing loadability is not negatively affected.

If the structure pattern is completed using 3 wires, the fabrication expression for the fabrication (meshing) method is expressed as a formatted type as follows (FIG. 52).

In order to indicate sequential order, an arrow (->), a comma (","), a null space ( ), etc. may be used. In the present disclosure, null space or line feed is used for simplicity. As can be seen later in the examples, because a fabrication (meshing) method has a repeating sequence, it can be expressed by an abbreviated format.

For the cross node too, a half cross which passes without crossing for control or optimization of specific stent performance may be used. The passing of a specific cross node may be up (or over) or down, and this cross node may be represented by $(i, j)_{xu}$ or $(i, j)_{xd}$ (FIG. 53). However, the fabrication (meshing) method of the multi-alterable structure pattern proposed by the present disclosure may be described and understood without special difficulty even when such expression is not used. Therefore, the expression for the cross node will be not used in the fabrication expression.

Hereinafter, the fabrication expression will be applied to simple examples.

The structure patterns of Model A consisting only of hooks and Model B consisting of a combination of hooks and crosses, which were described above in the description of the compression ratio depending on structure patterns, will be described as examples. The primitive planar matrix formulas for the two models are shown in FIG. 54. For simplicity, the case where the exponent of a primitive node pattern is 1 (i.e., n = 1) will be described first (for reference, the primitive planar matrix formula is the structure pattern for the stent body).

Model A is a very simple and monotonous repeating structure pattern consisting only of hooks. If the upper interface node pattern is [-H-H-H-H-H-H-H-H-H-H-H-H-H-] and the lower interface node pattern is [H-H-H-H-H-H-H-H-H-H-H-H-H-H] (FIG. 55), the fabrication expression with no twisted line from the start point to the end point with the hook at the center is as follows (FIG. 56). A planar mesh wire-meshed according to the sequence of the fabrication expression is shown in FIG. 57. The planar mesh is very useful in visualizing the hooks and crosses after the wire meshing.

The fabrication expression of FIG. 56 is an abbreviated form. The total expression is S1(1, 2),(2, 3),(1, 4),(2, 5),(1, 6),(2, 7),(1, 8),(2, 9),(1, 10). Looking at the coordinates (i, j) of the hook node position marked by the horizontal node line I and the vertical node line j, it can be seen that i is repeated in the order of 2->1->2 and j corresponds to arithmetic progression with common difference of 1. Accordingly, it can be abbreviated by $(i, j+1)_{i=2->i->2,j=2}$ to 25.

Model B is a structure pattern consisting of a combination of hooks and crosses. If the upper interface node pattern is

[H-H-H-H-H-H-H-H-H-H-H-H-H-H
-C-C-C-C-C-C-C-C-C-C-C-C-C-]

and the lower interface node pattern is [H-H-H-H-H-H-H-H-H-H-H-H-H-H], the planar matrix of Model B is as shown in FIG. 58. The fabrication expression with no twisted line from the start point to the end point with the hook at the center is as follows (FIG. 59). And, a planar mesh wire-meshed according to the sequence of the fabrication expression is shown in FIG. 60.

For Model B, the case where the exponent of a primitive node pattern n = 2 is as follows. When the interface node pattern is the same as above when n = 1, the primitive planar matrix formula and the planar matrix are as shown in FIG. 61. The fabrication expression and a planar mesh wire-meshed according to the fabrication expression are shown in FIG. 62 and FIG. 63.

As described above, a specific wire meshing structure pattern obtained by the multi-alterable structure patterning method proposed by the present disclosure may be expressed by a primitive planar matrix formula and, if necessary, the total structure pattern including an adequate interface node pattern may be expressed by a planar matrix. The fabrication (meshing) method may be expressed by a formatted fabrication expression and the finally completed wire-mesh stent structure pattern has a systematic sequence that can be identified from the planar mesh.

Hereinafter, a flow chart for systematically determining the fabrication expression for the fabrication (meshing) method will be described briefly.

The fabrication (meshing) method for determining the fabrication expression may be configured by using the primitive planar matrix formula and the node position information of hooks and crosses in the planar matrix. As described above, a planar matrix has horizontal node lines expressed by i, from 1 to $N_i$, and vertical node lines expressed by j, from 1 to $N_j$. Therefore, the position of all hooks and crosses in the planar matrix may be expressed by (i, j). The hook or cross at the node position (i, j) may be distinguished as C(i, j) for the cross and H(i, j) for the hook.

Because the node line width and the node line height are expressed to have the same equal distance in the planar matrix even when the distance is different, a virtual node line for adjusting equal distance is unnecessary. Accordingly, it is easier than to determine the fabrication expression in the planar mesh.

If the planar matrix is constituted with the same node line width or node line height, movement can be determined simply by the movement by several spaces in the horizontal direction (Δi) or by the movement by several spaces in the vertical direction (delta-j space, Δj) without having to use movement angle or directional angle. Therefore, the moved position can be easily expressed logically or algorithmically in the form of (i, j).

In order to move from the current node position to the next node position, the information of node type at the current position is necessary. If the node type at the current position is a cross node, it is to be determined whether to accept as a cross node and pass through to a neighbor horizontal node line or to reject as a cross node and move to a neighbor cross node. Otherwise, if it is a hook node, it is to be determined which type of technical hook (horizontal hook, vertical hook, open hook, half hook or turning hook) will be applied.

To summarize the fabrication (meshing) method, the fabrication expression capable of configuring a planar mesh may be completed through a repeated procedure of moving by Δi and Δj using start node position, movement direction and node type information. The flow chart is shown in detail in FIGS. 64a-64c.

Method of multi-alterable wire-mesh stent structure patterning

Next, the 'patterning method of multi-alterable structure with loadability assured' presented by the present disclosure is described. Hereinafter, it will be referred to as 'multi-alterable structure patterning'.

As described earlier, the stent is a medical device system in which a stent and a delivery device are coupled. The delivery device passes through natural orifices of the human body such as mouth, anus, urinary organs, eye, nose, ear, etc. as well as abdomen, skin tissues and blood vessels and is often used together with surgical instruments such as an endoscope, a cystoscope, etc.

Because the stent should be inserted into the human body, the penetrated hole should be as mall as possible to minimize the patient's pain.

In order to satisfy these basic requirements, a 'stent structure pattern with superior loading compressibility into a delivery device' is necessary.

In the present disclosure, to satisfy these basic requirements, microscopic structural analysis of the wire-mesh stent structure is conducted through modeling and the following main elements are identified through the quantitative investigation of compression ratio depending on the number and arrangement of hook and cross nodes, which are the key elements of the wire-mesh stent structure.

First, the formation of twisted line during fabrication (meshing) should be avoided. Second, interference between neighboring hook and cross nodes should be avoided during compression. Third, it is the hook node that mainly affects the compression ratio. Fourth, the adequate number of hooks is 3-8 per the radial section of the stent, i.e., per the horizontal node line. Fifth, the symmetry of the global arrangement of hooks should be maintained unless for special design purposes. Sixth, the symmetry should be close to a circle so that the sustainability of circular circumference can be ensured during the compression.

To summarize, it is recommended that 'there is no twisted line or interference between nodes and the symmetric circularity of 3-8 hook nodes per horizontal node line is ensured during the compression'. This is the basic statement of requirements for the 'multi-alterable structure patterning' proposed by the present disclosure.

In the present disclosure, the following concepts are introduced for 'multi-alterable structure patterning' satisfying the basic requirements: 1) primitive n-gon; 2) rotation; 3) section layer; 4) skip; 5) combination; 6) outburst.

The multi-alterability can be largely classified into formatted multi-alterability and unformatted multi-alterability. The biggest difference lies in repetition. For the formatted multi-alterability, repetition is conducted in a standardized form. In contrast, for the unformatted multi-alterability, repetition is conducted randomly.

The concept presented here ensures non-interference, compressibility, symmetry and circularity for both formatted and unformatted multi-alterability.

The six concepts are explained briefly first and then specific application will be described later.

The concept of 'primitive n-gon' is introduced based on the analysis result that the adequate number of hooks per the radial section of the wire-mesh stent, i.e., the horizontal node line, is 3-8. Primitive n-gons from triangle to octagon are introduced as primitive shapes (FIG. 65). The primitive n-gon is a cyclic polygon such that it can be inscribed to the stent diameter. Basically a regular polygon always inscribed to a circle is adopted as the polygon and an irregular polygon inscribed to the circle is adopted additionally in order to extend the multi-alterability of the 'primitive n-gon'. Because the circle is the circumference of the stent, the circumference of the stent is the circumcircle of the primitive n-gon.

In addition, the 'primitive n-gon' is a concept for satisfying the condition that the symmetry of the global arrangement of hooks should be retained and the symmetry should be such that the sustainability of circular circumference, i.e., circularity of the stent radial section is ensured during the compression of the stent.

Also, it is a concept that can satisfy the requirement of no interference between neighboring hook nodes during the compression.

The concept of 'primitive n-gon' has been introduced based on the analysis result that the adequate number of hooks per the horizontal node line of the wire-mesh stent is 3-8. However, because the actual number of hooks may be from 1 to 8, the concept including a dot (n = 1) and a line (n = 2) will be referred to as 'primitive n-geo'. Unless specified otherwise, a 'primitive n-geo' including two or less hooks will not be included in the primitive planar matrix formula but will be treated in the interface node pattern.

The 'rotation' is related to the rotational movement of the 'primitive n-gon'. Although it is a concept introduced mainly to ensure multi-alterability, it can additionally satisfy the conditions of symmetry and circularity.

The direction of the 'rotation' may be either forward (clockwise) or reverse (counterclockwise) and the degree of rotation angle may be set freely as long as there is no mutual interference between hook nodes during compression.

The 'section layer' is a concept that the 'primitive n-gon' is present in a plurality of independent layers retaining individual characteristics and is for ensuring multi-alterability. If the number of the 'section layers' having individual characteristics is increased, the number of cases of multi-alterability is also increased. It is also a concept for ensuring the conditions of symmetry and circularity.

The 'skip' is a concept that the 'primitive n-gon' does not exist in a given section layer and is for ensuring multi-alterability.

The 'combination' refers to a combination of multi-alterability. It is a concept introduced for amplification or multiplication of multi-alterability.

The 'outburst' is a concept for ensuring unformatted multi-alterability. It is for increasing the degree of freedom (DOF) of unformatted alterability by endowing randomness when selecting parameters.

These concepts are embodied as follows.

First, let's suppose that a stent is seen along the axial direction so that the radial section can be seen, as in FIG. 66. Let the radial section with no hook or cross node present be a node radial section. Then, the node radial section is identical to the horizontal node line in a planar matrix. That is to say, if the node radial section is spread (unfolded), it becomes the horizontal node line. Accordingly, the number of the node radial sections is identical to that of the horizontal node lines. Also, the concept of the 'section layer' is identical to the horizontal node line. To summarize node radial section = section layer = horizontal node line.

In FIG. 67, the node radial sections are aligned sequentially and isometrically along the axial direction. That is to say, a plurality of 'section layers' to which the individual characteristics for multi-alteration are to be endowed are formed. As the number of the 'section layers' to which the individual characteristics are to be endowed is increased, the number of cases of multi-alteration is also increased.

Inscribed 'primitive n-gons' may be aligned in the node radial sections. An example of aligning triangles in the node radial sections is shown in FIG. 68.

The 'primitive n-gon' is a basic element which determines the position and symmetry of hook nodes. Therefore, if it is assumed that a hook node exists at each vertex of the 'primitive n-gon', the symmetric hook array satisfying the condition identified through microscopic structural analysis of the wire-mesh stent structure proposed by the present disclosure that the adequate number of hook nodes per horizontal node line, i.e., per node radial section, is 3-8 can be ensured systematically.

For example, FIG. 68 is obtained if hook nodes are aligned on the vertices of primitive triangles in FIG. 67. FIG. 69 is obtained if other 'primitive n-gons' are applied.

If the sequentially aligned node radial sections are seen isometrically from the axial direction, all the hook nodes positioned at the respective node radial sections look identical because all the hook nodes located at the respective vertices of the 'primitive n-gons' are in the same phase while retaining symmetry (FIG. 70).

However, if the second concept, i.e., 'rotation', of multi-alterability is applied, multi-alterability can be further ensured because the phase of the hook nodes positioned at the vertices of the 'primitive n-gons' can be changed. The phase shifting may be achieved through movement in the rotation direction, i.e., clockwise (cw) or counterclockwise (ccw), and may be controlled more finely with the rotation angle.

A simple example of primitive regular triangles to help understanding is shown in FIG. 71. For convenience's sake, let the rotation direction be forward (clockwise) and the rotation angle ($\theta_{rot}$) be 90°. Then, the first node radial section is rotated by 0°, the second by 90°, the third by 180° and the fourth by 270°. The fifth node radial section is rotated by 360° and has the same phase as the first node radial section. The same pattern is repeated continuously and the node radial sections with the same phase are indistinguishable when seen from the axial direction.

As can be seen this example of primitive regular triangles, various multi-alterability can be ensured by changing the rotation angle ($\theta_{rot}$). For other 'primitive n-gons', the multi-alterability can be ensured in the same way. The degree of basic alteration that can be applied to the node radial section with the rotation angle ($\theta_{rot}$), i.e., the number of angle division ($N_{ang}$), is given by $N_{ang} = \| 360/\theta_{rot} \|$. As will be described later, multi-alterability can be achieved by applying the concept of mathematical 'combination' (${}_nC_k$) of the rotation angle.

Because the 'rotation' has directionality of forward or reverse, directionality may be set for the angle division or a combination thereof to ensure multi-alterability. For example, if the rotation angle is 90°, the accumulated rotation

angle ($\theta_{accu}$) is cycled in the order of 0° -> 90° -> 180° -> 270° -> 360° (0°). 90° and 180° may be set as forward (clockwise, cw) direction and 270° and 360° may be set as reverse (counterclockwise, ccw) direction.

Depending on the node structure pattern formed when multi-alterability is ensured by a combination of 'rotation' in the forward (cw) direction and the reverse (ccw) direction, an interface node pattern for connection between the forward direction node structure pattern and the reverse direction node structure pattern may be necessary.

In order to ensure unformatted multi-alterability, unformatted structure patterning may be conducted by randomly changing the accumulated rotation angle ($\theta_{accu}$) by applying the concept of 'outburst'.

The expression for the 'rotation' may be expressed as follows to endow directionality to the respective accumulated rotation angles. For simplicity of expression, the forward (clockwise, cw) direction is represented by R and the reverse (counterclockwise, ccw) direction is represented by L.

$$R_{rot} = (\theta_{rot} \times i,j)_{\ i = 1 \text{ to } N.ang,\ j = L \text{ or } R}$$

For the above example, $R_{rot}$ = (0, R) (90, R) (180, R), (270, L), (360, L).

In addition, symmetry can be ensured by combining the concepts of 'rotation' and 'section layer'. If a rotation angle of 90° and 4 section layers are applied to a primitive triangle, 12 hook nodes are aligned symmetrically. If a stent having such as wire-mesh stent structure pattern is compressed and loaded into a delivery device, it is easier to ensure circularity because the compressed circumference becomes a regular dodecagonal circumference (FIG. 72). If only the 'section layer' is applied, not the 'rotation', the compressed circumference becomes a regular triangular circumference. In this case, it is difficult to ensure circularity even when the compression ratio is identical (number of total nodes, $N_t = N_h + N_c$).

If a 'primitive n-gon' having more vertices is applied to the combination of 'rotation' and 'section layer', finer symmetry and circularity can be ensured. But, because this is disadvantageous in terms of compression ratio, adjustment may be necessary depending on the specific function and performance required for the wire-mesh stent and the inner diameter of the delivery device into which the stent is loaded. Of course, the increase of friction in the delivery device that may occur due to decreased compression ratio may be reduced by 'skip' and 'combination' as will described later.

The concept of 'skip' is for ensuring multi-alterability. The 'skip' refers to intentionally missing or pushing a specific node radial section, i.e., the node pattern element formed in the 'section layer'. Missing means passing the node pattern by and pushing means adding one 'section layer' with no hook node and then proceeding the original node pattern.

A skipping pattern may be formed by mathematical combination (${}_nC_k$), rather than simply one or two 'skips', depending on the number of section layers ($N_{lay}$) or the number of horizontal node lines expressed in the primitive planar matrix formula. In addition, the degree of freedom (DOF) of multi-alterability can be further extended through missing and pushing.

In the foregoing example of primitive regular triangles for helping understanding, a regular dodecagonal compressed circumference was configured by applying 4 'section layers' when the rotation angle ($\theta_{rot}$) was 90°. The 'skip' refers to 'skipping' a 'section layer' corresponding to a specific accumulated rotation angle.

For example, if the rotation angle ($\theta_{rot}$) is 90°, the rotation angles accumulated at the respective 'section layers' are 0°, 90°, 180°, 270° and 360° (=0°). FIG. 73 shows a case wherein the 'section layer' at 180° is 'skipped'. **A** shows missing skip and **B** shows pushing skip.

FIG. 74 shows the primitive planar matrix formula for this example. This primitive planar matrix formula is a very simplified expression using hook and null nodes only without cross nodes. In the actual application, cross nodes are formed naturally because a wire crosses. Accordingly, only cross and null nodes exist in the 'skipped' 'section layer', or the horizontal node line. The cross and null nodes formed naturally by the multi-alterable structure patterning are called dummy nodes. And, a horizontal node line having dummy nodes is called a dummy node line and is not expressed in the primitive planar matrix formula. However, in the planar mesh, the dummy node lines are distinguishably marked as $1_{dum}$, $2_{dum}$, etc.

It was described above that a skipping pattern may be determined by mathematical combination (${}_nC_k$) of 'skip'. For a combination of two, i.e., k = 2, if the first 'skip' occurs at an accumulated rotation angle ($\theta_{accu}$) of 180° and the second 'skip' occurs at an accumulated rotation angle ($\theta_{accu}$) of 90°, a primitive planar matrix formula shown in FIG. 75 can be obtained. Because independent primitive planar matrix formulas mean different structure patterns, the primitive planar matrix formulas shown in FIG. 74 and FIG. 75 are different wire-mesh stent structure patterns derived from the concept of 'skip'. For a mathematical combination with $k \geq 2$, symmetric circularity can be ensured during compression.

When the concept of 'outburst' of multi-alterability is applied to 'skip', unformatted multi-alterability can be ensured through randomness. Here, it is important to establish a policy for random selection. For example, it is to be determined first whether the total 'section layers' will be 'skipped' or the repeated section layer occurring when a rotation angle is applied will be 'skipped'. In addition, it is to be determined how many 'section layers' will be 'skipped' through random selection.

Next, the concept of 'combination' of multi-alterability is described. The 'combination' can be largely classified into parameter combination and pattern combination. The parameter combination refers to mathematical combination ($_nC_k$) of a 'primitive n-gon', rotation and 'skip'. The pattern combination is a combination of primitive planar matrix formulas generated from parameter combination and an interface node pattern may be necessary (FIG. 76).

The pattern combination can be configured by normal combination, symmetry combination or reverse symmetry combination of pattern of primitive planar matrix formulas. The normal combination refers to a combination without change in the primitive planar matrix formula, the symmetry combination refers to a combination in which the primitive planar matrix formulas are inverted to achieve symmetry, and the reverse symmetry combination refers to a combination in which the primitive planar matrix formulas are inverted and applied in reverse order (FIG. 77a and FIG. 77b).

An example of the parameter combination is to apply one or more 'primitive n-gon' to multi-alterable structure patterning. For example, a primitive regular triangle and a primitive regular tetragon can be applied simultaneously. FIG. 78 shows an example wherein the rotation angle is ($\theta_{rot}$) 90°.

Lastly, the 'outburst' is a concept of applying randomness to ensure unformatted multi-alterability. However, the randomness devised in the present disclosure is not the confusing mass randomness. It is a concept introduced to increase the degree of freedom (DOF) of multi-alterability by creating outburst which gives unexpected change in the factor and order of formatted multi-alterability, like the mutation of a base sequence. The randomness can be achieved from the selection of 'primitive n-gon', 'rotation' and 'skip'. As described above with regard to the concept of 'skip', it is very important to determine in advance how and to what extent the randomness will be applied.

The multi-alterability is related with versatility. Because the versatility is defined as a multiplication of a factor by the degree of freedom ($D_f$), i.e., f (versatility) = factor x $D_f$, the number of cases of formatted multi-alterability ($N_{def}$) that can be obtained from the concept is as follows.

$$N_{def} = {}_{N.gon}C_{k.g} \times N_{rot.d} \times {}_{N.ang}C_{k.a} \times N_{lay} \times {}_{N.lay}C_{k.s} \times N_{skip}$$

$$= N_{gon}!/(k_g!(N_{gon} - k_g)!) \times N_{rot.d} \times N_{ang}!/(k_a!(N_{ang} - k_a)!) \times N_{lay} \times N_{lay}!/(k_s!(N_{lay} - k_s)!) \times N_{skip}$$

For unformatted multi-alterability, the number of cases of unformatted multi-alterability ($N_{undef}$) is as follows.

$$N_{def} = {}_{N.gon}C_{k.g} \times N_{rot.d} \times {}_{N.ang}C_{k.a} \times N_{lay} \times {}_{N.lay}C_{k.s} \times N_{skip}$$

$$= N_{gon}!/(k_g!(N_{gon} - k_g)!) \times N_{rot.d} \times N_{ang}!/(k_a!(N_{ang} - k_a)!) \times N_{lay} \times N_{lay}!/(k_s!(N_{lay} - k_s)!) \times N_{skip}$$

where,

$N_{def}$ : number of cases of formatted multi-alterability
$N_{gon}$ (or N.gon) : number of primitive n-gon (=6)
${}_{N.gor}C_{kg}$ : $k_g$-combination of primitive n-gon
$k_g$ (or k.g) : number of primitive n-gons applied ($1 \le k_g \le 6$)
$N_{rotd}$ : number of rotation direction (=2)
$\theta_{rot}$: rotation angle (irregular: $\theta_{rot}$ = arbitrary, regular: $\theta_{rot}$ = except { ‖360/vertex of n-gon‖ x n < 360})
$N_{ang}$ (or N.ang) : number of angle division ($N_{ang} = \|360/\theta_{rot}\|$)
${}_{N.ang}C_{ka}$ : $k_a$-combination of angles
$k_a$ (or k.a) : number of angles applied (≥1)
$N_{lay}$ (or N.lay) : number of layers in the primitive planar matrix formula

$$(\ge 2, \{\text{if } \theta_{rot} = 0, N_{lay} \ge N_{gon.selected}\}, \{\text{if } \theta_{rot} > 0, N_{gon.selected} \le N_{lay} \le N_{ang}\})$$

${}_{N.lay}C_{ks}$ : $k_s$-combination of section layer skipped
$k_s$ (or k.s) : number of section layer skipped ($1 \le k_s \le N_{lay} - 2$)
$N_{skip}$: number of skipping method (=3, none, missing & pushing)

$$N_{undef} = N_{gon}^{N.lay} \times N_{ang}^{N.lay} \times N_{rot.d}^{N.lay} \times N_{skip}^{N.lay}$$

As can be expected from the above equations, quite a variety of multi-alterability can be ensured through the

concept of multi-alterable structure patterning proposed by the present disclosure.

All the primitive planar matrix formulas derived from the concept described above are expressed by a planar matrix in combination with an adequate interface node pattern. Because the movement of a wire in the planar matrix is of 6-way sandglass type (⧖) or 8-way radial type ( ✳), a wire-meshed planar mesh can be obtained. That is to say, all the multi-alterable wire-mesh stent structure of the present disclosure can be obtained.

Next, prior to describe the examples to which the above-described concept is applied actually, the method of aligning cross nodes is described briefly. The cross nodes may be aligned along the sides of a 'primitive n-gon' and the number of cross nodes varies depending on the desired function and performance of the wire-mesh stent.

Although the cross nodes are actually aligned along the circumcircle passing the vertices of the 'primitive n-gon', i.e., the circumference of the stent, it is intuitive to align them along the sides of the 'primitive n-gon' when designing a node pattern in the multi-alterable structure patterning.

For example, FIG. 79 shows the primitive planar matrix formula of the foregoing example wherein 3 cross nodes are aligned on the sides of a primitive regular triangle and a rotation angle of 90° has been applied. For the superposed 'primitive n-gons', only the first 'primitive n-gon' is marked. The graphical symbolic expression for a cross node is X.

Although the evaluation of the symmetry and circularity of the multi-alterable structure pattern should be conducted on the planar matrix, if the number of primitive section layers ($N_{lay}$) excluding the skip node line is 4 or greater, they may also be evaluated from the primitive node pattern expressed by the primitive planar matrix formula, which implicitly expresses the core structure pattern. But, if the number of primitive section layers constituting the primitive node pattern is less than 4, the value of the exponent of a primitive node pattern (n) should be changed to satisfy the condition that the number of the primitive section layers ($N_{lay}$) is 4 or greater.

Because the symmetry is the factor for evaluating how uniformly the hooks are arranged when seen from the axial direction, both the position and amount of the hooks should be considered in the evaluation. As seen from FIG. 80, although it seems that symmetry is retained as the hooks are arranged uniformly when seen from the axial direction, the number of the hooks arranged in the same phase ($N_{h.phase}$) may be different. Therefore, both the position and amount should be considered.

Because the symmetry is for evaluating the degree of uniform distribution (dispersion) of the hooks, the variance of the hook nodes is evaluated. There are three variance factors or variance variables to be evaluated, including two amount variables and on position variable:

1) number of hooks per horizontal node line, $N_{h.horiz.node.i\text{-}th}$
2) number of hooks per vertical node line, $N_{h.vert.node.j\text{-}th}$
3) distance between neighbor hooks per horizontal node line, $\Delta_{h.horiz.node.i\text{-}th}$

The distance between neighbor hooks per horizontal node line ($\Delta_{h.horiz.node.i\text{-}th}$) means the angle contained between neighbor hooks ($\theta_{H\text{-}H}$) at the center of the axial direction. Since the division angle between nodes ($\theta_{div}$) is expressed by the distance between vertical node lines, i.e., the node line width ($W_{n.line}$), in the primitive node pattern and the node line width is expressed by the equal distance, the following relation is established if the number of vertical node lines between neighbor hooks is $N_{H\text{-}H.vert}$.

$$\theta_{H\text{-}H} = N_{H\text{-}H.vert} \times \theta_{div} = N_{H\text{-}H.vert} \times W_{n.line}$$

$$\theta_{H\text{-}H} / \theta_{div} = N_{H\text{-}H.vert}$$

Accordingly, the distance between neighbor hooks per horizontal node line ($\Delta_{h.horiz.node.i\text{-}th}$) can be evaluated simply with the number of vertical node lines between neighbor hooks ($N_{H\text{-}H.vert}$).

The number of hooks per vertical node line ($N_{h.vert.node.j\text{-}th}$) is a variable reflecting the variation of the number of hooks existing in the same phase. The variation of the j-th vertical node line in which the whole vertical node line is composed of null node is excluded from calculation.

Because all the three variance variables are independent and uncorrelated variables, each of the variance variables can be summed arithmetically. Accordingly, a quantitative evaluation that can be compared dimensionlessly is possible by converting the variance to a standard deviation and dividing by a mean value to give a coefficient of variation (CV) value. That is to say, the symmetry ($Q_{symm}$) can be evaluated with the average coefficient variation ($CV_{avg}$) value for the three variance variables. The symmetry is good as the average coefficient variation is closer to 0. For convenience of recognition, the equation of symmetry evaluation is calculated by $Q_{symm} = 1 - (CV_{N.h.horiz} + CV_{N.h.vert} + CV\Delta_{.h.horiz})/3$. The symmetry is good as this value is closer to 1. In the present disclosure, it is recommended that the

value is at least 0.5.

Next, a formula for evaluation of quantitative symmetry ($Q_{symm}$) is summarized briefly.

$$Q_{symm} = 1 - ( CV_{N.h.horiz} + CV_{N.h.vert} + CV_{\Delta.h.horiz} ) / 3$$

where,

$CV_{N.h.horiz} = (SD/MV)_{N.h.horiz}$
$CV_{N.h.vert} = (SD/MV)_{N.h.vert}$
$CV_{\Delta.h.horiz} = (SD/MV)_{\Delta h.horiz} = \Sigma(CV_{\Delta h.horizy\text{-}th})_{i,\ i=1\ to\ N.lay} / N_{lay}$

CV : coefficient of variation
SD : standard deviation
MV : mean value

N.h.horiz: no. of hooks for horizontal node line
N.h.vert: no. of hooks for vertical node line
$\Delta$-h.horiz : distance between neighbor hooks for horizontal node line
$\Delta$.h.horiz.i-th : distance between neighbor hooks per i-th horizontal node line
N.lay (or $N_{lay}$) : no. of primitive section layer applied (> = 4, except for the skip node line)
$N_{H\text{-}H.vert}$ : no. of vertical node line between neighbor hooks per horizontal node line
$\Delta$.h.horiz = $N_{H\text{-}H.vert}$

note)

- except the vertical line that all node is configured of null node
- higher $Q_{symm}$ value is better (best value is 1)
- recommended $Q_{symm}$ value is over 0.5 at minimum

Since the circularity is for evaluating the sustainability of circular circumference of the stent radial section upon compression of the stent (FIG. 81), a structure pattern consisting of many hooks when seen from the axial direction may be favorable. However, the presence of many hooks in a single section layer is disadvantageous in terms of compression ratio. Therefore, it is preferred to sustain circularity by uniformly dispersing the hoods for different section layers.

Basically, the circularity can be evaluated by comparing with the ratio of the circumference to the diameter (pi, $\pi$). Since pi ($\pi$) is the ratio of the circumference (S) of a circle to its diameter (D), i.e., $\pi$ = S/D, the ratio may be compared relatively with the circumference. For example, as the number of the sides of a regular n-gon inscribed to a circle with a diameter of D is increased, the relationship "total length of sides ≒ circumference" is established.

Accordingly, assuming a regular n-gon inscribed to a circle with a diameter of D, the simple circularity ($Q_{circ.simple}$) can be evaluated with the value obtained by dividing the sum of the length of the sides of the regular n-gon ($S_{gon}$) by the circumference (S, S = $\pi$D), i.e., $Q_{circ.simple} = S_{gon} / S$.

The length of one side (s) of the regular n-gon inscribed to the circle with a diameter of D can be calculated by the equation s = D sin(180/n), where n is the number of the sides. Therefore, the total length of the sides is given by $S_{gon}$ = n x s = n x D sin(180/n).

Accordingly, the simple circularity can be calculated simply by $Q_{circ.simple} = S_{gon} / S$ = n x D sin(180/n) / $\pi$D = n x sin(180/n)/$\pi$.

However, the simple circularity assuming a regular n-gon inscribed to a circle is inadequate for evaluation of circularity when the number of hooks seen from the axial direction is different from the number of hooks present in the same phase, i.e., when variation is included. In this case, evaluation should be made in consideration of the variation of the number and arrangement of the hooks.

As in the evaluation of symmetry, the following three variables are required for the evaluation of circularity, which are independent and uncorrelated variables.

1) total number of hooks on the axial view, $N_{h.radial}$
2) perpendicular distance of neighbor hooks on the axial view, $\Delta_{h.radial}$
3) number of hooks per vertical node line, $N_{h.vert.node.j\text{-}th}$

The total number of hooks on the axial view ($N_{h.radial}$) is identical to the number of vertical node lines on which

hooks exist. It is a variable suitable to apply the concept of a regular n-gon inscribed to a circle, which is assumed for the simple circularity ($Q_{circ.simple}$).

The concept and application of the number of hooks per vertical node line ($N_{h.vert.node.j\text{-}th}$) are the same as those for the symmetry evaluation. Therefore, the vertical node line composed only of null node is excluded from calculation.

For the perpendicular distance of neighbor hooks on the axial view ($\Delta_{h.radial}$), the number of vertical node lines between neighboring hooks is used as in the symmetry evaluation. However, unlike the symmetry evaluation where the distance between hooks on the same horizontal node line is used, the vertical distance between a pair of neighboring vertical node lines is used in the circularity evaluation.

Accordingly, the circularity ($Q_{circ}$) can be evaluated by reflecting the variation of hook arrangement and the number of hooks existing in the same phase to the simple circularity ($Q_{circ.simple}$). That is, Qcirc = $Q_{circ.simple}$ X (1 - $CV_{avg}$).

Here, $CV_{avg}$ is the average coefficient of variation for the number of hooks ($N_{h.vert.node.j\text{-}th}$) existing in the same phase as the hook arrangement ($\Delta_{h.radial}$). (1 - $CV_{avg}$) corresponds to the hooks configuring a regular n-gon with respect to the axial direction since the state of complete uniformity with no variation corresponds to $CV_{avg}$ = 0. That is, $Q_{circ}$ = $Q_{circ.simple}$.

Next, the equation for evaluating quantitative circularity ($Q_{circ}$) is summarized briefly.

$$Q_{circ} = Q_{circ.simple} \times (1 - CV_{avg})$$

where,

$Q_{circ.simple}$ = n x sin(180/n)/$\pi$
$CV_{avg}$ = ($CV_{\Delta h.radial}$ + $CV_{N.h.vert}$) / 2
$CV_{\Delta.h.radial}$ = $(SD/MV)_{\Delta.h.radial}$
$CV_{N.h.vert}$ = $(SD/MV)_{N.h.vert}$
n = N.h.radial (or $N_{h.radial}$)

$Q_{circ.simple}$ : simple circularity (based on regular n-gon)
$CV_{avg}$ : average coefficient of variation
CV : coefficient of variation
SD: standard deviation
MV: mean value
n : no. of sides of regular n-gon ( = total no. of hooks on the axial view)

N.h.radial : total no. of hooks on the axial view
$\Delta$.h.radial: perpendicular distance of neighbor hooks between pair.vert.node on the axial view
pair.vert.node: pair vertical node between neighbored j-th and (j+$\alpha$)-th vertical node on which hook exists
N.h.vert: no. of hooks for vertical node line

note)

- except the vertical line that all node is configured of null node
- higher $Q_{circ}$ value is better (best value is 1)
- recommended $Q_{circ}$ value is over 0.4 at minimum

Briefly, the four phases of the procedure of the multi-alterable structure patterning described above are summarized as: patterning policy phase, parameter design phase, patterning configuration phase and fabrication expression phase. Its flow chart is shown in FIGS. 82a-82e, in more detail in FIGS. 83a-83d.

Hereinafter is given examples wherein the multi-alterable structure patterning method of the present disclosure described above is applied.

Example 1 (REG-TRIA-90-L-12)

An example of multi-alterable structure patterning (REG-TRIA-90-L-12) wherein the 'primitive n-gon' is a regular triangle, the rotation angle is 90°, the rotation direction is reverse (ccw, left), there is no 'skip', 'combination' or 'outburst', the loop type is open loop type and the exponent of a primitive node pattern is 2 (n = 2) is shown in FIG. 84a, FIG. 84b and FIG. 84c.

Example 2 (REG-TRIA-180-L-12)

An example of multi-alterable structure patterning (REG-TRIA-180-L-12) wherein the 'primitive n-gon' is a regular triangle, the rotation angle is 180°, the rotation direction is reverse (ccw, left), there is no 'skip', 'combination' or 'outburst', the loop type is closed loop type and the exponent of a primitive node pattern is 4 (n = 4) is shown in FIG. 85a, FIG. 85b and FIG. 85c.

A result of applying the equation of symmetry evaluation to Example 1 (REG-TRIA-90-L-12) and Example 2 (REG-TRIA-180-L-12) of the present disclosure is shown in FIG. 86a and FIG. 86b.

A result of applying the equation of circularity evaluation to Example 1 (REG-TRIA-90-L-12) and Example 2 (REG-TRIA-180-L-12) of the present disclosure is shown in FIG. 87a and FIG. 87b.

Example 3 (REG-TRIA-15-R-COMB-12)

An example of multi-alterable structure patterning (REG-TRIA-15-R-COMB-12) wherein the 'primitive n-gon' is a regular triangle, the rotation angle is 15°, the rotation direction is forward (cw, right), the 'combination' is parameter combination for the rotation angle, there is no 'skip' or 'outburst', the loop type is open loop type and the exponent of a primitive node pattern is 2 (n = 2) is shown in FIG. 88a, FIG. 88b and FIG. 88c.

Example 4 (REG-TETR-18-R-SKIP-20)

An example of multi-alterable structure patterning (REG-TETR-18-R-SKIP-20) wherein the 'primitive n-gon' is a regular tetragon, the rotation angle is 18°, the rotation direction is forward (cw, right), the 'skip' is missing skip, there is no 'combination' or 'outburst', the loop type is open loop type and the exponent of a primitive node pattern is 2 (n = 2) is shown in FIG. 89a, FIG. 89b and FIG. 89c.

Example 5 (REG-TETR-45-RL-SKIP-20)

An example of multi-alterable structure patterning (REG-TETR-45-RL-SKIP-20) wherein the 'primitive n-gon' is a regular tetragon, the rotation angle is 45°, the rotation direction is forward (cw, right) and reverse (ccw, left) alternatingly, the 'skip' is pushing skip, there is no 'combination' or 'outburst', the loop type is closed loop type and the exponent of a primitive node pattern is 3 (n = 3) is shown in FIG. 90a, FIG. 90b and FIG. 90c.

Example 6 (REG-PENT-27-RL-COMB-20)

An example of multi-alterable structure patterning (REG-PENT-24-RL-COMB-20) wherein the 'primitive n-gon' is a regular pentagon, the rotation angle is 27°, the rotation direction is forward (cw, right) and reverse (ccw, left) alternatingly, the 'combination' is parameter combination for the rotation direction, there is no 'skip' or 'outburst', the loop type is open loop type and the exponent of a primitive node pattern is 1 (n = 1) is shown in FIG. 91a, FIG. 91b and FIG. 91c.

Example 7 (REG-TRPH-0-L-COMB-12)

An example of multi-alterable structure patterning (REG-TRPH-0-L-COMB-12) wherein the 'primitive n-gon' is a regular triangle, a regular tetragon, a regular pentagon and a regular hexagon, the rotation angle is 0°, there is no rotation direction, the 'combination' is parameter combination for the primitive n-gon and the cross, there is no 'skip' or 'outburst', the loop type is open loop type and the exponent of a primitive node pattern is 3 (n = 3) is shown in FIG. 92a, FIG. 92b, FIG. 92c and FIG. 92d.

Example 8 (REG-TRPH-0-L-COMB-SYMM-12)

An example of multi-alterable structure patterning (REG-TRPH-0-L-COMB-SYMM-12) wherein the design parameters are identical to those of Example 7 (REG-TRPH-0-L-COMB) and the patterning policy is also similar but the 'combination' is both parameter combination and pattern combination (pattern combination = symmetry combination) and the exponent of a primitive node pattern is 1 (n = 1) is shown in FIG. 93a, FIG. 93b and FIG. 93c.

Example 9 (REG-HXTR-30/90-LR/L-COMB-PATN-18/12)

In this example, a multi-alterable structure pattern is formed by configuring a compound structure pattern from different structure patterns, i.e., by combining primitive planar matrix formulas. The REG-TRIA-90-L-12 model and the

REG-HEXA-30-LR-18 model, wherein the exponent of a primitive node pattern is 1 (n = 1) was combined in the form of [REG-HEXA-30-LR-18] + [REG-TRIA-90-L-12] + [REG-HEXA-30-LR-18]. The multi-alterability policy applied to the REG-HEXA-30-LR-18 model is as follows.

The 'primitive n-gon' is a regular hexagon, the rotation angle is 30°, the rotation direction is reverse (ccw, left) and forward (cw, right) alternatingly, the 'combination' is parameter combination for the rotation direction, there is no 'skip' or 'outburst', the loop type is open loop type, the exponent of a primitive node pattern is 1 (n = 1) and a half hook is used for the connection of different compound structure nodes.

This example of multi-alterable structure patterning (REG-HXTR-30/90-LR/L-COMB-PATN-18/12) is shown in FIG. 94a, FIG. 94b and FIG. 94c.

Example 10 (IRR-TRPH-20-L-COMB-14)

In this example, a multi-alterable structure pattern is formed by aligning the total number of hooks of an irregular 'primitive n-gon' uniformly in the radial direction. The multi-alterability policy applied to the IRR-TRPH-20-L-COMB-14 model is as follows.

The 'primitive n-gon' is an irregular triangle, an irregular tetragon, an irregular pentagon and an irregular hexagon, the rotation angle is 20° (18 x 20° = 360°), the rotation direction is reverse (ccw, left), the 'combination' is parameter combination for the primitive n-gon and the cross, there is no 'skip' or 'outburst', the loop type is open loop type, the exponent of a primitive node pattern is 1 (n = 1) and a half cross is used to ensure the connection of the irregular n-gon.

This example of multi-alterable structure patterning (IRR-TRPH-20-L-COMB-14) is shown in FIG. 95a, FIG. 95b, FIG. 95c and FIG. 95d.

Example 11 (IRR-TETR-NA-L-COMB-REVS-8/10/14)

In this example, a multi-alterable structure pattern is configured by reverse symmetry combination of an 8-node, 10-node and 14-node compound structure patterns using an irregular tetragon.

This example of multi-alterable structure patterning (IRR-TETR-NA-L-COMB-REVS-8/10/14) is shown in FIG. 96a, FIG. 96b and FIG. 96c.

Materials for applying method for patterning of multi-alterable wire-mesh stent structure

This multi-alterable structure patterning technique can be applied to any biocompatible metal-based, synthetic polymer-based or natural polymer-based material from which a thread-like wire can be fabricated for application to a wire-mesh stent.

The metal-based material includes a nickel-titanium (Ni-Ti) shape memory alloy), a martensitic nickel-titanium (NiTi) shape memory alloy, stainless steel, tantalum, tungsten (W), gold (Au), platinum, silver (Ag), nickel, titanium (Ti), chromium (Cr), a cobalt-chromium (Co-Cr) alloy, a platinum-chromium (Pt-Cr) alloy, a platinum-iridium (Pt-Ir) alloy, a magnesium alloy, etc.

The synthetic polymer-based material may be a degradable and/or non-degradable material.

Examples of the degradable polymer material that may be used include an aliphatic polyester such as poly(lactic acid) and a copolymer thereof, poly(glycolic acid) and a copolymer thereof, poly(hydroxy butyrate), poly(ε-caprolactone) and a copolymer thereof, poly(alkylene succinate), polyanhydride, poly(ortho ester), etc.

And, examples of the non-degradable polymer material that may be used include polyamide (nylon), polycyanoacrylate, polyphosphazene, thermoplastic polyurethane, low-density polyethylene, poly(vinyl alcohol), poly(ethylene oxide), poly(hydroxyethyl methacrylate), poly(methyl methacrylate), poly(tetrafluoroethylene) (PTFE), polydimethylsiloxane, poly(ethylene oxide-b-propylene oxide), poly(vinyl methyl ether), poly(N-alkyl acrylamide), polyethylene terephthalate, polypropylene, etc.a

The natural polymer-based polymer material includes a protein-based polymer such as collagen, albumin, silk protein, etc., a poly(amino acid) such as poly(L-lysine), poly(L-glutamic acid) and poly(aspartic acid), a polysaccharide and a derivative thereof, a polymer of botanic origin such as carboxymethyl cellulose, cellulose sulfate, agarose, alginate, carrageenan, etc., a polymer of human or animal origin such as hyaluronic acid, heparin, glycosaminoglycan, etc., a polymer of microbial origin such as dextran and a derivative thereof, chitosan and a derivative thereof, etc.

The metal and polymer wire materials may be used in combination depending on particular purposes. It is also possible to use specific metal and polymer wire materials for specific structure patterns only because compound structure patterns can be formed easily.

Those skilled in the art will appreciate that the conceptions and specific embodiments disclosed in the foregoing description may be readily utilized as a basis for modifying or designing other embodiments for carrying out the same purposes of the present disclosure. Those skilled in the art will also appreciate that such equivalent embodiments do

not depart from the spirit and scope of the disclosure as set forth in the appended claims.

[Industrial Applicability]

The present disclosure microscopically analyzes and quantifies a basic structure of a wire-mesh stent from a viewpoint of integrating, rather than separating, a stent and a delivery device which are main components of a stent system, especially in order to ensure the loadability that can be applied to the delivery device, which is a practical necessity. As a result, a wire-mesh stent defined by a compression ratio ($r_{comp}$) can be fabricated.

## Claims

1. A method for calculating a diameter $D_{ob}$ of a wire-mesh stent after compression of the stent, wherein
$D_{ob} = 2R_{ob}$, wherein the $R_{ob}$ represents a radius of the outmost boundary, the radius of the outmost boundary ($R_{ob}$) being defined by the following Formula 1:

**Formula 1**

$$R_{ob} = \frac{R_{tb}}{\cos(180/N_{xo})},$$

and
in Formula 1, the $R_{tb}$ represents an inradius of the outmost boundary and the $N_{xo}$ represents the number of virtual hook nodes tangential on the outmost boundary.

2. The method for calculating a diameter of a stent after compression according to claim 1 wherein
the $N_{xo}$ is defined by the following Formula 2:

**Formula 2**

$$N_{xo} = \left\| \frac{180}{\tan^{-1}(W_{hook}/(2R_{tb}))} \right\|,$$

the $W_{hook}$ represents the nominal width of a hook node and is defined by the following Formula 3:

**Formula 3**

$$W_{hook} = \Phi_w \times SF_{wh}$$

wherein
in Formula 3, the $\Phi_w$ represents the diameter of a wire, and the $SF_{wh}$ represents the width scale factor of a hook node and has a value of 3.0-3.6, and
in Formula 2, the $R_{tb}$ represents the inradius of the outmost boundary.

3. The method for calculating a diameter of a stent after compression according to claim 2,
wherein the $R_{tb}$ is defined by the following Formula 4 and the number of virtual hook nodes tangential on the outmost boundary ($N_{xo}$) is an integer satisfying the boundary condition of the following Formula 5:

**Formula 4**

$$R_{tb} = R_{ib} + H_{hook}$$

**Formula 5**

$$\left\| \frac{2\pi R_{tb}}{W_{hook}} \right\| \leq N_{xo} \leq \left\| \frac{2\pi R_{ob}}{W_{hook}} \right\|$$

wherein
in Formula 4, the $R_{ib}$ represents the radius of the inmost boundary and the $H_{hook}$ represents the nominal height of a hook node.

**4.** The method for calculating a diameter of a stent after compression according to claim 2 or 3,
wherein
in Formula 2, if $(R_{ob} - R_{tb}) << H_{hook}$, the number of virtual hook nodes tangential on the outmost boundary ($N_{xo}$) is calculated by the following Formula 6:

**Formula 6**

$$N_{xo} = \| 2\pi R_{tb}/W_{hook} \|.$$

**5.** The method for calculating a diameter of a stent after compression according to claim 3,
wherein
in Formula 4, the radius of the inmost boundary ($R_{ib}$) is defined by the following Formula 7 and the nominal height of a hook node ($H_{hook}$) is defined by the following Formula 8:

**Formula 7**

$$R_{ib} = \frac{W_{hook}}{2 \bullet \tan(180/N_{xi})}$$

**Formula 8**

$$H_{hook} = \Phi_{w} \times SF_{hh}$$

wherein
in Formula 7, the $N_{xi}$, represents the number of virtual hook nodes tangential on the inmost boundary, and
in Formula 8, the $SF_{hh}$ represents the height scale factor of a hook node and has a value of 2.7-3.3.

**6.** The method for calculating a diameter of a stent after compression according to claim 5, wherein the number of virtual hook nodes tangential on the inmost boundary ($N_{xi}$) is the largest round to the nearest integer satisfying the boundary condition of the following Formula 9:

**Formula 9**

$$N_{h} < \mathrm{Max}\ \{ N_{xi} \} \leq \| (W_{avg} \times N_{t}) / W_{hook} \|$$

wherein the $W_{avg}$ represents the average nominal width of node per section of the stent, the $N_{t}$ represents the total

number of node per section of the stent, and the $N_h$ represents the number of hook node per section of the stent.

7. The method for calculating a diameter of a stent after compression according to claim 6, wherein the $W_{avg}$ is defined by the following Formula 11 and the $N_t$ is defined by the following Formula 12:

**Formula 11**

$$W_{avg} = W_{total} / N_t$$

**Formula 12**

$$N_t = N_h + N_c$$

wherein
in Formula 11, the $W_{total}$ represents the sum total of nominal width of all nodes per section of the stent, and
in Formula 12, the $N_c$ represents the number of cross node per section of the stent.

8. The method for calculating a diameter of a stent after compression according to claim 7, wherein the $W_{total}$ is defined by the following Formula 13:

**Formula 13**

$$W_{total} = W_{hook} \times N_h + W_{cross} \times N_c$$

wherein
in Formula 13, the $W_{cross}$ is represents the nominal width of a cross node and is defined by the following Formula 14:

**Formula 14**

$$W_{cross} = \Phi_w \times SF_{wc}$$

wherein
in Formula 14, the $SF_{wc}$ represents the width scale factor of a cross node and has a value of 1.7-2.3.

9. The method for calculating a diameter of a stent after compression according to claim 1, wherein the number of hooks is an integer from 3 to 8 per radial section of the stent.

10. The method for calculating a diameter of a stent after compression according to claim 1, wherein the stent is made of a material selected from a group consisting of a metal, a synthetic polymer and a natural polymer.

11. The method for calculating a diameter of a stent after compression according to claim 10, wherein the metal is selected from a group consisting of a nickel-titanium (Ni-Ti) shape memory alloy, a martensitic nickel-titanium (Ni-Ti) shape memory alloy, stainless steel, tantalum, tungsten (W), gold (Au), platinum, silver (Ag), nickel, titanium (Ti), chromium (Cr), a cobalt-chromium (Co-Cr) alloy, a platinum-chromium (Pt-Cr) alloy, a platinum-iridium (Pt-Ir) alloy and a magnesium alloy.

12. The method for calculating a diameter of a stent after compression according to claim 10, wherein the synthetic polymer is a degradable or non-degradable polymer or a combination thereof.

13. The method for calculating a diameter of a stent after compression according to claim 12, wherein the degradable polymer is selected from a group consisting of poly(lactic acid) and a copolymer thereof, poly(glycolic acid) and a copolymer, poly(hydroxy butyrate), poly(ε-caprolactone) and a copolymer thereof, poly(alkylene succinate), polyanhydride and poly(ortho ester).

**14.** The method for calculating a diameter of a stent after compression according to claim 12, wherein the non-degradable polymer is selected from a group consisting of polyamide (nylon), polycyanoacrylate, polyphosphazene, thermoplastic polyurethane, low-density polyethylene, poly(vinyl alcohol), poly(ethylene oxide), poly(hydroxyethyl methacrylate), poly(methyl methacrylate), poly(tetrafluoroethylene) (PTFE), polydimethylsiloxane, poly(ethylene oxide-b-propylene oxide)), poly(vinyl methyl ether), poly(N-alkyl acrylamide), polyethylene terephthalate and polypropylene.

**15.** The method for calculating a diameter of a stent after compression according to claim 10, wherein the natural polymer is selected from a group consisting of collagen, albumin, silk protein, poly(L-lysine), poly(L-glutamic acid), poly(aspartic acid), polysaccharide and a derivative thereof, carboxymethyl cellulose, cellulose sulfate, agarose, alginate, carrageenan, hyaluronic acid, heparin, glycosaminoglycan, dextran and a derivative thereof, and chitosan and a derivative thereof.

**16.** A method for manufacturing a wire-mesh stent, comprising:

(a) a phase of confirming a planar matrix wherein a plurality of nodes formed by horizontal node lines and vertical node lines crossing each other are represented by coordinates (i, j);
(b) a phase of selecting a start node position (S#(i, j)) by identifying the number of the horizontal node lines, the number of the vertical node lines, node position information, node shape information and figure-cutting line information;
(c) a phase of selecting a movement type of wire meshing from sandglass-type movement and radial movement and selecting one basic movement direction (D.mv) from the start node position (S#(i, j)) of the phase (c);
(d) a phase of moving to a neighbor node along the basic movement direction (D.mv) of the phase (c); and
(e) a phase of completing wire meshing by selecting one basic movement direction (D.mv) at the neighbor node of the phase (d) and selecting the start node position (S#(i, j)) as an end node position (E#(i, j)).

**17.** The method for manufacturing a wire-mesh stent according to claim 16, wherein, if sandglass-type movement is selected in the phase (c), the basic movement direction is selected from a group consisting of horizontal to left ($H_L$), horizontal to right ($H_R$), diagonal to left up ($D_{LU}$), diagonal to left down ($D_{LD}$), diagonal to right up ($D_{RU}$) and diagonal to right down ($D_{RD}$).

**18.** The method for manufacturing a wire-mesh stent according to claim 16, wherein, if radial movement is selected in the phase (c), the basic movement direction is selected from a group consisting of horizontal to left ($H_L$), horizontal to right ($H_R$), diagonal to left up ($D_{LU}$), diagonal to left down ($D_{LD}$), diagonal to right up ($D_{RU}$), diagonal to right down ($D_{RD}$), vertical to up ($V_U$) and vertical to down ($V_D$).

**19.** The method for manufacturing a wire-mesh stent according to claim 16, wherein the method further comprises, after the phase (d), (d-1) a phase of repeating moving from the neighbor node of the phase (d) to another neighbor node along the basic movement direction (D.mv) of the phase (c).

**20.** The method for manufacturing a wire-mesh stent according to claim 16, wherein the neighbor node of the phase (d) has a node shape of a hook or a cross, and the hook is selected from horizontal hook, vertical hook, horizontal open hook, vertical open hook, horizontal half hook and vertical half hook or is selected from in-coil half hook, out-coil half hook, band half hook, in-band half hook, out-band half hook, fork half hook, in-track half hook, out-track half hook, close-turning half hook and gap-turning half hook.

**21.** The method for manufacturing a wire-mesh stent according to claim 16, wherein the method further comprises, after the phase (e), (f) a phase of expressing a fabrication expression and verifying a planar mesh.

**22.** A multi-alterable structure patterning method of a wire-mesh stent, wherein, when a radial section in which a hook node or a cross node is present is defined as a node radial section and a primitive n-gon with the hook node arranged at the vertex is defined, the primitive n-gon is inscribed to the node radial section, and
which comprises

(a) a phase of selecting a primitive n-gon;
and comprises at least one of
(b) a phase of rotating each of the node radial section with respect to a neighboring node radial section about the center of the node radial section by a predetermined rotation angle along a predetermined rotation direction;

(c) a phase of skipping a specific node radial section from the node radial section; and
(d) a phase of combining two or more of the primitive n-gon, two or more of the predetermined rotation direction, two or more of the predetermined rotation angle, or two or more of the specific node radial section.

**23.** The multi-alterable structure patterning method of a wire-mesh stent according to claim 22, wherein the primitive n-gon is a regular or irregular polygon, and the n is at least one integer from 3 to 8.

**24.** The multi-alterable structure patterning method of a wire-mesh stent according to claim 22, wherein the predetermined rotation angle ($\theta_{rot}$) is within a range defined by the following Formula 15, and the predetermined rotation direction is a clockwise direction (forward direction) or a counterclockwise direction (reverse direction):

**Formula 15**

$$0 \leq \theta_{rot} < 360°$$

wherein
in Formula 15, if the primitive n-gon is a regular polygon, $\theta_{rot}$ is not a rotation angle corresponding to an integer multiple of $\| 360/\text{vertex}(n) \|$.

**25.** The multi-alterable structure patterning method of a wire-mesh stent according to claim 22, wherein the number of the node radial sections is an integer which is equal to or greater than the number of the selected primitive n-gon if the predetermined rotation angle ($\theta_{rot}$) is 0° and is equal to or smaller than the number of angle division ($N_{ang}$) and is equal to or greater than the number of the selected primitive n-gon if the predetermined rotation angle ($\theta_{rot}$) > 0, and the number of angle division satisfies the following Formula 16:

**Formula 16**

$$N_{ang} = \| 360°/\theta_{rot} \| .$$

**26.** The multi-alterable structure patterning method of a wire-mesh stent according to claim 22, wherein, in the phase of skipping a specific node radial section from the node radial section, the skip is selected from missing skip and pushing skip,
wherein
the missing skip refers to missing a node pattern of the specific node radial section, and
the pushing skip refers to conducting a node pattern of the specific node radial section after adding a node radial section without a hook node.

**27.** The multi-alterable structure patterning method of a wire-mesh stent according to claim 22, wherein a wire-mesh stent formed by the multi-alterable structure patterning method of a wire-mesh stent has a symmetry ($Q_{symm}$) defined by the following Formula 17:

**Formula 17**

$$Q_{symm} = 1 - (CV_{N.h.horiz} + CV_{N.h.vert} + CV_{\Delta.h.horiz})/3$$

wherein $CV_{N.h.horiz} = (SD/MV)_{N.h.horiz}$ (Formula 18), $CV_{N.h.vert} = (SD/MV)_{N.h.vert}$ (Formula 19), $CV_{\Delta.h.horiz} = (SD/MV)_{\Delta.h.horiz} = \sum(CV_{\Delta.h.horiz.i\text{-}th})_{i,\ i=1\ to\ N.lay} / N_{lay}$ (Formula 20) and $\Delta$.h.horiz = $N_{H\text{-}H.vert}$ (Formula 21), wherein the CV is a coefficient of variation, the SD is a standard deviation, the MV is a mean value, the N.h.horiz is the number of hooks per horizontal node line, the N.h.vert is the number of hooks per vertical node line, the $\Delta$.h.horiz is the distance between neighbor hooks per horizontal node line, the $\Delta$.h.horiz.i-th is the distance between neighbor hooks per i-th horizontal node line, the N.lay (or $N_{lay}$) is the number of four or more radial sections except the skip node line(no. of primitive section layer applied), and the $N_{H\text{-}H.vert}$ is the number of vertical node lines between neighbor hooks per horizontal node line(no. of vertical node line between neighbor hooks per horizontal node line).

**28.** The multi-alterable structure patterning method of a wire-mesh stent according to claim 27, wherein a vertical node

line in which the whole vertical node line is composed of null node is excluded from the calculation of Formula 17.

**29.** The multi-alterable structure patterning method of a wire-mesh stent according to claim 27, wherein the symmetry ($Q_{symm}$) is 0.5 or greater.

**30.** The multi-alterable structure patterning method of a wire-mesh stent according to claim 27, wherein the $N_{H\text{-}H.vert}$ of Formula 21 is defined by the following Formula 22:

## Formula 22

$$N_{H\text{-}H.vert} = \theta_{H\text{-}H} / \theta_{div}$$

wherein the $\theta_{H\text{-}H} = N_{H\text{-}H.vert} \times \theta_{div} = N_{H\text{-}H.vert} \times W_{n.line}$ (Formula 23), the $\theta_{H\text{-}H}$ is an angle contained between neighbor hooks, the $\theta_{div}$ is a division angle between nodes, and the $W_{n.line}$ is a node line width.

**31.** The multi-alterable structure patterning method of a wire-mesh stent according to claim 22, wherein a wire-mesh stent formed by the multi-alterable structure patterning method of a wire-mesh stent has a circularity ($Q_{circ}$) defined by the following Formula 24:

## Formula 24

$$Q_{circ} = Q_{circ.simple} \times (1 - CV_{avg})$$

wherein $Q_{circ.simple} = n \times \sin(180°/n)/\pi$ (Formula 25), $CV_{avg} = (CV_{\Delta h.radial} + CV_{N.h.vert})/2$ (Formula 26), $CV_{\Delta.h.radial} = (SD/MV)_{\Delta.h.radial}$-(Formula 27), $CV_{N.h.vert} = (SD/MV)_{N.h.vert}$ (Formula 28) and n = N.h.radial (or $N_{h.radial}$) (Formula 29), wherein the $Q_{circ.simple}$ is simple circularity assuming a regular n-gon, the $CV_{avg}$ is an average coefficient of variation, the CV is a coefficient of variation, the SD is a standard deviation, the MV is a mean value, the n is the number of the sides of the regular n-gon ( = total number of hooks on the axial view), the $N_{.h.radial}$ is the total number of hooks on the axial view, the $_{\Delta.h.radial}$ is the perpendicular distance of neighbor hooks on the axial view, and the $N_{.h.vert}$ is the number of hooks per vertical node line.

**32.** The multi-alterable structure patterning method of a wire-mesh stent according to claim 31, wherein a vertical node line in which the whole vertical node line is composed of null node is excluded from the calculation of Formula 24.

**33.** The multi-alterable structure patterning method of a wire-mesh stent according to claim 31, wherein the circularity ($Q_{circ}$) is 0.4 or greater.

FIG. 1

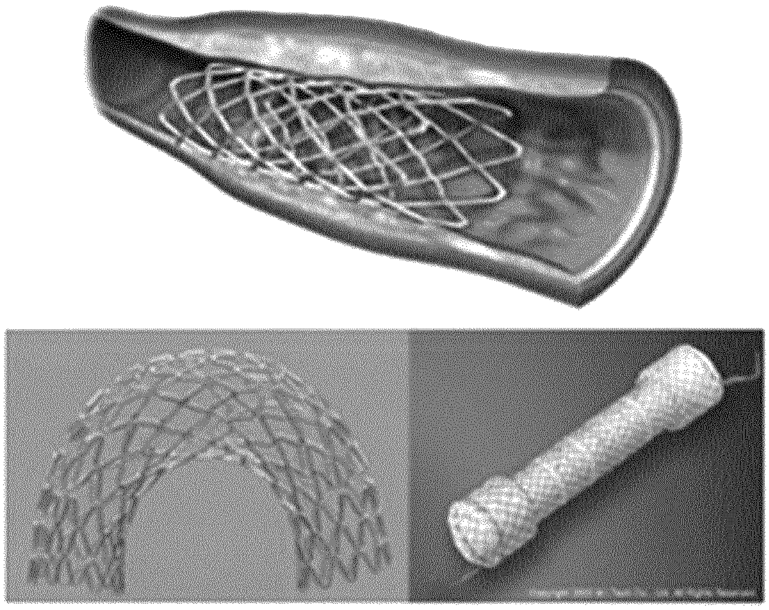

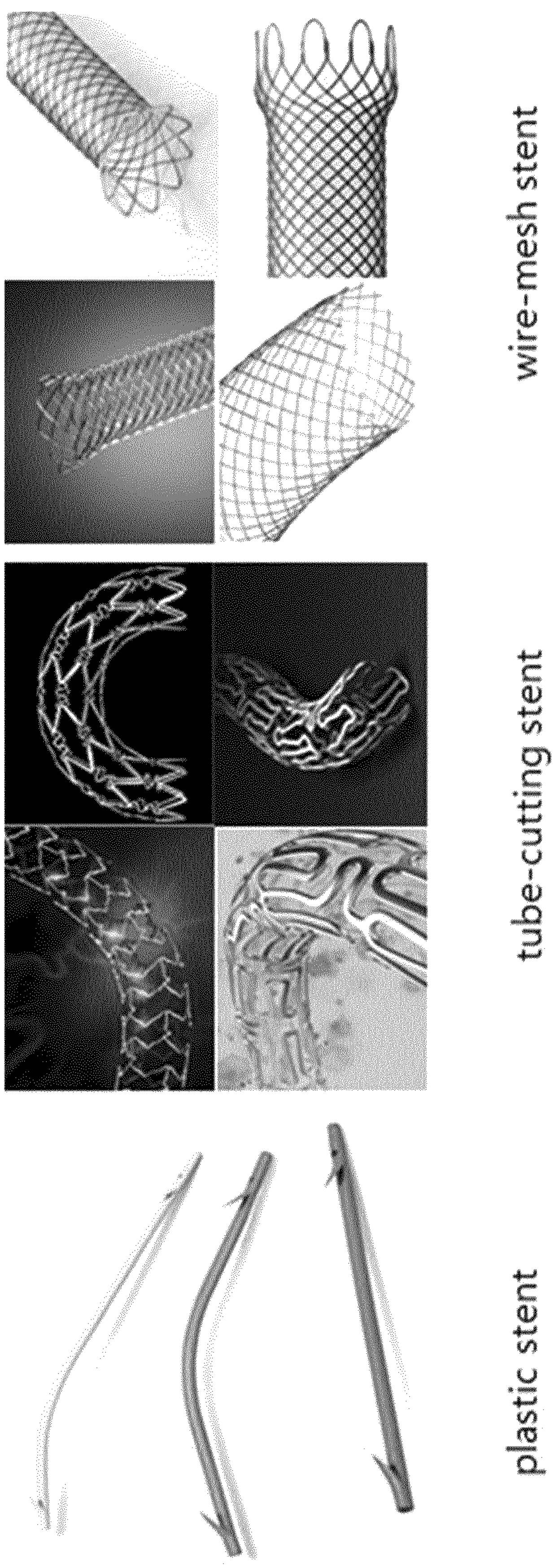


FIG. 2

FIG. 3

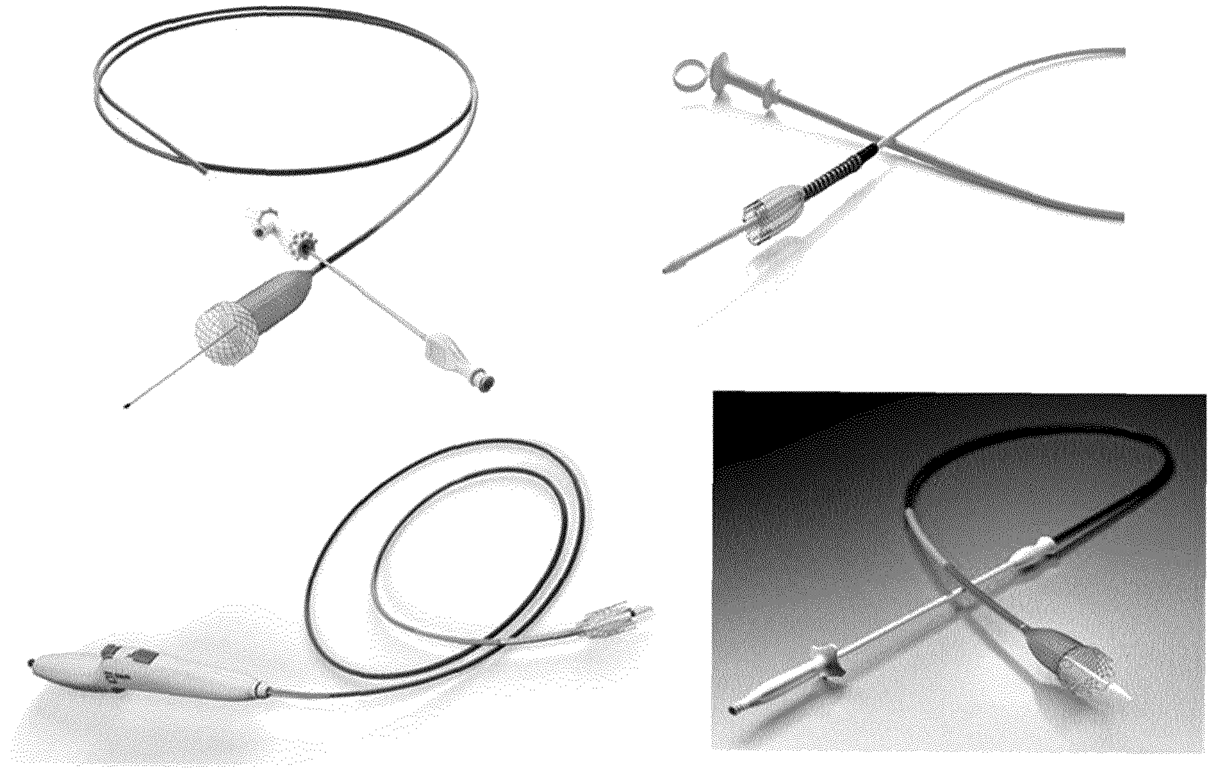

FIG. 4

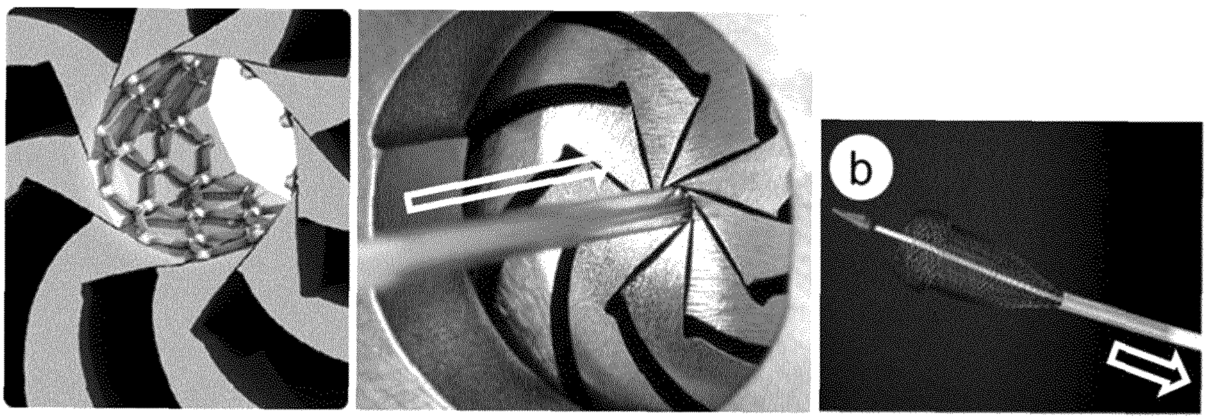


a) push type of loading

b) pull type of loading

FIG. 5

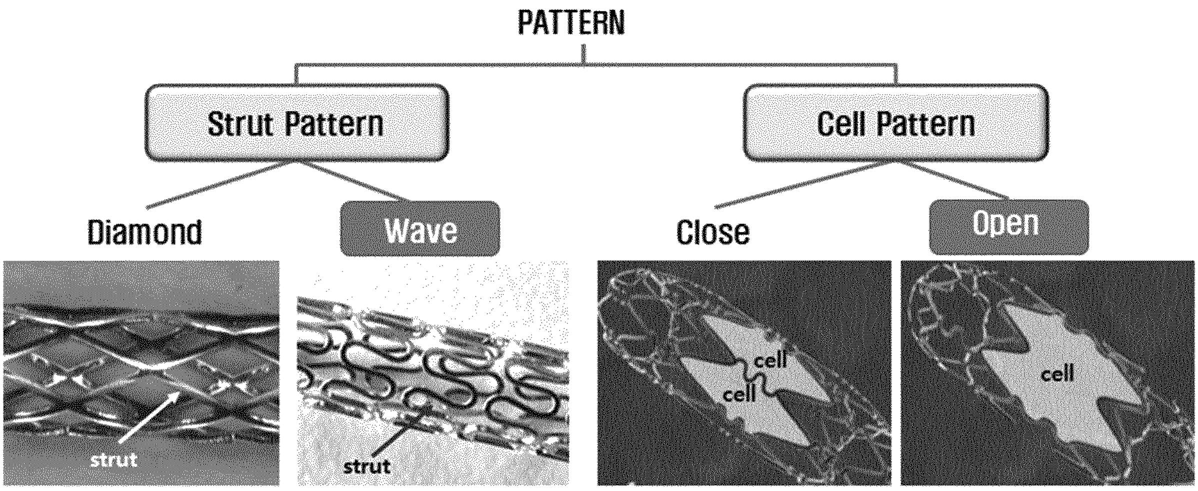


FIG. 6

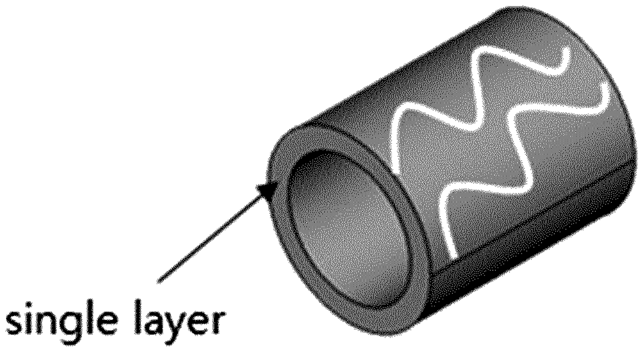


FIG. 7

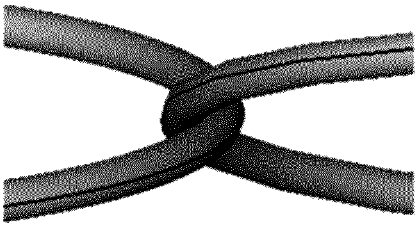

a) hook

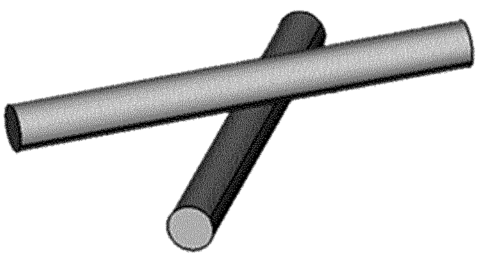

b) cross

FIG. 8

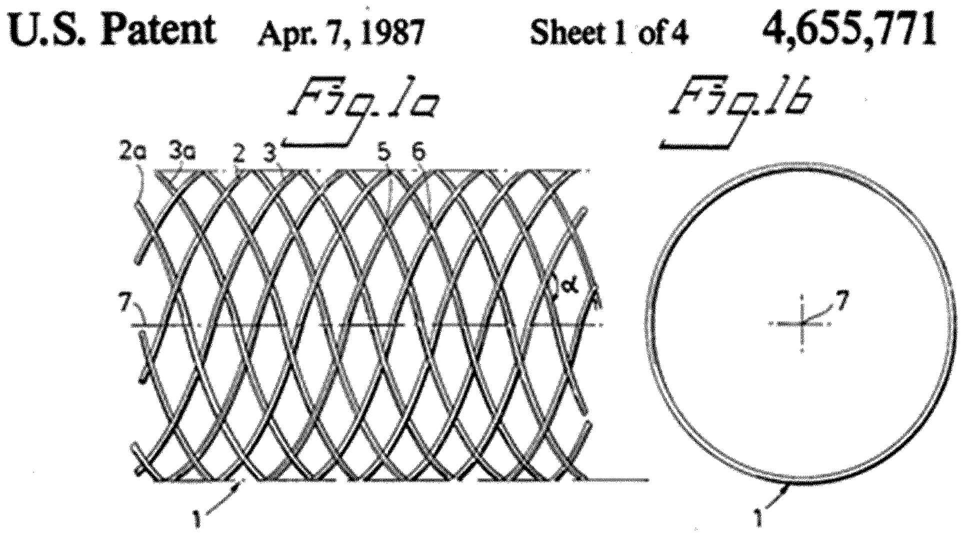
U.S. Patent Apr. 7, 1987 Sheet 1 of 4 4,655,771
Fig. 1a
Fig. 1b
2a
3a
2
3
5
6
7
α
1

FIG. 9

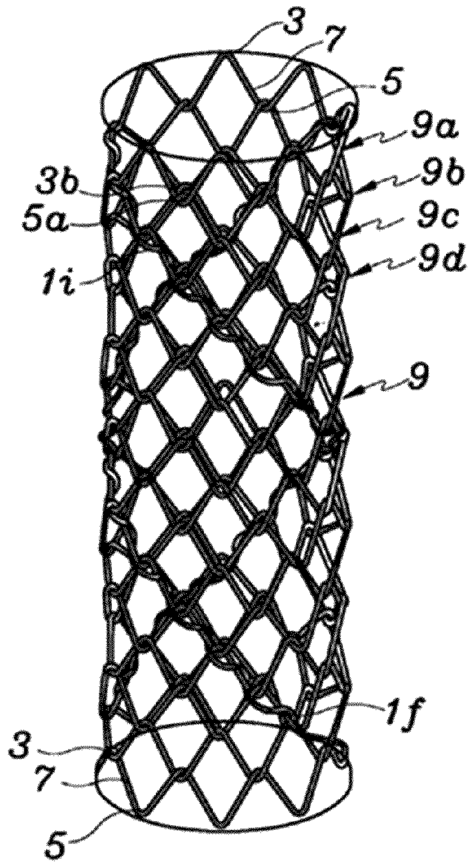
3
7
5
9a
9b
9c
9d
3b
5a
1i
9
1f

FIG. 10

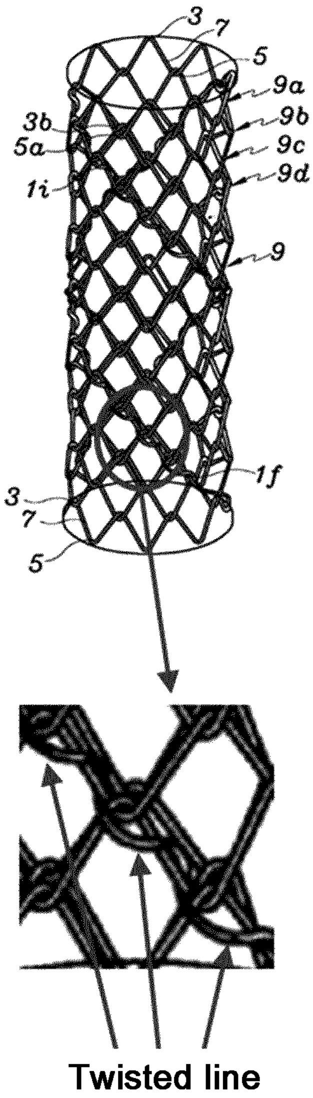
3
7
5
9a
3b
9b
5a
9c
9d
1i
9
1f
3
7
5
Twisted line

FIG. 11

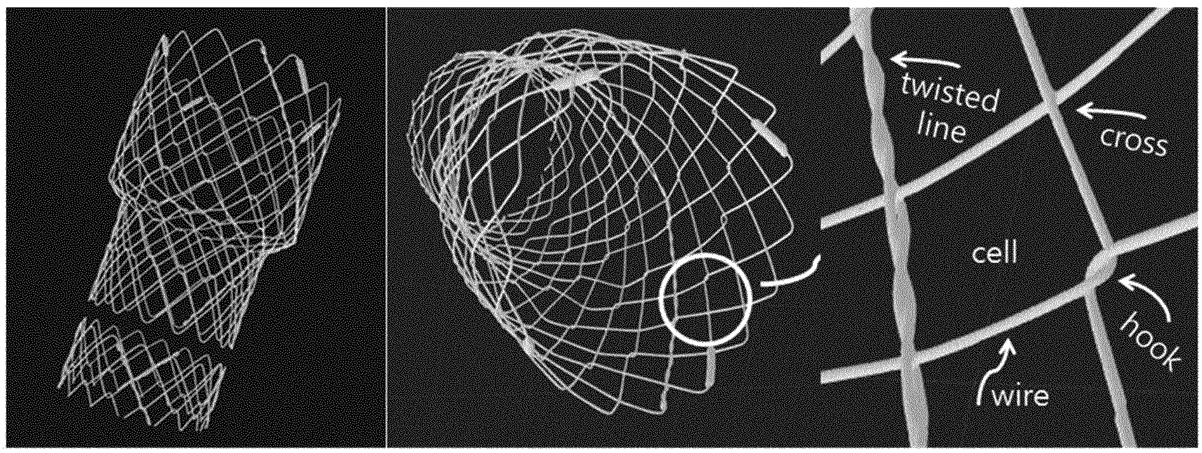
twisted
line
cross
cell
hook
wire

FIG. 12

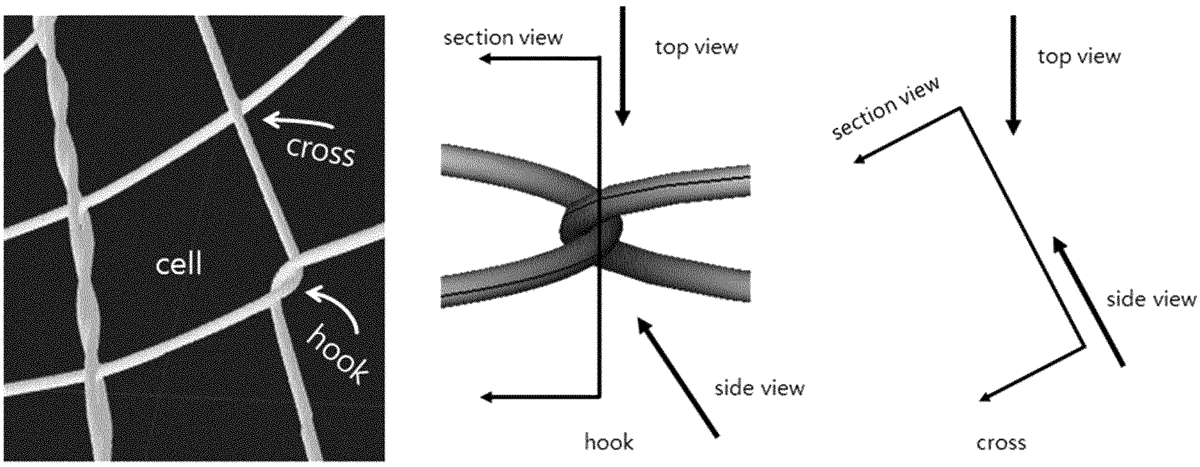
cross
cell
hook
section view
top view
side view
hook
top view
section view
side view
cross

FIG. 13

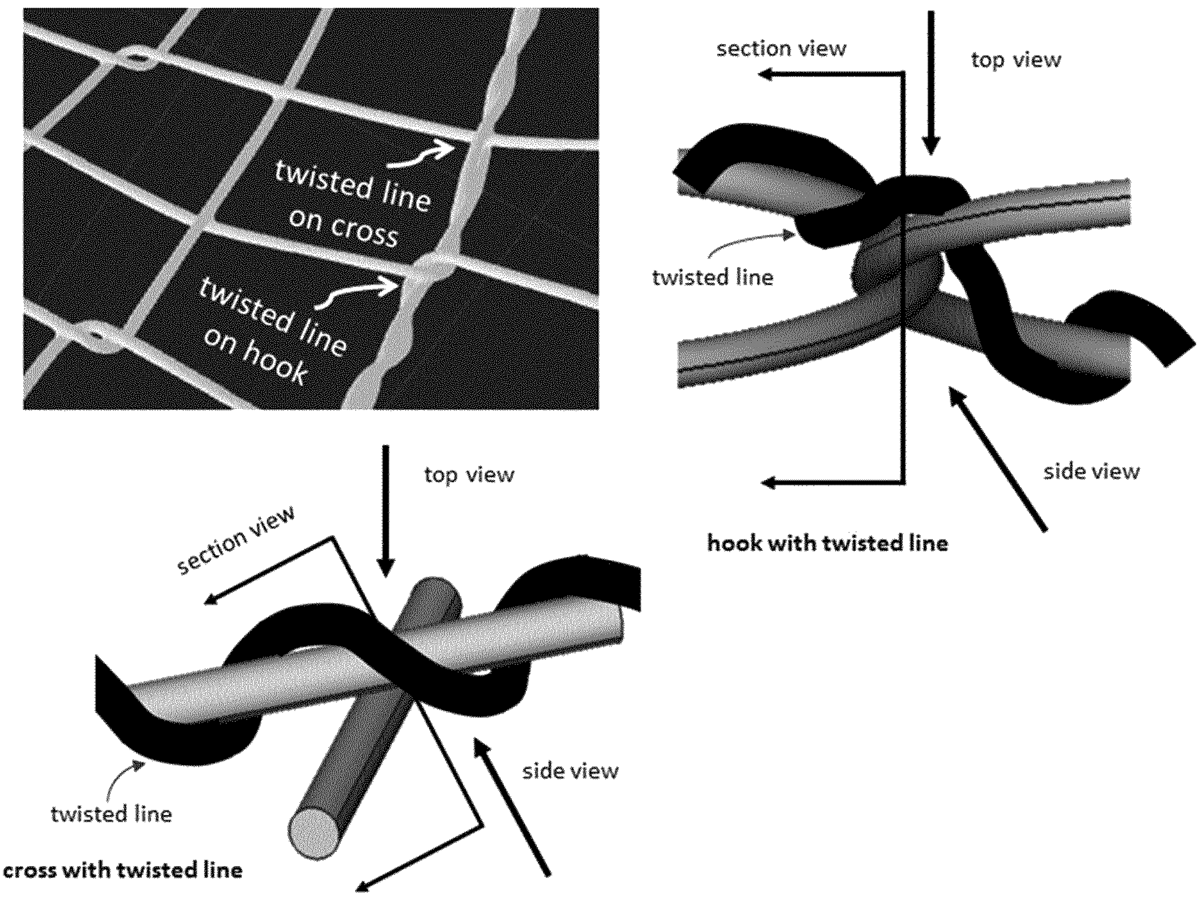
twisted line on cross
twisted line on hook
section view
top view
twisted line
side view
hook with twisted line
top view
section view
side view
twisted line
cross with twisted line

FIG. 14

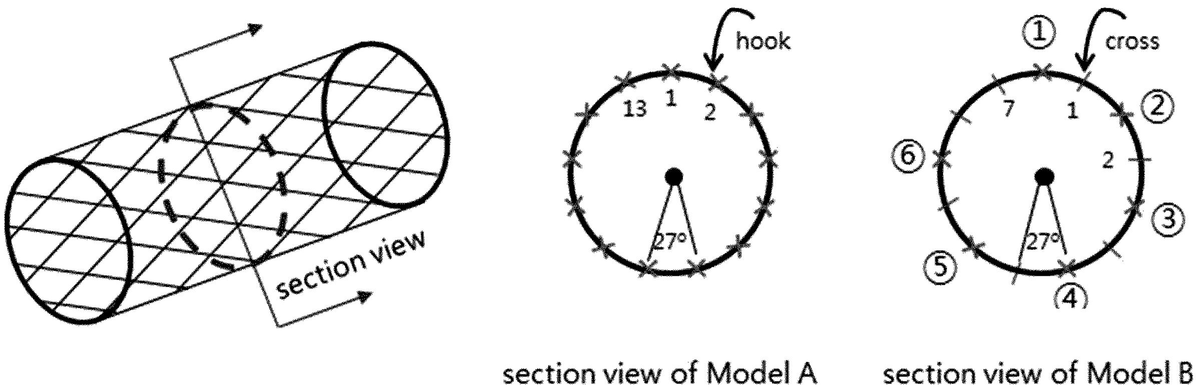
section view
hook
13
1
2
27°
①
cross
7
1
②
⑥
2
③
⑤
27°
④
section view of Model A
section view of Model B

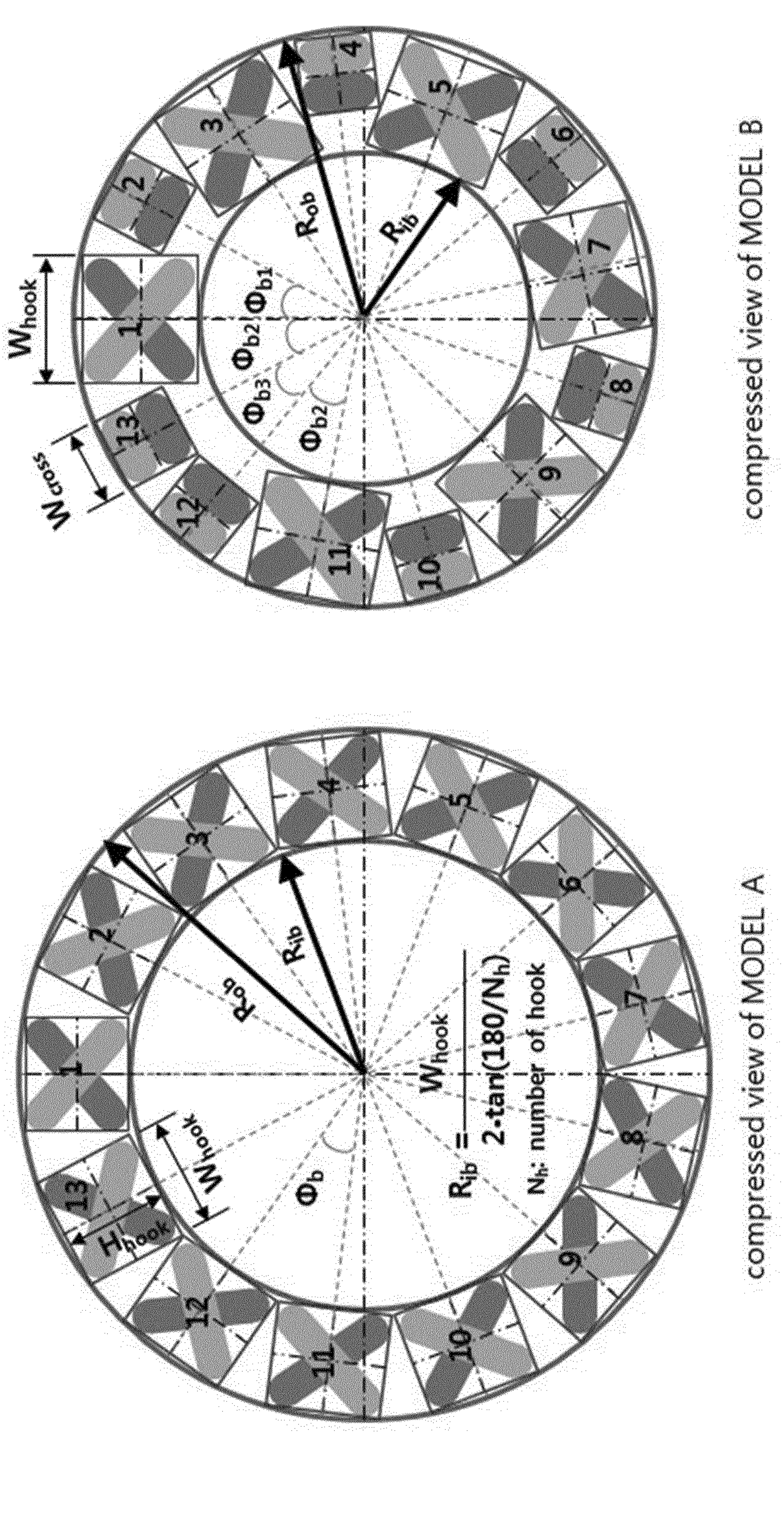

$R_{ib} = \frac{W_{hook}}{2 \cdot tan(180/N_h)}$
$N_h$: number of hook
$R_{ob}$
$R_{ib}$
$W_{hook}$
$H_{hook}$
$\Phi_b$
1 2 3 4 5 6 7 8 9 10 11 12 13 14
compressed view of MODEL A
$W_{hook}$
$W_{cross}$
$R_{ob}$
$R_{ib}$
$\Phi_{b1}$
$\Phi_{b2}$
$\Phi_{b3}$
1 2 3 4 5 6 7 8 9 10 11 12 13
compressed view of MODEL B


FIG. 15

FIG. 16

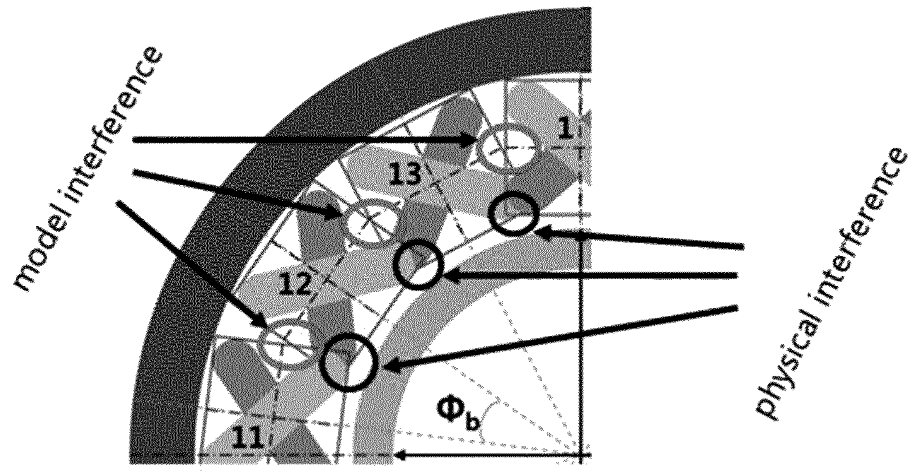
model interference
physical interference
1
13
12
11
$\Phi_b$

FIG. 17

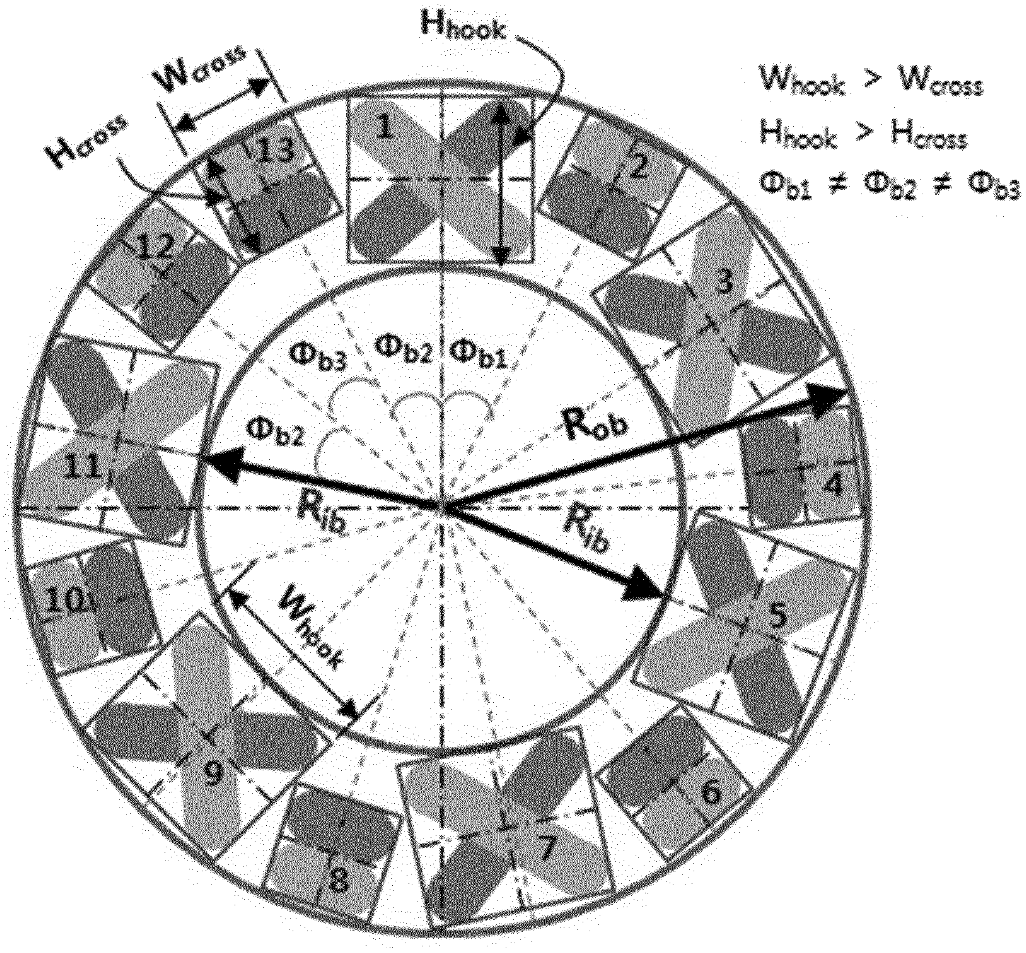
$H_{hook}$
$W_{cross}$
$H_{cross}$
$W_{hook} > W_{cross}$
$H_{hook} > H_{cross}$
$\Phi_{b1} \neq \Phi_{b2} \neq \Phi_{b3}$
$\Phi_{b3}$ $\Phi_{b2}$ $\Phi_{b1}$
$\Phi_{b2}$
$R_{ob}$
$R_{ib}$
$R_{ib}$
$W_{hook}$
1
2
3
4
5
6
7
8
9
10
11
12
13

FIG. 18

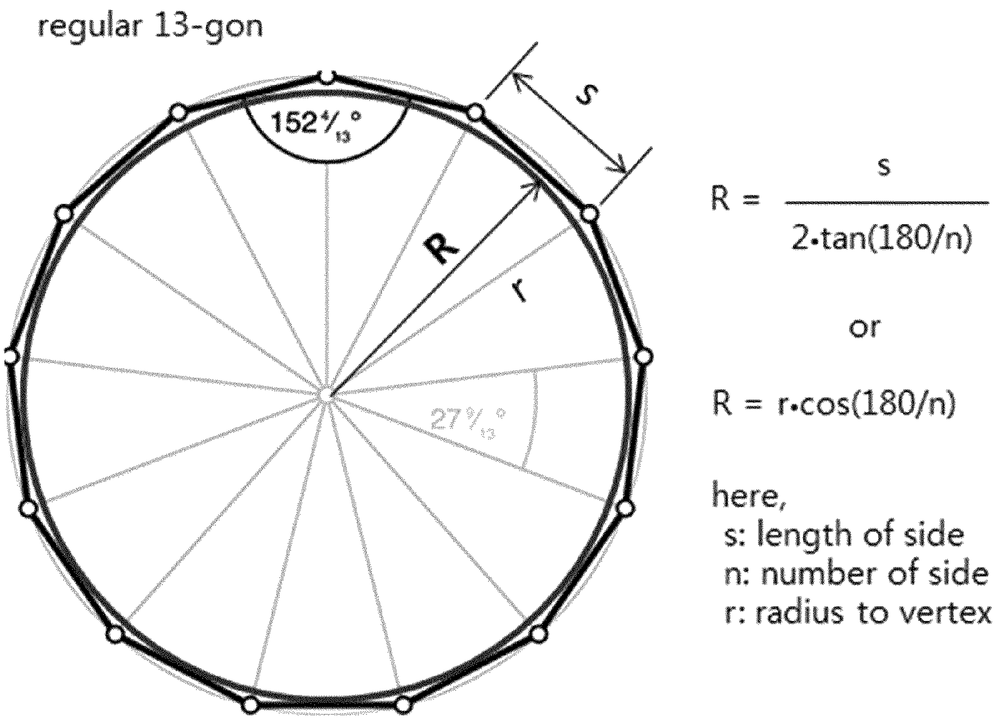
regular 13-gon
152 4/13 °
s
R
r
27 9/13 °
R = s / 2•tan(180/n)
or
R = r•cos(180/n)
here,
s: length of side
n: number of side
r: radius to vertex

FIG. 19

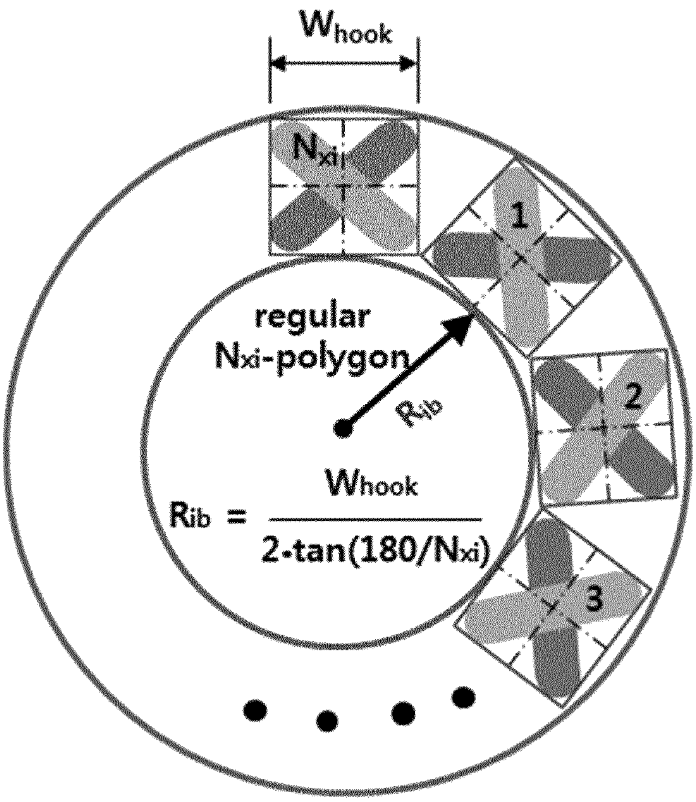
Whook
Nxi
1
2
3
regular
Nxi-polygon
Rib
Rib = Whook / 2•tan(180/Nxi)

FIG. 20

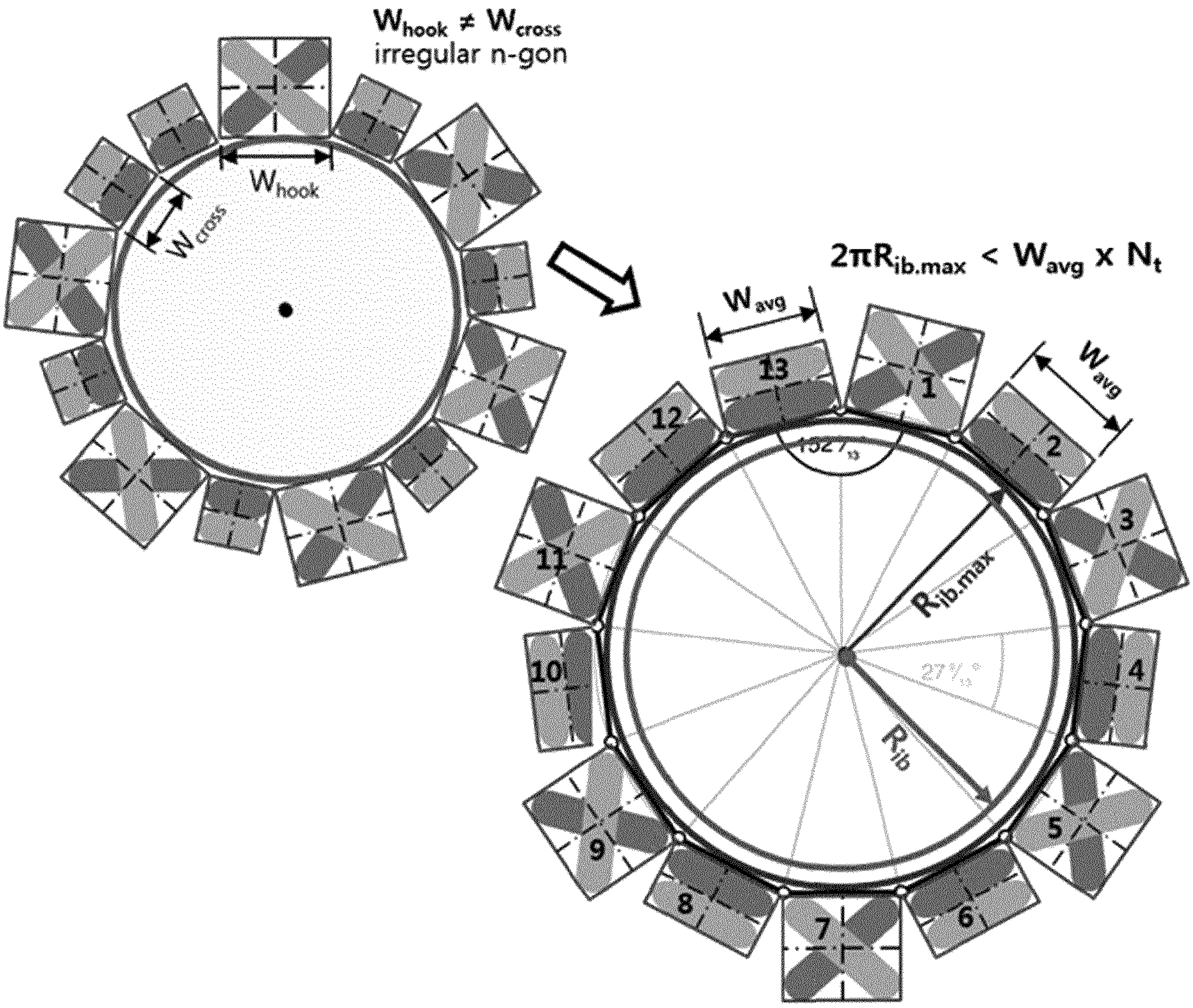
$W_{hook} \neq W_{cross}$
irregular n-gon
$W_{hook}$
$W_{cross}$
$2\pi R_{ib.max} < W_{avg} \times N_t$
$W_{avg}$
$W_{avg}$
1
2
3
4
5
6
7
8
9
10
11
12
13
$R_{ib.max}$
$R_{ib}$

FIG. 21

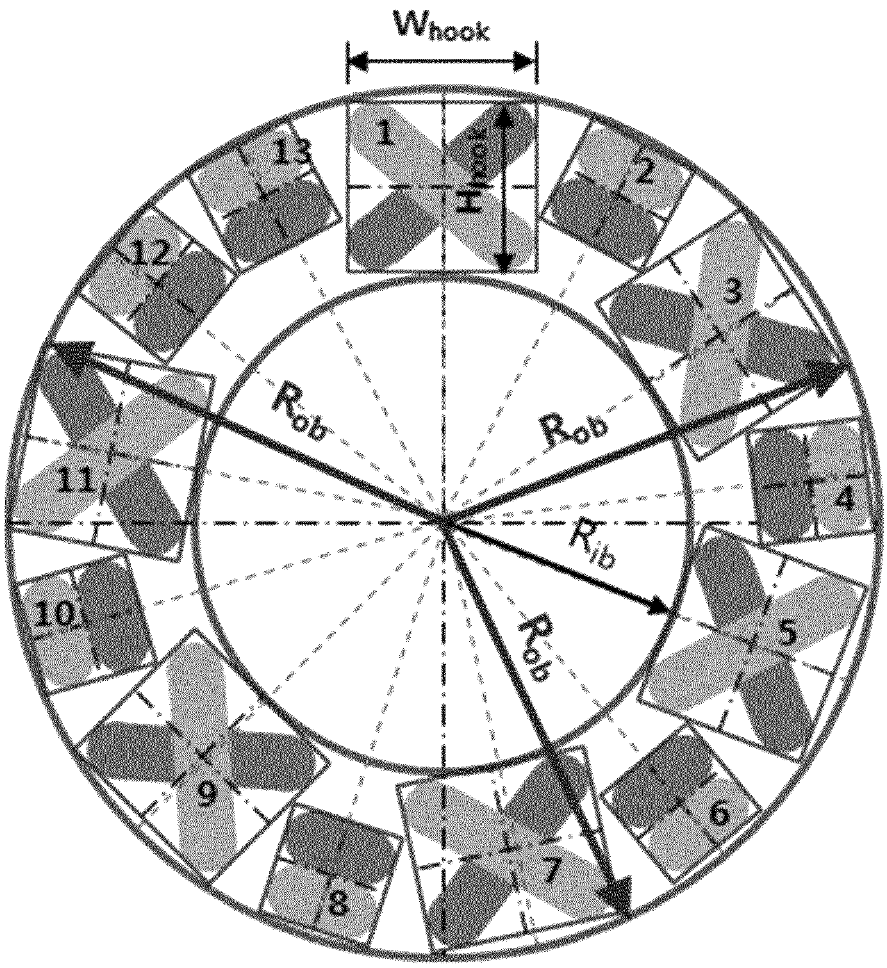

FIG. 22

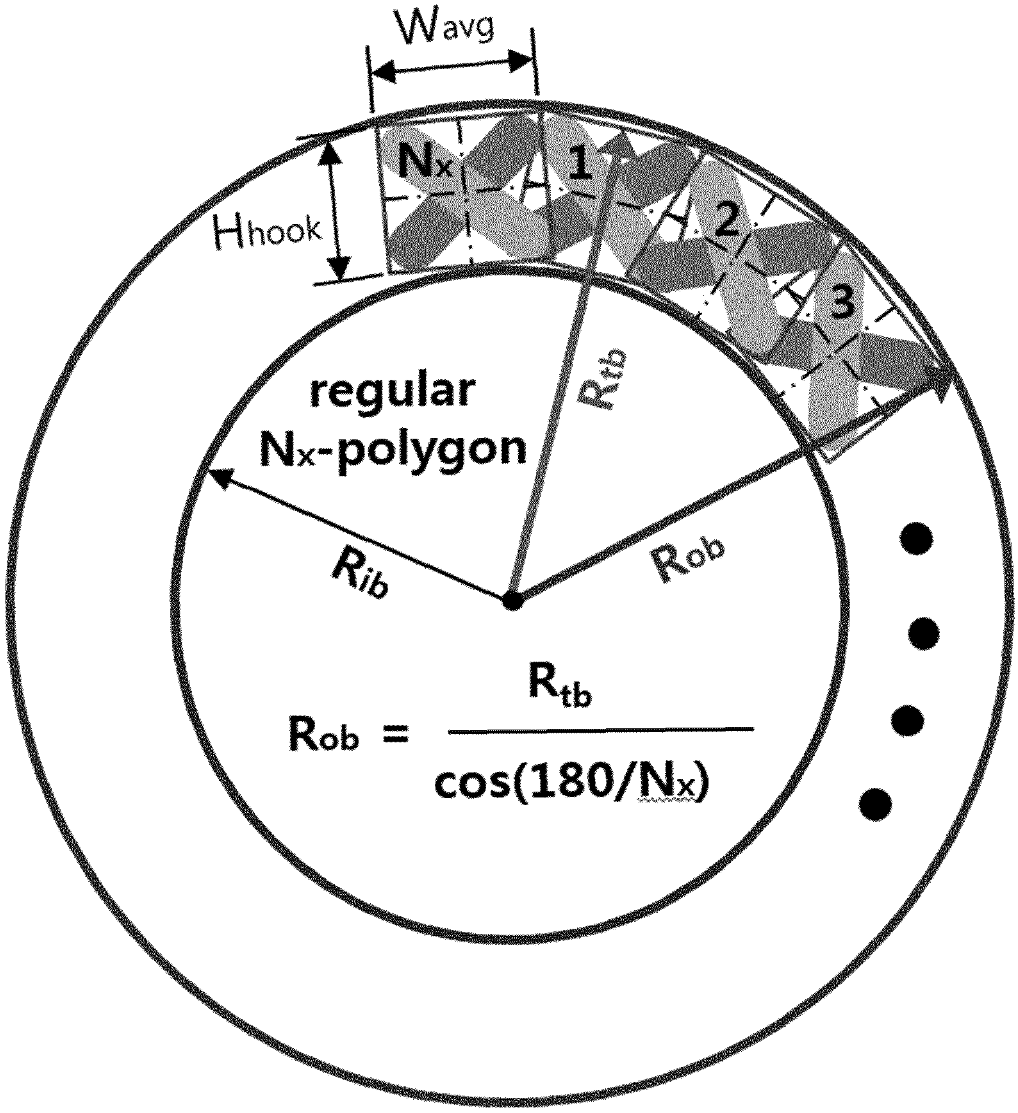

Wavg
Hhook
Nx
1
2
3
Rtb
regular
Nx-polygon
Rib
Rob
Rob = Rtb / cos(180/Nx)

FIG. 23

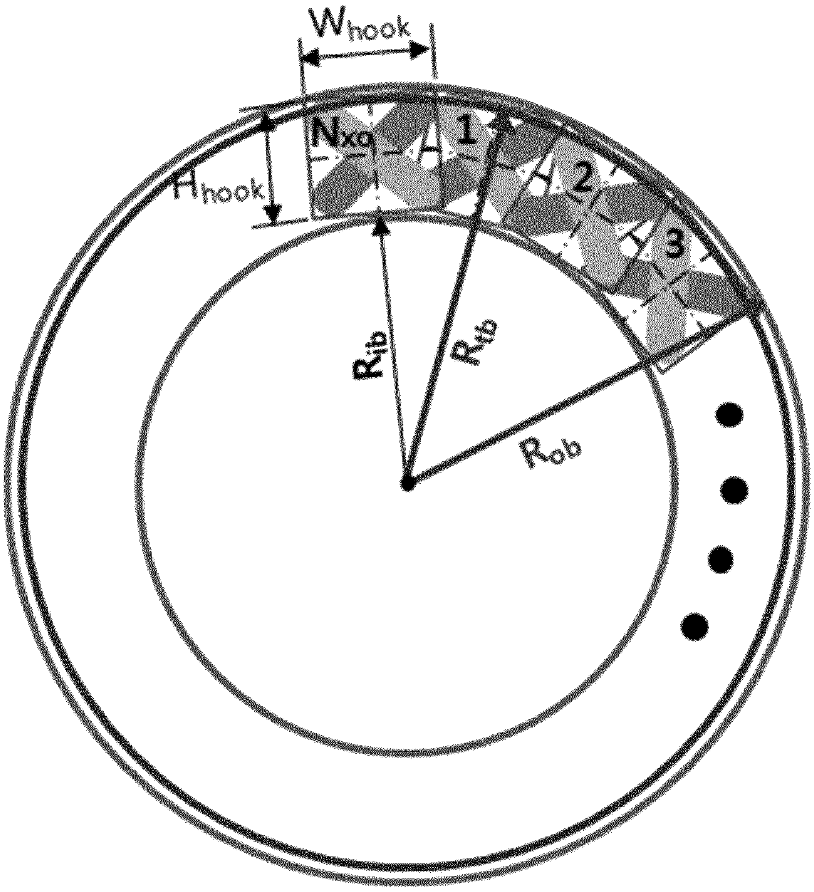
W hook
H hook
N xo
1
2
3
R ib
R tb
R ob

FIG. 24

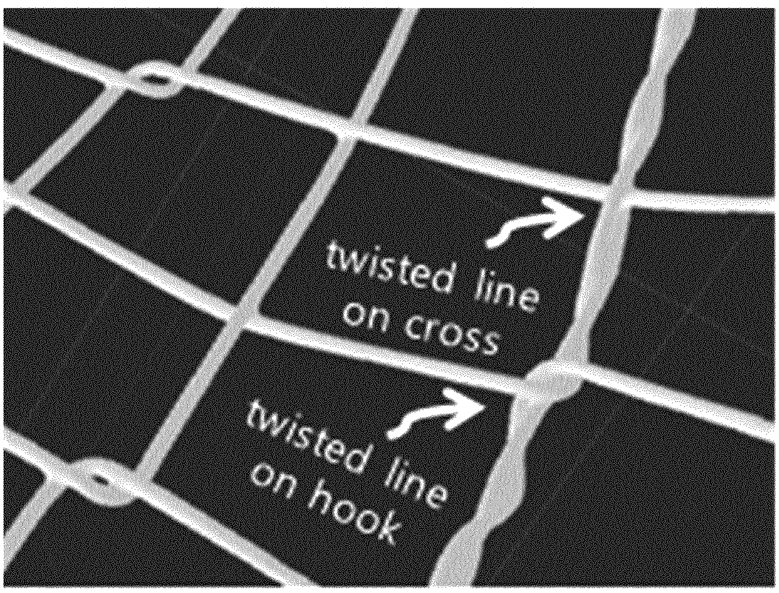
twisted line
on cross
twisted line
on hook

FIG. 25

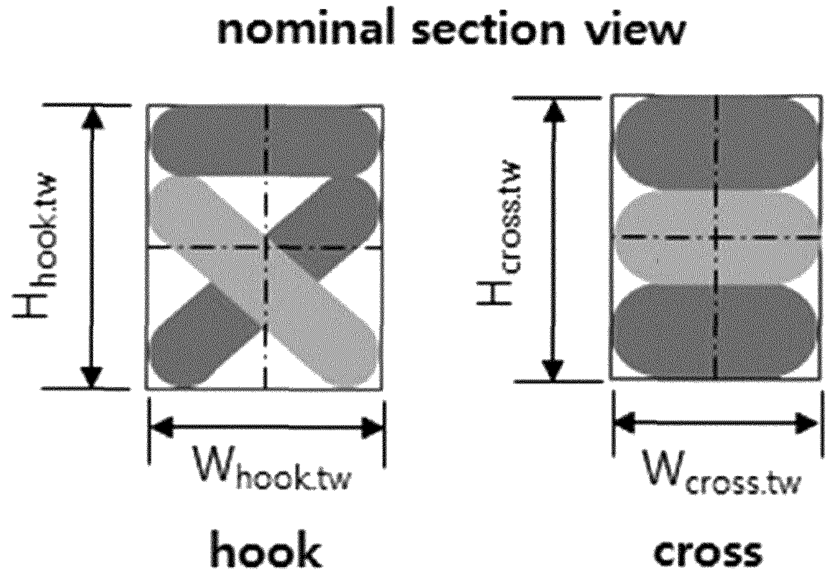
nominal section view
$H_{hook.tw}$
$W_{hook.tw}$
hook
$H_{cross.tw}$
$W_{cross.tw}$
cross

FIG. 26

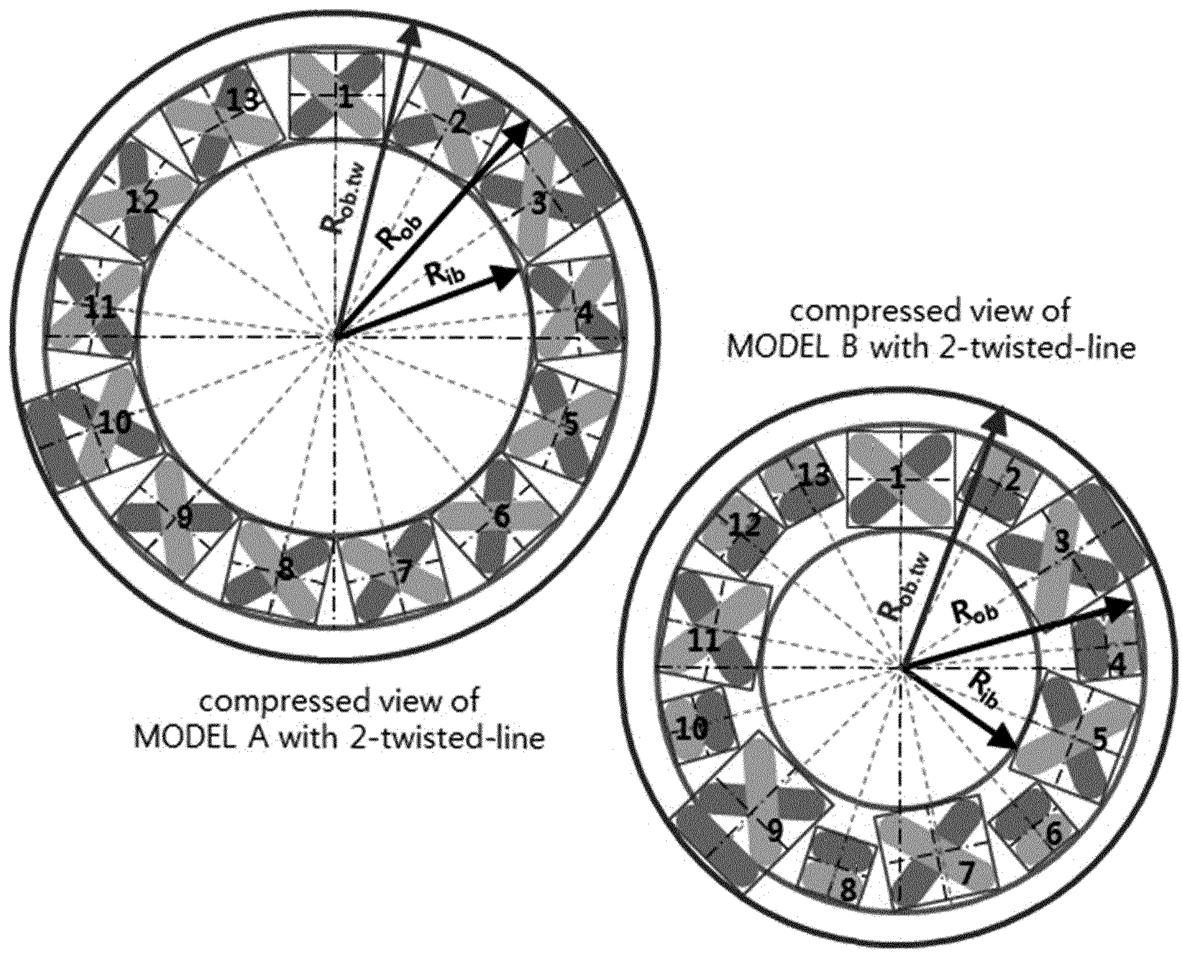
1
2
3
4
5
6
7
8
9
10
11
12
13
$R_{ob.tw}$
$R_{ob}$
$R_{ib}$
compressed view of
MODEL A with 2-twisted-line
compressed view of
MODEL B with 2-twisted-line

FIG. 27

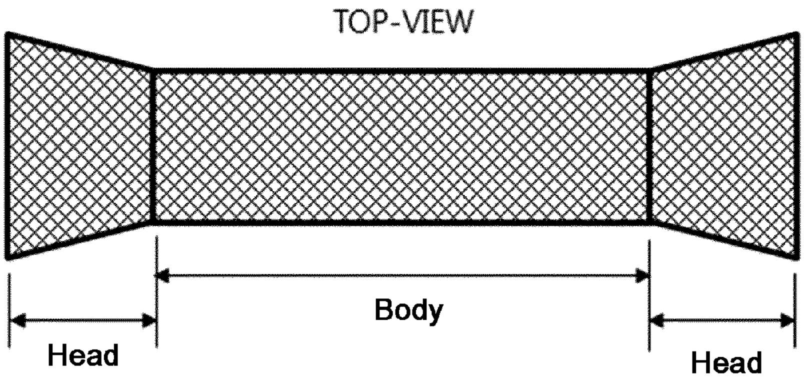


FIG. 28

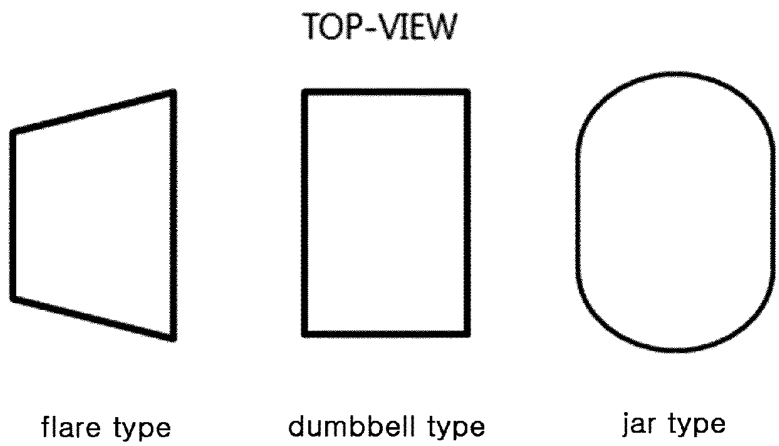


FIG. 29

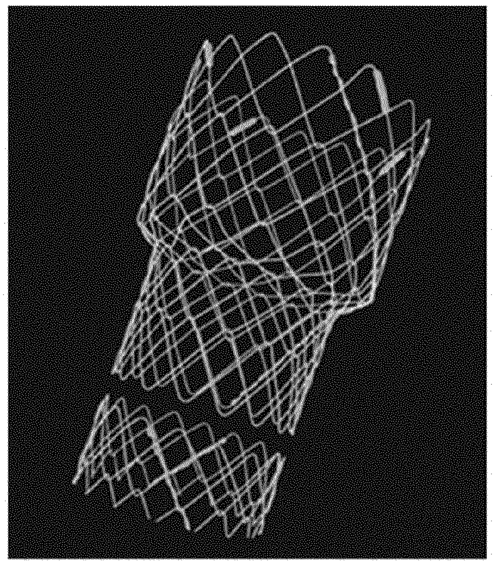

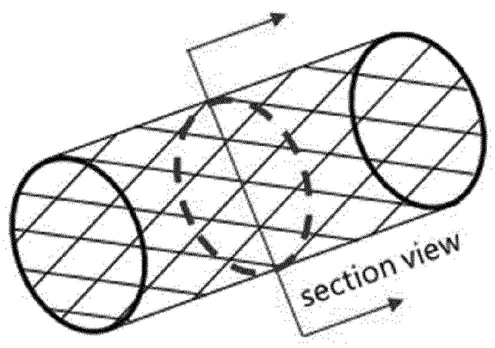

FIG. 30

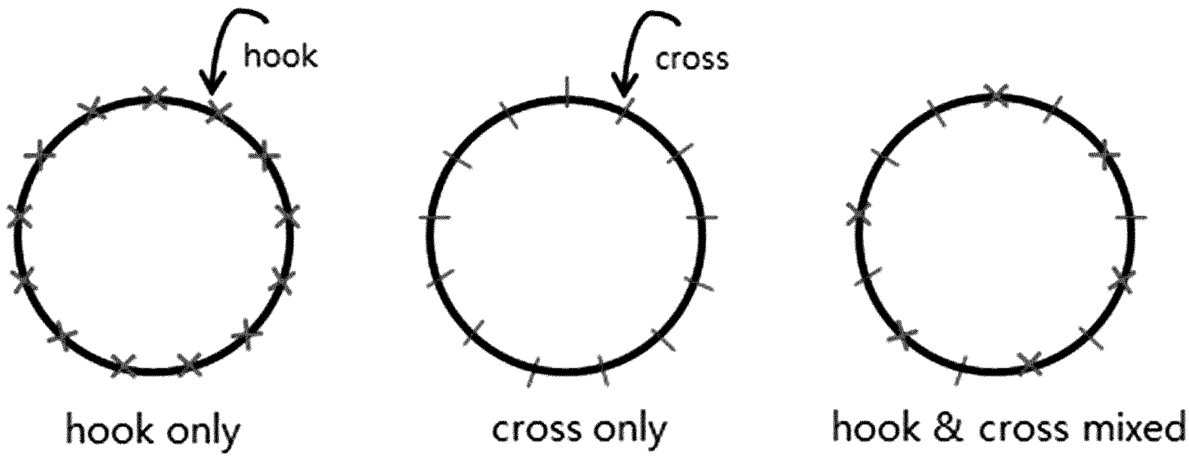


FIG. 31

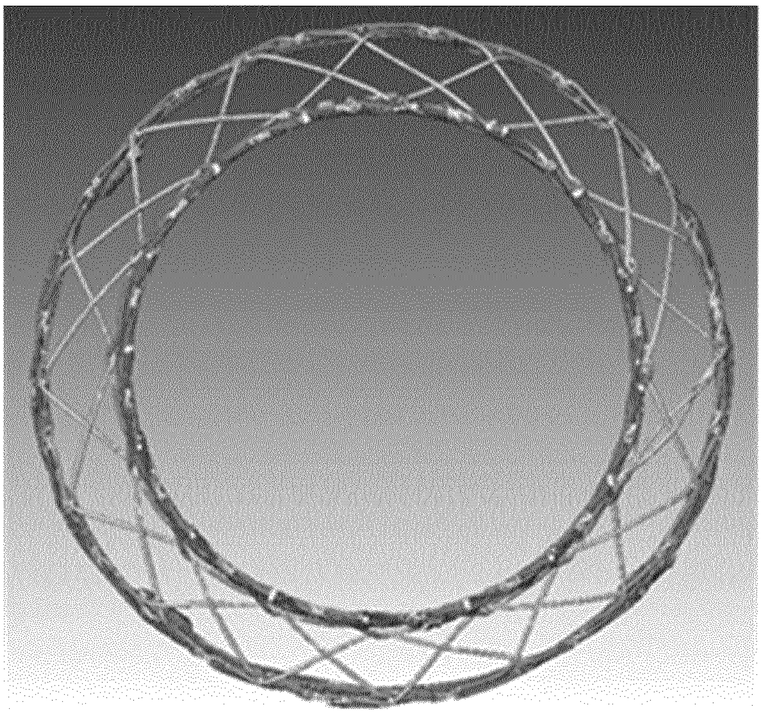

FIG. 32

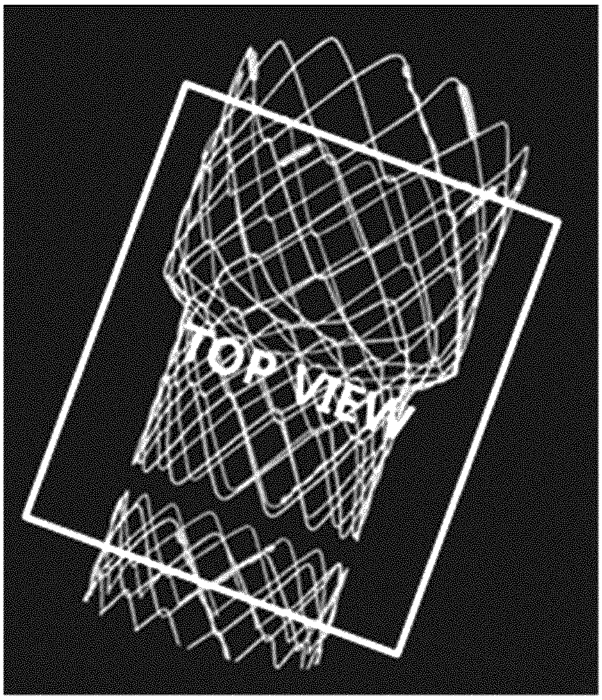


FIG. 33

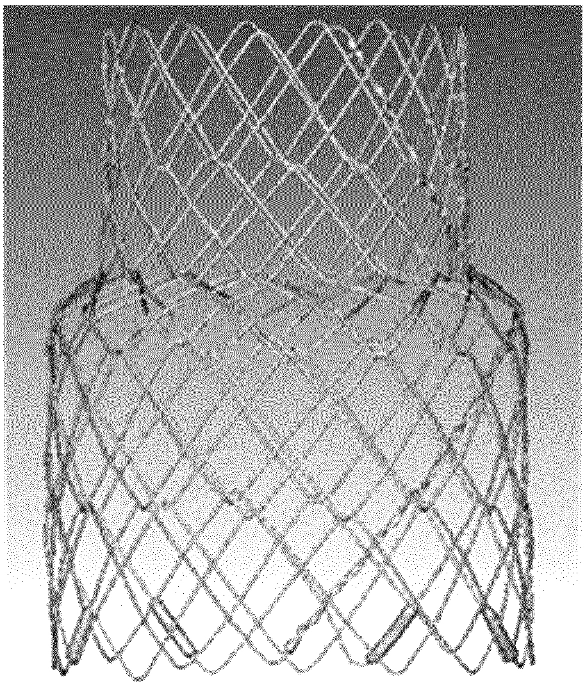

FIG. 34

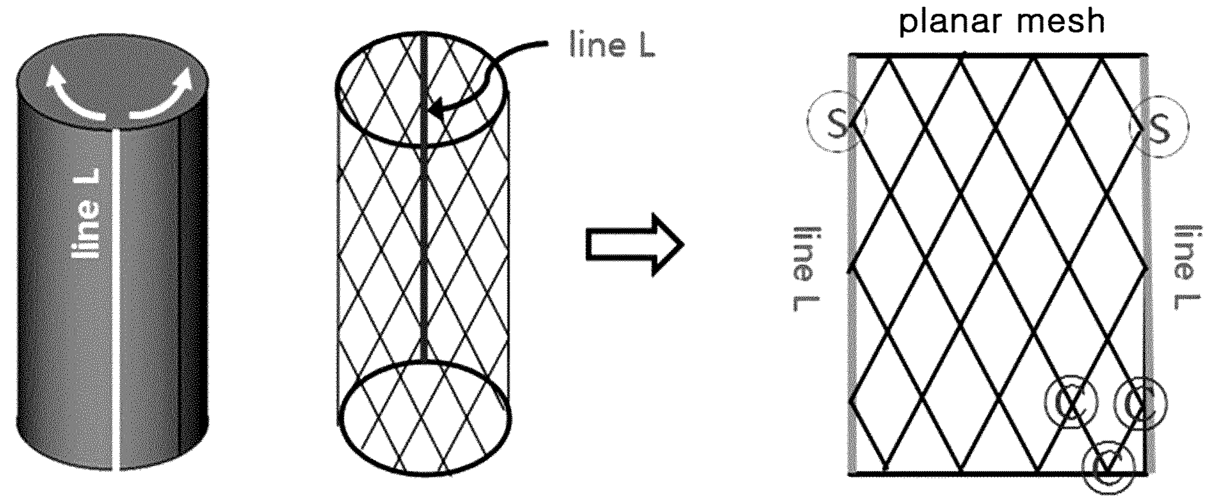
line L
line L
planar mesh
S
S
line L
line L
C
C
C

FIG. 35

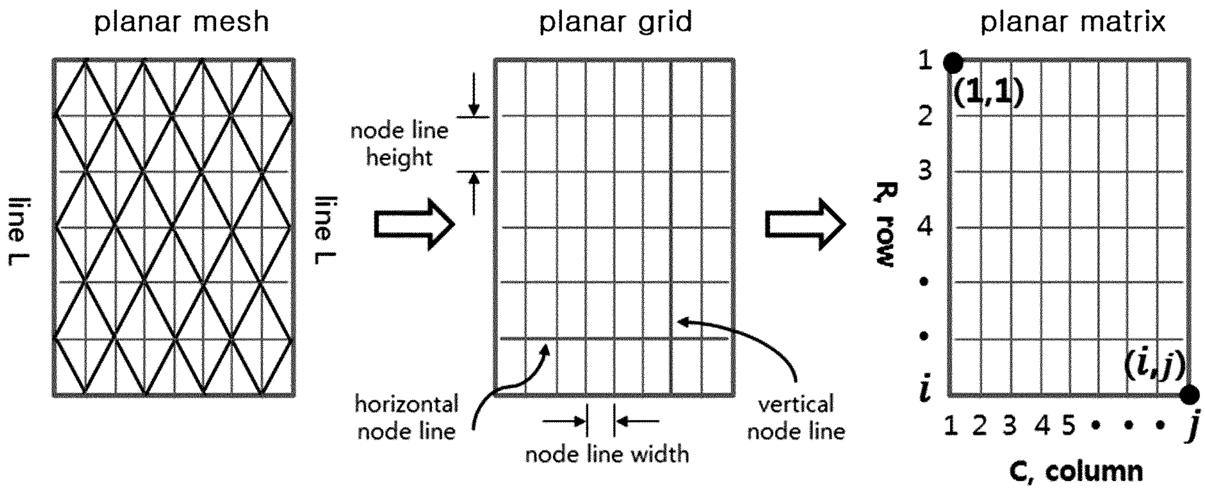
planar mesh
line L
line L
planar grid
node line height
horizontal node line
node line width
vertical node line
planar matrix
R, row
1
2
3
4
•
•
i
(1,1)
(i,j)
1 2 3 4 5 • • • j
C, column

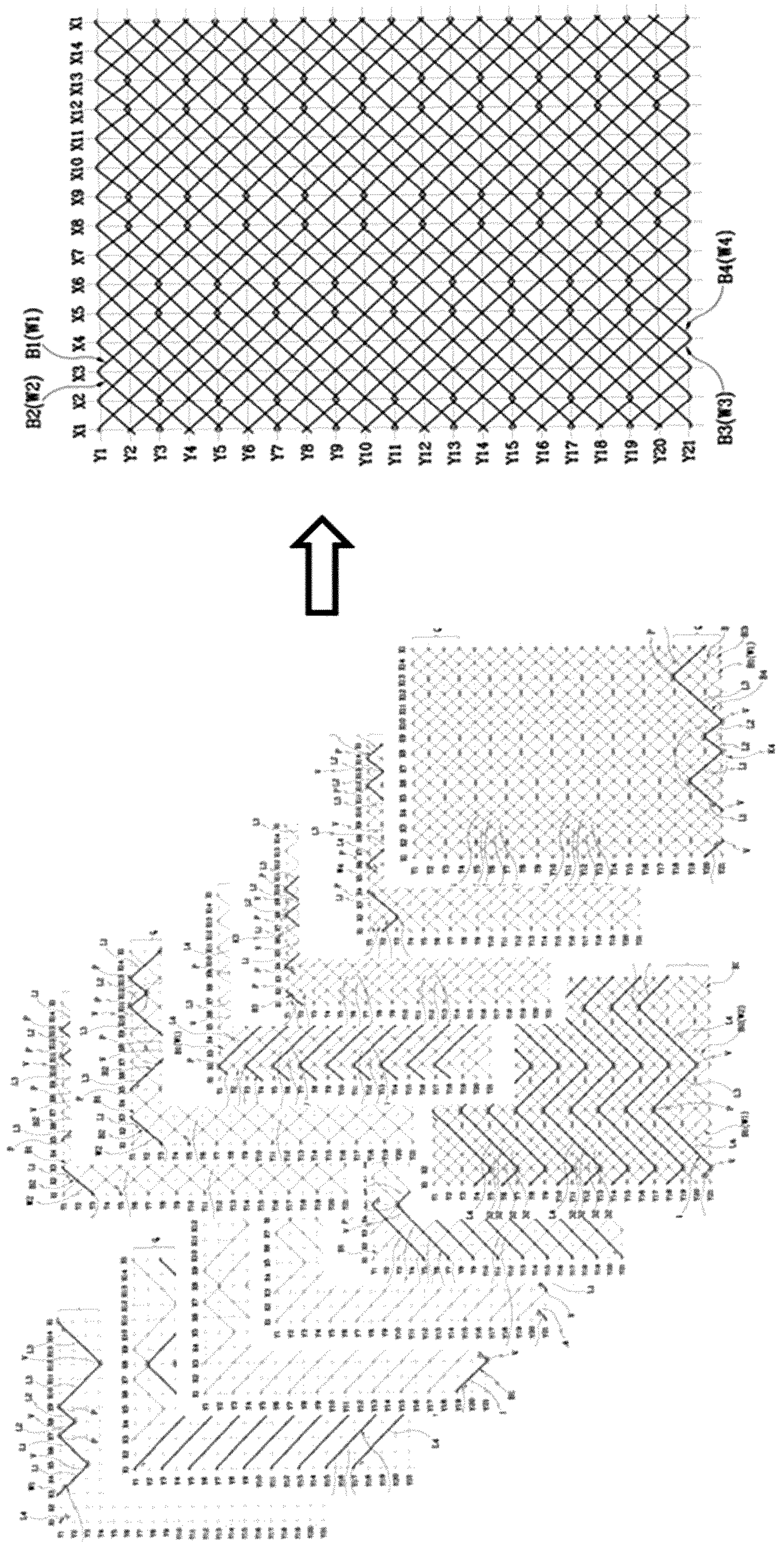
B1(W1)
B2(W2)
B3(W3)
B4(W4)
X1
X2
X3
X4
X5
X6
X7
X8
X9
X10
X11
X12
X13
X14
X1
Y1
Y2
Y3
Y4
Y5
Y6
Y7
Y8
Y9
Y10
Y11
Y12
Y13
Y14
Y15
Y16
Y17
Y18
Y19
Y20
Y21

FIG. 36

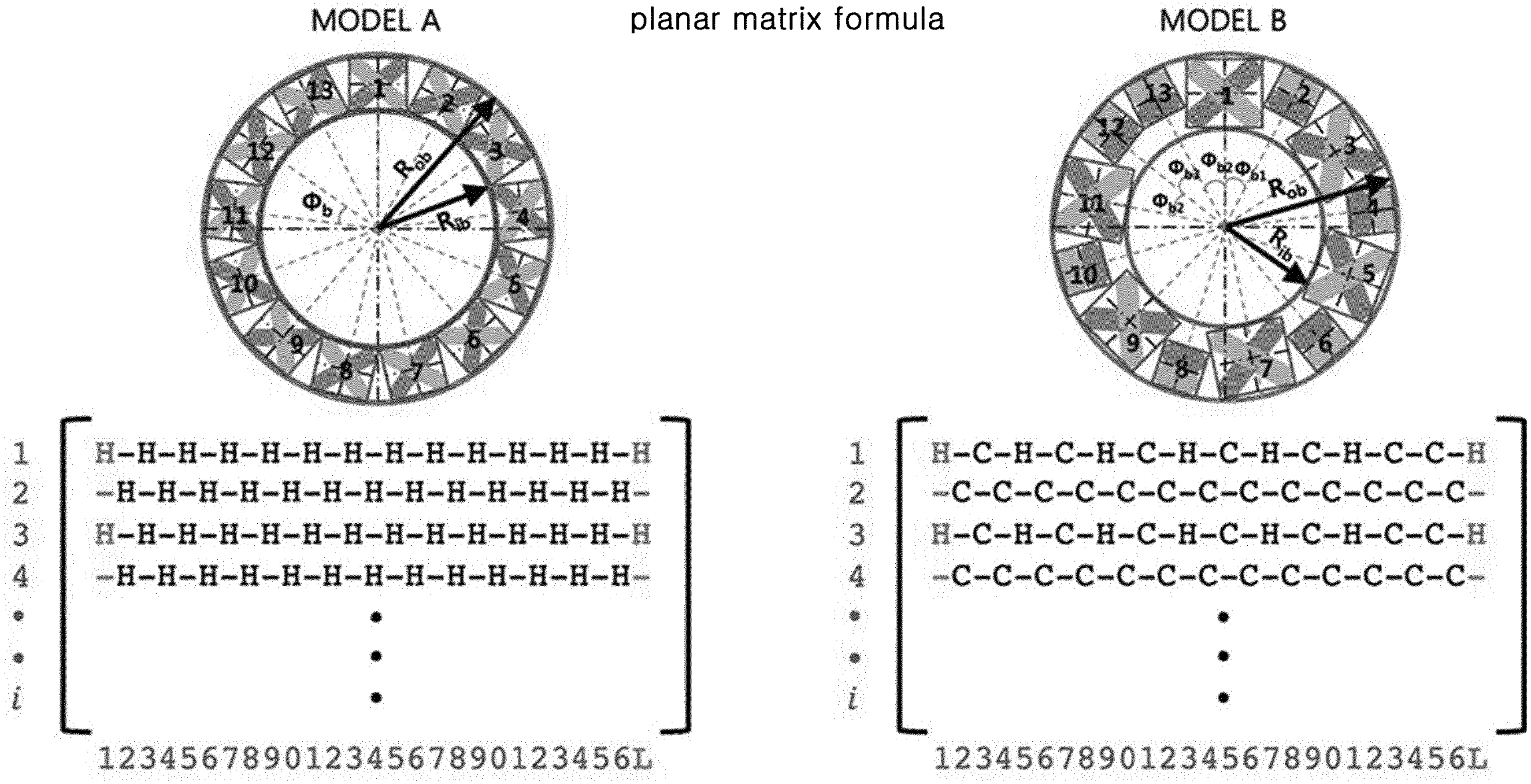
MODEL A
planar matrix formula
MODEL B
$R_{ob}$
$R_{ib}$
$\Phi_b$
$\Phi_{b1}$
$\Phi_{b2}$
$\Phi_{b3}$
1 H-H-H-H-H-H-H-H-H-H-H-H-H-H
2 -H-H-H-H-H-H-H-H-H-H-H-H-H-
3 H-H-H-H-H-H-H-H-H-H-H-H-H-H
4 -H-H-H-H-H-H-H-H-H-H-H-H-H-
i
12345678901234567890123456L
1 H-C-H-C-H-C-H-C-H-C-H-C-C-H
2 -C-C-C-C-C-C-C-C-C-C-C-C-C-
3 H-C-H-C-H-C-H-C-H-C-H-C-C-H
4 -C-C-C-C-C-C-C-C-C-C-C-C-C-
i
12345678901234567890123456L

FIG. 37

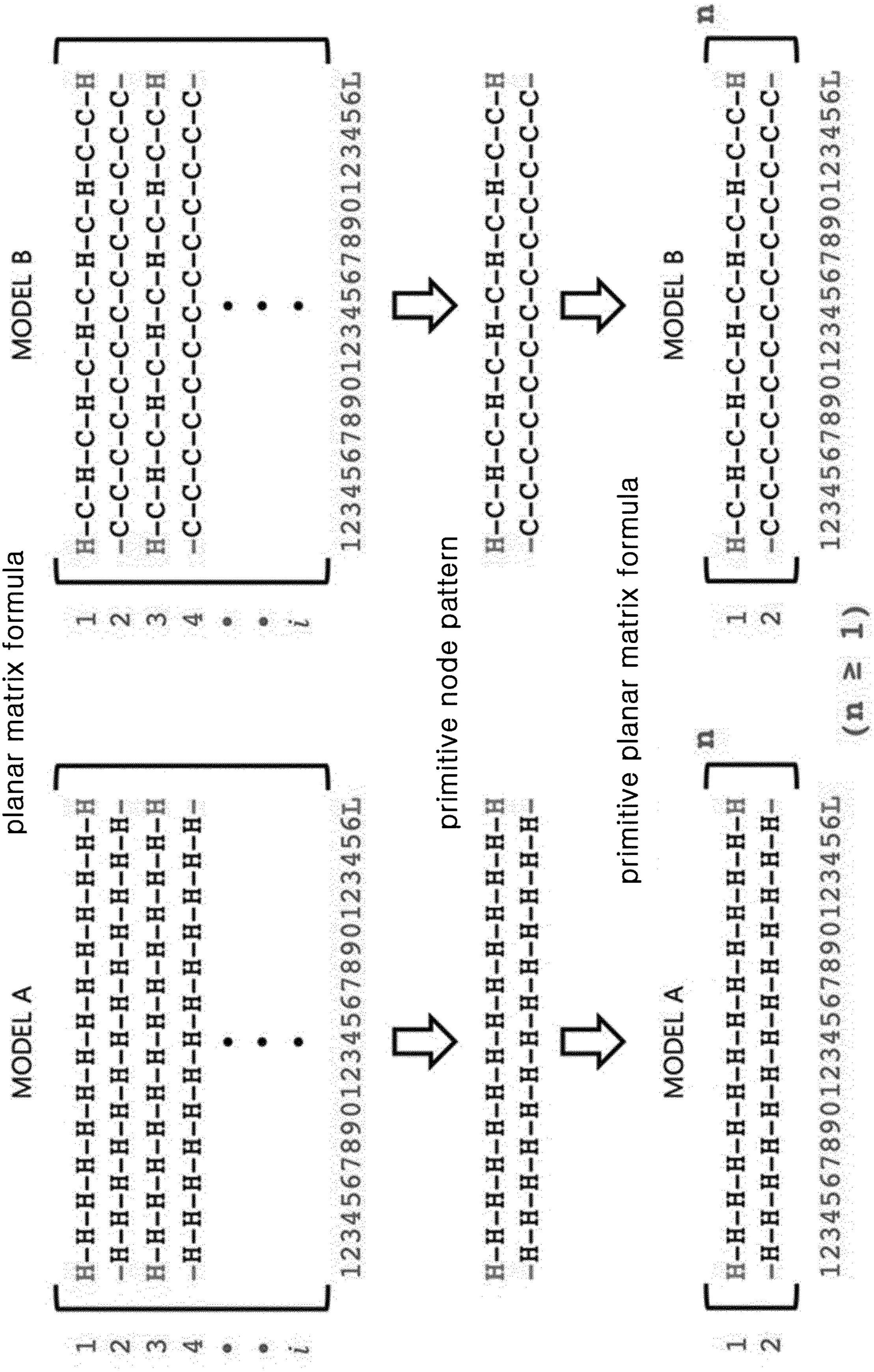
planar matrix formula
MODEL A
MODEL B
primitive node pattern
primitive planar matrix formula
MODEL A
MODEL B
(n ≥ 1)

FIG. 38

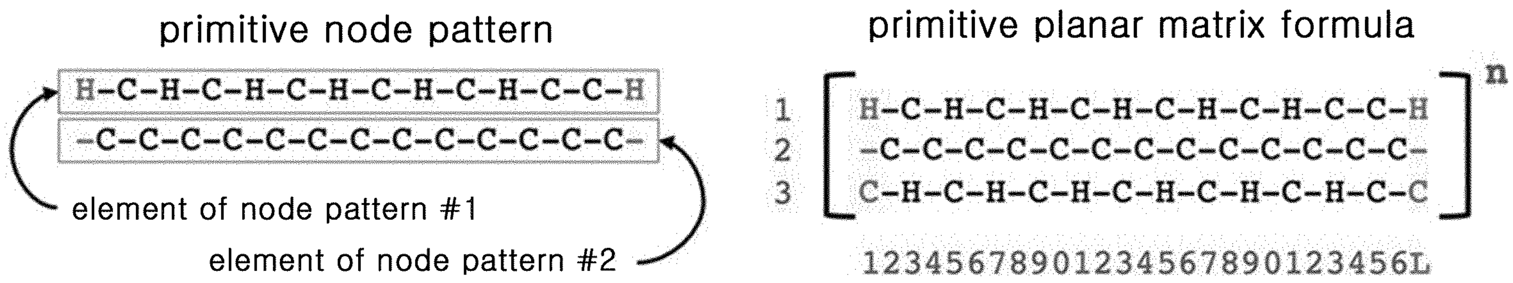
primitive node pattern
H-C-H-C-H-C-H-C-H-C-H-C-C-H
-C-C-C-C-C-C-C-C-C-C-C-C-C-
element of node pattern #1
element of node pattern #2
primitive planar matrix formula
1 H-C-H-C-H-C-H-C-H-C-H-C-C-H
2 -C-C-C-C-C-C-C-C-C-C-C-C-C-
3 C-H-C-H-C-H-C-H-C-H-C-H-C-C
n
12345678901234567890123456L

FIG. 39

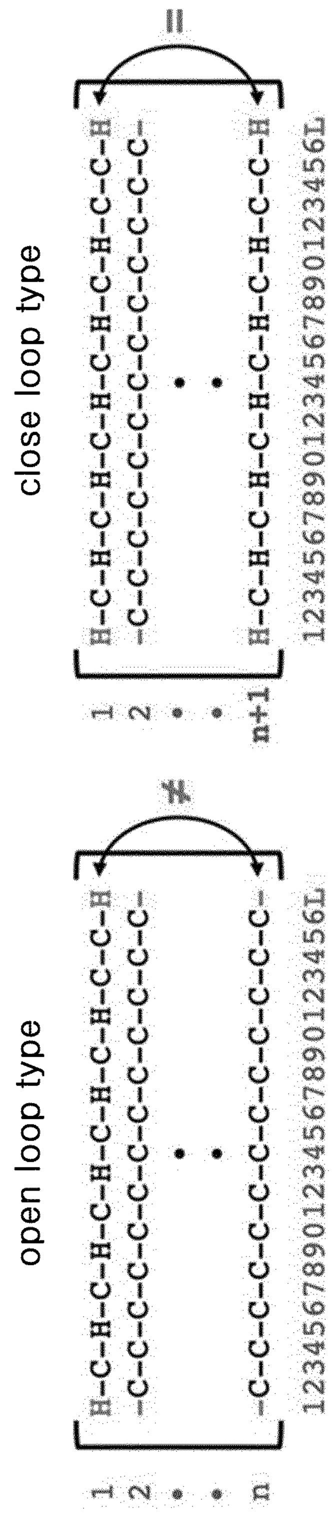


FIG. 40

FIG. 41

primitive planar matrix formula

```
   [                                        ]n
1  [ H-C-H-C-H-C-H-C-H-C-H-C-C-H            ]
2  [ -C-C-C-C-C-C-C-C-C-C-C-C-C-            ]
   [                                        ]c
     123456789012345678901234567890123456L
```

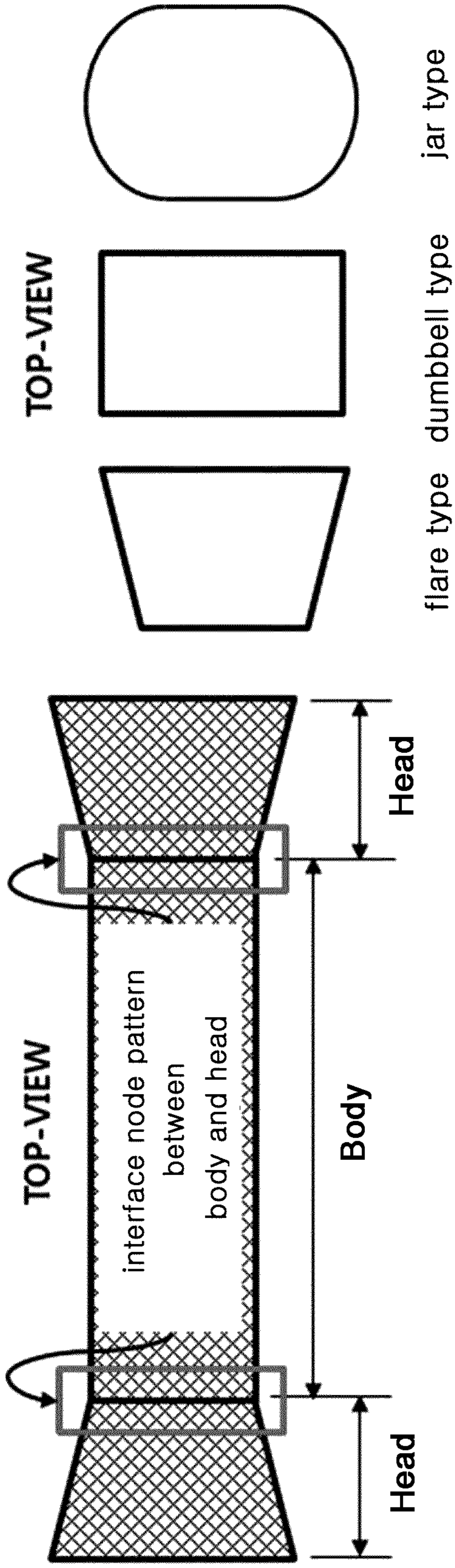
TOP-VIEW
jar type
dumbbell type
flare type
TOP-VIEW
interface node pattern between body and head
Head
Body
Head

FIG. 42

FIG. 43

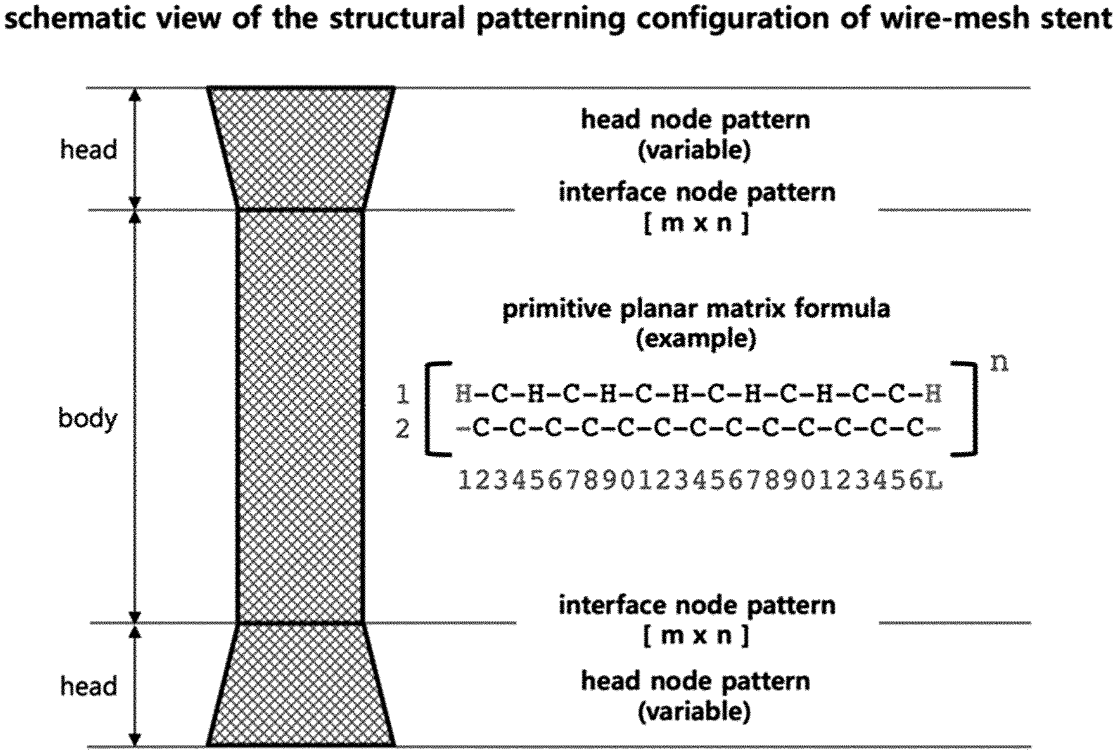
schematic view of the structural patterning configuration of wire-mesh stent
head
body
head
head node pattern
(variable)
interface node pattern
[ m x n ]
primitive planar matrix formula
(example)
1 H-C-H-C-H-C-H-C-H-C-H-C-C-H
2 -C-C-C-C-C-C-C-C-C-C-C-C-C-
123456789012345678901234567890123456L
n
interface node pattern
[ m x n ]
head node pattern
(variable)

FIG. 44

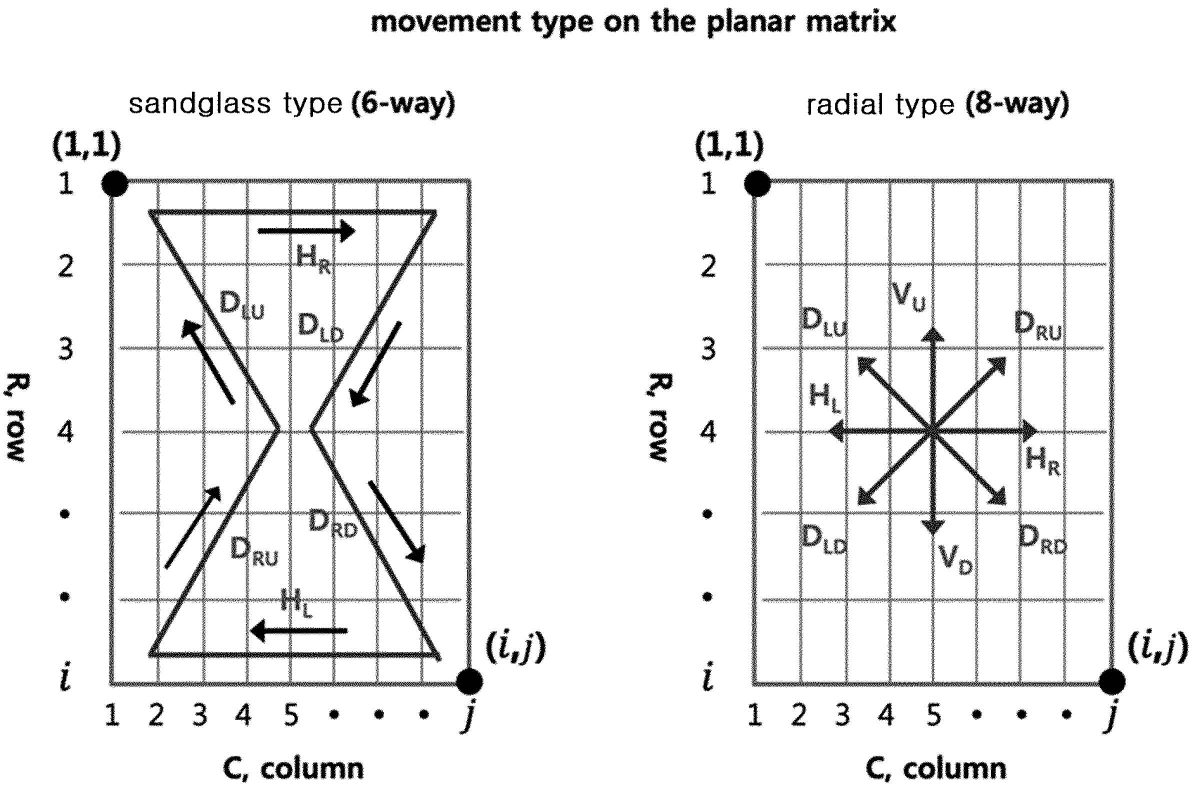
movement type on the planar matrix
sandglass type (6-way)
radial type (8-way)
(1,1)
(i,j)
R, row
C, column
1 2 3 4 5 • • • j
1 2 3 4 • • i
$H_R$
$H_L$
$D_{LU}$
$D_{LD}$
$D_{RU}$
$D_{RD}$
$V_U$
$V_D$

FIG. 45

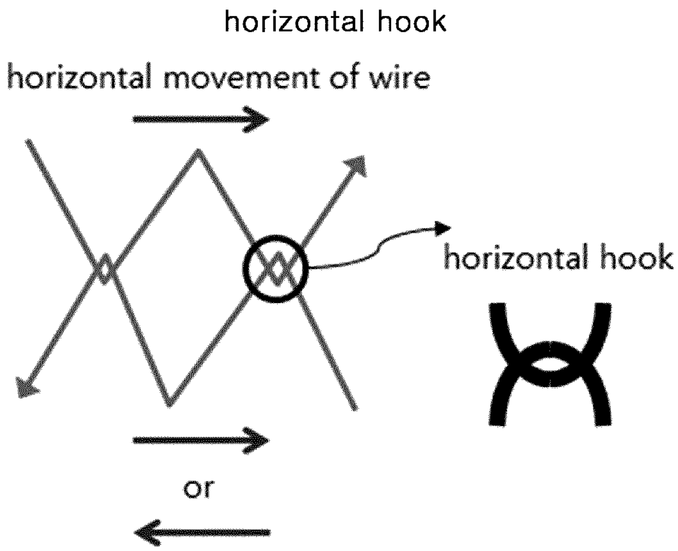
horizontal hook
horizontal movement of wire
horizontal hook
or

FIG. 46

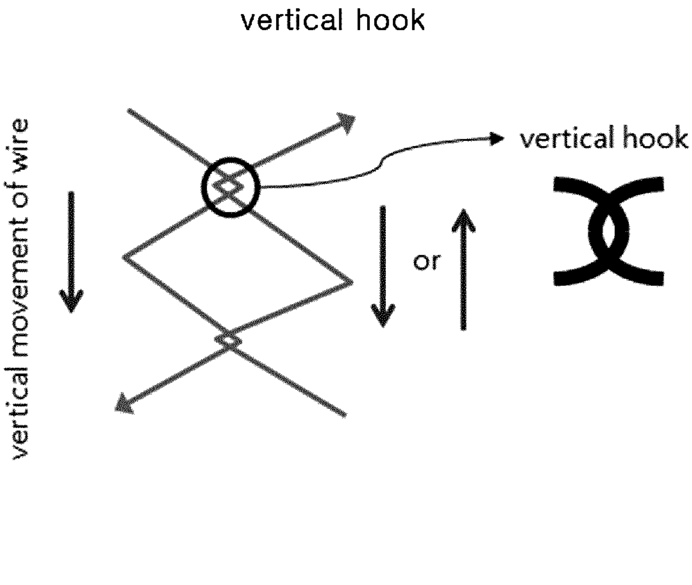
vertical hook
vertical movement of wire
vertical hook
or

FIG. 47

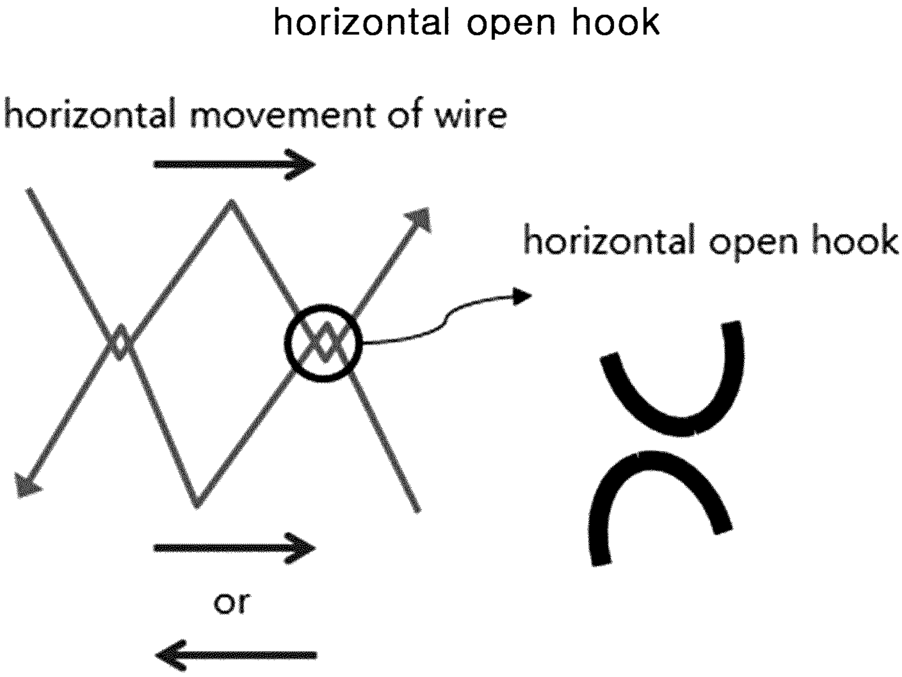
horizontal open hook
horizontal movement of wire
horizontal open hook
or

FIG. 48

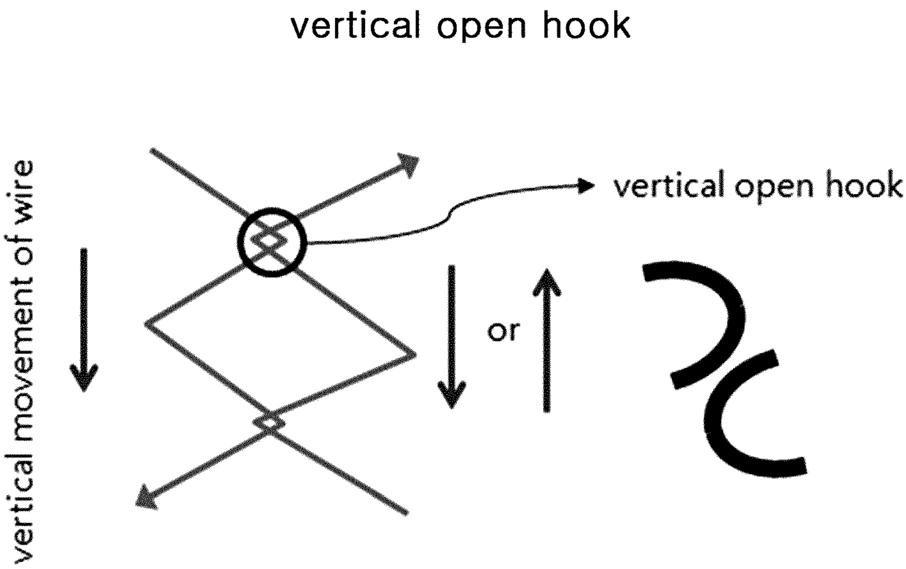
vertical open hook
vertical movement of wire
vertical open hook
or

FIG. 49

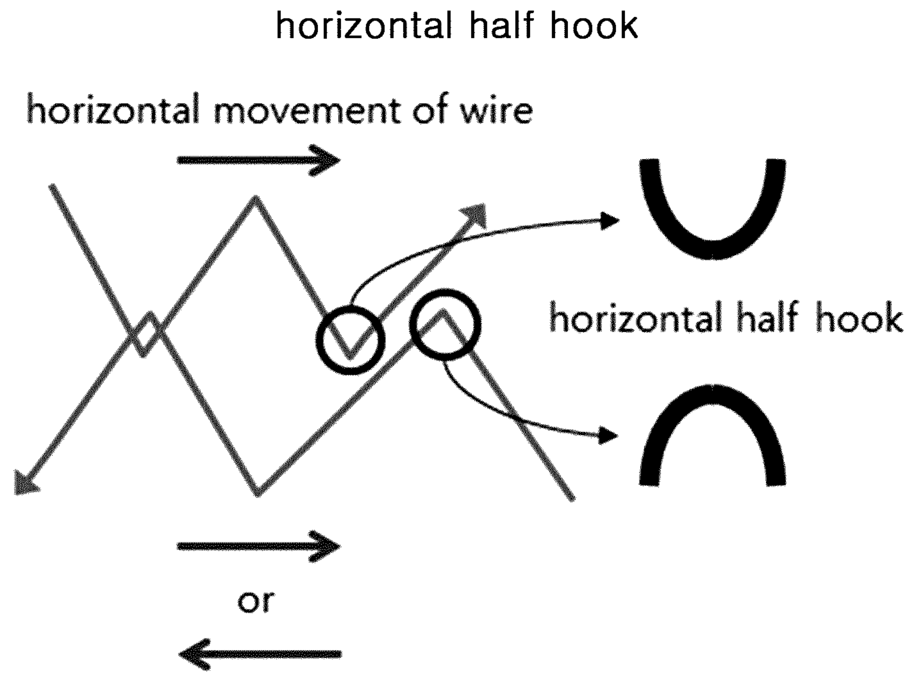
horizontal half hook
horizontal movement of wire
horizontal half hook
or

FIG. 50

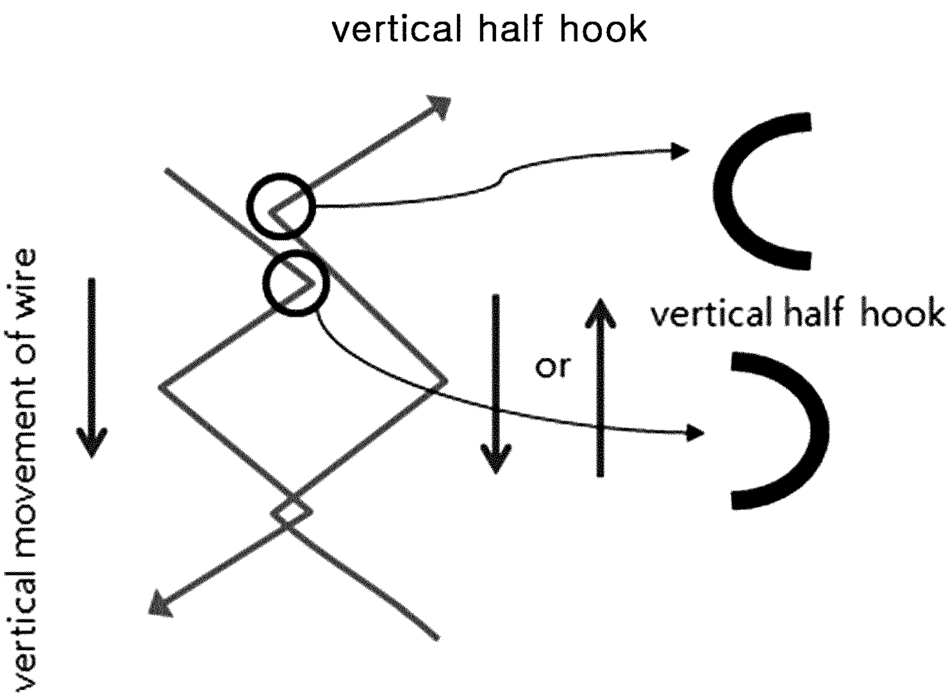
vertical half hook
vertical movement of wire
or
vertical half hook

FIG. 51

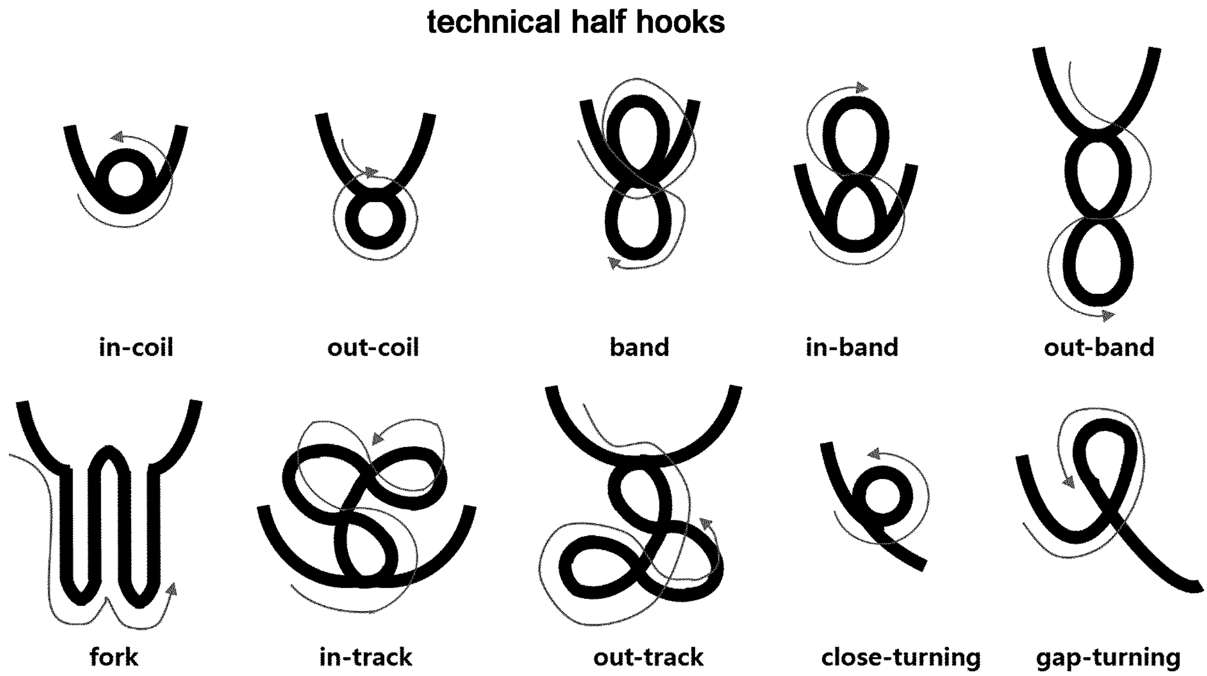


FIG. 52

fabrication expression

**S1(1, 1)**
( , ) ( , ) ( , ) ( , ) ......
**E1(1, 1)**

**S2(1, 2)**
( , ) ( , ) ( , ) ( , ) ......
**E2(1, 2)**

**S3(1, 3)**
( , ) ( , ) ( , ) ( , ) ......
**E3(1, 3)**

FIG. 53

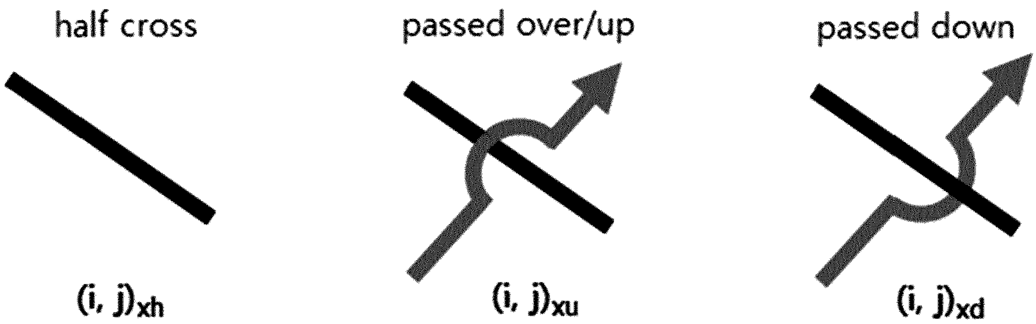

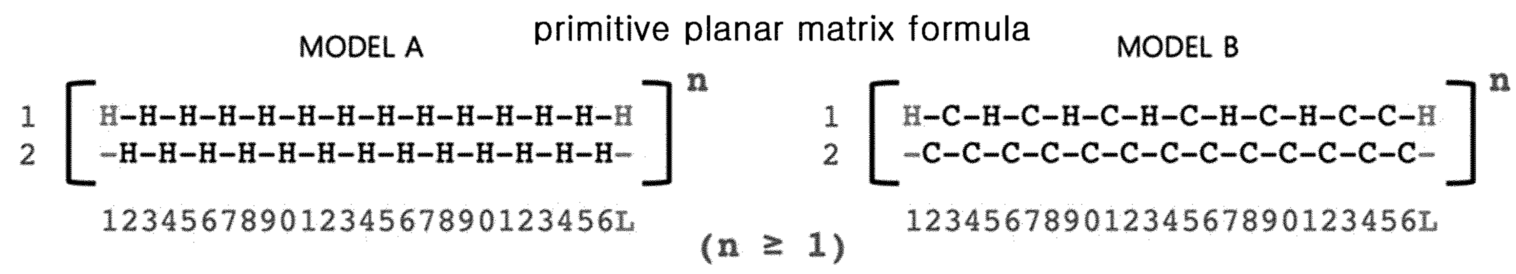
primitive planar matrix formula
MODEL A
1 H-H-H-H-H-H-H-H-H-H-H-H-H-H
2 -H-H-H-H-H-H-H-H-H-H-H-H-H-
n
12345678901234567890123456L
(n ≥ 1)
MODEL B
1 H-C-H-C-H-C-H-C-H-C-H-C-C-H
2 -C-C-C-C-C-C-C-C-C-C-C-C-C-
n
12345678901234567890123456L

FIG. 54

FIG. 55

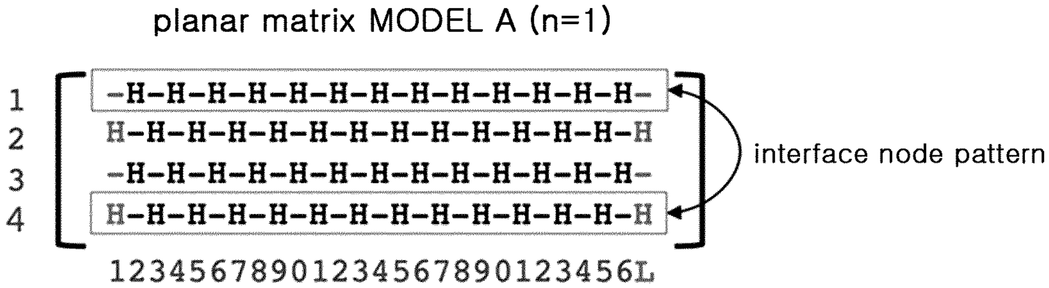


FIG. 56

fabrication expression of MODEL A(n=1)

**S1(1,2)**

(i, j+1) i = 2->1->2, j = 2 to 25

$(2, L)_V$

(i, j+1) i = 3->2->3, j = 25 to 2

$(3, 2)_V$

(i, j+1) i = 4->3->4, j = 2 to 25

(4, L)

$(3, 2)_V$

$(2, 1)_V$

**E1(1,2)**

FIG. 57

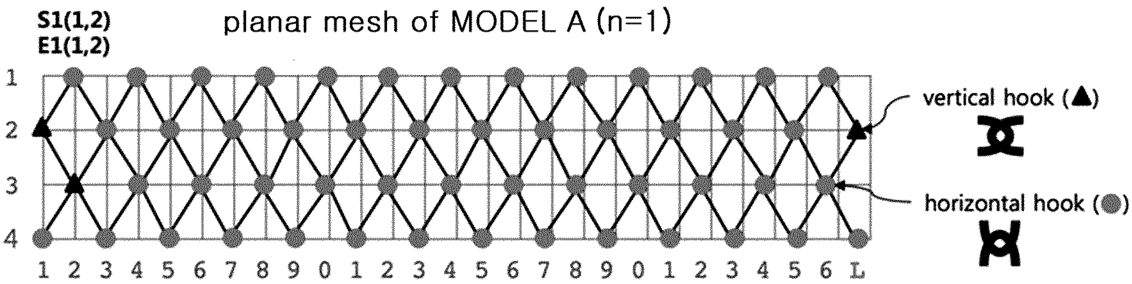

FIG. 58

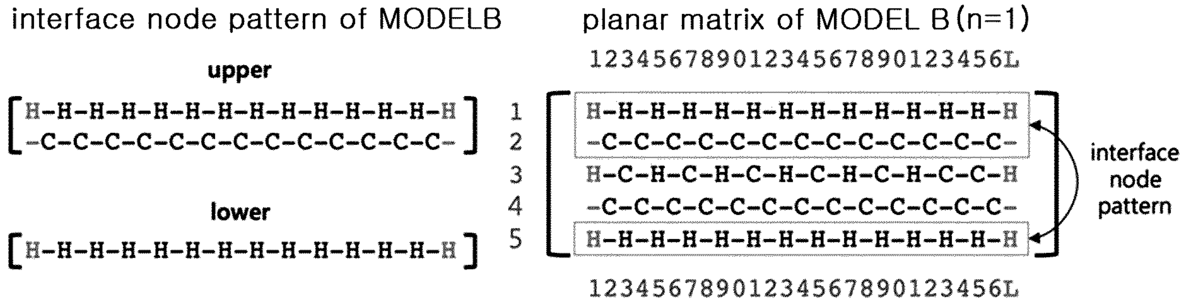


FIG. 59

fabrication expression of MODEL B (n=1)

**S1(1,1)**

(i, 4j+1) i=5->1->5, j=1 to 6

(3,L)

(i, 2j+1) i=1->3->1, j=1 to 11

(5.L)

(i, 4j+1) i=1->5->1, j=1 to 6

(3,L)

(i, 2j+1) i=5->3->5, j=1 to 11

**E1(1,1)**

FIG. 60

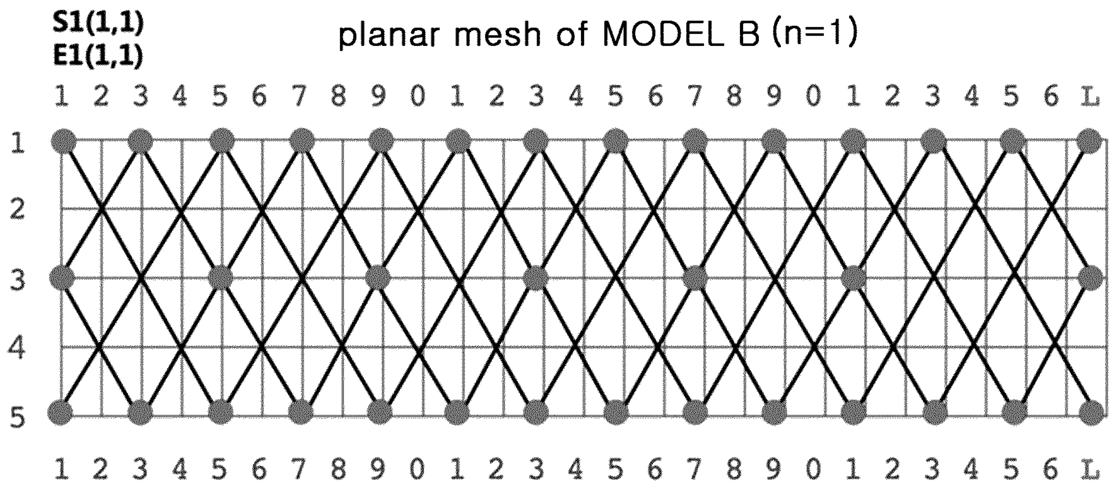

FIG. 61

primitive planar matrix formula
of MODEL B (n=2)

```
1 [ H-C-H-C-H-C-H-C-H-C-H-C-C-H ] 2
2 [ -C-C-C-C-C-C-C-C-C-C-C-C-C- ]
    123456789012345678901234 56L
```

interface node pattern of MODEL B

**upper**

```
[ H-H-H-H-H-H-H-H-H-H-H-H-H-H ]
[ -C-C-C-C-C-C-C-C-C-C-C-C-C- ]
```

**lower**

```
[ H-H-H-H-H-H-H-H-H-H-H-H-H-H ]
```

planar mesh of MODEL B (n=2)

```
    123456789012345678901234 56L
1 [ H-H-H-H-H-H-H-H-H-H-H-H-H-H ]
2 [ -C-C-C-C-C-C-C-C-C-C-C-C-C- ]
3 [ H-C-H-C-H-C-H-C-H-C-H-C-C-H ]
4 [ -C-C-C-C-C-C-C-C-C-C-C-C-C- ]
5 [ H-C-H-C-H-C-H-C-H-C-H-C-C-H ]
6 [ -C-C-C-C-C-C-C-C-C-C-C-C-C- ]
7 [ H-H-H-H-H-H-H-H-H-H-H-H-H-H ]
    123456789012345678901234 56L
```

FIG. 62

fabrication expression of MODEL B (n=2)

**S1(1,1)**

(i, 4j+1) $_{i=5->1->5,\ j=1\ to\ 6}$

(3,L)

(i, 2j+1) $_{i=1->3->1,\ j=1\ to\ 11}$

(5.L)

(i, 4j+1) $_{i=1->5->1,\ j=1\ to\ 6}$

(7,L)

(i, 4j-3) $_{i=7->3->7,\ j=1\ to\ 7}$

(5,L)

(i, 2j+1) $_{i=7->5->7,\ j=1\ to\ 11}$

(3,L)

(i, 4j+1) $_{i=7->3->7,\ j=1\ to\ 5}$

**E1(1,L)**

**Note: (1,1) = (1,L)**

FIG. 63

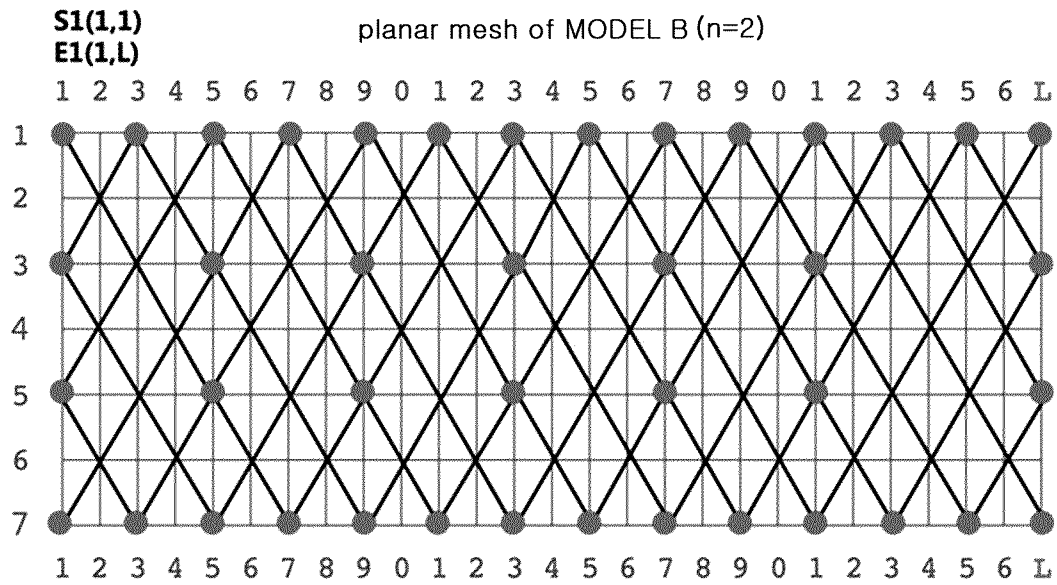
S1(1,1)
E1(1,L)
planar mesh of MODEL B (n=2)
1 2 3 4 5 6 7 8 9 0 1 2 3 4 5 6 7 8 9 0 1 2 3 4 5 6 L
1
2
3
4
5
6
7
1 2 3 4 5 6 7 8 9 0 1 2 3 4 5 6 7 8 9 0 1 2 3 4 5 6 L

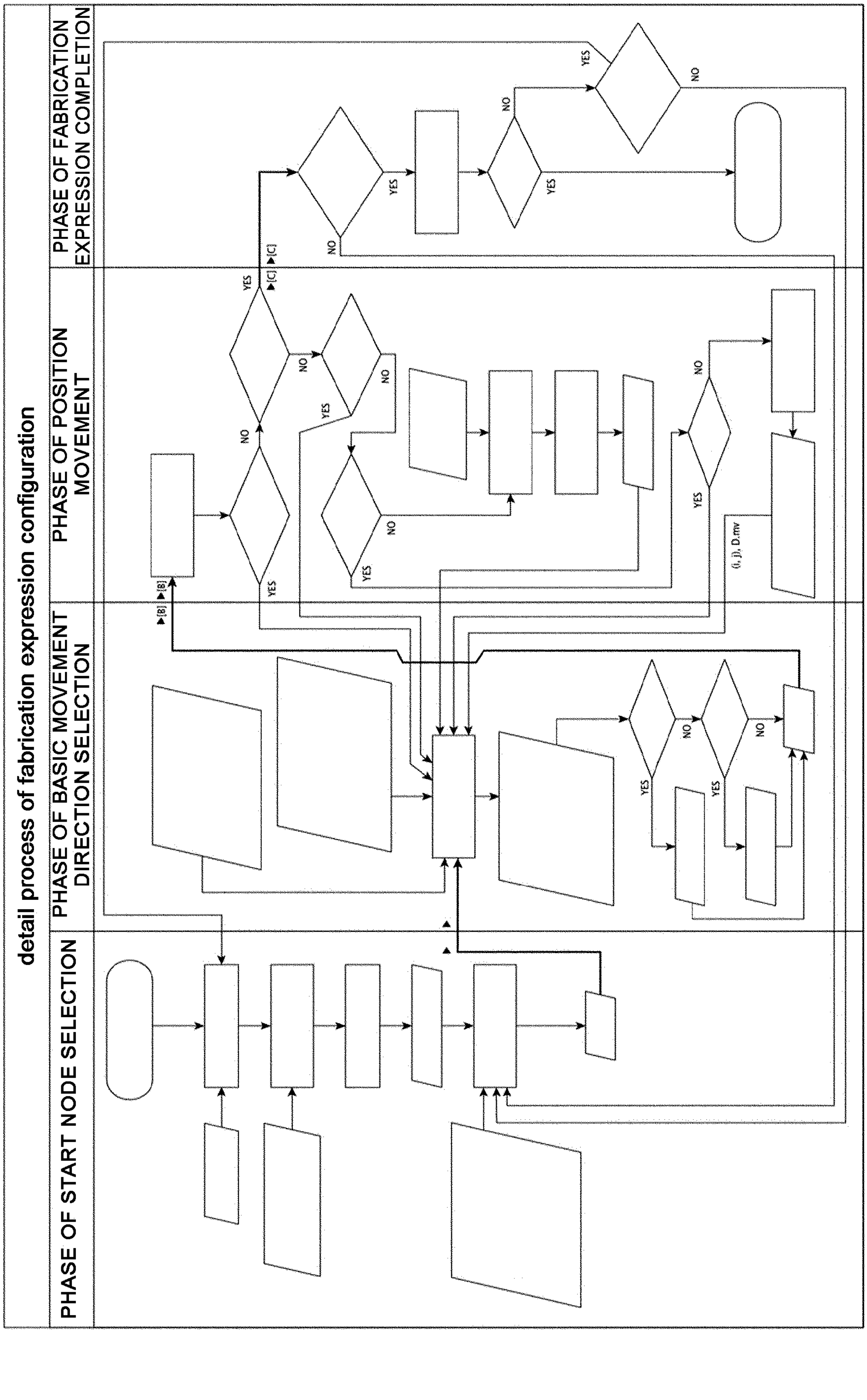
detail process of fabrication expression configuration
PHASE OF START NODE SELECTION
PHASE OF BASIC MOVEMENT DIRECTION SELECTION
PHASE OF POSITION MOVEMENT
PHASE OF FABRICATION EXPRESSION COMPLETION
[B]
[C]
(i, j), D.mv
YES
NO

FIG. 64A

FIG. 64B

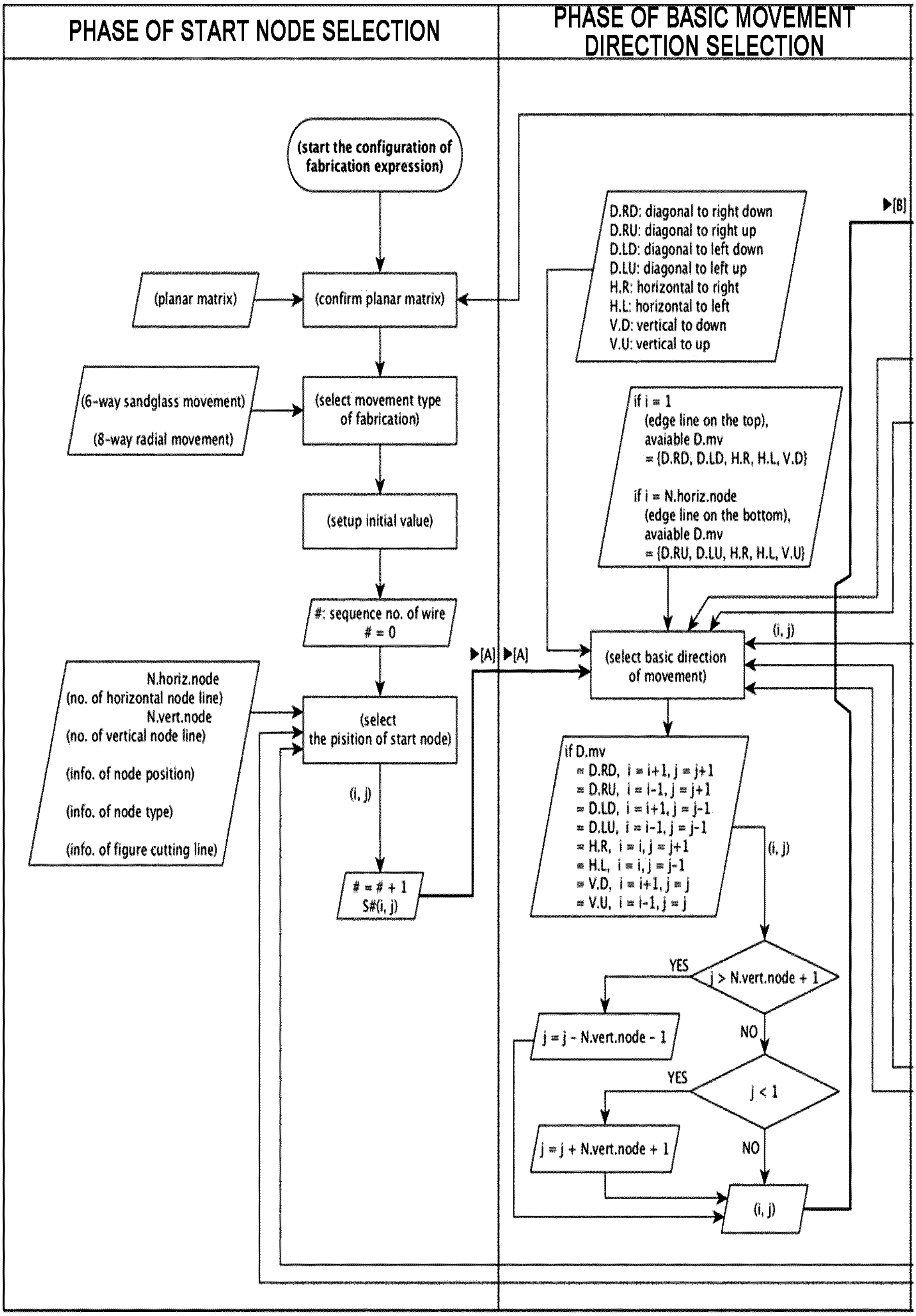

PHASE OF START NODE SELECTION
PHASE OF BASIC MOVEMENT DIRECTION SELECTION
(start the configuration of fabrication expression)
(planar matrix)
(confirm planar matrix)
(6-way sandglass movement)
(8-way radial movement)
(select movement type of fabrication)
(setup initial value)
#: sequence no. of wire
# = 0
N.horiz.node
(no. of horizontal node line)
N.vert.node
(no. of vertical node line)
(info. of node position)
(info. of node type)
(info. of figure cutting line)
(select the pisition of start node)
(i, j)
# = # + 1
S#(i, j)
▶[A] ▶[A]
D.RD: diagonal to right down
D.RU: diagonal to right up
D.LD: diagonal to left down
D.LU: diagonal to left up
H.R: horizontal to right
H.L: horizontal to left
V.D: vertical to down
V.U: vertical to up
▶[B]
if i = 1
(edge line on the top),
avaiable D.mv
= {D.RD, D.LD, H.R, H.L, V.D}
if i = N.horiz.node
(edge line on the bottom),
avaiable D.mv
= {D.RU, D.LU, H.R, H.L, V.U}
(i, j)
(select basic direction of movement)
if D.mv
= D.RD, i = i+1, j = j+1
= D.RU, i = i-1, j = j+1
= D.LD, i = i+1, j = j-1
= D.LU, i = i-1, j = j-1
= H.R, i = i, j = j+1
= H.L, i = i, j = j-1
= V.D, i = i+1, j = j
= V.U, i = i-1, j = j
(i, j)
YES
j > N.vert.node + 1
j = j - N.vert.node - 1
NO
YES
j < 1
j = j + N.vert.node + 1
NO
(i, j)

FIG. 64C

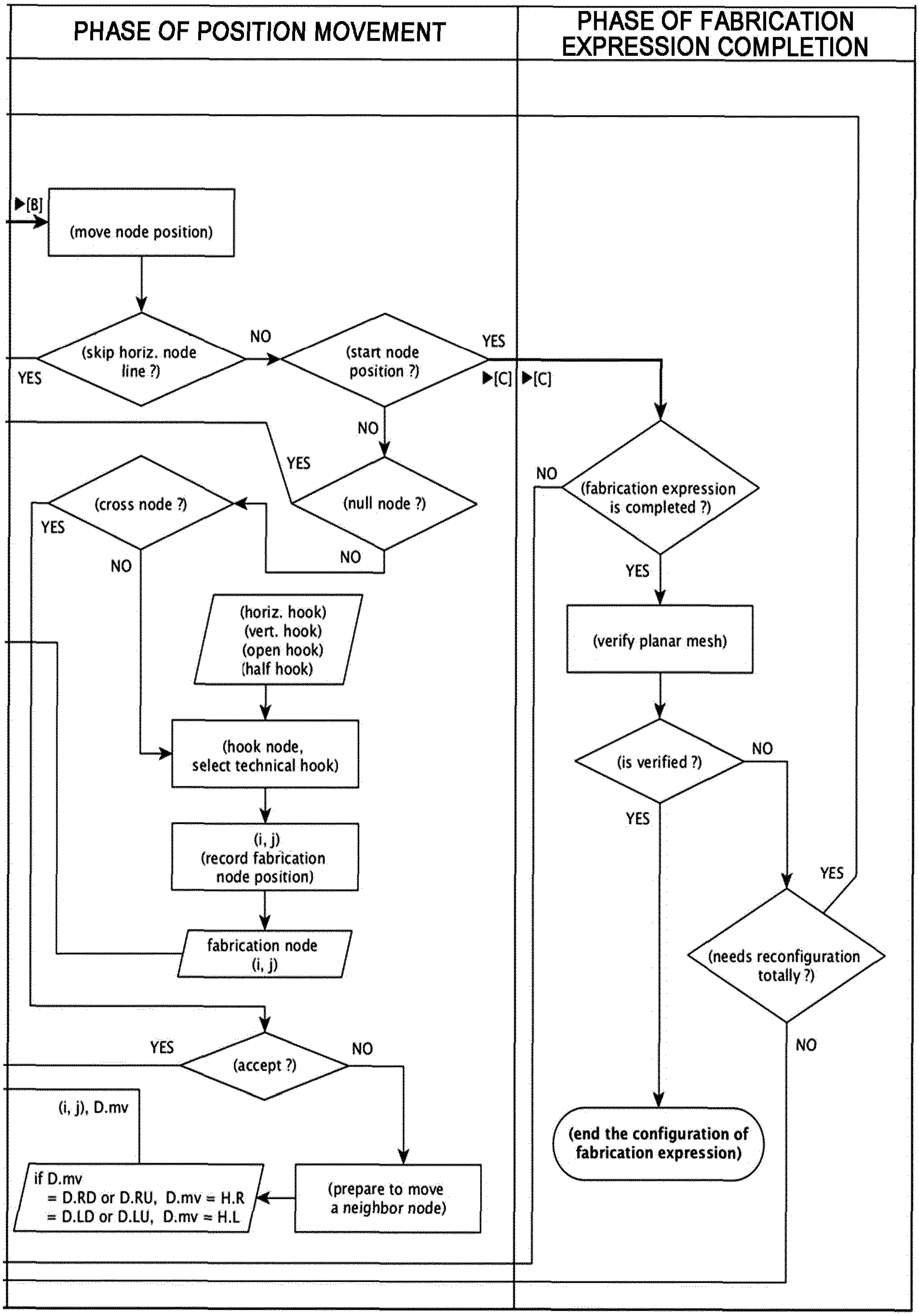
PHASE OF POSITION MOVEMENT
PHASE OF FABRICATION EXPRESSION COMPLETION
[B]
(move node position)
(skip horiz. node line ?)
YES
NO
(start node position ?)
YES
[C]
[C]
NO
(null node ?)
YES
(cross node ?)
YES
NO
NO
(horiz. hook)
(vert. hook)
(open hook)
(half hook)
(hook node, select technical hook)
(i, j)
(record fabrication node position)
fabrication node
(i, j)
(accept ?)
YES
NO
(i, j), D.mv
if D.mv
= D.RD or D.RU, D.mv = H.R
= D.LD or D.LU, D.mv = H.L
(prepare to move a neighbor node)
NO
(fabrication expression is completed ?)
YES
(verify planar mesh)
(is verified ?)
NO
YES
YES
(needs reconfiguration totally ?)
NO
(end the configuration of fabrication expression)

FIG. 65

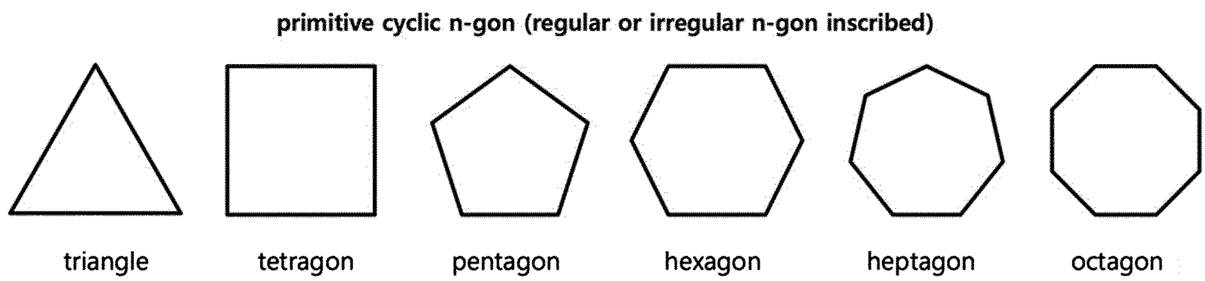
primitive cyclic n-gon (regular or irregular n-gon inscribed)
triangle
tetragon
pentagon
hexagon
heptagon
octagon

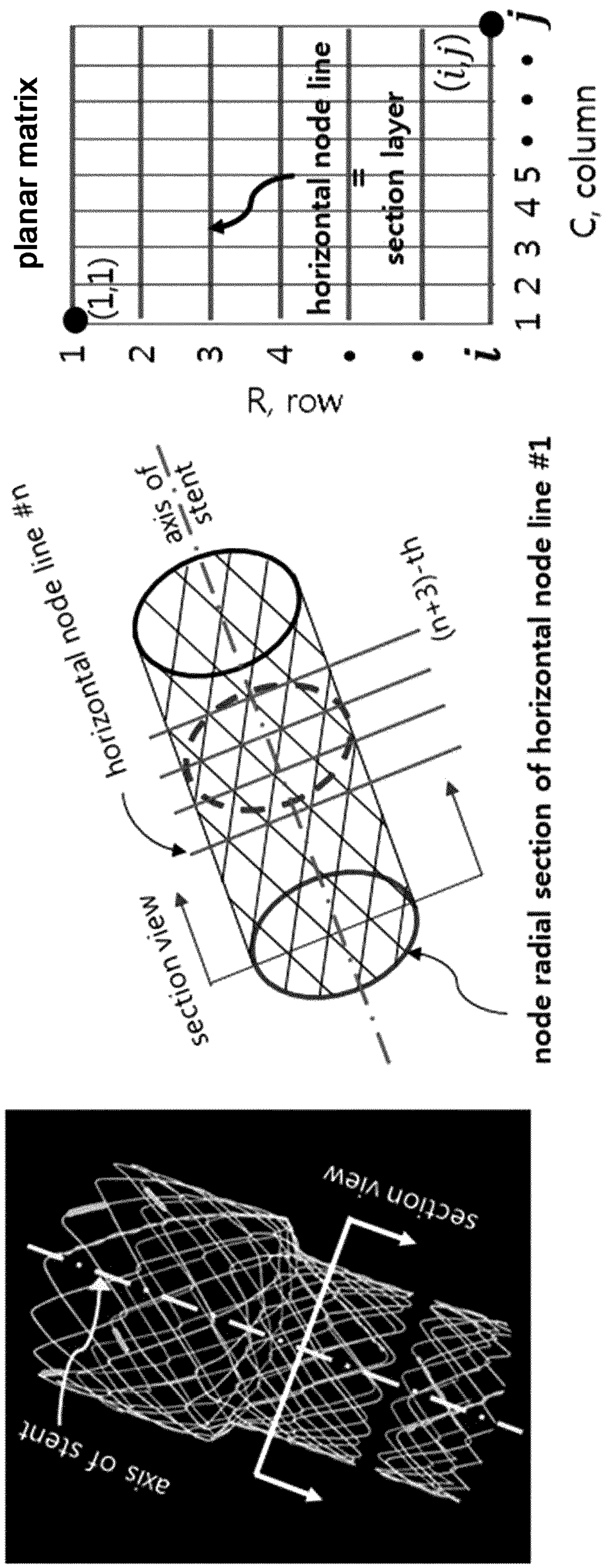
planar matrix
(1,1)
horizontal node line
=
section layer
(i,j)
1 2 3 4 5 • • • j
C, column
1 2 3 4 • • i
R, row
horizontal node line #n
axis of stent
(n+3)-th
section view
node radial section of horizontal node line #1
axis of stent
section view

FIG. 66

FIG. 67

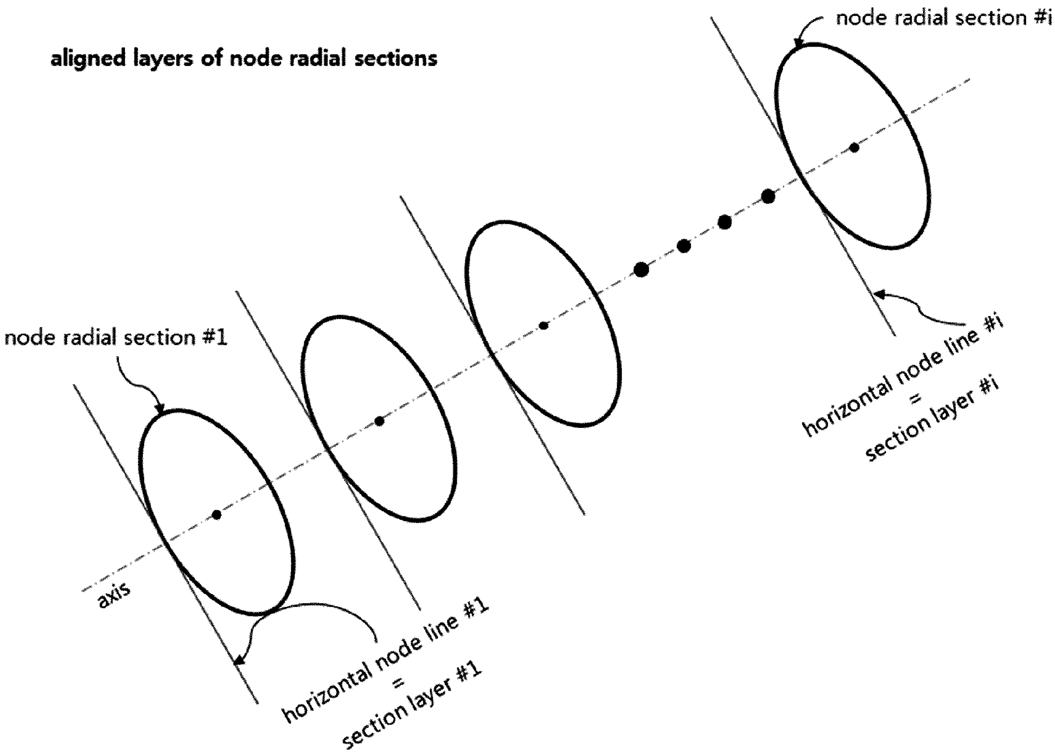
aligned layers of node radial sections
node radial section #i
node radial section #1
axis
horizontal node line #1
=
section layer #1
horizontal node line #i
=
section layer #i

FIG. 68

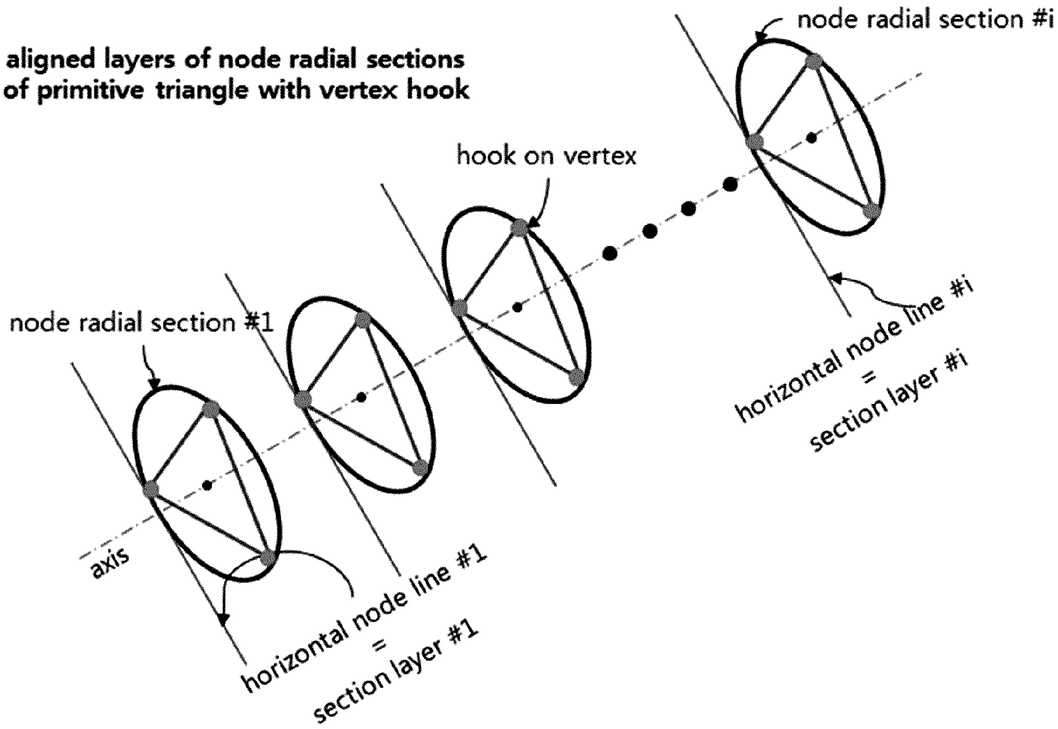
aligned layers of node radial sections
of primitive triangle with vertex hook
node radial section #i
hook on vertex
node radial section #1
axis
horizontal node line #1
=
section layer #1
horizontal node line #i
=
section layer #i

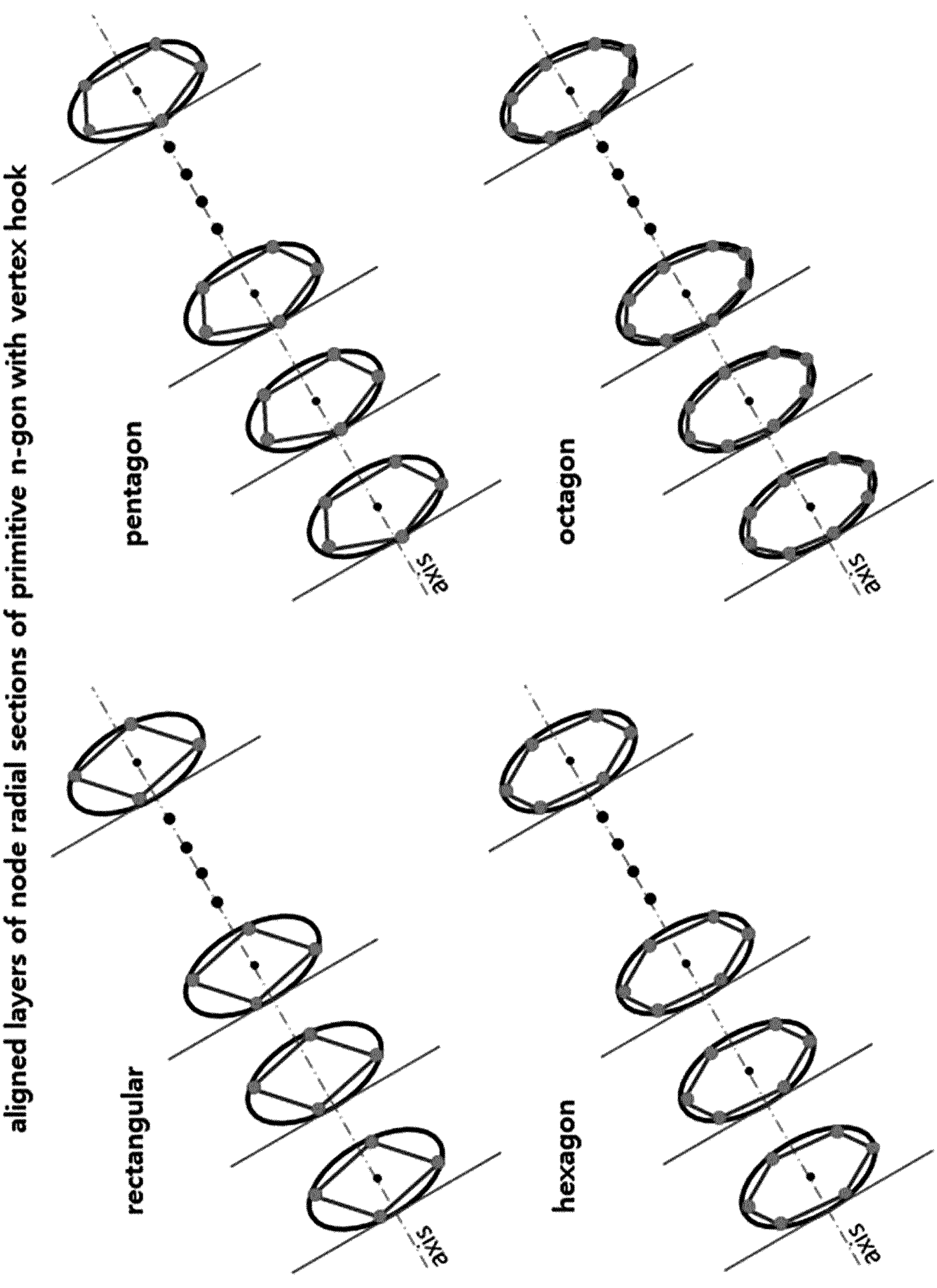
aligned layers of node radial sections of primitive n-gon with vertex hook
rectangular
pentagon
hexagon
octagon
axis
axis
axis
axis

FIG. 69

FIG. 70

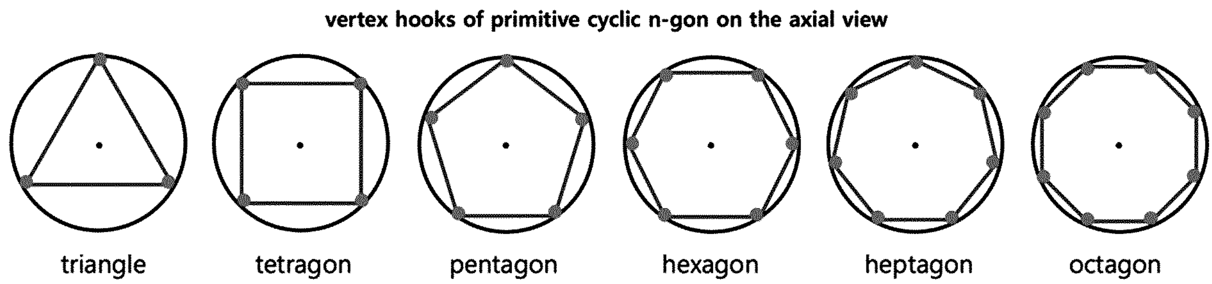
vertex hooks of primitive cyclic n-gon on the axial view
triangle
tetragon
pentagon
hexagon
heptagon
octagon

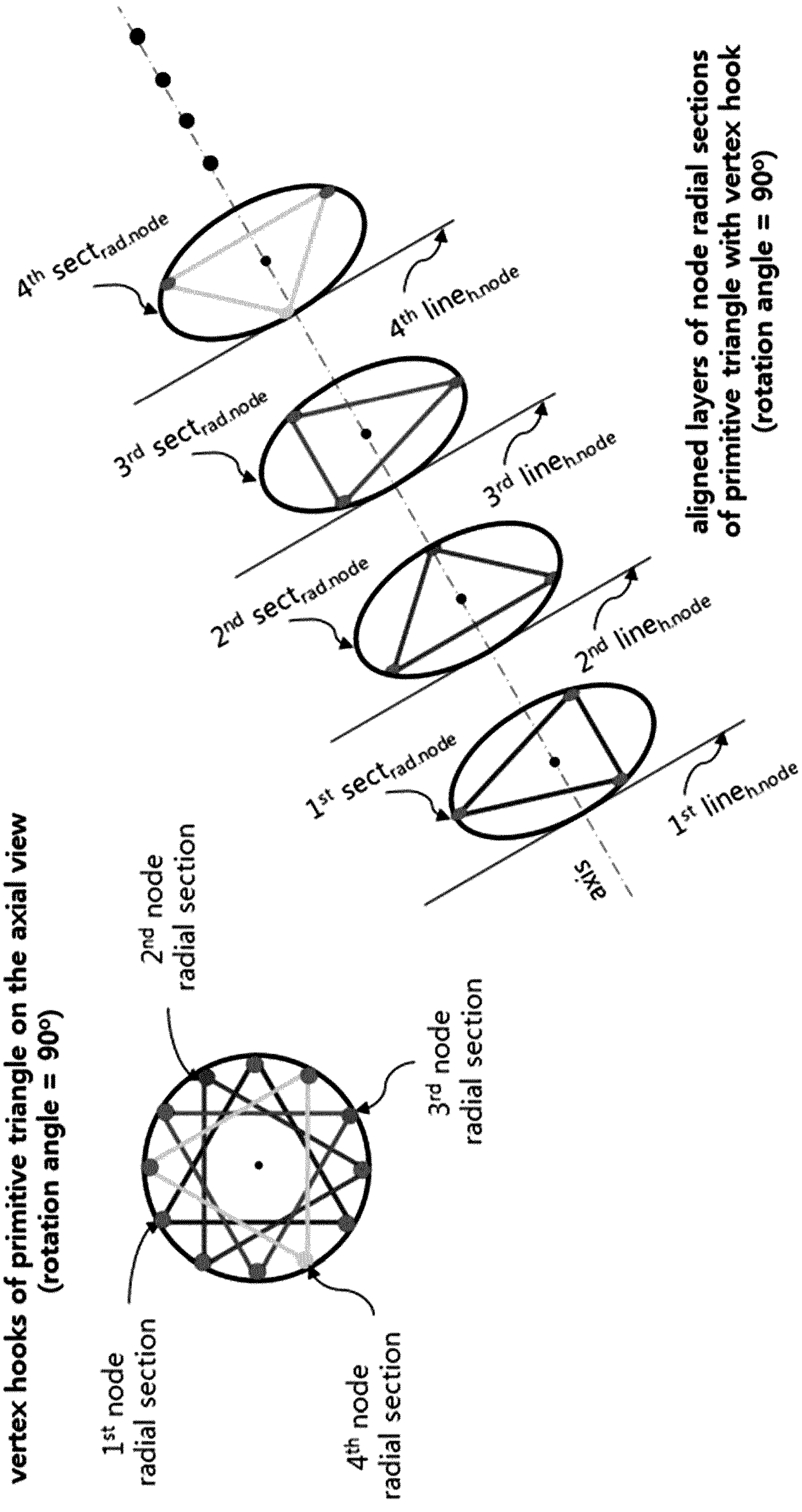
vertex hooks of primitive triangle on the axial view
(rotation angle = 90°)
1st node radial section
2nd node radial section
3rd node radial section
4th node radial section
aligned layers of node radial sections of primitive triangle with vertex hook
(rotation angle = 90°)
1st sect rad.node
2nd sect rad.node
3rd sect rad.node
4th sect rad.node
1st line h.node
2nd line h.node
3rd line h.node
4th line h.node
axis

FIG. 71

FIG. 72

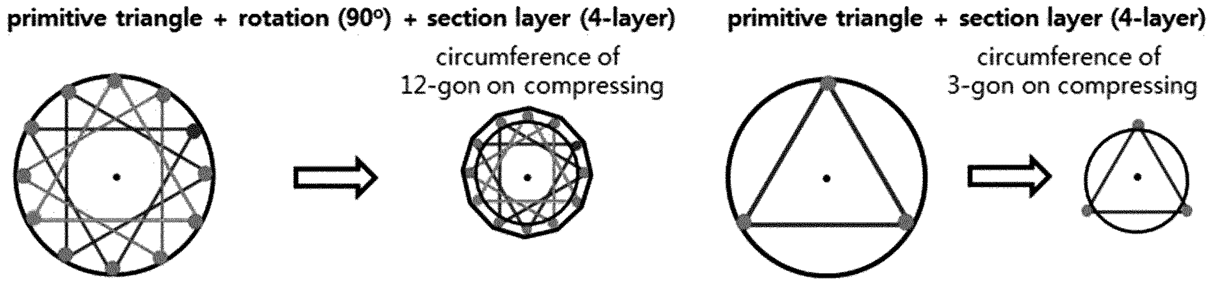
primitive triangle + rotation (90°) + section layer (4-layer)
circumference of
12-gon on compressing
primitive triangle + section layer (4-layer)
circumference of
3-gon on compressing

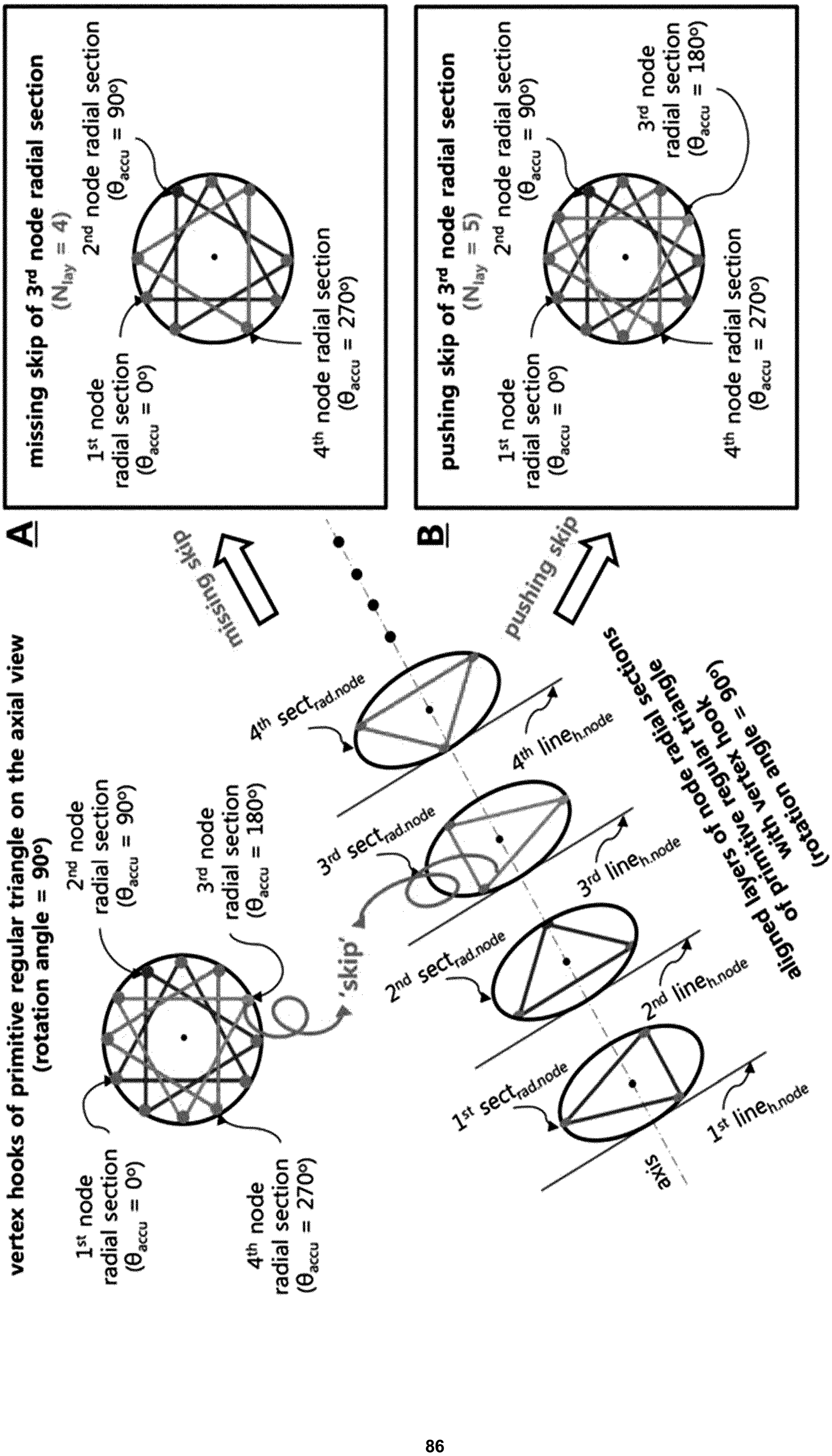
vertex hooks of primitive regular triangle on the axial view
(rotation angle = 90°)
1st node radial section ($\theta_{accu}$ = 0°)
2nd node radial section ($\theta_{accu}$ = 90°)
3rd node radial section ($\theta_{accu}$ = 180°)
4th node radial section ($\theta_{accu}$ = 270°)
'skip'
1st $sect_{rad.node}$
2nd $sect_{rad.node}$
3rd $sect_{rad.node}$
4th $sect_{rad.node}$
1st $line_{h.node}$
2nd $line_{h.node}$
3rd $line_{h.node}$
4th $line_{h.node}$
axis
aligned layers of node radial sections of regular triangle with primitive vertex hook
(rotation angle of primitive vertex hook = 90°)
missing skip
pushing skip
A
missing skip of 3rd node radial section
($N_{lay}$ = 4)
1st node radial section ($\theta_{accu}$ = 0°)
2nd node radial section ($\theta_{accu}$ = 90°)
4th node radial section ($\theta_{accu}$ = 270°)
B
pushing skip of 3rd node radial section
($N_{lay}$ = 5)
1st node radial section ($\theta_{accu}$ = 0°)
2nd node radial section ($\theta_{accu}$ = 90°)
3rd node radial section ($\theta_{accu}$ = 180°)
4th node radial section ($\theta_{accu}$ = 270°)

FIG. 73

**simplified primitive planar matrix formula of primitive regular triangle with 'skip' ($\theta_{rot}$ = 90°, k=1)**

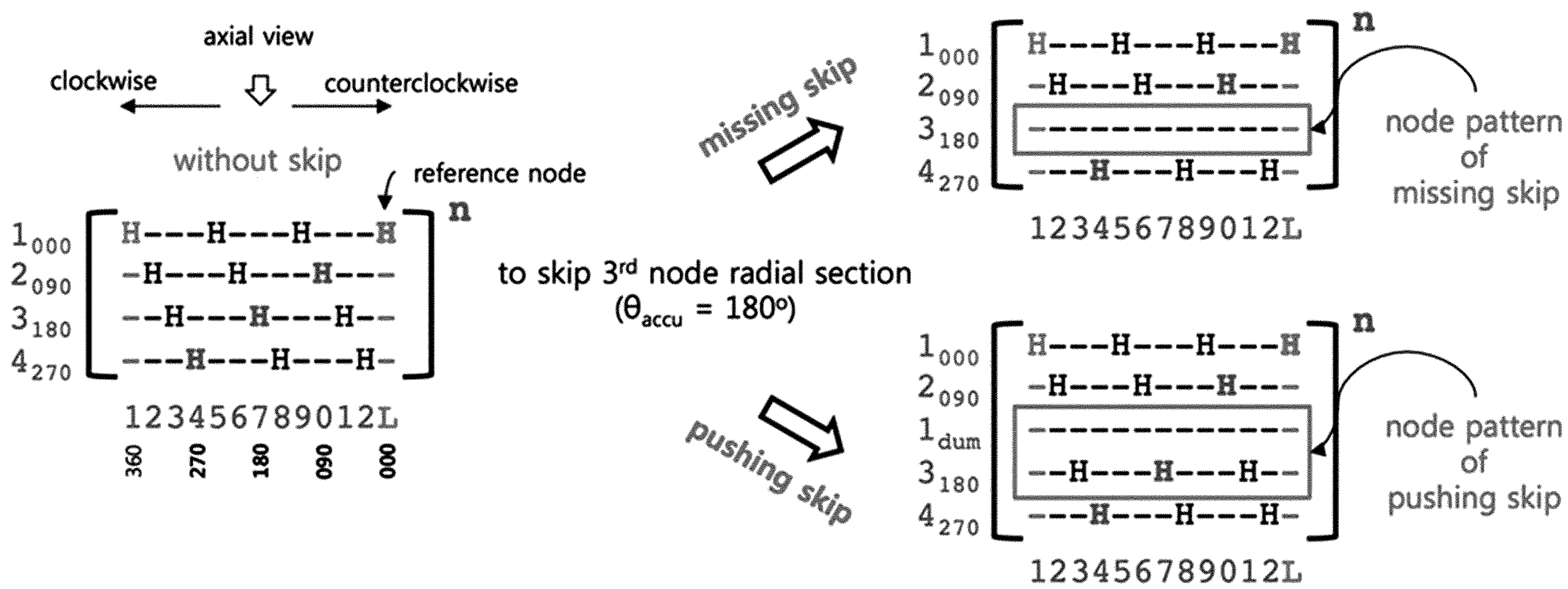


FIG. 74

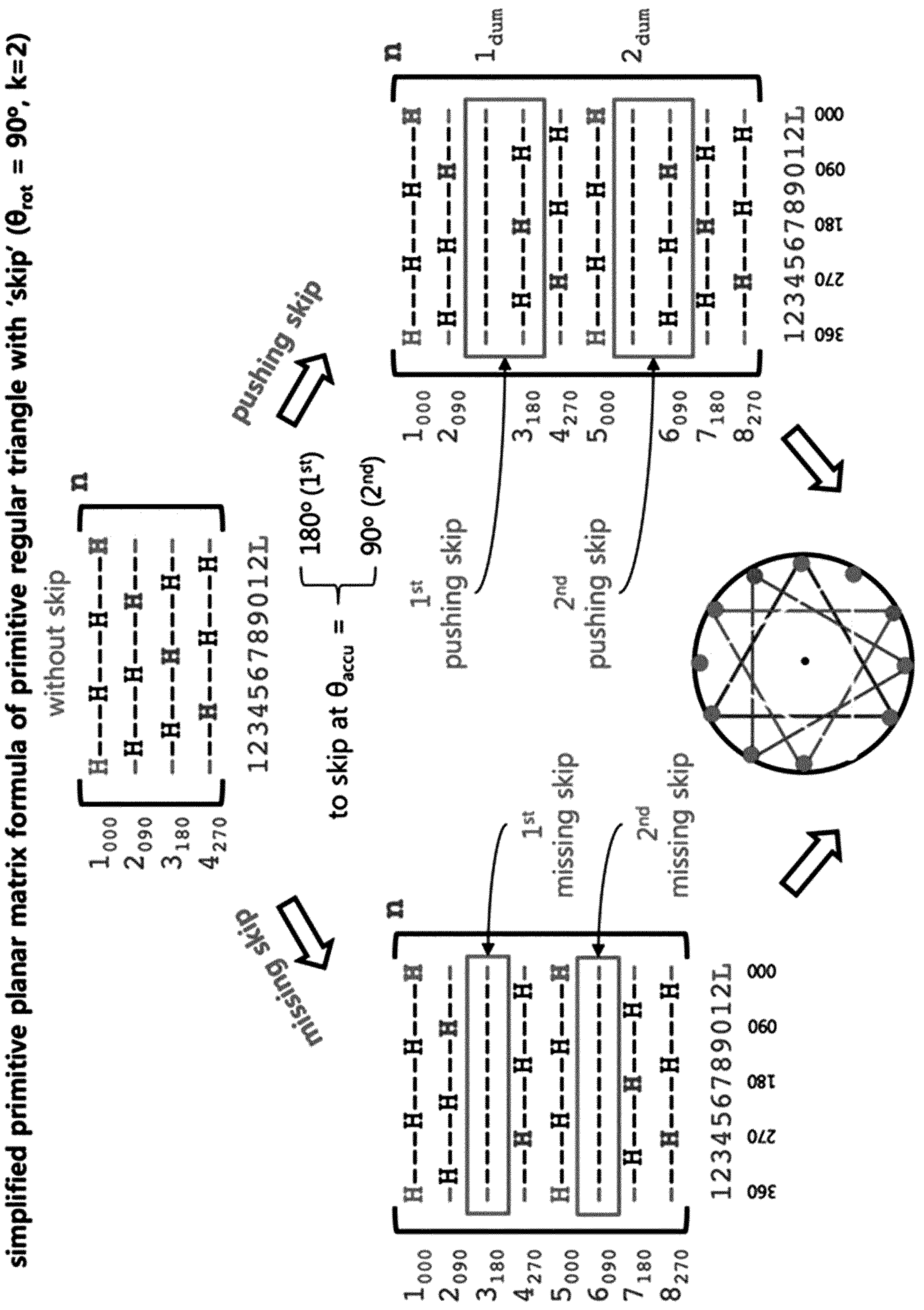
simplified primitive planar matrix formula of primitive regular triangle with 'skip' ($\theta_{rot}$ = 90°, k=2)
without skip
to skip at $\theta_{accu}$ = 180° (1st) 90° (2nd)
pushing skip
missing skip
1st pushing skip
2nd pushing skip
1st missing skip
2nd missing skip
$1_{dum}$
$2_{dum}$
123456789012L
360 270 180 090 000

FIG. 75

FIG. 76

**pattern combination**

[ primitive planar matrix formula A ]$^{n}$

+

[ interface node pattern ]

+

[ primitive planar matrix formula B ]$^{n}$

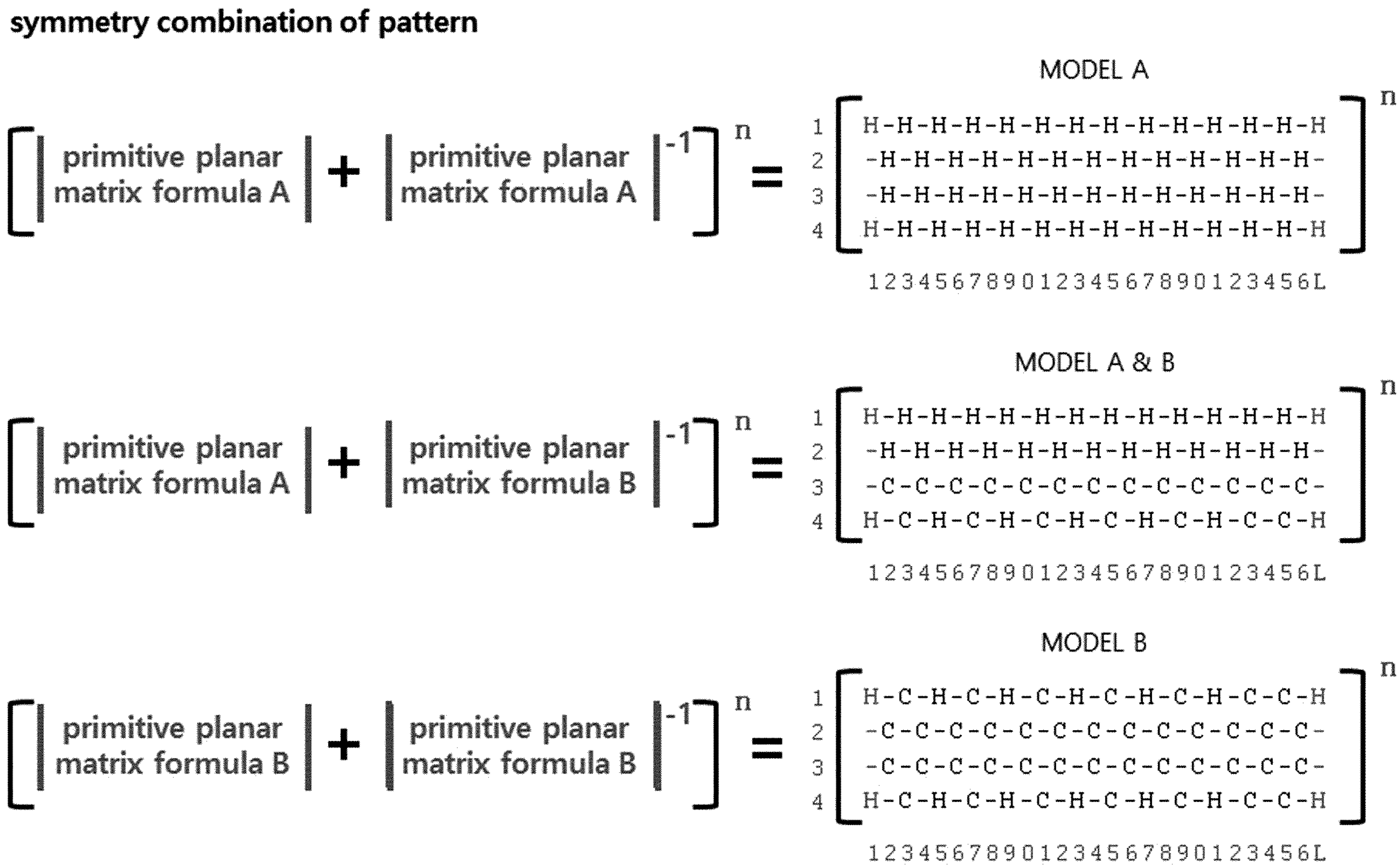
symmetry combination of pattern
MODEL A
primitive planar matrix formula A
+
primitive planar matrix formula A
-1
n
=
1 H-H-H-H-H-H-H-H-H-H-H-H-H-H
2 -H-H-H-H-H-H-H-H-H-H-H-H-H-
3 -H-H-H-H-H-H-H-H-H-H-H-H-H-
4 H-H-H-H-H-H-H-H-H-H-H-H-H-H
n
12345678901234567890123456L
MODEL A & B
primitive planar matrix formula A
+
primitive planar matrix formula B
-1
n
=
1 H-H-H-H-H-H-H-H-H-H-H-H-H-H
2 -H-H-H-H-H-H-H-H-H-H-H-H-H-
3 -C-C-C-C-C-C-C-C-C-C-C-C-C-
4 H-C-H-C-H-C-H-C-H-C-H-C-C-H
n
12345678901234567890123456L
MODEL B
primitive planar matrix formula B
+
primitive planar matrix formula B
-1
n
=
1 H-C-H-C-H-C-H-C-H-C-H-C-C-H
2 -C-C-C-C-C-C-C-C-C-C-C-C-C-
3 -C-C-C-C-C-C-C-C-C-C-C-C-C-
4 H-C-H-C-H-C-H-C-H-C-H-C-C-H
n
12345678901234567890123456L

FIG. 77A

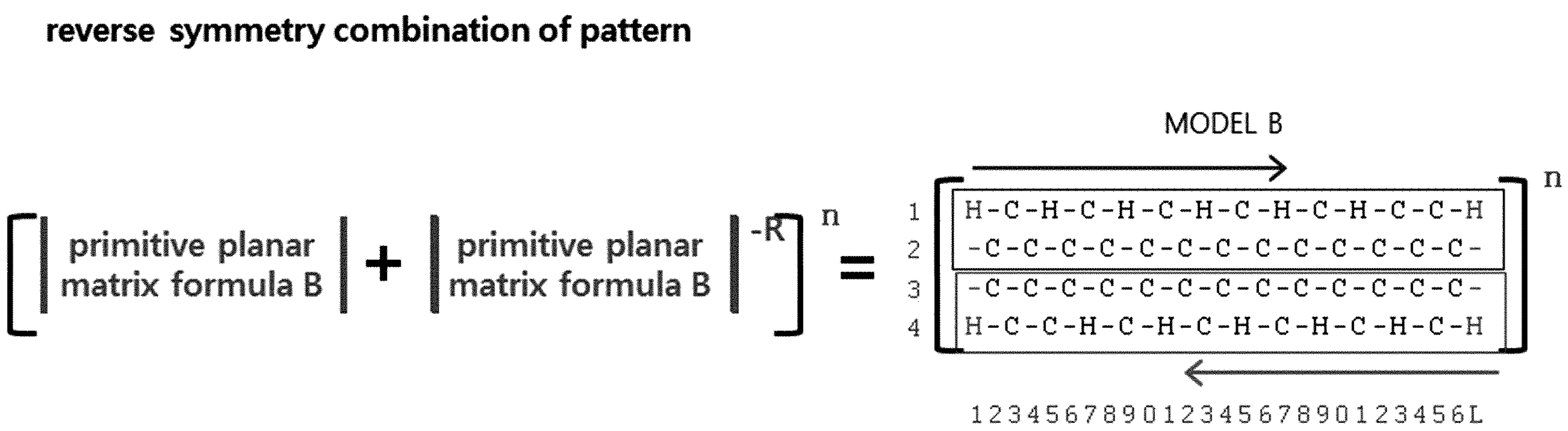
reverse symmetry combination of pattern
primitive planar matrix formula B
+
primitive planar matrix formula B
-R
n
=
MODEL B
n
1 H-C-H-C-H-C-H-C-H-C-H-C-C-H
2 -C-C-C-C-C-C-C-C-C-C-C-C-C-
3 -C-C-C-C-C-C-C-C-C-C-C-C-C-
4 H-C-C-H-C-H-C-H-C-H-C-H-C-H
1234567890123456789012345 6L

FIG. 77B

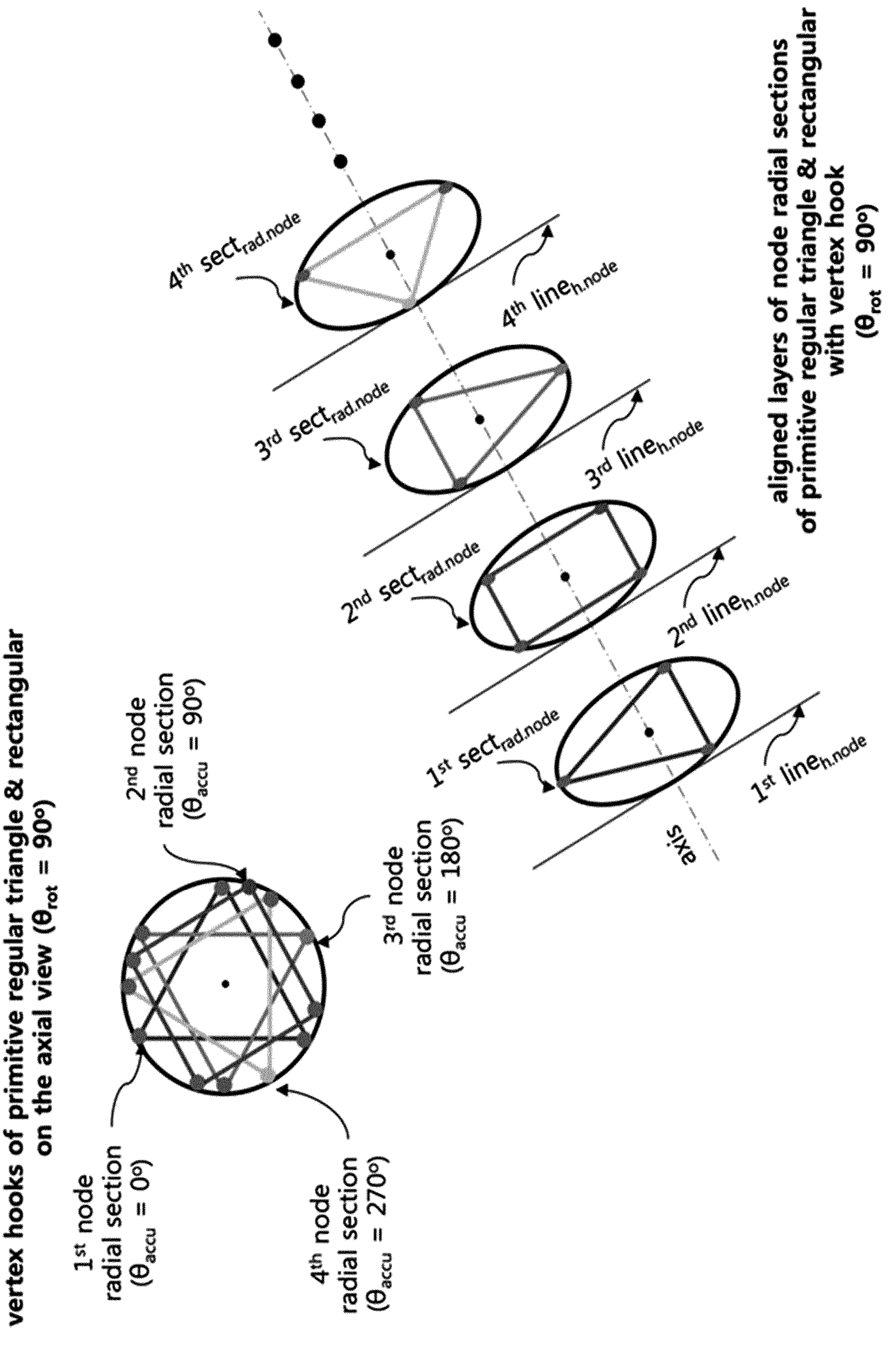
vertex hooks of primitive regular triangle & rectangular on the axial view ($\theta_{rot}$ = 90°)
1st node radial section ($\theta_{accu}$ = 0°)
2nd node radial section ($\theta_{accu}$ = 90°)
3rd node radial section ($\theta_{accu}$ = 180°)
4th node radial section ($\theta_{accu}$ = 270°)
1st sect$_{rad.node}$
2nd sect$_{rad.node}$
3rd sect$_{rad.node}$
4th sect$_{rad.node}$
1st line$_{h.node}$
2nd line$_{h.node}$
3rd line$_{h.node}$
4th line$_{h.node}$
axis
aligned layers of node radial sections of primitive regular triangle & rectangular with vertex hook ($\theta_{rot}$ = 90°)

FIG. 78

FIG. 79

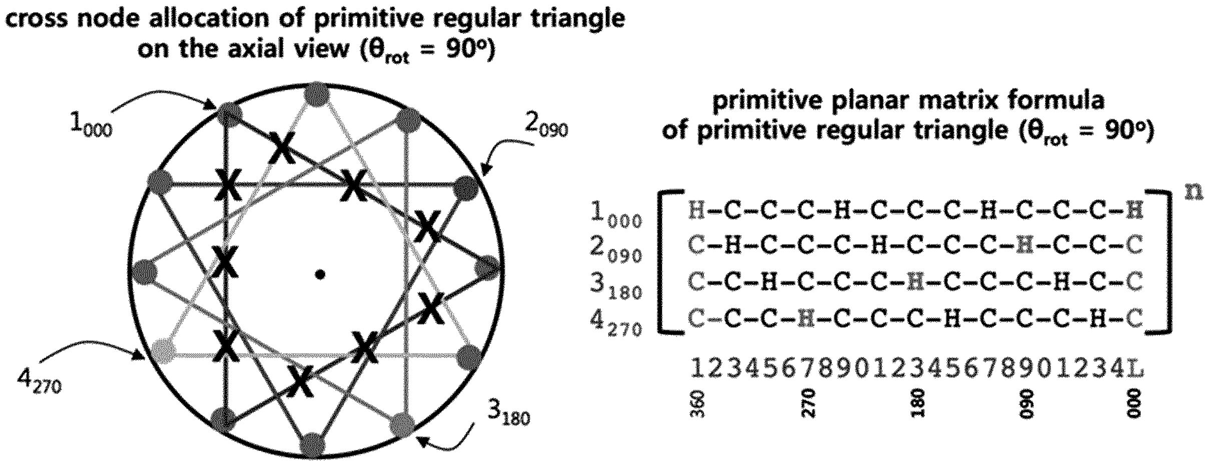
cross node allocation of primitive regular triangle
on the axial view ($\theta_{rot}$ = 90°)
$1_{000}$
$2_{090}$
$3_{180}$
$4_{270}$
primitive planar matrix formula
of primitive regular triangle ($\theta_{rot}$ = 90°)
$1_{000}$ H-C-C-C-H-C-C-C-H-C-C-C-H
$2_{090}$ C-H-C-C-C-H-C-C-C-H-C-C-C
$3_{180}$ C-C-H-C-C-C-H-C-C-C-H-C-C
$4_{270}$ C-C-C-H-C-C-C-H-C-C-C-H-C
n
1234567890123456789012 34L
360
270
180
090
000

FIG. 80

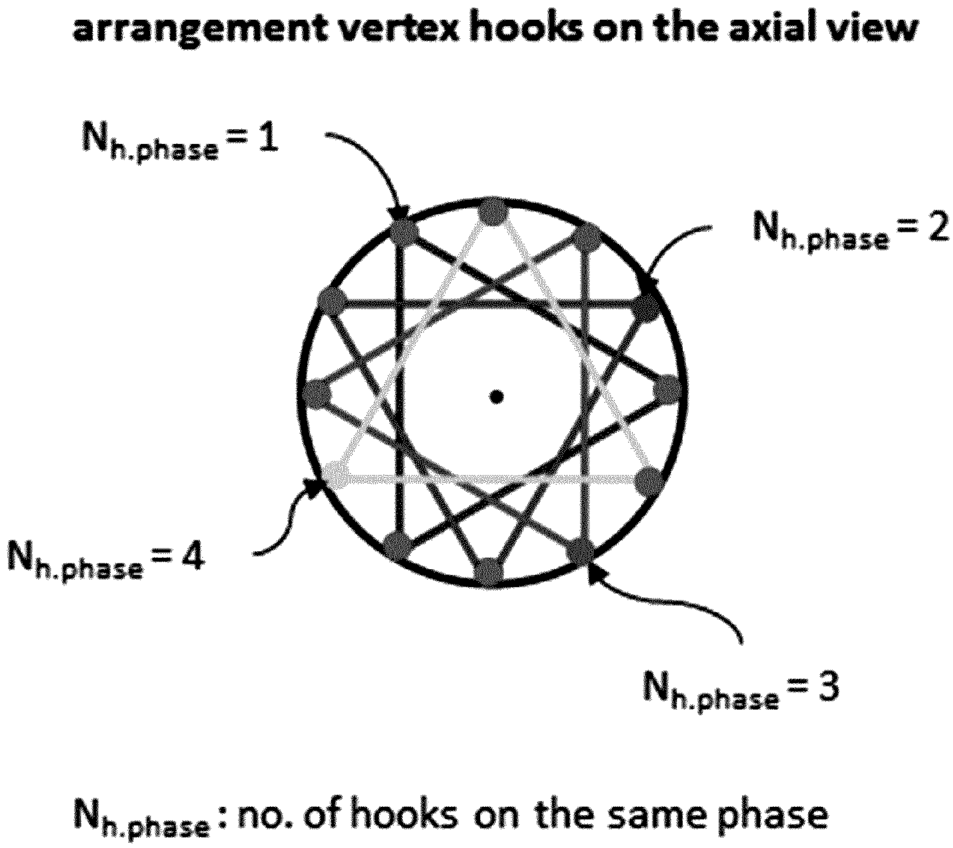
arrangement vertex hooks on the axial view
$N_{h.phase}$ = 1
$N_{h.phase}$ = 2
$N_{h.phase}$ = 4
$N_{h.phase}$ = 3
$N_{h.phase}$ : no. of hooks on the same phase

FIG. 81

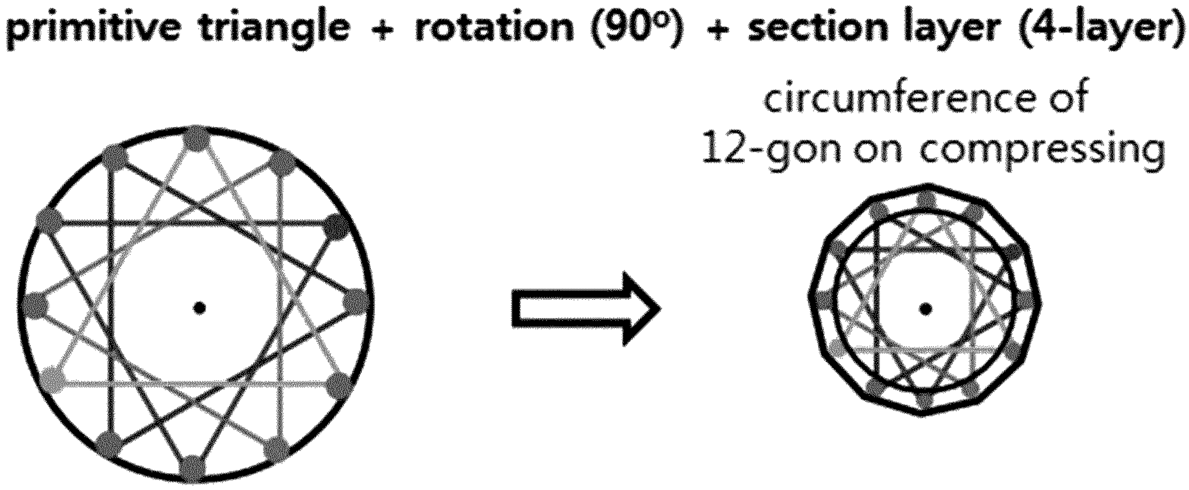
primitive triangle + rotation (90°) + section layer (4-layer)
circumference of
12-gon on compressing

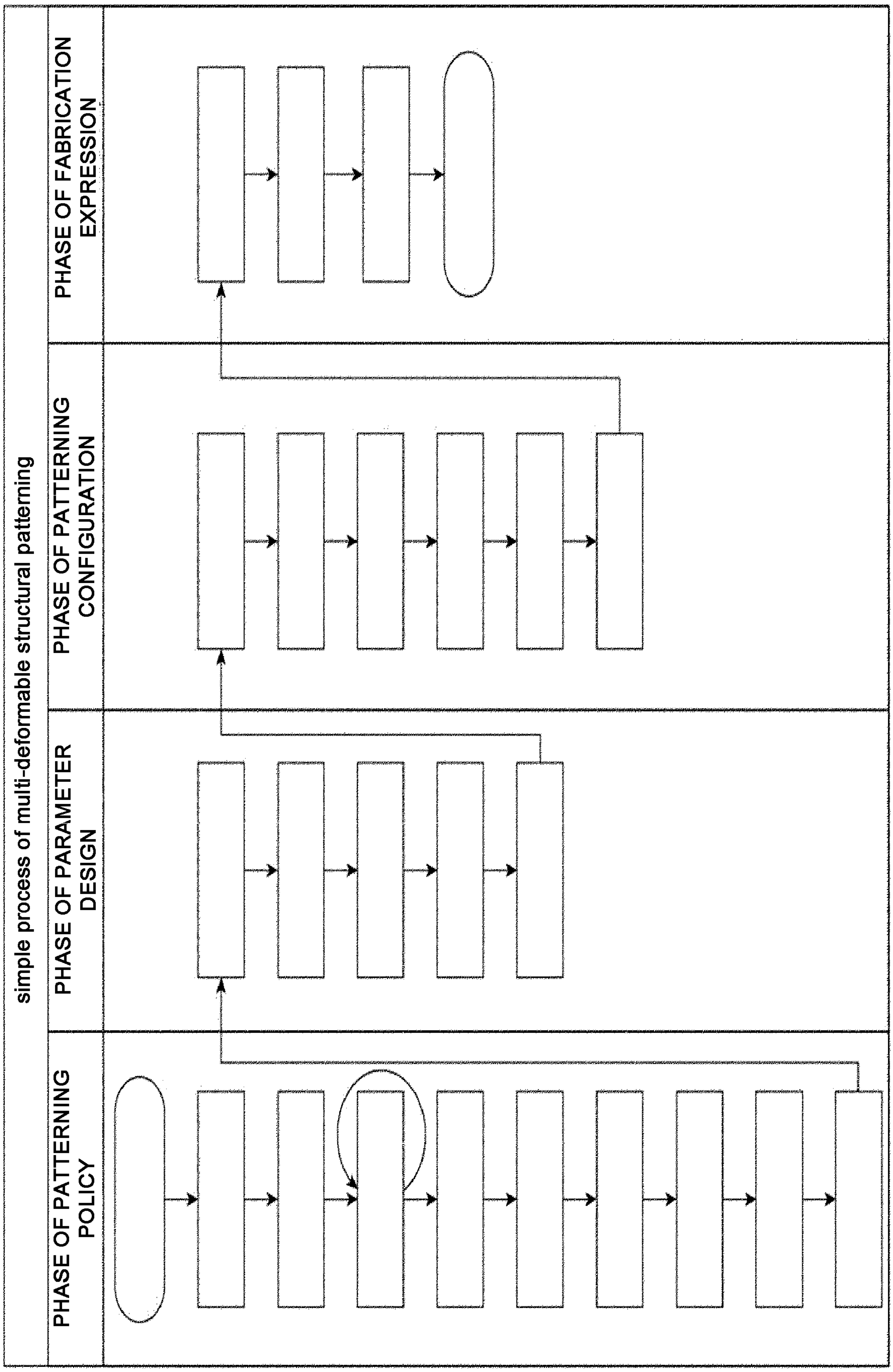
simple process of multi-deformable structural patterning
PHASE OF PATTERNING POLICY
PHASE OF PARAMETER DESIGN
PHASE OF PATTERNING CONFIGURATION
PHASE OF FABRICATION EXPRESSION

FIG. 82A

FIG. 82B

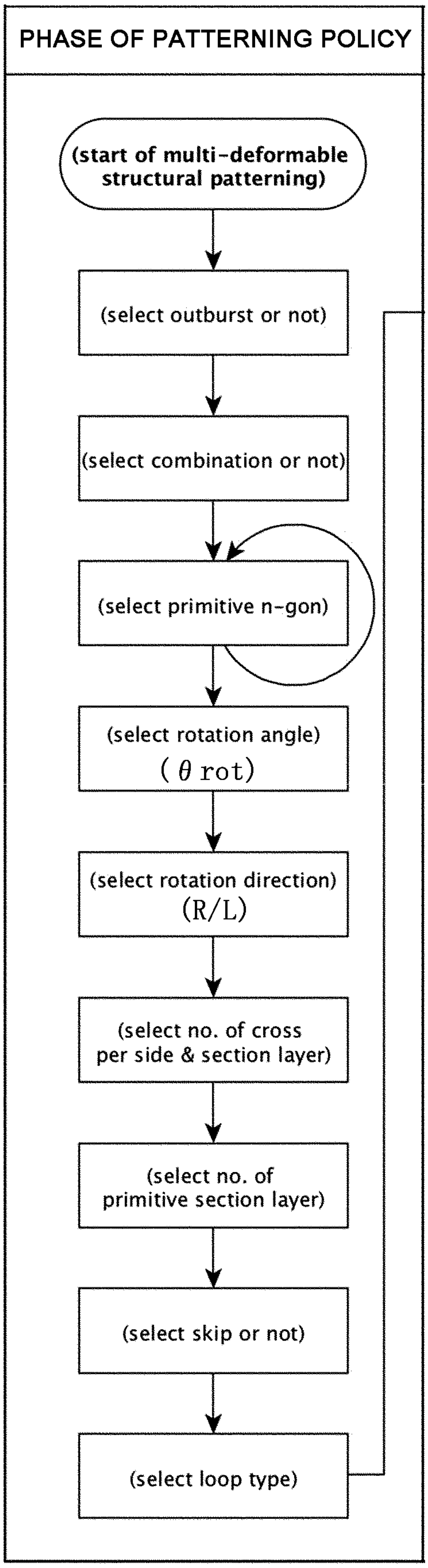
PHASE OF PATTERNING POLICY
(start of multi-deformable structural patterning)
(select outburst or not)
(select combination or not)
(select primitive n-gon)
(select rotation angle)
( θ rot)
(select rotation direction)
(R/L)
(select no. of cross per side & section layer)
(select no. of primitive section layer)
(select skip or not)
(select loop type)

FIG. 82C

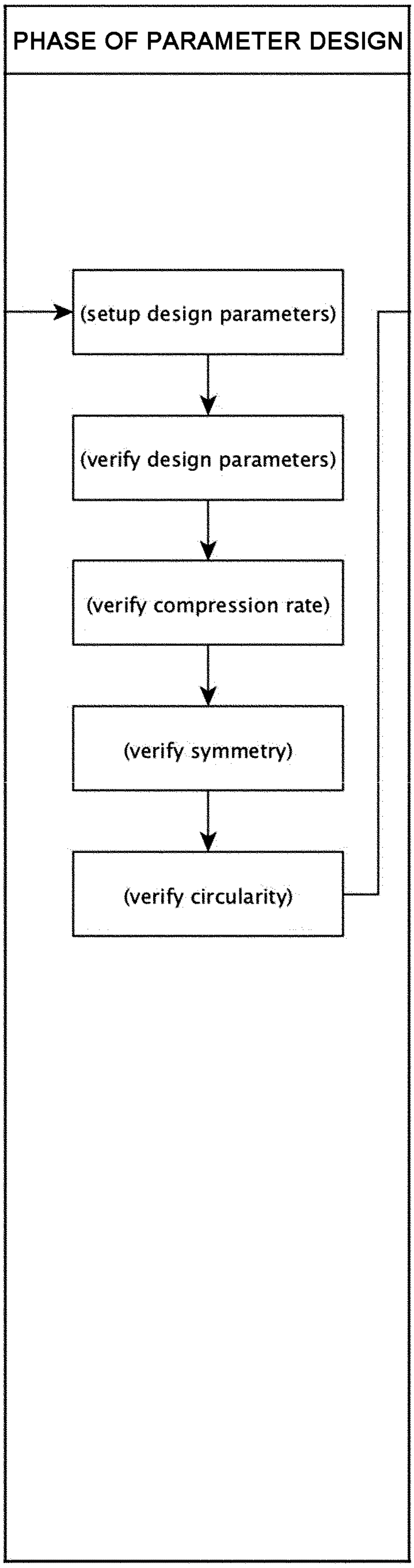
PHASE OF PARAMETER DESIGN
(setup design parameters)
(verify design parameters)
(verify compression rate)
(verify symmetry)
(verify circularity)

FIG. 82D

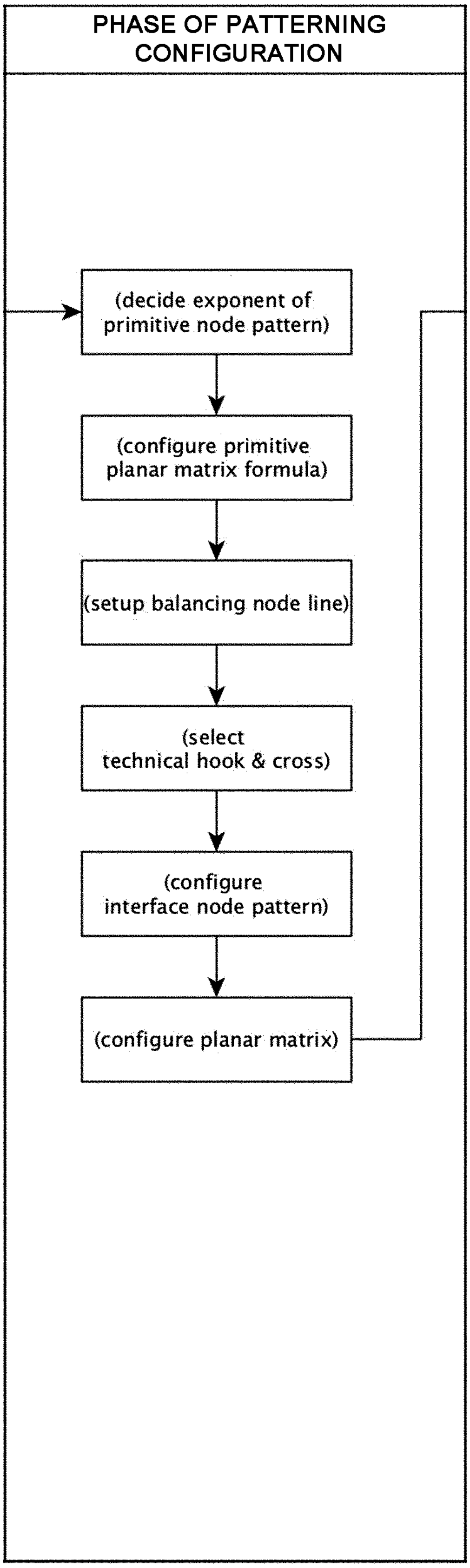
PHASE OF PATTERNING CONFIGURATION
(decide exponent of primitive node pattern)
(configure primitive planar matrix formula)
(setup balancing node line)
(select technical hook & cross)
(configure interface node pattern)
(configure planar matrix)

FIG. 82E

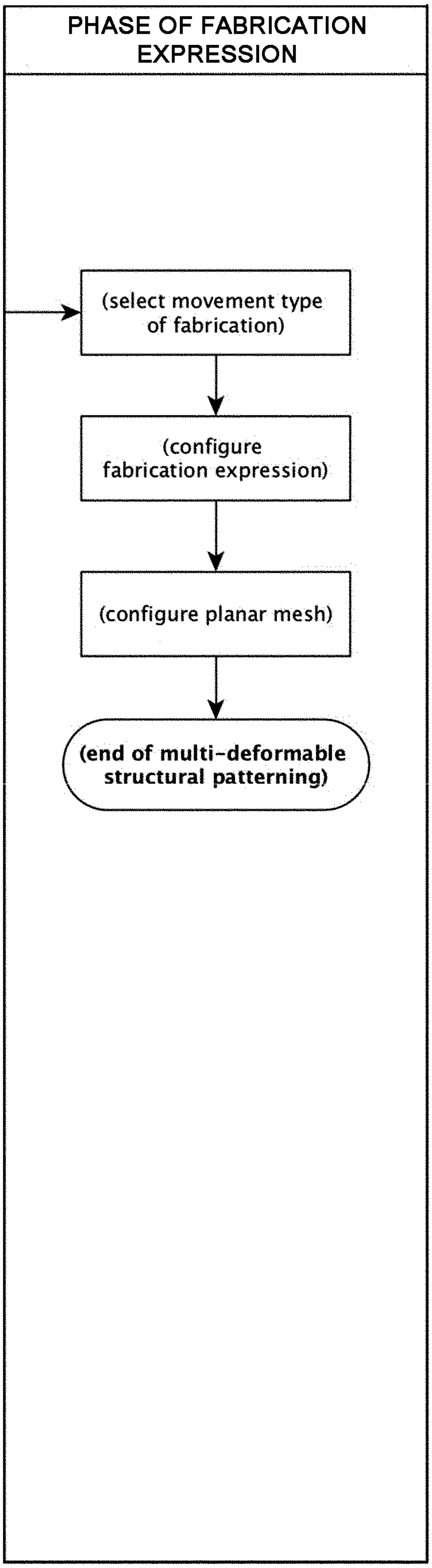
PHASE OF FABRICATION EXPRESSION
(select movement type of fabrication)
(configure fabrication expression)
(configure planar mesh)
(end of multi-deformable structural patterning)

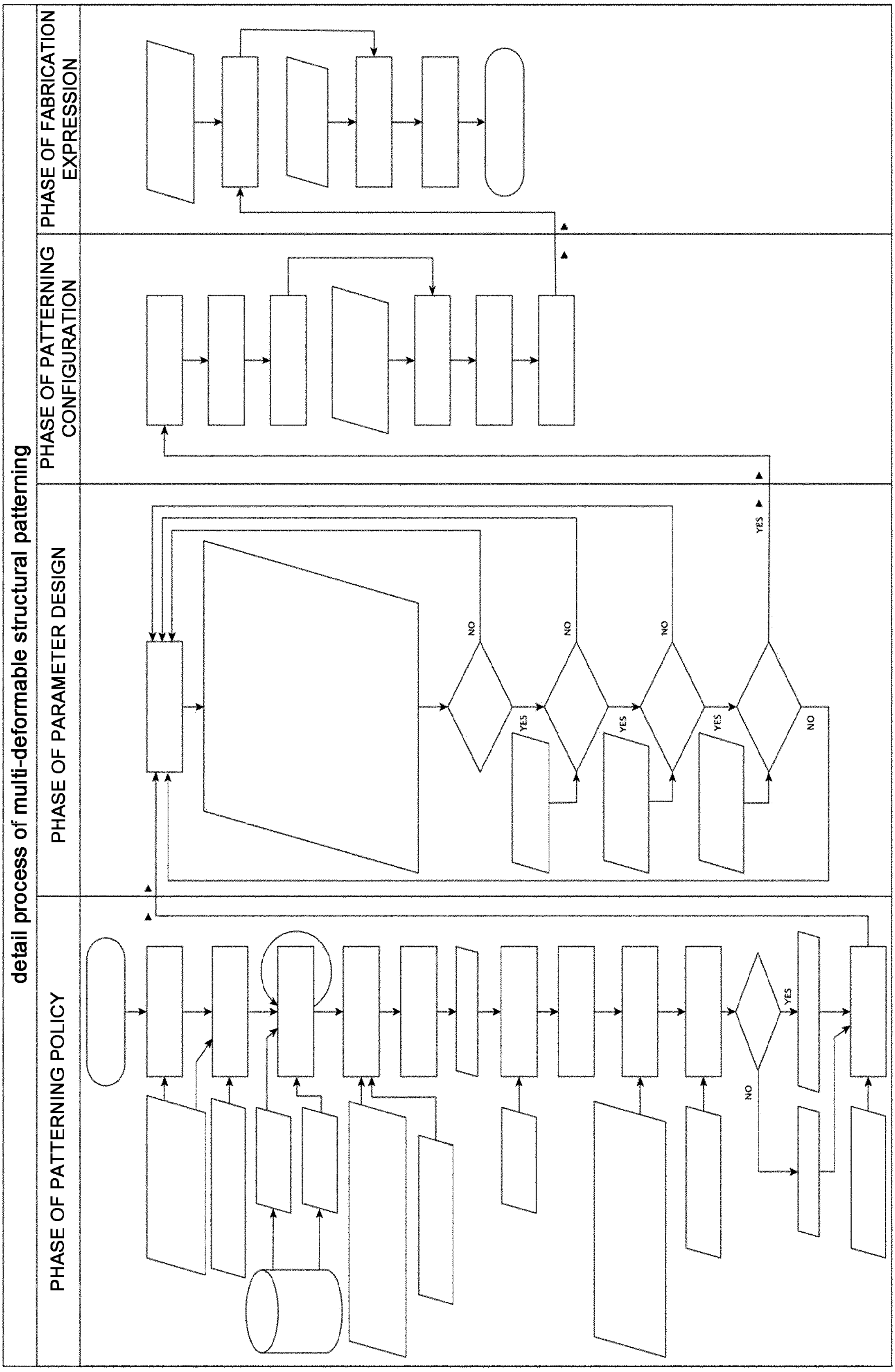
detail process of multi-deformable structural patterning
PHASE OF PATTERNING POLICY
PHASE OF PARAMETER DESIGN
PHASE OF PATTERNING CONFIGURATION
PHASE OF FABRICATION EXPRESSION
YES
NO
YES
NO
YES
NO
YES
NO
YES
NO

FIG. 83A

FIG. 83B

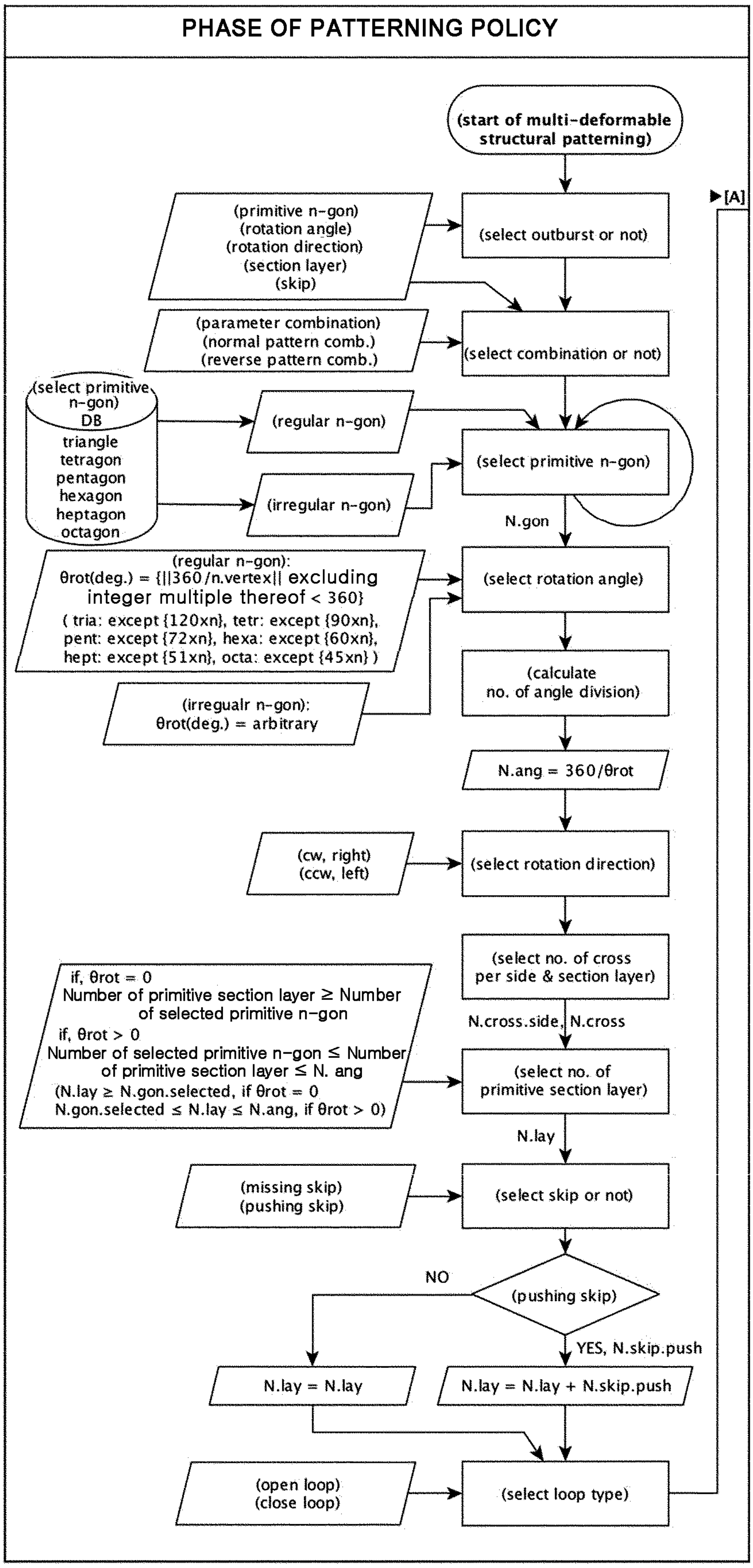
PHASE OF PATTERNING POLICY
(start of multi-deformable structural patterning)
[A]
(primitive n-gon)
(rotation angle)
(rotation direction)
(section layer)
(skip)
(select outburst or not)
(parameter combination)
(normal pattern comb.)
(reverse pattern comb.)
(select combination or not)
(select primitive n-gon) DB
triangle
tetragon
pentagon
hexagon
heptagon
octagon
(regular n-gon)
(irregular n-gon)
(select primitive n-gon)
N.gon
(regular n-gon):
θrot(deg.) = {||360/n.vertex|| excluding integer multiple thereof < 360}
( tria: except {120xn}, tetr: except {90xn}, pent: except {72xn}, hexa: except {60xn}, hept: except {51xn}, octa: except {45xn} )
(irregualr n-gon):
θrot(deg.) = arbitrary
(select rotation angle)
(calculate no. of angle division)
N.ang = 360/θrot
(cw, right)
(ccw, left)
(select rotation direction)
(select no. of cross per side & section layer)
N.cross.side, N.cross
if, θrot = 0
Number of primitive section layer ≥ Number of selected primitive n-gon
if, θrot > 0
Number of selected primitive n-gon ≤ Number of primitive section layer ≤ N. ang
(N.lay ≥ N.gon.selected, if θrot = 0
N.gon.selected ≤ N.lay ≤ N.ang, if θrot > 0)
(select no. of primitive section layer)
N.lay
(missing skip)
(pushing skip)
(select skip or not)
NO
(pushing skip)
YES, N.skip.push
N.lay = N.lay
N.lay = N.lay + N.skip.push
(open loop)
(close loop)
(select loop type)

FIG. 83C

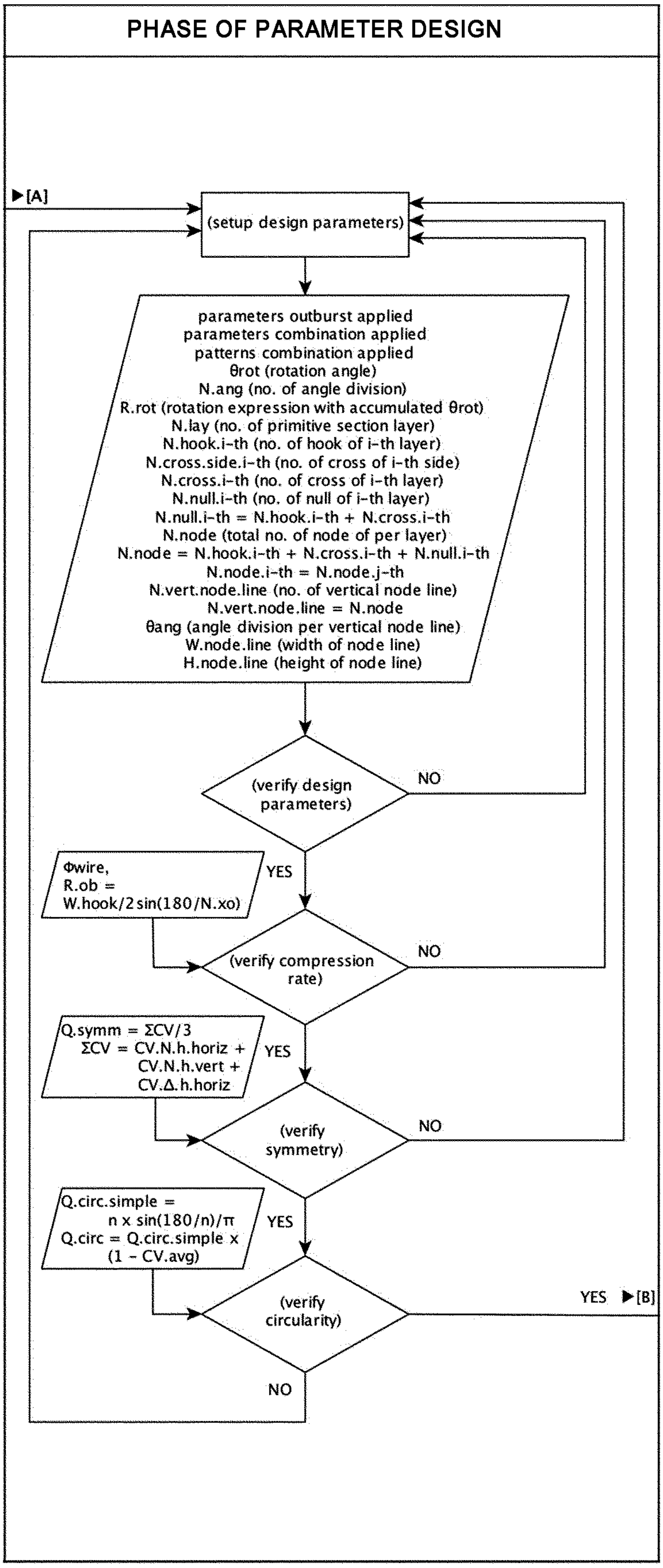
PHASE OF PARAMETER DESIGN
[A]
(setup design parameters)
parameters outburst applied
parameters combination applied
patterns combination applied
θrot (rotation angle)
N.ang (no. of angle division)
R.rot (rotation expression with accumulated θrot)
N.lay (no. of primitive section layer)
N.hook.i-th (no. of hook of i-th layer)
N.cross.side.i-th (no. of cross of i-th side)
N.cross.i-th (no. of cross of i-th layer)
N.null.i-th (no. of null of i-th layer)
N.null.i-th = N.hook.i-th + N.cross.i-th
N.node (total no. of node of per layer)
N.node = N.hook.i-th + N.cross.i-th + N.null.i-th
N.node.i-th = N.node.j-th
N.vert.node.line (no. of vertical node line)
N.vert.node.line = N.node
θang (angle division per vertical node line)
W.node.line (width of node line)
H.node.line (height of node line)
(verify design parameters)
NO
YES
Φwire,
R.ob =
W.hook/2 sin(180/N.xo)
(verify compression rate)
NO
YES
Q.symm = ΣCV/3
ΣCV = CV.N.h.horiz +
CV.N.h.vert +
CV.Δ.h.horiz
(verify symmetry)
NO
YES
Q.circ.simple =
n x sin(180/n)/π
Q.circ = Q.circ.simple x
(1 - CV.avg)
(verify circularity)
YES [B]
NO

FIG. 83D

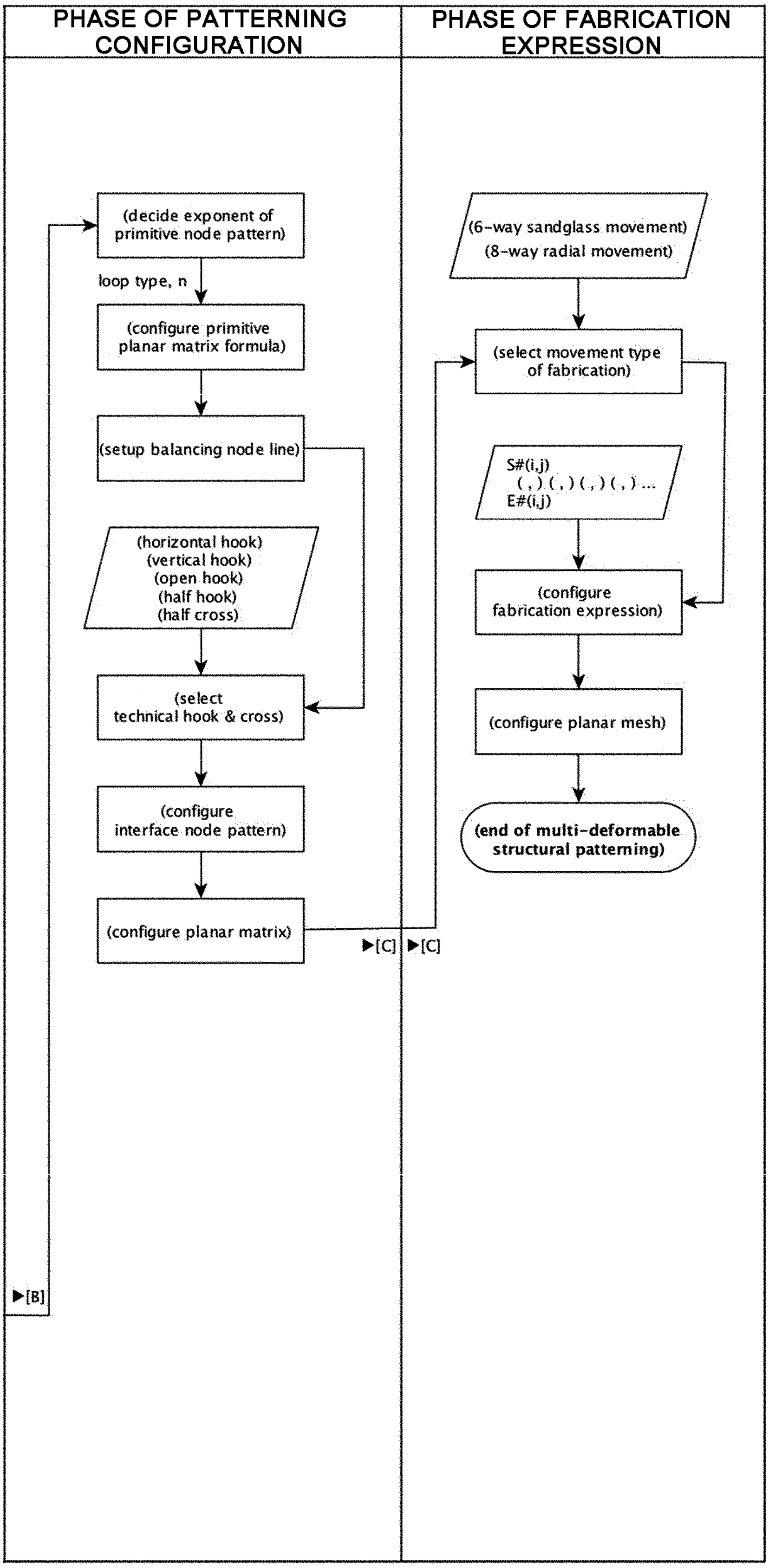
PHASE OF PATTERNING CONFIGURATION
PHASE OF FABRICATION EXPRESSION
(decide exponent of primitive node pattern)
loop type, n
(configure primitive planar matrix formula)
(setup balancing node line)
(horizontal hook)
(vertical hook)
(open hook)
(half hook)
(half cross)
(select technical hook & cross)
(configure interface node pattern)
(configure planar matrix)
▶[C]
▶[C]
▶[B]
(6-way sandglass movement)
(8-way radial movement)
(select movement type of fabrication)
S#(i,j)
( , ) ( , ) ( , ) ( , ) ...
E#(i,j)
(configure fabrication expression)
(configure planar mesh)
(end of multi-deformable structural patterning)

**NAME OF PATTERNING: REG-TRIA-90-L-12**

**POLICY OF PATTERNING**

| | |
|---|---|
| primitive n-gon: | REGULAR TRIANGLE |
| rotation angle: | 90° |
| rotation direction: | LEFT (counterclockwise, left to right on planar matrix) : (0,L) (90,L) (180,L) (270,L) |
| skip: | NO |
| combination: | NO |
| outburst: | NO |
| loop type: | OPEN |

**PARAMETER OF DESIGN**

no. of angle division: 4(4)
no. of primitive section layer: 4
no. of hook node per layer(h): 3
no. of cross node on side: 3
no. of cross node per layer(c): 9
no. of null node per layer(n): 12
total no. of node(h+c+n): 24
no. of vertical node line: 24L
angle division per vertical node line: 15°
width of horizontal node line: EQUAL
height of vertical node line: EQUAL
exponent of primitive node pattern: 2

axial view

**primitive planar matrix formula**

```
        ┌                         ┐ 2
1 000   H-C-C-C-H-C-C-C-H-C-C-C-H
2 090   C-C-C-H-C-C-C-H-C-C-C-H-C
3 180   C-C-H-C-C-C-H-C-C-C-H-C-C
4 270   C-H-C-C-C-H-C-C-C-H-C-C-C
        └                         ┘
        1234567890123456789012345L
        000     090     180     270     360
```

**interface node pattern**

**upper**

[H-H-H-H-H-H-H-H-H-H-H-H-H]

**lower**

[H-H-H-H-H-H-H-H-H-H-H-H-H]

FIG. 84A

## NAME OF PATTERNING: REG-TRIA-90-L-12

### planar matrix

```
          1234567890123456789012 34L
1upp   [ H-H-H-H-H-H-H-H-H-H-H-H-H ]
2000   [ H-C-C-C-H-C-C-C-H-C-C-C-H ]
3090   [ C-C-C-H-C-C-C-H-C-C-C-H-C ]
4180   [ C-C-H-C-C-C-H-C-C-C-H-C-C ]
5270   [ C-H-C-C-C-H-C-C-C-H-C-C-C ]
6000   [ H-C-C-C-H-C-C-C-H-C-C-C-H ]
7090   [ C-C-C-H-C-C-C-H-C-C-C-H-C ]
8180   [ C-C-H-C-C-C-H-C-C-C-H-C-C ]
9270   [ C-H-C-C-C-H-C-C-C-H-C-C-C ]
0low   [ H-H-H-H-H-H-H-H-H-H-H-H-H ]
          1234567890123456789012 34L
          000   090   180   270   360
```

### fabrication expression (n=2)

S1(1,1)
(i, -6j+25) $_{i=10->1->10,\ j=1\ to\ 3}$
E1(1,L)

**S2(1,3)**
(3,7) (2,9) (4,13) (3,15) (5,19) (4,21) (6,L)
(5,3) (7,7) (6,9) (8,13) (7,15) (9,19) (8,21) (10,L)
(9,3) (10,5) (1,23) $(2,L)_V$
(3,23) (1,19) (2,17) (1,15) (10,21) (9.19) (10,17)
(8,13) (9.11) (7,7) (8.5) (6,1) (7,23) (5,19) (6,17)
(4,13) (5,11) (3,7) (4,5) $(2,1)_V$
**E2(1,3)**

**S3(1,5)**
(i, -6j+29) $_{i=10->1->10,\ j=1\ to\ 3}$
**E3(1,5)**

**S4(1,9)**
(10,3) (1,21) (10,15)
**E4(1,9)**

FIG. 84B

FIG. 84C

**NAME OF PATTERNING: REG-TRIA-90-L-12**

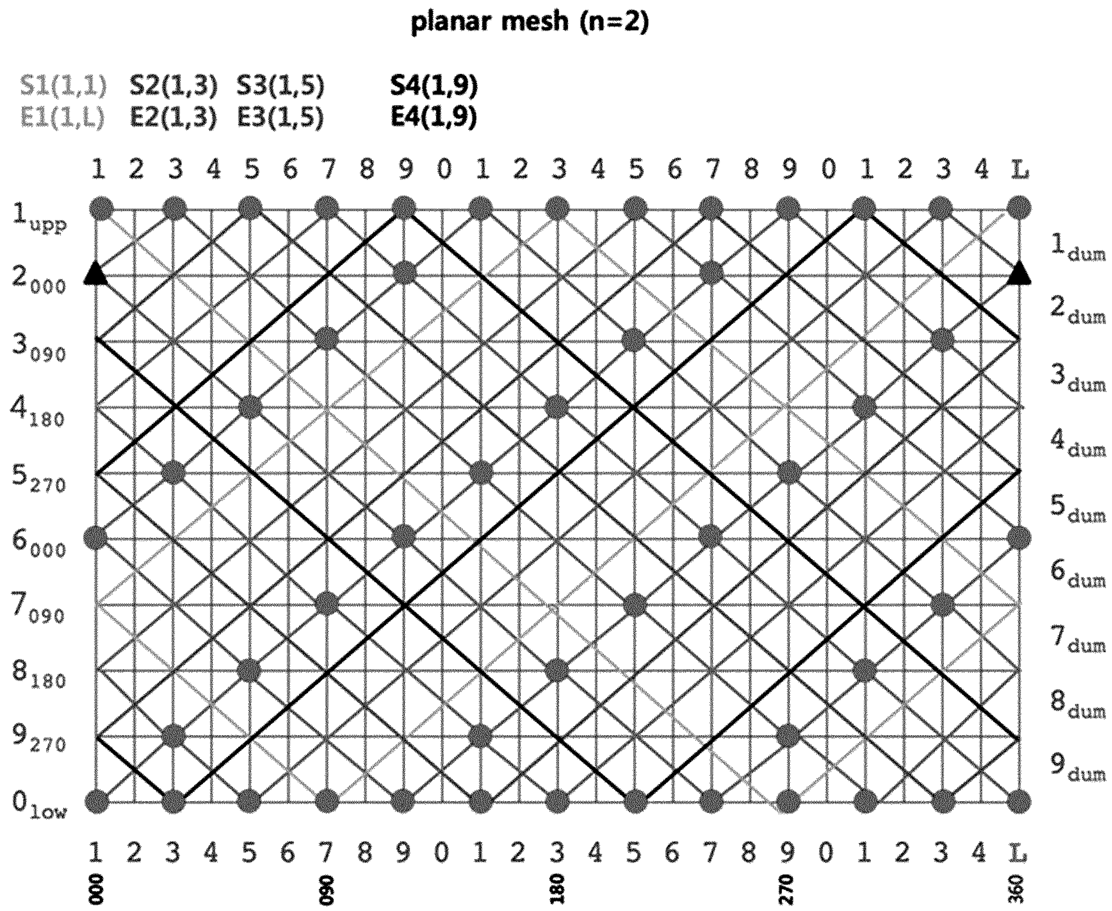

**NAME OF PATTERNING: REG-TRIA-180-L-12**

**POLICY OF PATTERNING**

| | |
|---|---|
| primitive n-gon: | REGULAR TRIANGLE |
| rotation angle: | 180° |
| rotation direction: | LEFT (counterclockwise, left to right on planar matrix) : (0,L) (180,L) |
| skip: | NO |
| combination: | NO |
| outburst: | NO |
| loop type: | CLOSE |

**PARAMETER OF DESIGN**

no. of angle division: 1(2)
no. of primitive section layer: 2
no. of hook node per layer(h): 3
no. of cross node on side: 3
no. of cross node per layer(c): 9
no. of null node per layer(n): 12
total no. of node(h+c+n): 24
no. of vertical node line: 24L
angle division per vertical node line: 15°
width of horizontal node line: EQUAL
height of vertical node line: EQUAL
exponent of primitive node pattern: 4

axial view

**primitive planar matrix formula**

$1_{000}$ [ H-C-C-C-H-C-C-C-H-C-C-C-H ] $^4$
$2_{180}$ [ C-C-H-C-C-C-H-C-C-C-H-C-C ] $_c$
123456789012345678901234L
000 090 180 270 360

**interface node pattern**

**upper**

[H-H-H-H-H-H-H-H-H-H-H-H-H]

**lower**

[H-H-H-H-H-H-H-H-H-H-H-H-H]

FIG. 85A

NAME OF PATTERNING: REG-TRIA-180-L-12

**planar matrix**

```
         12345678901234567890l234L
1 upp  [ H-H-H-H-H-H-H-H-H-H-H-H-H ]
2 000  [ H-C-C-C-H-C-C-C-H-C-C-C-H ]
3 180  [ C-C-H-C-C-C-H-C-C-C-H-C-C ]
4 360  [ H-C-C-C-H-C-C-C-H-C-C-C-H ]
5 180  [ C-C-H-C-C-C-H-C-C-C-H-C-C ]
6 360  [ H-C-C-C-H-C-C-C-H-C-C-C-H ]
7 180  [ C-C-H-C-C-C-H-C-C-C-H-C-C ]
8 360  [ H-C-C-C-H-C-C-C-H-C-C-C-H ]
9 180  [ C-C-H-C-C-C-H-C-C-C-H-C-C ]
0 000  [ H-C-C-C-H-C-C-C-H-C-C-C-H ]
1 low  [ H-H-H-H-H-H-H-H-H-H-H-H-H ]
         123456789012345678901234L
         000    090    180    270    360
```

**fabrication expression (n=4)**

S1(1,1)
(i, 4j+1) $_{i=3->1->3, j=1 \text{ to } 4}$
$(3,21)_V$
(7,13) (3,5) (7,21) (3,13) (7,5) (11,13) (7,21) (11,5) (7,13) (11,21)
$(7,5)_V$ $(3,21)_V$
E1(1,L)

S2(11,L)
(i, -4j+25) $_{i=9->11->9, j=1 \text{ to } 4}$
$(9,5)_V$
(5,13) (9,21) (5,5) (9,13) $(5,21)_V$
(1,13) (5,5) (1,21) (5,13) 1,5) $(5,21)_V$ $(9,5)_V$
E2(11,1)

S3(1,7)
(2,9) (1,11) (4,17) (1,23) (2,L) (1,3) (4,9) (1,15) (2,17) (1,19) $(4,L)_V$
(8,17) (4,9) $(8,1)_V$
(11,7) (10,9) (11,11) (8,17) (11,23) $(10,L)_V$
(6,17) (10,9) $(6,1)_V$
(2,9) (6,17) (2,L) (6,9) (2,17) $(6,L)_V$
(10,17) (6,9) $(10,1)_V$
(11,3) (8,9) (11,15) (10,17) (11,19) $(8,L)_V$
(4,17) (8,9) $(4,1)_V$
E3(1,7)

FIG. 85B

FIG. 85C

**NAME OF PATTERNING: REG-TRIA-180-L-12**

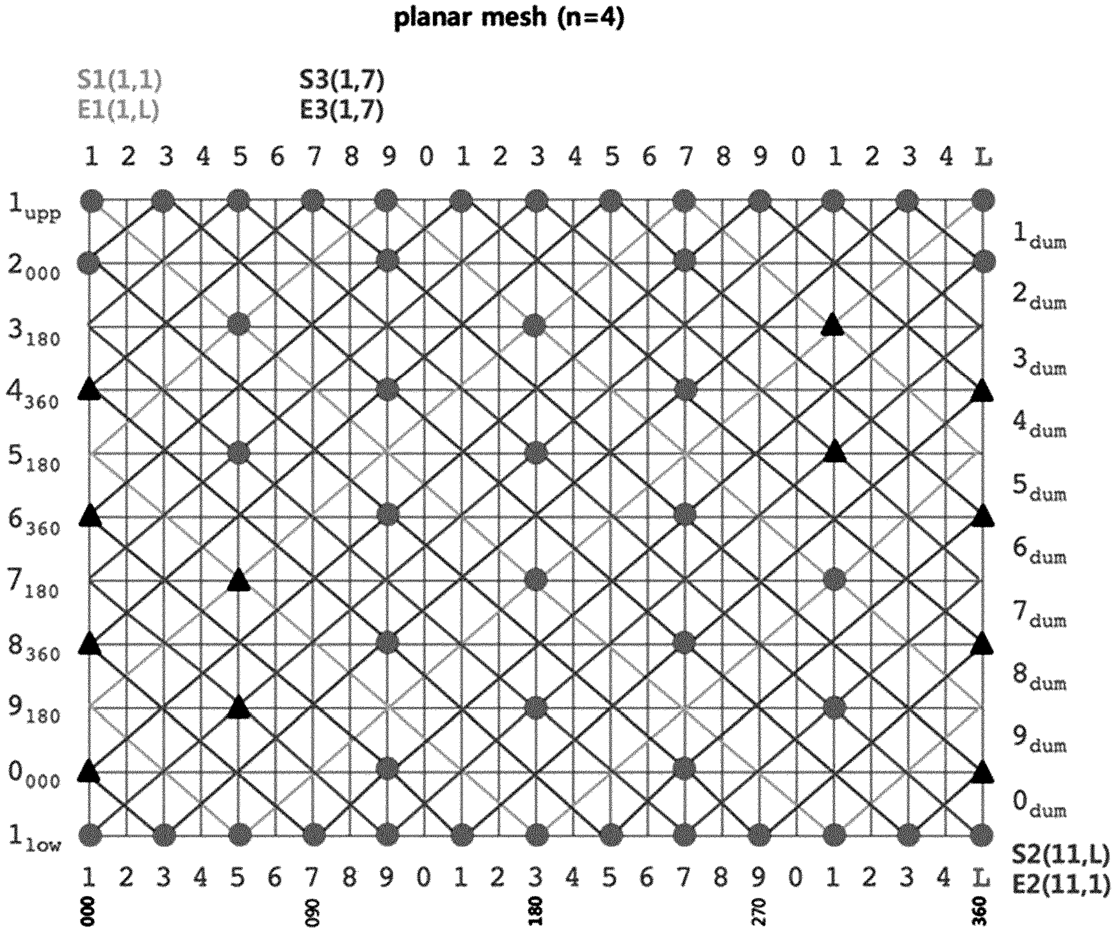

FIG. 86A

**NAME OF PATTERN : REG-TRIA-90-L-12**

**primitive planar matrix formula**

$$1_{000}\ \begin{bmatrix} H-C-C-C-H-C-C-C-H-C-C-C-H \\ C-C-C-H-C-C-C-H-C-C-C-H-C \\ C-C-H-C-C-C-H-C-C-C-H-C-C \\ C-H-C-C-C-H-C-C-C-H-C-C-C \end{bmatrix}^{1}$$

```
1000  H-C-C-C-H-C-C-C-H-C-C-C-H
2090  C-C-C-H-C-C-C-H-C-C-C-H-C
3180  C-C-H-C-C-C-H-C-C-C-H-C-C
4270  C-H-C-C-C-H-C-C-C-H-C-C-C
      1234567890123456789012 34L
      000   090   180   270   360
```

**1)** **$CV_{N.h.horiz}$**

horiz.node 1 2 3 4

N.h(#) 3 3 3 3

MV = 3

SD = 0

CV = 0

**2)** **$CV_{N.h.vert}$**

vert.node 1 - 3 - 5 - 7 - 9 - 1 - 3 - 5 - 7 - 9 - 1 - 3 -

N.h(#) 1 - 1 - 1 - 1 - 1 - 1 - 1 - 1 - 1 - 1 - 1 - 1 -

MV = 1

SD = 0

CV = 0

**3)** **$CV_{\Delta.h.horiz}$**

| horiz.node | Δ.h.horiz (separated by comma) | MV | SD | CV |
|---|---|---|---|---|
| 1 | 7, 7, 7 | 7 | 0 | 0 |
| 2 | 7, 7, 7 | 7 | 0 | 0 |
| 3 | 7, 7, 7 | 7 | 0 | 0 |
| 4 | 7, 7, 7 | 7 | 0 | 0 |

ΣCV = 0

ΣCV/N.lay = 0

**3)** **$Q_{symm}$**

$Q_{symm} = 1 - (CV_{N.h.horiz} + CV_{N.h.vert} + CV_{\Delta.h.horiz})/3 = 1 - 0/3 = 1$

FIG. 86B

**NAME OF PATTERN : REG-TRIA-180-L-12**

**primitive planar matrix formula**

```
       ┌                                        ┐ 2
1000   │ H-C-C-C-H-C-C-C-H-C-C-C-H              │
2180   │ C-C-H-C-C-C-H-C-C-C-H-C-C              │
       └                                        ┘ C
         1234567890123456789012 34L
         000      090      180      270      360
```

⟹

```
1000   ┌ H-C-C-C-H-C-C-C-H-C-C-C-H ┐
2180   │ C-C-H-C-C-C-H-C-C-C-H-C-C │
3000   │ H-C-C-C-H-C-C-C-H-C-C-C-H │
4180   │ C-C-H-C-C-C-H-C-C-C-H-C-C │
5000   └ H-C-C-C-H-C-C-C-H-C-C-C-H ┘
         1234567890123456789012 34L
         000      090      180      270      360
```

**1)** $CV_{N.h.horiz}$

```
horiz.node   1 2 3 4 5
N.h(#)       3 3 3 3 3
MV = 3
SD = 0
CV = 0
```

**2)** $CV_{N.h.vert}$

```
vert.node    1 - 3 - 5 - 7 - 9 - 1 - 3 - 5 - 7 - 9 - 1 - 3 -
N.h(#)       3 - 0 - 2 - 0 - 3 - 0 - 2 - 0 - 3 - 0 - 2 - 0 -
MV = 1.25
SD = 1.36
CV = 1.09
```

**3)** $CV_{\Delta.h.horiz}$

| horiz.node | Δ.h.horiz (separated by comma) | MV | SD | CV |
|---|---|---|---|---|
| 1 | 7, 7, 7 | 7 | 0 | 0 |
| 2 | 7, 7, 7 | 7 | 0 | 0 |
| 3 | 7, 7, 7 | 7 | 0 | 0 |
| 4 | 7, 7, 7 | 7 | 0 | 0 |
| 5 | 7, 7, 7 | 7 | 0 | 0 |

ΣCV = 0

ΣCV/N.lay = 0

**3)** $Q_{symm}$

$Q_{symm} = 1 - (CV_{N.h.horiz} + CV_{N.h.vert} + CV_{\Delta.h.horiz})/3 = 1 - 1.09/3 = 1 - 0.36 = 0.64$

**NAME OF PATTERN : REG-TRIA-90-L-12**

**primitive planar matrix formula**

$$1_{000}\ \text{H-C-C-C-H-C-C-C-H-C-C-C-H}$$
$$2_{090}\ \text{C-C-C-H-C-C-C-H-C-C-C-H-C}$$
$$3_{180}\ \text{C-C-H-C-C-C-H-C-C-C-H-C-C}$$
$$4_{270}\ \text{C-H-C-C-C-H-C-C-C-H-C-C-C}$$

1234567890123456789012345L

000 090 180 270 360

1) $N_{h.radial}$

$n = N.h.radial = 12$

2) $Q_{circ.simple}$

$Q_{circ.simple} = n \times \sin(180/n)/\pi$

$= 12 \times \sin(180/12)/\pi$

$= 0.989$

3) $CV_{N.h.vert}$

| | |
|---|---|
| vert.node | 1 - 3 - 5 - 7 - 9 - 1 - 3 - 5 - 7 - 9 - 1 - 3 - |
| N.h(#) | 1 - 1 - 1 - 1 - 1 - 1 - 1 - 1 - 1 - 1 - 1 - 1 - |

MV = 1

SD = 0

CV = 0

4) $CV_{\Delta.h.radial}$

| | | | | | | | | | | | |
|---|---|---|---|---|---|---|---|---|---|---|---|
| pair.vert.node | 1-3 | 3-5 | 5-7 | 7-9 | 9-1 | 1-3 | 3-5 | 5-7 | 7-9 | 9-1 | 1-3 |
| Δ.h.radial | 1 | 1 | 1 | 1 | 1 | 1 | 1 | 1 | 1 | 1 | 1 |

MV = 1

SD = 0

CV = 0

5) $CV_{avg}$

$CV_{avg} = (CV_{N.h.vert} + CV_{\Delta.h.radial})/2 = 0/2 = 0$

6) $Q_{circ}$

$Q_{circ} = Q_{circ.simple} \times (1 - CV_{avg}) = 0.989 \times (1 - 0) = 0.989$

FIG. 87A

**NAME OF PATTERN : REG-TRIA-180-L-12**

**primitive planar matrix formula**

```
1000 [ H-C-C-C-H-C-C-C-H-C-C-C-H ] 2
2180 [ C-C-H-C-C-C-H-C-C-C-H-C-C ] c
       1234567890123456789012345L
       000    090    180    270    360
```

⇨

```
1000 [ H-C-C-C-H-C-C-C-H-C-C-C-H ]
2180 [ C-C-H-C-C-C-H-C-C-C-H-C-C ]
3000 [ H-C-C-C-H-C-C-C-H-C-C-C-H ]
4180 [ C-C-H-C-C-C-H-C-C-C-H-C-C ]
5000 [ H-C-C-C-H-C-C-C-H-C-C-C-H ]
       1234567890123456789012345L
       000    090    180    270    360
```

1) $N_{h.radial}$

n = N.h.radial = 12

2) $Q_{circ.simple}$

$Q_{circ.simple} = n \times \sin(180/n)/\pi = 12 \times \sin(180/12)/\pi = 0.989$

3) $CV_{N.h.vert}$

vert.node 1 - 3 - 5 - 7 - 9 - 1 - 3 - 5 - 7 - 9 - 1 - 3 -

N.h(#) 3 - 0 - 2 - 0 - 3 - 0 - 2 - 0 - 3 - 0 - 2 - 0 -

MV = 1.25

SD = 1.36

CV = 1.09

4) $CV_{\Delta.h.radial}$

| pair.vert.node | 1-3 | 3-5 | 5-7 | 7-9 | 9-1 | 1-3 | 3-5 | 5-7 | 7-9 | 9-1 | 1-3 |
|---|---|---|---|---|---|---|---|---|---|---|---|
| Δ.h.radial | 3 | 3 | 3 | 3 | 3 | 3 | 3 | 3 | 3 | 3 | 3 |

MV = 3

SD = 0

CV = 0

5) $CV_{avg}$

$CV_{avg} = (CV_{N.h.vert} + CV_{\Delta.h.radial})/2 = (1.09 + 0)/2 = 0.545$

6) $Q_{circ}$

$Q_{circ} = Q_{circ.simple} \times (1 - CV_{avg}) = 0.989 \times (1 - 0.545) = 0.449$

FIG. 87B

**NAME OF PATTERNING: REG-TRIA-15-R-COMB-12**

**POLICY OF PATTERNING**

| | |
|---|---|
| primitive n-gon: | REGULAR TRIANGLE |
| rotation angle: | 15° |
| rotation direction: | RIGHT (clockwise, right to left on planar matrix) |
| skip: | NO |
| combination: | YES |
| combination item(type): | ROTATION ANGLE (PARAMETER) , (0,R) (45,R) (60,R) (105,R) |
| outburst: | NO |
| loop type: | OPEN |

**PARAMETER OF DESIGN**

no. of angle division: 4(24)
no. of primitive section layer: 4
no. of hook node per layer(h): 3
no. of cross node on side: 3
no. of cross node per layer(c): 9
no. of null node per layer(n): 12
total no. of node(h+c+n): 24
no. of vertical node line: 24L
angle division per vertical node line: 15°
width of horizontal node line: EQUAL
height of vertical node line: EQUAL
exponent of primitive node pattern: 2

axial view

**primitive planar matrix formula**

$1_{000}$ H-C-C-C-H-C-C-C-H-C-C-C-H
$2_{045}$ -C-C-H-C-C-C-H-C-C-C-H-C-
$3_{060}$ C-C-H-C-C-C-H-C-C-C-H-C-C
$4_{105}$ -H-C-C-C-H-C-C-C-H-C-C-C-
(exponent 2)

1234567890123456789 01234L

360 270 180 105 090 060 045 000

**interface node pattern**

**upper**

[-H-H-H-H-H-H-H-H-H-H-H-H-]

**lower**

[H-H-H-H-H-H-H-H-H-H-H-H-H]

FIG. 88A

# NAME OF PATTERNING: REG-TRIA-15-R-COMB-12

## planar matrix

```
        12345678901234567890 1234L
1upp  [ H-H-H-H-H-H-H-H-H-H-H-H-H ]
2000  [ H-C-C-C-H-C-C-C-H-C-C-C-H ]
3045  [ -C-C-H-C-C-C-H-C-C-C-H-C- ]
4060  [ C-C-H-C-C-C-H-C-C-C-H-C-C ]
5105  [ -H-C-C-C-H-C-C-C-H-C-C-C- ]
6000  [ H-C-C-C-H-C-C-C-H-C-C-C-H ]
7045  [ -C-C-H-C-C-C-H-C-C-C-H-C- ]
8060  [ C-C-H-C-C-C-H-C-C-C-H-C-C ]
9105  [ -H-C-C-C-H-C-C-C-H-C-C-C- ]
0low  [ H-H-H-H-H-H-H-H-H-H-H-H-H ]
        12345678901234567890 1234L
        360  270  180  105 090 060 045 000
```

## fabrication expression (n=2)

S1(1,2)
(4,5) (3,6) (6,9) (5,10) (8,13) (7,14) (10,17) (9,18)
(10,19) (8,21) (10,23) (4,5) (9,10) (2,17) (7,12)
(1,4) (3,6) (1,8) (2,9) (1,10) (4,13) (3,14) (6,17)
(5,18) (8,21) (7,22) (10,L) (9,2) (10,3) (8,5) (10,7)
(4,13) (9,18) (2,L) (7,6) (1,12) (3,14) (1,16) (2,17)
(1,18) (4,21) (3,22) (6,L) (5,2) (8,5) (7,6) (10,9)
(9,10) (10,11) (8,13) (10,15) (4,21) (9,2) (2,9)
(7,14) (1,20) (3,22) (1,14) (2,L)
E1(1,2)

S2(10,21)
$(i, -4j+21)_{i=6\to 10\to 6,\ j=1 \text{ to } 5}$
E2(10,21)

S3(1,22)
$(i, 4j-2)_{i=5\to 1\to 5,\ j=1 \text{ to } 5}$
E3(1,22)

FIG. 88B

FIG. 88C

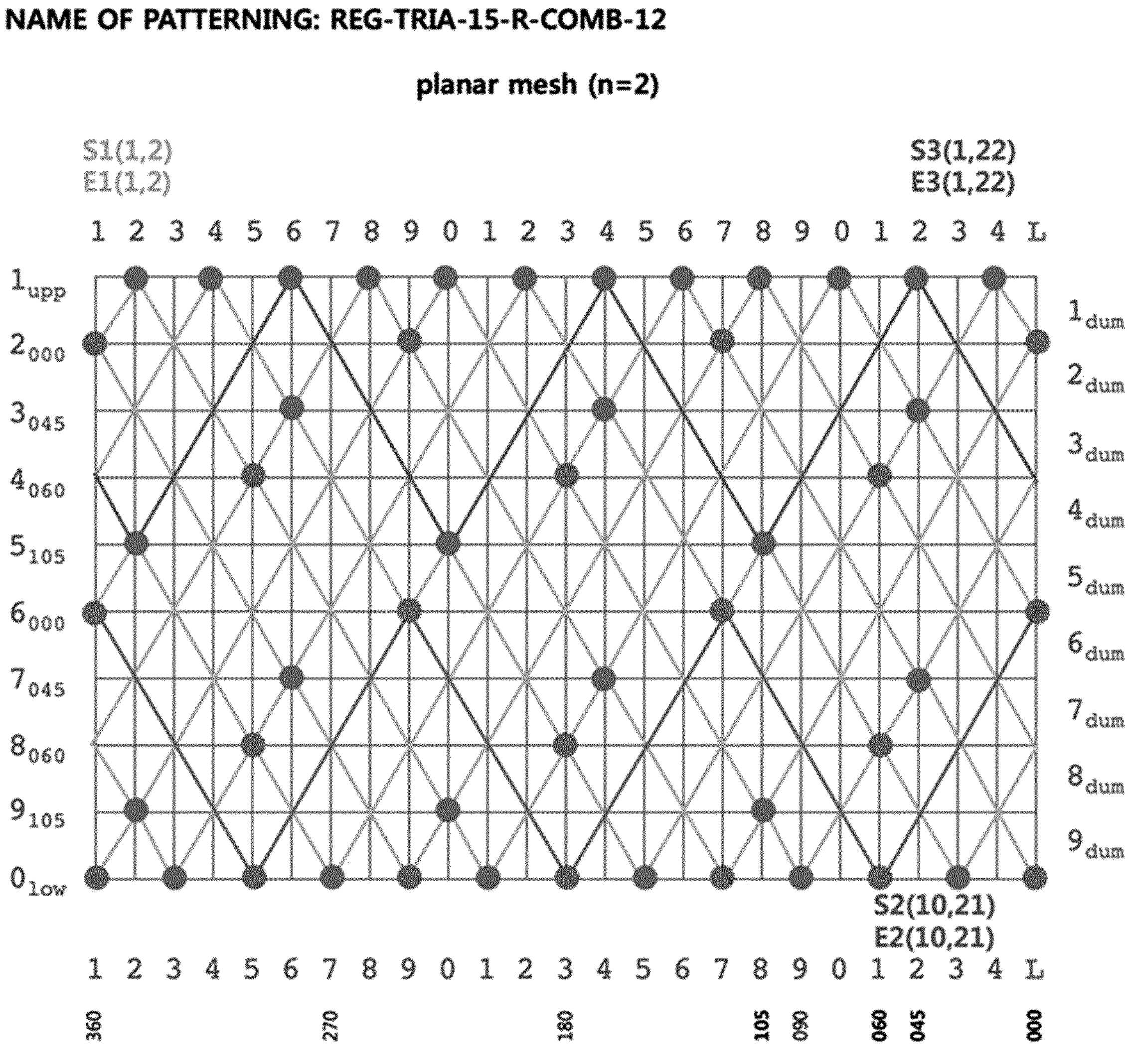
NAME OF PATTERNING: REG-TRIA-15-R-COMB-12
planar mesh (n=2)
S1(1,2)
E1(1,2)
S3(1,22)
E3(1,22)
1 2 3 4 5 6 7 8 9 0 1 2 3 4 5 6 7 8 9 0 1 2 3 4 L
1upp
2000
3045
4060
5105
6000
7045
8060
9105
0low
1dum
2dum
3dum
4dum
5dum
6dum
7dum
8dum
9dum
S2(10,21)
E2(10,21)
1 2 3 4 5 6 7 8 9 0 1 2 3 4 5 6 7 8 9 0 1 2 3 4 L
360
270
180
105
090
060
045
000

**NAME OF PATTERNING: REG-TETR-18-R-SKIP-20**

**POLICY OF PATTERNING**

| | |
|---|---|
| primitive n-gon: | REGULAR TETRAGON |
| rotation angle: | 18° |
| rotation direction: | RIGHT (clockwise, right to left on planar matrix) , (0,R) (18,R) (36,R) (54,R) (72,R) |
| skip: | YES |
| skip type: | MISSING |
| combination: | NO |
| outburst: | NO |
| loop type: | OPEN |

**PARAMETER OF DESIGN**

no. of angle division: 5(20)
no. of primitive section layer: 5
no. of hook node per layer(h): 4
no. of cross node on side: 4
no. of cross node per layer(c): 16
no. of null node per layer(n): 20
total no. of node(h+c+n): 40
no. of vertical node line: 40L
angle division per vertical node line: 9°
section layer of missing: 3rd layer
width of horizontal node line: EQUAL
height of vertical node line: EQUAL
exponent of primitive node pattern: 2

axial view
⇩
**primitive planar matrix formula**

```
           [                                         ] 2
1 000      H-C-C-C-C-H-C-C-C-C-H-C-C-C-C-H-C-C-C-C-H
2 018      C-C-C-C-H-C-C-C-C-H-C-C-C-C-H-C-C-C-C-H-C
3 036      C-C-C-C-C-C-C-C-C-C-C-C-C-C-C-C-C-C-C-C-C    3rd layer of section for missing skip
4 054      C-C-H-C-C-C-C-H-C-C-C-C-H-C-C-C-C-H-C-C-C
5 072      C-H-C-C-C-C-H-C-C-C-C-H-C-C-C-C-H-C-C-C-C
           1234567890123456789012345678901234567890L
           360  315  270  225  180  135  090 072 054 036 018 000
```

FIG. 89A

**NAME OF PATTERNING: REG-TETR-18-R-SKIP-20**

**interface node pattern**

**upper**

[H-H-H-H-H-H-H-H-H-H-H-H-H-H-H-H-H-H-H-H-H]

**lower**

[H-H-H-H-H-H-H-H-H-H-H-H-H-H-H-H-H-H-H-H-H]

**planar matrix**

```
       12345678901234567890L
1upp   H-H-H-H-H-H-H-H-H-H-H-H-H-H-H-H-H-H-H-H-H
2000   H-C-C-C-C-H-C-C-C-C-H-C-C-C-C-H-C-C-C-C-H
3018   C-C-C-C-H-C-C-C-C-H-C-C-C-C-H-C-C-C-C-H-C
4036   C-C-C-C-C-C-C-C-C-C-C-C-C-C-C-C-C-C-C-C-C
5054   C-C-H-C-C-C-C-H-C-C-C-C-H-C-C-C-C-H-C-C-C
6072   C-H-C-C-C-C-H-C-C-C-C-H-C-C-C-C-H-C-C-C-C
7000   H-C-C-C-C-H-C-C-C-C-H-C-C-C-C-H-C-C-C-C-H
8018   C-C-C-C-H-C-C-C-C-H-C-C-C-C-H-C-C-C-C-H-C
9036   C-C-C-C-C-C-C-C-C-C-C-C-C-C-C-C-C-C-C-C-C
0054   C-C-H-C-C-C-C-H-C-C-C-C-H-C-C-C-C-H-C-C-C
1072   C-H-C-C-C-C-H-C-C-C-C-H-C-C-C-C-H-C-C-C-C
2low   H-H-H-H-H-H-H-H-H-H-H-H-H-H-H-H-H-H-H-H-H
       12345678901234567890L
       360 315 270 225 180 135 090 072 054 036 018 000
```

**fabrication expression (n=2)**

S1(1,5)
(3,9) (2,11) (7,21) (6,23) (11,33) (12,39) (1,21) (12,3)
(1,25) (3,29) (2,31) (7,L) (6,3) (11,13) (10,15) (12,19)
(1,L) (12,23)
E1(1,5)

**S2(1,15)**
(3,19) (2,21) (7,31) (6,33) (11,3) (10,5) (12,9) (1,31)
(12,13) (1,35)
(3,39) (2,L) (7,11) (6,13) (11,23) (10,25) (12,29) (1,11)
(12,33)
**E2(1,15)**

**S3(1,3)**
(6,13) (5,15) (10,25) (8,29) (12,37) (1,19) (2,21) (1,23)
(6,33) (5,35) (10,5) (8,9) (12,17) (1,39) (2,L)
**E3(1,3)**

**S4(1,27)**
(5,35) (3,39) (8,9) (7,11) (12,21) (11,23) (12,25) (1,7)
(5,15) (3,19) (8,29) (7,31) (12,L) (11,3) (12,5)
**E4(1,27)**

S5(1,37)
(5,5) (3,9) (8,19) (7,21) (12,31) (11,33) (12,35) (1,17)
(5,25) (3,29) (8,39) (7,L) (12,11) (11,13) (12,15)
E5(1,37)

**S6(1,9)**
(2,11) (1,13) (6,23) (5,25) (10,35) (8,39) (12,7) (1,29)
(2,31) (1,33) (6,3) (5,5) (10,15) (8,19) (12,27)
**E6(1,9)**

FIG. 89B

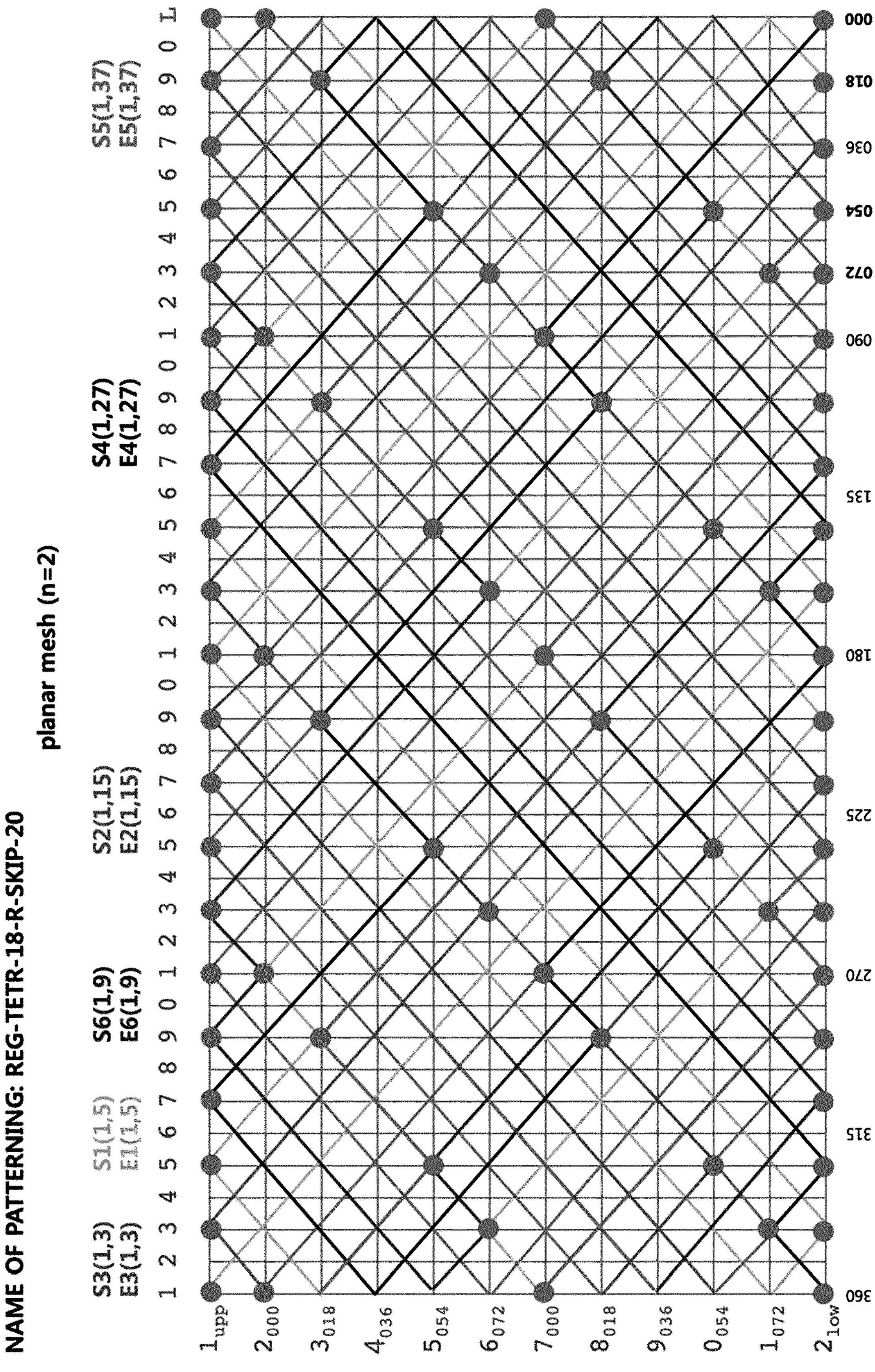
NAME OF PATTERNING: REG-TETR-18-R-SKIP-20
planar mesh (n=2)
S3(1,3)
E3(1,3)
S1(1,5)
E1(1,5)
S6(1,9)
E6(1,9)
S2(1,15)
E2(1,15)
S4(1,27)
E4(1,27)
S5(1,37)
E5(1,37)
000
018
036
054
072
090
135
180
225
270
315
360

FIG. 89C

**NAME OF PATTERNING: REG-TETR-45-RL-SKIP-20**

**POLICY OF PATTERNING**

| | |
|---|---|
| primitive n-gon: | REGULAR TETRAGON |
| rotation angle: | 45° |
| rotation direction: | RIGHT-LEFT BY TURNS<br>: (0,R) (45,L) |
| skip: | YES |
| skip type: | PUSHING |
| combination: | NO |
| outburst: | NO |
| loop type: | CLOSE |

**PARAMETER OF DESIGN**

no. of angle division: 1(8)
no. of primitive section layer: 3
no. of hook node per layer(h): 4
no. of cross node on side: 4
no. of cross node per layer(c): 16
no. of null node per layer(n): 20
total no. of node(h+c+n): 40
angle division per vertical node line: 9°(lcm(90,45))
no. of vertical node line: 40L
no. of dummy node line: 0
section layer of pushing: 3rd layer
width of horizontal node line: EQUAL
height of vertical node line: EQUAL
exponent of primitive node pattern: 3

axial view

⇩

**primitive planar matrix formula**

```
1000 [ H-C-C-C-C-H-C-C-C-C-H-C-C-C-C-H-C-C-C-C-H ] 3
2045 [ -C-C-H-C-C-C-C-H-C-C-C-C-H-C-C-C-C-H-C-C- ]
3skp [ --------------------------------------- ] C
       1234567890123456789012345678901234567890L
       360  315  270  225  180  135  090  045  000
```

3rd layer of section for pushing skip

FIG. 90A

**NAME OF PATTERNING: REG-TETR-45-RL-SKIP-20**

**interface node pattern**

**upper**

```
[H-H-H-H-H-H-H-H-H-H-H-H-H-H-H-H-H-H-H-H-H]
```

**lower**

```
[H-H-H-H-H-H-H-H-H-H-H-H-H-H-H-H-H-H-H-H-H]
```

**planar matrix**

```
        1234567890123456789012345678901234567890L
1upp    H-H-H-H-H-H-H-H-H-H-H-H-H-H-H-H-H-H-H-H-H
2000    H-C-C-C-C-H-C-C-C-C-H-C-C-C-C-H-C-C-C-C-H
3045    -C-C-H-C-C-C-C-H-C-C-C-C-H-C-C-C-C-H-C-C-
4skp    -----------------------------------------
5000    H-C-C-C-C-H-C-C-C-C-H-C-C-C-C-H-C-C-C-C-H
6045    -C-C-H-C-C-C-C-H-C-C-C-C-H-C-C-C-C-H-C-C-
7skp    -----------------------------------------
8000    H-C-C-C-C-H-C-C-C-C-H-C-C-C-C-H-C-C-C-C-H
9045    -C-C-H-C-C-C-C-H-C-C-C-C-H-C-C-C-C-H-C-C-
0skp    -----------------------------------------
1000    H-C-C-C-C-H-C-C-C-C-H-C-C-C-C-H-C-C-C-C-H
2low    H-H-H-H-H-H-H-H-H-H-H-H-H-H-H-H-H-H-H-H-H
        1234567890123456789012345678901234567890L
        360      315     270      225     180      135     090     045     000
```

**fabrication expression (n=3)**

S1(1,1)
(i, 5j+1) $_{i=3->1->3,\ j=1\ to\ 6}$
$(3,36)_V$
(i, -5j+36) $_{i=5->3->5,\ j=1\ to\ 6}$
$(5,1)_V$ (12,19) (11,21) (12,23) $(5,L)_V$ $(3,36)_V$
E1(1,L)

**S2(1,15)**
(9,26) (1,7) (12,35) (2,21) (12,7) (1,25) (9,16) (1,37)
(12,25) (2,11) (12,37) (1,25) (9,6) (1,27) (2,15) (2,L)
(12,27) (1,15) (9,36) (1,17) (1,25) (2,31) (12,17)
**E2(1,15)**

**S3(1,9)**
(2,11) (1,13) (6,26) (1,39) (2,L) (1,3) (6,16) (1,29)
(2,31) (1,33) (6,6) (1,19) (2,21) (1,23) $(6,36)_V$
(8,31) (6,26) (8,21) (6,16) (8,11) (6,6) $(8,1)_V$
(12,11) (8,21) (12,31) $(8,L)_V$ $(6,36)_V$
**E3(1,9)**

**S4(12,13)**
$(11,11)_V$
(i, 5j+6) $_{i=9->11->9,\ j=1\ to\ 5}$
$(11,L)_V$ (12,39) (5,21) (12,3) $(11,1)_V$
(9,6) $(11,11)_V$
(12,9) (5,31)
**E4(12,13)**

**S5(12,21)**
(8,11) (12,1) (8,31)
**E5(12,21)**

**S6(12,33)**
(11,31) (12,29) (5,11)
**E6(12,33)**

FIG. 90B

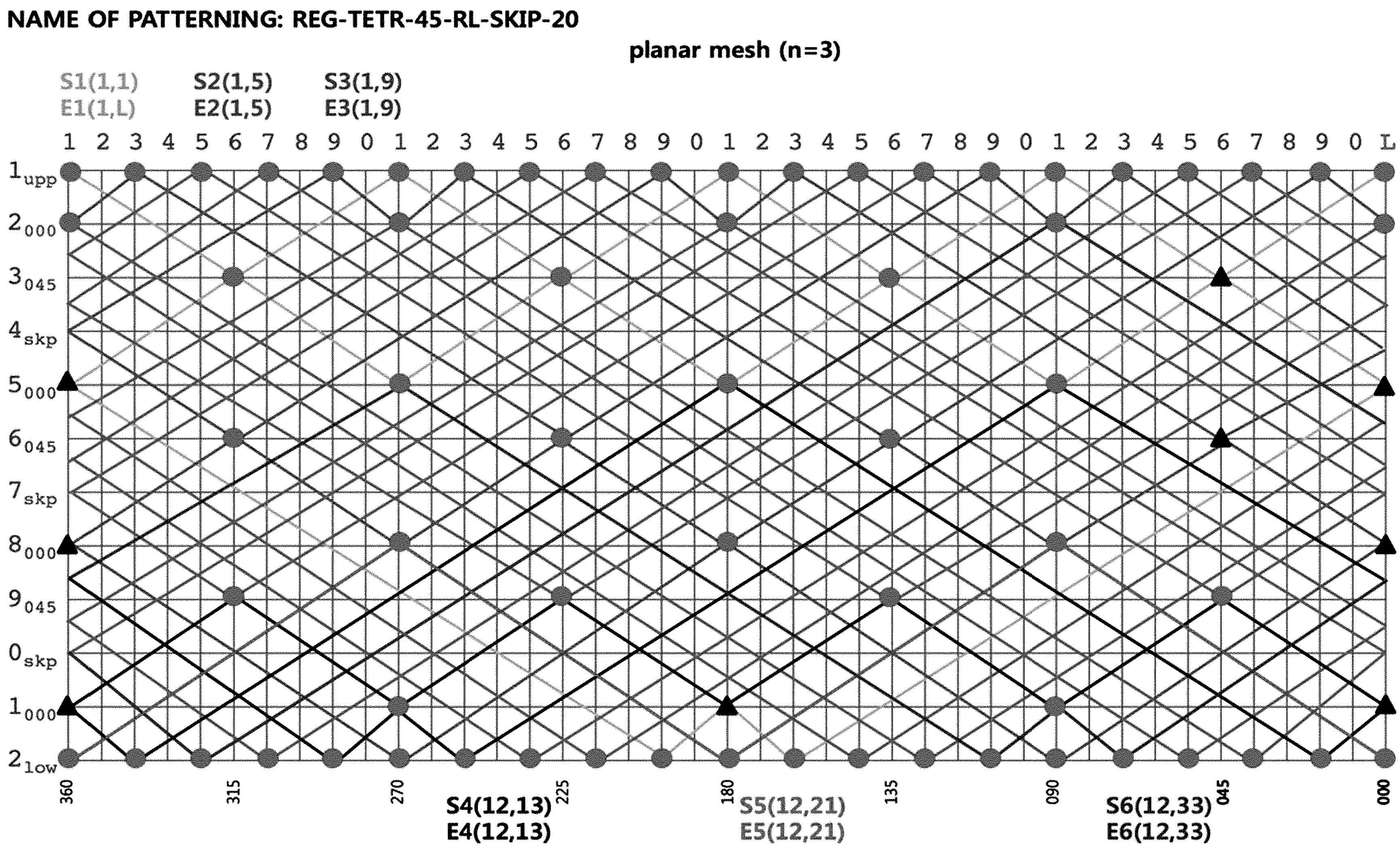
NAME OF PATTERNING: REG-TETR-45-RL-SKIP-20
planar mesh (n=3)
S1(1,1) E1(1,L)
S2(1,5) E2(1,5)
S3(1,9) E3(1,9)
1 2 3 4 5 6 7 8 9 0 1 2 3 4 5 6 7 8 9 0 1 2 3 4 5 6 7 8 9 0 1 2 3 4 5 6 7 8 9 0 L
1upp
2000
3045
4skp
5000
6045
7skp
8000
9045
0skp
1000
2low
360
315
270
225
180
135
090
045
000
S4(12,13) E4(12,13)
S5(12,21) E5(12,21)
S6(12,33) E6(12,33)

FIG. 90C

**NAME OF PATTERNING: REG-PENT-27-RL-COMB-20**

**POLICY OF PATTERNING**

primitive n-gon: REGULAR PENTAGON
rotation angle: 27°
rotation direction: RIGHT-LEFT BY TURNS
, (0,R) (27,R) (54,R) (81,R) (108,R) $|_{R\text{->}L}$
skip: NO
combination: YES
combination item(type): ROTAION DIRECTION (PARAMETER)
outburst: NO
loop type: OPEN

**PARAMETER OF DESIGN**

no. of angle division: 4(13)
no. of primitive section layer: 10
no. of hook node per layer(h): 5
no. of cross node on side: 3
no. of cross node per layer(c): 15
no. of null node per layer(n): 20
total no. of node(h+c+n): 40
no. of vertical node line: 40L
angle division per vertical node line: 9°
width of horizontal node line: EQUAL
height of vertical node line: EQUAL
exponent of primitive node pattern: 1

**primitive planar matrix formula**

```
     1_000 [ H-C-C-C-H-C-C-C-H-C-C-C-H-C-C-C-H-C-C-C-H ] 1
     2_027   -C-C-H-C-C-C-H-C-C-C-H-C-C-C-H-C-C-C-H-C-
R    3_054   C-H-C-C-C-H-C-C-C-H-C-C-C-H-C-C-C-H-C-C-C
     4_081   -C-C-C-H-C-C-C-H-C-C-C-H-C-C-C-H-C-C-C-H-
     5_108   C-C-H-C-C-C-H-C-C-C-H-C-C-C-H-C-C-C-H-C-C
     6_000   H-C-C-C-H-C-C-C-H-C-C-C-H-C-C-C-H-C-C-C-H
     7_027   -C-H-C-C-C-H-C-C-C-H-C-C-C-H-C-C-C-H-C-C-
L    8_054   C-C-C-H-C-C-C-H-C-C-C-H-C-C-C-H-C-C-C-H-C
     9_081   -H-C-C-C-H-C-C-C-H-C-C-C-H-C-C-C-H-C-C-C-
     0_108 [ C-C-H-C-C-C-H-C-C-C-H-C-C-C-H-C-C-C-H-C-C ]

           1234567890123456789012345678901234567890L
R          360 315 270 225 180 108 081 054 027 000
L          000 027 054 081 108 245 180 225 270 000
```

FIG. 91A

**NAME OF PATTERNING: REG-PENT-27-RL-COMB-20**

**interface node pattern**

**upper**

```
[-H-H-H-H-H-H-H-H-H-H-H-H-H-H-H-H-H-H-H-H-]
```

**middle**

```
[-C-C-C-C-C-C-C-C-C-C-C-C-C-C-C-C-C-C-C-C-]
```

**lower**

```
[-H-H-H-H-H-H-H-H-H-H-H-H-H-H-H-H-H-H-H-H-]
```

**planar matrix**

```
              1234567890123456789012345678901234567890L
    1upp     [-H-H-H-H-H-H-H-H-H-H-H-H-H-H-H-H-H-H-H-H-
  ┌ 2000      H-C-C-C-H-C-C-C-H-C-C-C-H-C-C-C-H-C-C-C-H
  │ 3027      -C-C-H-C-C-C-H-C-C-C-H-C-C-C-H-C-C-C-H-C-
R │ 4054      C-H-C-C-C-H-C-C-C-H-C-C-C-H-C-C-C-H-C-C-C
  │ 5081      -C-C-C-H-C-C-C-H-C-C-C-H-C-C-C-H-C-C-C-H-
  └ 6108      C-C-H-C-C-C-H-C-C-C-H-C-C-C-H-C-C-C-H-C-C
    7mid      -C-C-C-C-C-C-C-C-C-C-C-C-C-C-C-C-C-C-C-C-
  ┌ 8000      H-C-C-C-H-C-C-C-H-C-C-C-H-C-C-C-H-C-C-C-H
  │ 9027      -C-H-C-C-C-H-C-C-C-H-C-C-C-H-C-C-C-H-C-C-
L │ 0054      C-C-C-H-C-C-C-H-C-C-C-H-C-C-C-H-C-C-C-H-C
  │ 1081      -H-C-C-C-H-C-C-C-H-C-C-C-H-C-C-C-H-C-C-C-
  └ 2108      C-C-H-C-C-C-H-C-C-C-H-C-C-C-H-C-C-C-H-C-C
    3low      -H-H-H-H-H-H-H-H-H-H-H-H-H-H-H-H-H-H-H-H-]
              1234567890123456789012345678901234567890L
           R  360 315 270 225 180 108 081 054 027 000
           L  000 027 054 081 108 245 180 225 270 360
```

**fabrication expression (n=1)**

S1(1,4)
(3,6) (1,8) (2,9) (1,10) (8,17) (3,22) (5,24) (1,28)
(3,30) (1,32) (2,33) (1,34) (8,2) (3,6) (5,8) (1,12)
(3,14) (1,16) (2,17) (1,18) (8,25) (3,30) (5,32) (1,36)
(3,38) (1,40) (2,L) (1,2) (8,9) (3,14) (5,16) (1,20)
(3,22) (1,24) (2,25) (1,26) (5,33) (3,38) (5,40)
E1(1,4)

S2(1,14)
(10,23) (8,25) (12,29) (10,31) (13,34) (4,3) (6,5) (2,9) $(4,11)_V$
(13,2) (10,39) (12,37) (8,33) (10,31) (1,22) (4,19) (2,17)
$(6,13)_V$ (11,18) (9,20) (13,24) (11,26) (13,28) (12,29) (13,30)
(6,37) (11,2) (9,4) (13,8) (11,10) (13,12) (12,13) (13,14)
(6,21) (11,26) (9,28) (13,32) (11,34) (13,36) (12,37) (13,38)
(6,5) (11,10) (9,12) (13,16) (11,18) (13,20) (12,21) (13,22)
(6,29) (11,34) (9,36) (13,40) (11,2) (13,4) (12,5) (13,6)
$(6,13)_V$, $(4,11)_V$
E2(1,14)

S3(1,30)
(10,39) (8,L) (12,5) (13,10) (4,19) (6,21) (2,25) $(4,27)_V$ (13,18)
(10,15) (12,13) (8,9) (10,7) (1,38) (4,35) (2,33) (6,29) $(4,27)_V$
E3(1,30)

**S4(13,26)**
(10,23) (12,21) (8,17) (10,15) (1,6) (4,3) (2,1) (6,37) (4,35)
**E4(13,26)**

S5(5,40)
(i, -4j+40) i=9->5->9, j=1 to 9
E5(5,40)

FIG. 91B

NAME OF PATTERNING: REG-PENT-27-RL-COMB-20

planar mesh (n=1)

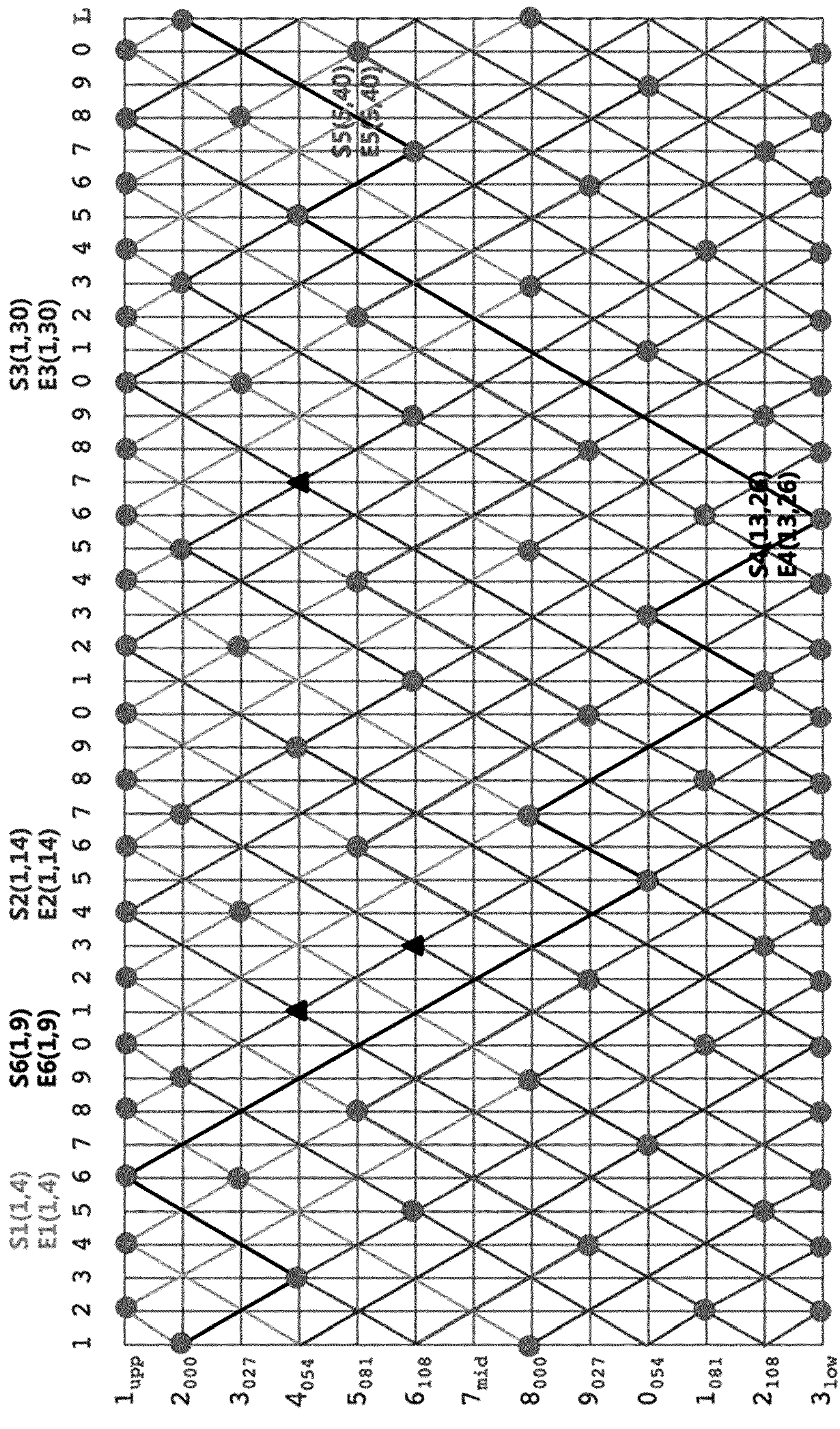


FIG. 91C

**NAME OF PATTERNING: REG-TRPH-0-L-COMB-12**

**POLICY OF PATTERNING**

| | |
|---|---|
| primitive n-gon: | REGULAR<br>TRIANGLE, RECTANGULAR<br>PENTAGON, HEXAGON |
| rotation angle: | 0° |
| rotation direction: | LEFT |
| skip: | NO |
| combination: | YES |
| combination item(type): | N-GON, CROSS (PARAMETER) |
| outburst: | NO |
| loop type: | OPEN |

**PARAMETER OF DESIGN**

no. of angle division: 0(0)
no. of primitive section layer: 4
angle division per vertical node line: 6° (lcm(120,90,72,60))
no. of vertical node line: 60L

no. of hook node on 3-gon per layer($h_3$): 3
no. of cross node on 3-gon side: 3
no. of cross node of 3-gon per layer($c_3$): 9
no. of null node of 3-gon per layer($n_3$): 12
total no. of node of 3-gon per layer($h_3+c_3+n_3$): 24

**PARAMETER OF DESIGN(cont.)**

no. of hook node on 4-gon per layer($h_4$): 4
no. of cross node on 4-gon side: 2
no. of cross node of 4-gon per layer($c_4$): 8
no. of null node of 4-gon per layer($n_4$): 12
total no. of node of 4-gon per layer($h_4+c_4+n_4$): 24

no. of hook node on 5-gon per layer($h_5$): 5
no. of cross node on 5-gon side: 1 or 2
no. of cross node of 5-gon per layer($c_5$): 7
no. of null node of 5-gon per layer($n_5$): 12
total no. of node of 5-gon per layer($h_5+c_5+n_5$): 24

no. of hook node on 6-gon per layer($h_6$): 6
no. of cross node on 6-gon side: 1
no. of cross node of 6-gon per layer($c_6$): 6
no. of null node of 6-gon per layer($n_6$): 12
total no. of node of 6-gon per layer($h_6+c_6+n_6$): 24

no. of dummy node per layer($n_d$): 36
no. of common superposed dummy node: 2($n_d$/n-1
)
width of horizontal node line: EQUAL
height of vertical node line: EQUAL
exponent of primitive node pattern: 2

FIG. 92A

NAME OF PATTERNING: REG-TRPH-0-L-COMB-12

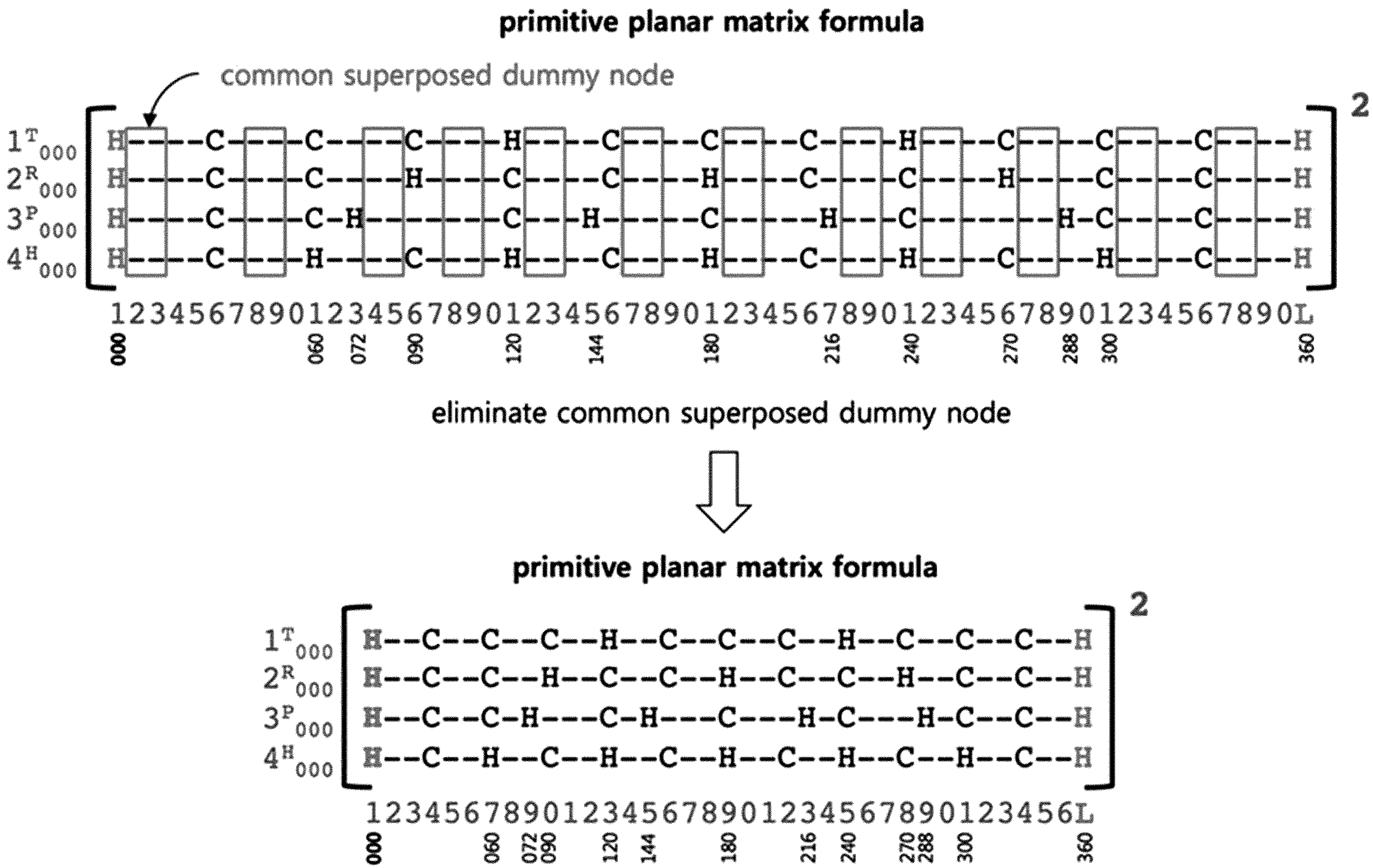


FIG. 92B

## NAME OF PATTERNING: REG-TRPH-0-L-COMB-12

### interface node pattern

**upper**

```
[H--H--H--H--H--H--H--H--H--H--H--H--H]
```

**lower**

```
[H--H--H--H--H--H--H--H--H--H--H--H--H]
```

### planar matrix

```
              1234567890123456789012345678901234556L
1^I_upp   [H--H--H--H--H--H--H--H--H--H--H--H--H
2^T_000    H--C--C--C--H--C--C--C--H--C--C--C--H
3^R_000    H--C--C--H--C--C--H--C--C--H--C--C--H
4^P_000    H--C--C-H---C-H---C---H-C---H-C--C--H
5^H_000    H--C--H--C--H--C--H--C--H--C--H--C--H
6^T_000    H--C--C--C--H--C--C--C--H--C--C--C--H
7^R_000    H--C--C--H--C--C--H--C--C--H--C--C--H
8^P_000    H--C--C-H---C-H---C---H-C---H-C--C--H
9^H_000    H--C--H--C--H--C--H--C--H--C--H--C--H
0^I_low    H--H--H--H--H--H--H--H--H--H--H--H--H]
              1234567890123456789012345678901234556L
              000 060 072 090 120 144 180 216 240 270 288 300 360
```

### fabrication expression (n=2)

S1(1,1)
(4,9) (1,19) $(4,29)_V$
(5,25) (4,23) (5,19) (4,15) (5,13) (4,9) (5,7) (3,1) (5,31) (7,L)
(5,7) (8,15) (7,9) $(8,23)_V$
(9.19) (8,5) (9,13) (8,9) $(9,7)_V$
(i, 3j+7) $_{i=10\text{->}9\text{->}10,\ j=1\ \text{to}\ 9}$
(9,L) (10,4) $(9,7)_V$
(7,1) (9,31) (8,29) (9,25) $(8,23)_V$ $(5,31)_V$ $(4,29)_V$
E1(1,L)

S2(10,31)
(3,10) (6,1) (3,28) (10,7) $(8,1)_V$
(1,22) (2,25) (1,28) (4,L) (1,10) $(2,13)_V$
(3,10) (1,4) (2,1) (1,34) (3,28) (2,25) (6,13) (2,1) (6,25) $(2,13)_V$
(1,16) $(8,L)_V$
E2(10,31)

S3(1,13)
$(3,19)_V$
(4,15) (1,7) (3,1) (1,31) (4,23) (3,19) (1,25) $(5,L)_V$
(8,29) $(5,1)_V$
E3(1,13)

**S4(10,25)**
(6,13) (7,10) (4,1) (7,28) (6,25) (10,13) (6,1)
**E4(10,25)**

S5(9,1)
(5,13) (7,19) (5,25)
E5(9,L)

FIG. 92C

FIG. 92D

**NAME OF PATTERNING: REG-TRPH-0-L-COMB-12**

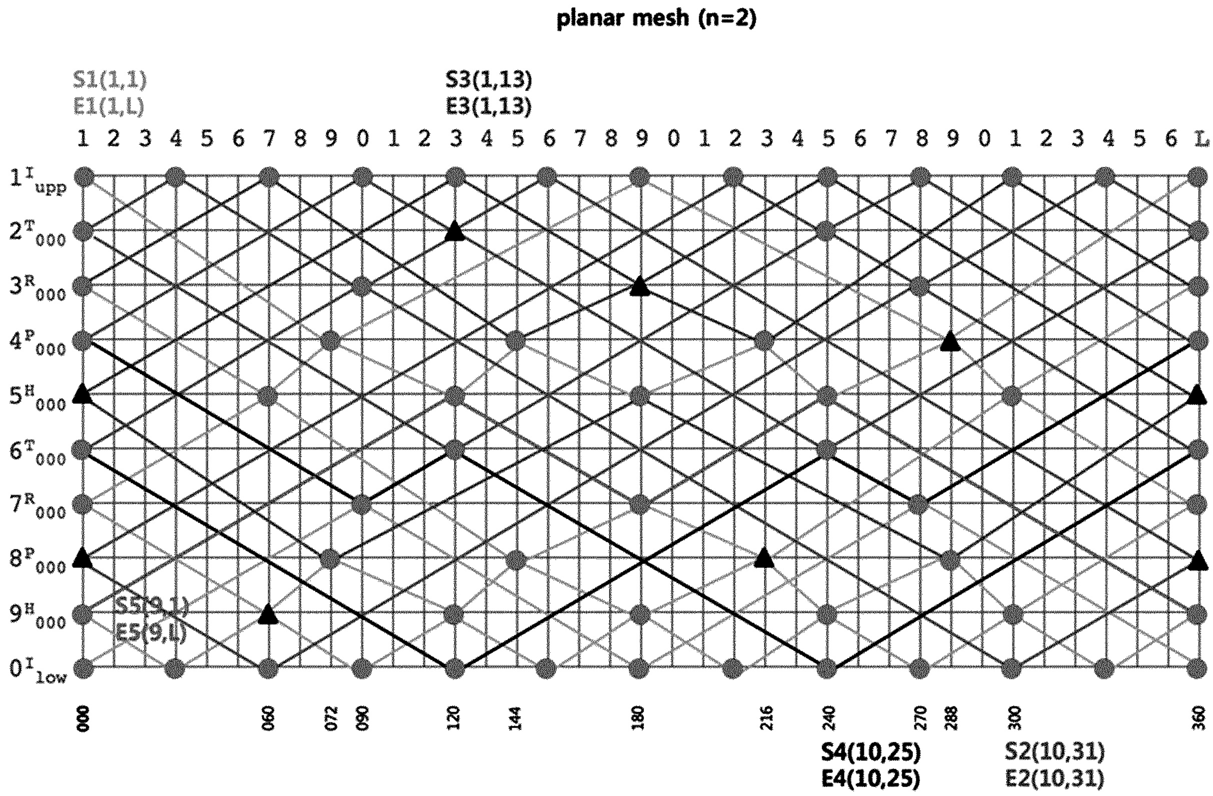

FIG. 93A

## NAME OF PATTERNING: REG-TRPH-0-L-COMB-SYMM-12

### POLICY OF PATTERNING

| | |
|---|---|
| primitive n-gon: | REGULAR<br>TRIANGLE, TETRAGON<br>PENTAGON, HEXAGON |
| rotation angle: | 0° |
| rotation direction: | LEFT |
| skip: | NO |
| combination: | YES |
| combination item(type): | N-GON, CROSS (PARAMETER)<br>MATRIX FORMULA$^{-1}$ (PATTERN) |
| outburst: | NO |
| loop type: | OPEN |

### PARAMETER OF DESIGN

THE SAME AS MODEL OF REG-TRPH-0-L-COMB

**primitive planar matrix formula**

$$\begin{bmatrix} \text{H--C--C--C--H--C--C--C--H--C--C--C--H} \\ \text{H--C--C--H--C--C--H--C--C--H--C--C--H} \\ \text{H--C--C-H---C-H---C---H-C---H-C--C--H} \\ \text{H--C--H--C--H--C--H--C--H--C--H--C--H} \\ \text{H--C--H--C--H--C--H--C--H--C--H--C--H} \\ \text{H--C--C-H---C-H---C---H-C---H-C--C--H} \\ \text{H--C--C--H--C--C--H--C--C--H--C--C--H} \\ \text{H--C--C--C--H--C--C--C--H--C--C--C--H} \end{bmatrix}^{1}$$

| Row | |
|---|---|
| $1^{T}_{000}$ | H--C--C--C--H--C--C--C--H--C--C--C--H |
| $2^{R}_{000}$ | H--C--C--H--C--C--H--C--C--H--C--C--H |
| $3^{P}_{000}$ | H--C--C-H---C-H---C---H-C---H-C--C--H |
| $4^{H}_{000}$ | H--C--H--C--H--C--H--C--H--C--H--C--H |
| $1^{H}_{000}$ | H--C--H--C--H--C--H--C--H--C--H--C--H |
| $2^{P}_{000}$ | H--C--C-H---C-H---C---H-C---H-C--C--H |
| $3^{R}_{000}$ | H--C--C--H--C--C--H--C--C--H--C--C--H |
| $4^{T}_{000}$ | H--C--C--C--H--C--C--C--H--C--C--C--H |

1234567890123456789012345678901234567890123456L

000 060 072 090 120 144 180 216 240 270 288 300 360

NAME OF PATTERNING: REG-TRPH-0-L-COMB-SYMM-12

interface node pattern

upper

```
[H--H--H--H--H--H--H--H--H--H--H--H--H]
```

lower

```
[H--H--H--H--H--H--H--H--H--H--H--H--H]
```

planar matrix

```
          1234567890123456789012345678901234 56L
1 I upp  [H--H--H--H--H--H--H--H--H--H--H--H--H]
2 T 000  [H--C--C--C--H--C--C--C--H--C--C--C--H]
3 R 000  [H--C--C--H--C--C--H--C--C--H--C--C--H]
4 P 000  [H--C--C-H---C-H---C---H-C---H-C--C--H]
5 H 000  [H--C--H--C--H--C--H--C--H--C--H--C--H]
6 H 000  [H--C--H--C--H--C--H--C--H--C--H--C--H]
7 P 000  [H--C--C-H---C-H---C---H-C---H-C--C--H]
8 R 000  [H--C--C--H--C--C--H--C--C--H--C--C--H]
9 T 000  [H--C--C--C--H--C--C--C--H--C--C--C--H]
0 I low  [H--H--H--H--H--H--H--H--H--H--H--H--H]
          1234567890123456789012345678901234 56L
          000 060 072 090 120 144 180 216 240 270 288 300 360
```

fabrication expression (n=1)

S1(1,1)
(4,9) (1,19) (4,29)$_V$
(5,25) (4,23) (5,19) (4,15) (5,13) (4,9) (5,7) (3,1)$_V$
(1,7) (4,15) (3,19) (4,23) (1,31) (3,L)$_V$ (5,31)$_V$
(7,L) (5,7) (10,22) (9,25) (10,28) (7,L) (10,10) (9,13) (10,16)
(5,31)$_V$ (4,29)$_V$
E1(1,L)

S2(10,L)
(7,29) (10,19) (7,9)$_V$
(6,13) (7,15) (6,19) (7,23) (6,25) (7,29) (6,31) (8,L) (6,7)$_V$
(4,1) (6,31) (1,16) (2,13) (1,10) (4,1) (1,28) (2,25) (1,22)
(6,7)$_V$ (7,9)$_V$
E2(10,1)

S3(1,13)
(3,19) (1,25) (5,L)$_V$
(8,28) (5,19) (8,10)$_V$
(9,13) (5,25) (9,L) (5,13) (9,25) (8,28) (10,34) (9,L) (10,4)
(8,10)$_V$ (5,1)$_V$
E3(1,13)

S4(10,7)
(8,1) (10,31) (7,23) (8,19)$_V$ (10,25) (6,L)$_V$ (3,28) (6,19) (3,10)
(6,1)$_V$ (10,13) (8,19)$_V$ (7,15)
E4(10,7)

S5(1,34)
(2,L)$_V$ (6,25) (2,13) (3,10) (1,4) (2,1)$_V$ (6,13) (2,25) (3,28)
E5(1,34)

FIG. 93B

FIG. 93C

**NAME OF PATTERNING: REG-TRPH-0-L-COMB-SYMM-12**

**planar mesh (n=1)**

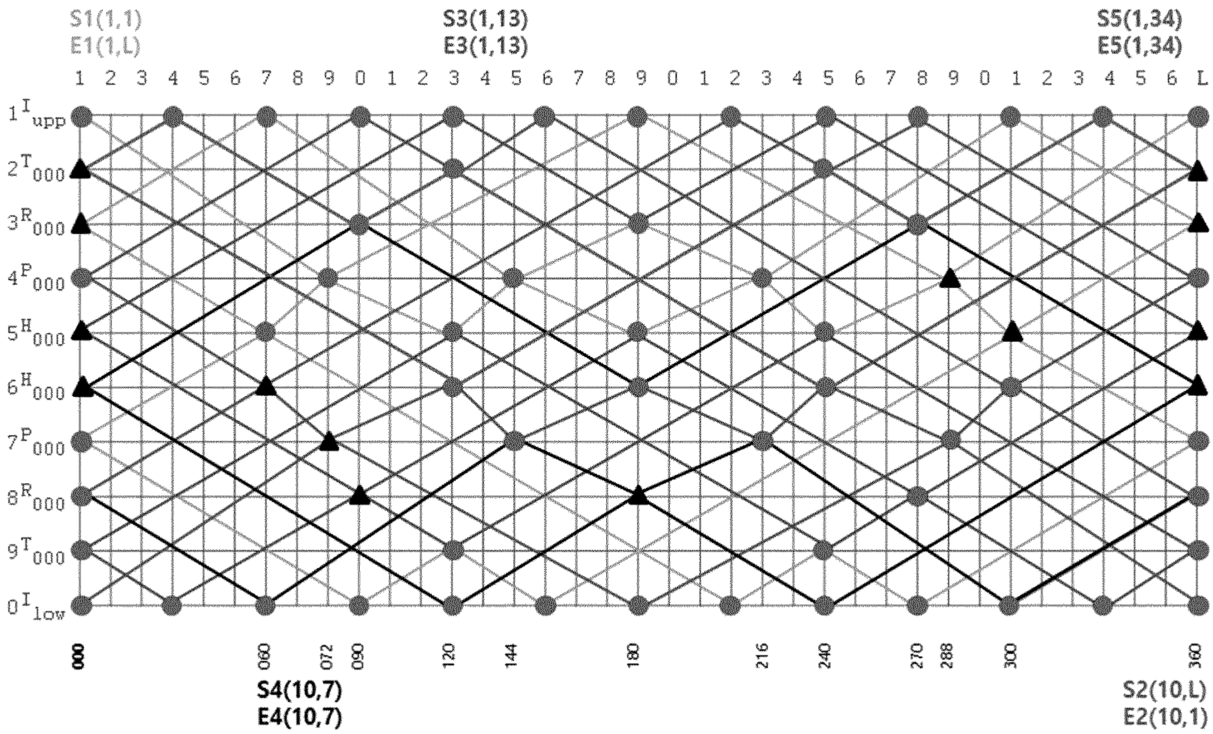

**NAME OF PATTERNING: REG-HXTR-30/90-LR/L-COMB-PATN-18/12**

**POLICY OF PATTERNING**

| | |
|---|---|
| primitive n-gon: | REGULAR HEXAGON, TRIANGLE |
| rotation angle: | 30° (HEXAGON), 90° (TRIANGLE) |
| rotation direction: | LEFT-RIGHT BY TURNS (HEXAGON)<br>LEFT (TRIANGLE) |
| skip: | NO |
| combination: | YES |
| combination item(type): | MATRIX FORMULA (PATTERN)<br>[HEXAGON] +<br>[TRIANGLE] +<br>[HEXAGON] |
| outburst: | NO |
| loop type: | OPEN |

**PARAMETER OF DESIGN**

TRIANGLE PATTERN: SIMILAR OF REG-TRIA-90-L-12
HEXAGON PATTERN: REG-HEXA-30-LR-18
- no. of angle division: 2(12)
- no. of primitive section layer: 4
- no. of hook node per layer(h): 6
- no. of cross node on side: 2
- no. of cross node per layer(c): 12
- no. of null node per layer(n): 18
- total no. of node(h+c+n): 36
- no. of vertical node line: 36L
- angle division per vertical node line: 10°
- width of horizontal node line: EQUAL
- height of vertical node line: EQUAL
- exponent of primitive node pattern: 1

**primitive planar matrix formula of REG-TRIA-90-L-12**

```
1000 [ H-C-C-C-H-C-C-C-H-C-C-C-H ] 1
2090 [ C-C-C-H-C-C-C-H-C-C-C-H-C ]
3180 [ C-C-H-C-C-C-H-C-C-C-H-C-C ]
4270 [ C-H-C-C-C-H-C-C-C-H-C-C-C ]
       1234567890123456789012 34L
       000   090   180   270   360
```

**primitive planar matrix formula of REG-HEXA-30-LR-18**

```
1000 [ H-C-C-H-C-C-H-C-C-H-C-C-H-C-C-H-C-C-H ] 1
2030 [ -C-H-C-C-H-C-C-H-C-C-H-C-C-H-C-C-H-C- ]
3000 [ H-C-C-H-C-C-H-C-C-H-C-C-H-C-C-H-C-C-H ]
4030 [ -C-H-C-C-H-C-C-H-C-C-H-C-C-H-C-C-H-C- ]
       123456789012345678901234567890123456L
       000 030 060 120 180 240 300 360
```

FIG. 94A

FIG. 94B

**NAME OF PATTERNING: REG-HXTR-30/90-LR/L-COMB-PATN-18/12**

**interface node pattern**

**upper**

```
[-H-H-H-H-H-H-H-H-H-H-H-H-H-H-H-H-H-H-]
```

**lower**

```
[H-H-H-H-H-H-H-H-H-H-H-H-H-H-H-H-H-H-H]
```

**middle 1 & 2**

```
[H-----H-----H-----------H-----H-----H]
[---H-----H-----H--H--H-----H-----H---]
```

**primitive planar matrix**

```
           1234567890123456789012345678901234567L
1^I_upp    -H-H-H-H-H-H-H-H-H-H-H-H-H-H-H-H-H-H-
2^H_000    H-C-C-H-C-C-H-C-C-H-C-C-H-C-C-H-C-C-H
3^H_030    -C-H-C-C-H-C-C-H-C-C-H-C-C-H-C-C-H-C-
4^H_000    H-C-C-H-C-C-H-C-C-H-C-C-H-C-C-H-C-C-H
5^H_030    -C-h-C-C-h-C-C-h-C-C-h-C-C-h-C-C-h-C-
6^I_mid    H-----H-----H-----------H-----H-----H
7^I_mid    ---H-----H-----H--H--H-----H-----H---
8^T_000    H--C--C--C--H--C--C--C--H--C--C--C--H
9^T_090    C--C--C--H--C--C--C--H--C--C--C--H--C
0^T_180    C--C--H--C--C--C--H--C--C--C--H--C--C
1^T_270    C--H--C--C--C--H--C--C--C--H--C--C--C
2^I_mid    H-----H-----H-----------H-----H-----H
3^I_mid    ---H-----H-----H--H--H-----H-----H---
4^H_030    h-C-C-h-C-C-h-C-C-h-C-C-h-C-C-h-C-C-h
5^H_000    -C-H-C-C-H-C-C-H-C-C-H-C-C-H-C-C-H-C-
6^H_030    H-C-C-H-C-C-H-C-C-H-C-C-H-C-C-H-C-C-H
7^H_030    -C-H-C-C-H-C-C-H-C-C-H-C-C-H-C-C-H-C-
8^I_low    H-H-H-H-H-H-H-H-H-H-H-H-H-H-H-H-H-H-H
           1234567890123456789012345678901234567L
```

000 030 060 090 120 180 240 270 300 360

**fabrication expression (n=1)**

S1(1,2)
(3,4) (1,6) (2,7) (1,8) (3,10) (1,12) (2,13) (1,14) (3,16)
(1,18) (2,19) (1,20) (3,22) (1,24) (2,25) (1,26) (3,28)
(1,30) (2,31) (1,32) (3,34)V
(i, -3j+34) i=6->3->6,j=1 to 10
(6,1)V (13,16) (6,31) (13,10) (6,25) (13,4) (7,19) (13,34)
(6,13) (13,28) (6,7) (13,22)V
(i, -3j+22) i=16->13->16,j=1 to 7
(16,25)V (18,27) (17,28) (16,31) (18,33) (17,34) (18,35) (16,L)
(i, j+2) i=18->17->18->16->18,j=1 to 15
(16,25)V (6,L)V (3,34)V (1,36) (2,L)
E1(1,2)

S2(18,L)
(i, -3j+37) i=15->18->15,j=1 to 10
(15,4)V (12,7) (15,10) (12,13) (15,16) (13,19) (15,22)
(12,25) (15,28) (12,31) (15,34) (12,L)V
(10,11) (11,28) (9,22) (10,19) (8,13) (9,10) (7,4) (8,1)
(7,34) (13,19) (11,16) (12,13) (10,7) (11,4) (9,34) (10,31)
(8,25) (9,22) (7,16) (8,13) (7,10) (12,31) (11,28) (12,25)
(10,19) (11,16) (9,10) (10,7) (8,1) (9,34) (7,28) (8,25)
(7,22) (12,7) (11,4) (12,1)V (15,4)V
E2(18,1)

S3(2,L)
(i, -3j+37) i=5->2->5,j=1 to 11
E3(2,1)

**S4(14,L)**
(i, -3j+37) i=17->14->17,j=1 to 11
**E4(14,1)**

S5(1,4)
(i, 3j+4) i=4->1->4, j=1 to 10
(4,L)V
(i, 3j+4) i=7>4->7, j=1 to 11
(4,1)V
E5(1,4)

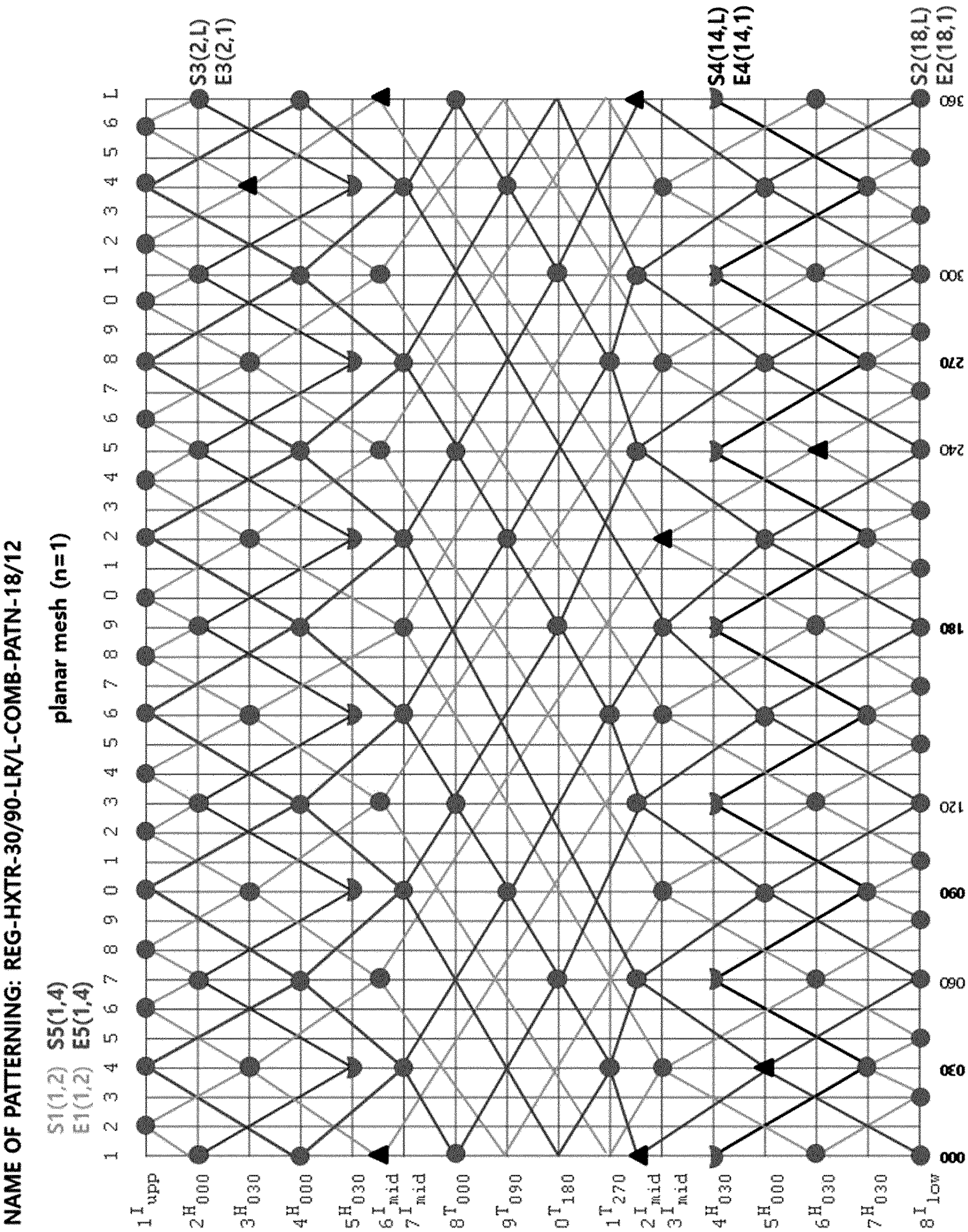
NAME OF PATTERNING: REG-HXTR-30/90-LR/L-COMB-PATN-18/12
planar mesh (n=1)
S1(1,2) S5(1,4)
E1(1,2) E5(1,4)
S3(2,L)
E3(2,1)
S4(14,L)
E4(14,1)
S2(18,L)
E2(18,1)
000 030 060 090 120 180 240 270 300 360

FIG. 94C

**NAME OF PATTERNING: IRR-TRPH-20-L-COMB-14**

**POLICY OF PATTERNING**

| | |
|---|---|
| primitive n-gon: | IRREGULAR |
| | TRIANGLE, RECTANGULAR |
| | PENTAGON, HEXAGON |
| rotation angle: | 20° |
| | TRI(0, 120, 240) |
| | REG(20, 100, 220, 340) |
| | PEN(40, 160, 200, 280, 320) |
| | HEX(60, 80, 140, 180, 260, 300) |
| rotation direction: | LEFT |
| skip: | NO |
| combination: YES | |
| combination item(type): | N-GON, CROSS (PARAMETER) |
| outburst: | NO |
| loop type: | OPEN |

**PARAMETER OF DESIGN**

no. of angle division: 18(18)
no. of primitive section layer: 8
angle division per vertical node line: 10°
no. of vertical node line: 36L

**PARAMETER OF DESIGN(cont.)**

no. of hook node on 3-gon per layer($h_3$): 3
no. of cross node of 3-gon per layer($c_3$): 11(full 7, half 8)
no. of null node of 3-gon per layer($n_3$): 22(full 18, half 8)
total no. of node of 3-gon per layer($h_3+c_3+n_3$): 36

no. of hook node on 4-gon per layer($h_4$): 4
no. of cross node of 4-gon per layer($c_4$): 10(full 9, half 2)
no. of null node of 4-gon per layer($n_4$): 22(full 21, half 2)
total no. of node of 4-gon per layer($h_4+c_4+n_4$): 36

no. of hook node on 5-gon per layer($h_5$): 5
no. of cross node of 5-gon per layer($c_5$): 9(full 6, half 6)
no. of null node of 5-gon per layer($n_5$): 22(full 19, half 6)
total no. of node of 5-gon per layer($h_5+c_5+n_5$): 36

no. of hook node on 6-gon per layer($h_6$): 6
no. of cross node of 6-gon per layer($c_6$): 8(full 6, half 4)
no. of null node of 6-gon per layer($n_6$): 22(full 20, half 4)
total no. of node of 6-gon per layer($h_6+c_6+n_6$): 36

width of horizontal node line: EQUAL
height of vertical node line: EQUAL
exponent of primitive node pattern: 1

FIG. 95A

FIG. 95B

**NAME OF PATTERNING: IRR-TRPH-20-L-COMB-14**

**primitive planar matrix formula**

12345678901234567890123456789012345 6L

1

$1^{T}_{000}$ H-c-C-c-C-c-H-c-C-c-C-c-H-C-C-c-C-c-H
$2^{R}_{000}$ --H-C-C---H-C-C---C-C-H-c-C---C-C-H--
$3^{P}_{000}$ C-c-H-c-C-c-C-c-H-c-H-C-C---H-C-H-c-C
$4^{H}_{000}$ C-C---H-H-C---H-C-H-c-C-c-H-c-H-c-C-C
$5^{T}_{000}$ H-c-C-c-C-c-H-c-C-C-C---H-C-C---C-C-H
$6^{R}_{000}$ c-H-C-C---H-C-C-c-C-c-H-c-C-c-C-c-H-c
$7^{P}_{000}$ C-C-H-c-C-c-C-C-H---H-C-C---H-C-H---C
$8^{H}_{000}$ c-C-c-H-H-C-c-H-c-H-c-C-c-H-c-H-c-C-c

123456789012345678901234567890123456L

000 060 120 180 240 300 360

**NAME OF PATTERNING: IRR-TRPH-20-L-COMB-14**

**interface node pattern**

**upper**

```
[H-H---H-H-H---H-H-H---H-H-H-H-H---H-H]
```

**lower**

```
[H---H-H-H-H-H---H-H-H---H-H-H---H-H-H]
```

**primitive planar matrix**

```
           123456789012345678901234567890123456L
1 I upp    H-H---H-H-H---H-H-H---H-H-H-H-H---H-H
2 T 000    H-c-C-c-C-c-H-c-C-c-C-c-H-C-C-c-C-c-H
3 R 000    --H-C-C---H-C-C---C-C-H-c-C---C-C-H--
4 P 000    C-c-H-c-C-c-C-c-H-c-H-C-C---H-C-H-c-C
5 H 000    C-C---H-H-C---H-C-H-c-C-c-H-c-H-c-C-C
6 T 000    H-c-C-c-C-c-H-c-C-C-C---H-C-C---C-C-H
7 R 000    c-H-C-C---H-C-C-c-C-c-H-c-C-c-C-c-H-c
8 P 000    C-C-H-c-C-c-C-C-H---H-C-C---H-C-H---C
9 H 000    c-C-c-H-H-C-c-H-c-H-c-C-c-H-c-H-c-C-c
0 I low    H---H-H-H-H-H---H-H-H---H-H-H---H-H-H
           123456789012345678901234567890123456L
           000          060          120          180          240          300          360
```

**fabrication expression (n=1)**

S1(1,1)
(5,9) (1,17) (10,35) (5,9) (10,19)
E1(1,L)

S2(1,3)
(6,13) (5,15) (8,21) (7,23) (9,27) (8,29) (9,31) (8,33)
(10,L) (8,5) (9,7) (7,11) (9,15) (8,17) (9,19) (8,21)
(10,25) (9,27) (10,29) (9,31) (10,33) (7,3) (8,5) (1,19)
(3,23) (2,25) (4,29) (1,35) $(2,L)_V$
(3,35) (1,31) (8,17) (6,13) (7,11) (5,7) (7,3) (6,1) (7,35)
(5,31) (10,21) (9,19) (10,17) (9,15) (10,13) (3,35) (4,33)
(1,27) (2,25) (1,23) (4,17) (2,13) (3,11) (1,7) (3,3) $(2,1)_V$
E2(1,3)

S3(1,19)
(10,27)
E3(1,19)

**S4(1,25)**
(2,13) (1,15) (4,21) (3,23) (5,27) (4,29) (5,31)
(4,33) (6,L) (4,5) (5,7) (3,11) (5,15) (4,17) (5,19)
(4,21) (6,25) (5,27) (8,33) (7,35) (10,5) (9,7) (10,9)
(5,19) (7,23) (6,25) (8,29) (3,3) (4,5)
**E4(1,25)**

S5(1,25)
(10,7) (9,9) (10,11) (1,29) (9,9)
E5(1,25)

FIG. 95C

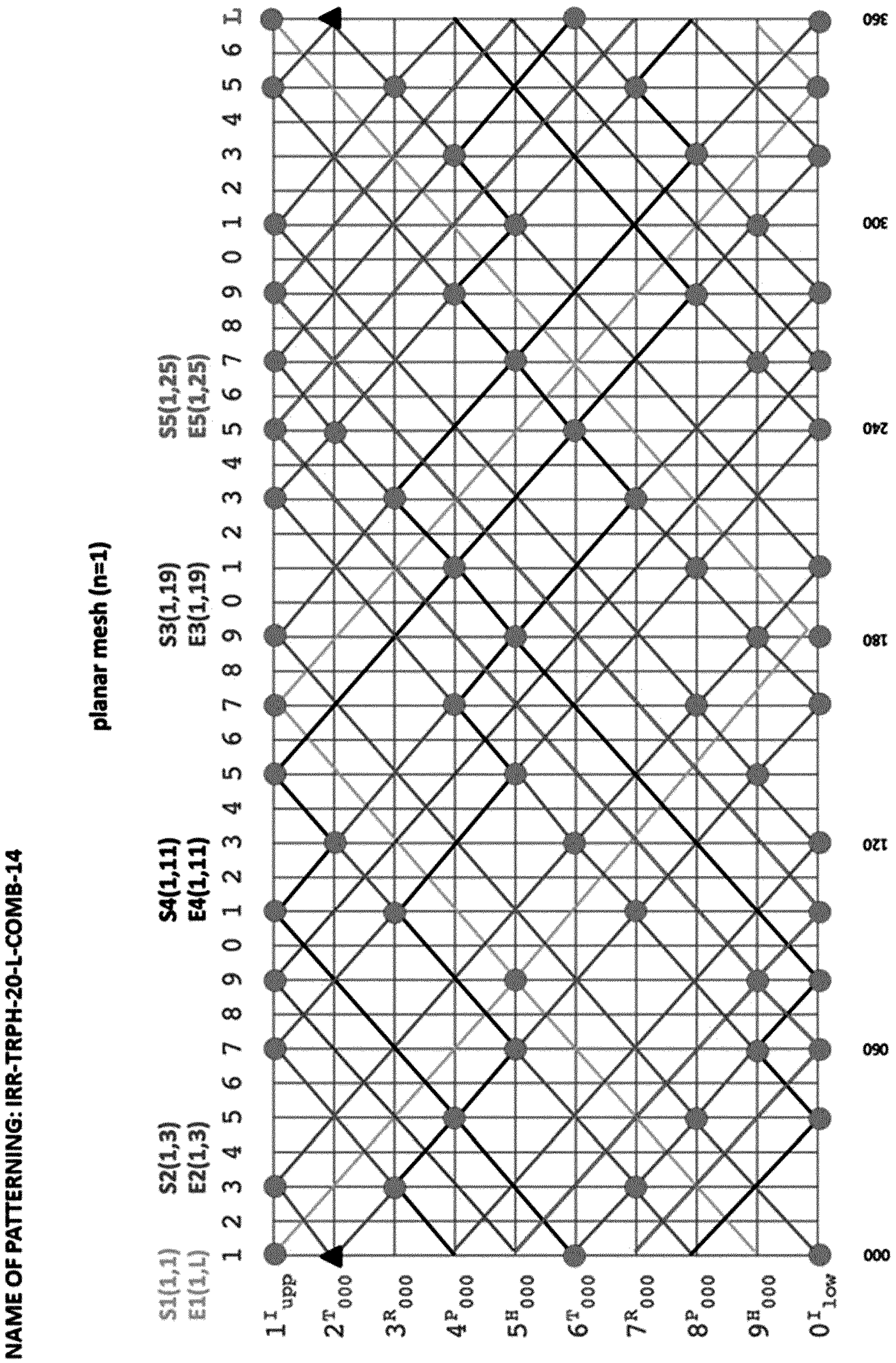
NAME OF PATTERNING: IRR-TRPH-20-L-COMB-14
planar mesh (n=1)
S1(1,1)
E1(1,L)
S2(1,3)
E2(1,3)
S4(1,11)
E4(1,11)
S3(1,19)
E3(1,19)
S5(1,25)
E5(1,25)
1 2 3 4 5 6 7 8 9 0 1 2 3 4 5 6 7 8 9 0 1 2 3 4 5 6 7 8 9 0 1 2 3 4 5 6 L
1 I upp
2 T 000
3 R 000
4 P 000
5 H 000
6 T 000
7 R 000
8 P 000
9 H 000
0 I low
000
060
120
180
240
300
360

FIG. 95D

FIG. 96A

**primitive planar matrix formula (A)**

```
        1    2    3    4    5    6    7    8   L08
        1   2   3   4   5   6   7   8   9   0   L10
        1  2  3  4  5  6  7  8  9  0  1  2  3  4  L14
                                                          1
1_08  [ H - H - H - H - H - H - H - H - H ]
2_08  [ C - H - H - H - C - C - C - H - C ]
3_10  [ C - C - h - C - H - C - H - H - h - H - C ]
4_14  [ C-H-h-H-C-h-H-C-C-h-C-C-h-C-C ]
5_14  [ C-C-C-H-C-H-C-H-C-H-C-C-C-C-C ]
6_14  [ H-C-h-H-C-h-C-C-C-h-C-H-h-C-H ]
7_10  [ H - C - h - H - H - C - H - C - h - C - H ]
8_08  [ C - H - C - H - C - H - C - H - C ]
        1  2  3  4  5  6  7  8  9  0  1  2  3  4  L14
        1   2   3   4   5   6   7   8   9   0   L10
        1    2    3    4    5    6    7    8   L08
```

FIG. 96B

**Reverse Symmetry Combination of Primitive Planar Matrix Formula A**

$$[A] + [A]^{-R} =$$

**planar matrix**

| | | |
|---|---|---|
| | 1 2 3 4 5 6 7 8 $L_{08}$ | |
| | 1 2 3 4 5 6 7 8 9 0 $L_{10}$ | |
| | 1 2 3 4 5 6 7 8 9 0 1 2 3 4 $L_{14}$ | |
| $1_{08}$ | H - H - H - H - H - H - H - H - H | |
| $2_{08}$ | C - H - H - H - C - C - C - H - C | |
| $3_{10}$ | C - C - h - C - H - C - H - H - h - H - C | |
| $4_{14}$ | C-H-h-H-C-h-H-C-C-h-C-C-h-C-C | |
| $5_{14}$ | C-C-C-H-C-H-C-H-C-H-C-C-C-C-C | |
| $6_{14}$ | H-C-h-H-C-h-C-C-C-h-C-H-h-C-H | |
| $7_{10}$ | H - C - h - H - H - C - H - C - h - C - H | |
| $8_{08}$ | C - H - C - H - C - H - C - H - C | $9_{08}$ |
| $9_{08}$ | C - H - C - H - C - H - C - H - C | $8_{08}$ |
| | H - C - h - C - H - C - H - H - h - C - H | $7_{10}$ |
| | H-C-h-H-C-h-C-C-C-h-C-H-h-C-H | $6_{14}$ |
| | C-C-C-C-C-H-C-H-C-H-C-H-C-C-C | $5_{14}$ |
| | C-C-h-C-C-h-C-C-H-h-C-H-h-H-C | $4_{14}$ |
| | C - H - h - H - H - C - H - C - h - C - C | $3_{10}$ |
| | C - H - C - C - C - H - H - H - C | $2_{08}$ |
| | H - H - H - H - H - H - H - H - H | $1_{08}$ |
| | $_{14}L$ 4 3 2 1 0 9 8 7 6 5 4 3 2 1 | |
| | $_{10}L$ 0 9 8 7 6 5 4 3 2 1 | |
| | $_{08}L$ 8 7 6 5 4 3 2 1 | |

**planar mesh**

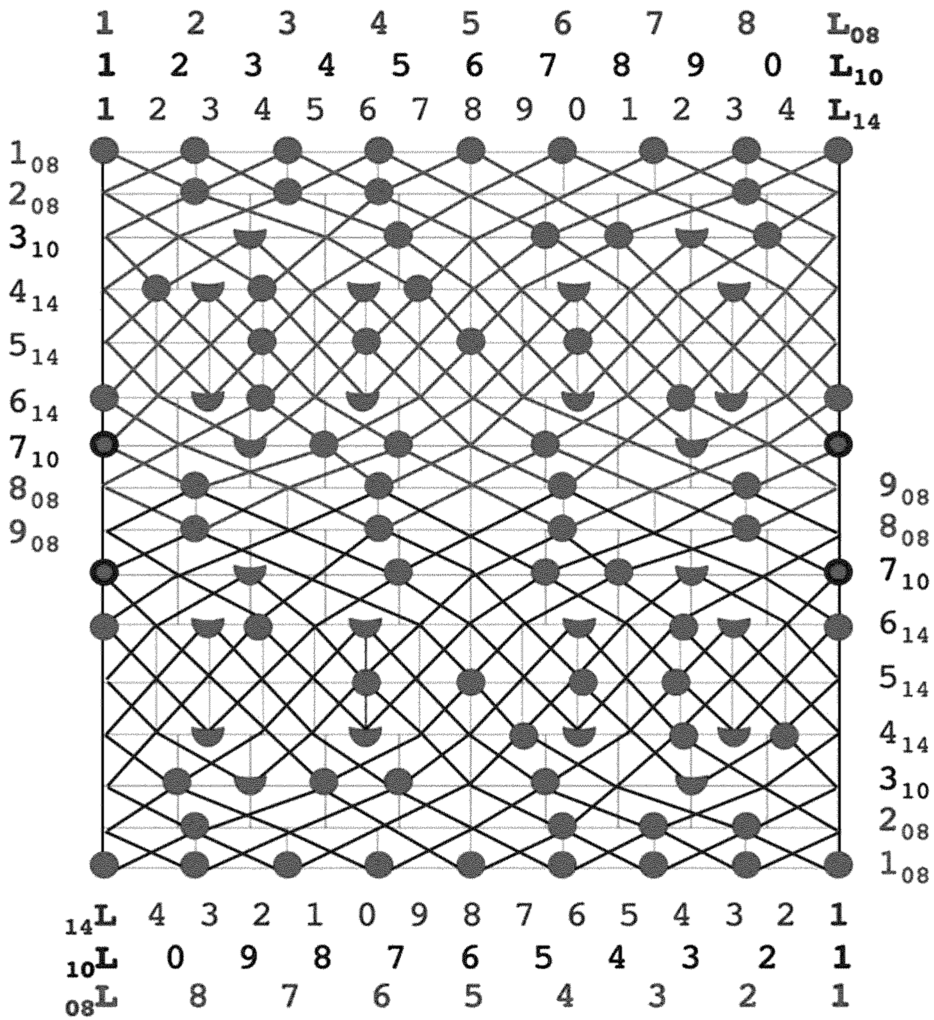


FIG. 96C

**fabrication expression (n=1)**

S1 $(7,L)_{10}$ & S2

$(3,8)_{10}$ (5,8) (4,7) (5,6) $(3,3)_{hh}$

(4,2) $(2,8)_{8}$ $(3,8)_{10}$ $(1,5)_{8}$ $(2,4)_{8}$ $(1,3)_{8}$ $(2,2)_{8}$ $(1,1)_{8}$

$(2,8)_{8}$ $(1,7)_{8}$ (4,7) $(2,2)_{8}$ (6,12) $(4,10)_{hh}$

$(7,5)_{10}$ (5,4) $(6,3)_{hh}$ $(3,10)_{10}$ $(6,10)_{hh}$

$(3,5)_{10}$ (5,4) $(4,3)_{hh}$ (6,1) $(4,13)_{hh}$

$(7,7)_{10}$ $(4,6)_{hh}$ (6,4) (4,2) $(7,9)_{10,hh}$

(5,10) $(8,4)_{8}$ $(7,4)_{10}$ $(8,2)_{8}$ $(7,1)_{10}$ $(8,8)_{8}$ (5,8)

$(7,4)_{10}$ (6,4) $(9,8)_{8}$ $(7,7)_{10}$ $(9,4)_{8}$ (6,1) $(9,6)_{8}$

$(7,5)_{10}$ $(9,2)_{8}$ (6,12) $(8,6)_{8}$ (5,6) $(7,3)_{10,hh}$ $(3,9)_{10,hh}$

(5,10) $(2,4)_{8}$ (4,4) $(1,8)_{8}$ $(3,7)_{10}$ $(1,4)_{8}$ $(2,3)_{8}$

$(1,2)_{8}$ $(3,10)_{10}$ $(1,6)_{8}$ $(3,5)_{10}$ $(2,3)_{8}$ $(6,13)_{hh}$

$(3,7)_{10}$ $(6,6)_{hh}$ (4,4)

E1 $(7,L)_{10}$ & S2

FIG. 97

## Node configuration of wired-mesh stent

| | Hook | Cross |
|---|---|---|
| **Height (side view)** | 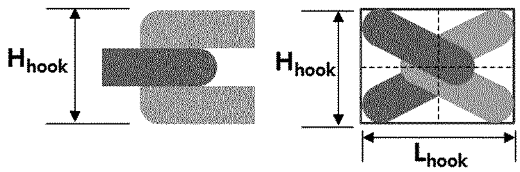 | 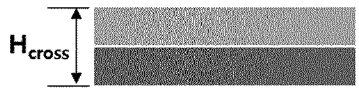 |
| Height scale factor ($SF_h$) | 3 | 2 |
| Nominal height | $H_{hook} = \Phi \times SF_{hh}$ | $H_{cross} = \Phi \times SF_{hc}$ |
| **Width & Length (top view)** | 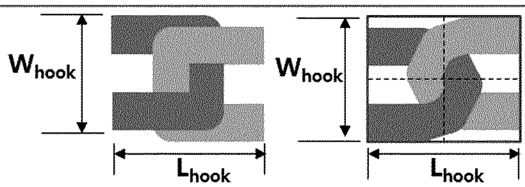 | 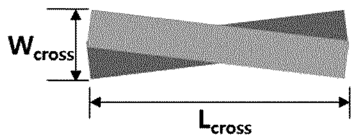 |
| Width scale factor ($SF_w$) | 3.3 | 2 |
| Nominal width | $W_{hook} = \Phi \times SF_{wh}$ | $W_{cross} = \Phi \times SF_{wc}$ |
| Length scale factor ($SF_l$) | 4 | 5 |
| Nominal length | $L_{hook} = \Phi \times SF_{lh}$ | $L_{cross} = \Phi \times SF_{lc}$ |
| **Nominal section** | 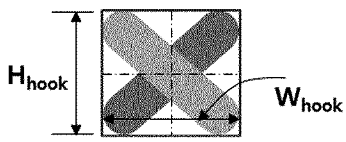 | 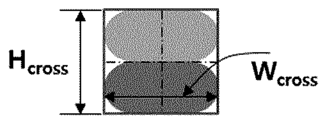 |

FIG. 98

**node configuration of twisted line**

| | on Hook | on Cross |
|---|---|---|
| **Height (side view)** | 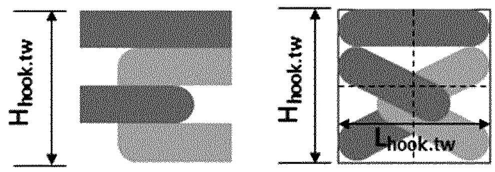 | 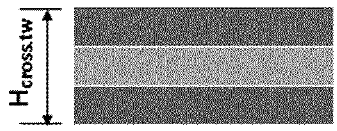 |
| Height scale factor ($SF_h$) | 4 | 3 |
| Nominal height | $H_{hook.tw} = \Phi \times SF_{hh.tw}$ | $H_{cross.tw} = \Phi \times SF_{hc.tw}$ |
| **Width & Length (top view)** | 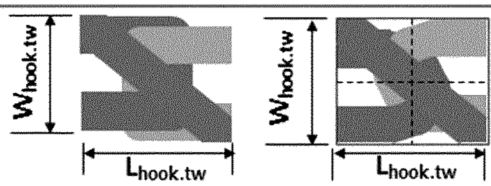 | 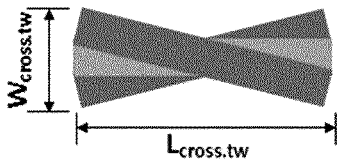 |
| Width scale factor ($SF_w$) | 3.3 | 3 |
| Nominal width | $W_{hook.tw} = \Phi \times SF_{wh.tw}$ | $W_{cross.tw} = \Phi \times SF_{wc.tw}$ |
| Length scale factor ($SF_l$) | 4 | 5 |
| Nominal length | $L_{hook.tw} = \Phi \times SF_{lh.tw}$ | $L_{cross.tw} = \Phi \times SF_{lc.tw}$ |
| **Nominal section** | 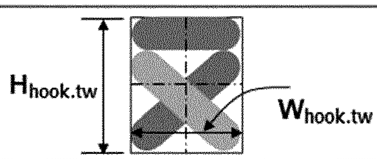 | 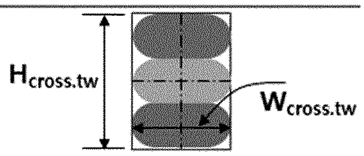 |

**INTERNATIONAL SEARCH REPORT**

International application No.
**PCT/KR2017/012057**

A. CLASSIFICATION OF SUBJECT MATTER

***A61F 2/91(2013.01)i, A61L 31/02(2006.01)i, A61L 31/04(2006.01)i, A61L 31/14(2006.01)i, A61F 2/915(2013.01)i***

According to International Patent Classification (IPC) or to both national classification and IPC

B. FIELDS SEARCHED

Minimum documentation searched (classification system followed by classification symbols)
A61F 2/91; A61F 2/86; A61F 2/88; A61F 2/82; A61F 2/89; A61M 29/02; A61L 31/02; A61L 31/04; A61L 31/14; A61F 2/915

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: stent, mesh, production, wire, node, coordinate, start, termination, pattern, matrix

C. DOCUMENTS CONSIDERED TO BE RELEVANT

| Category* | Citation of document, with indication, where appropriate, of the relevant passages | Relevant to claim No. |
|---|---|---|
| X | KR 10-1382263 B1 (SHIN, Kyong Min et al.) 07 April 2014<br>See paragraphs [0020]-[0059]; claims 1-3; figures 1-13. | 16-21 |
| A | KR 10-2016-0110893 A (UTAH-INHA DDS & ADVANCED THERAPEUTICS RESEARCH CENTER et al.) 22 September 2016<br>See the entire document. | 16-21 |
| A | KR 10-2013-0106115 A (M.I TECH CO., LTD.) 27 September 2013<br>See the entire document. | 16-21 |
| A | KR 10-0633020 B1 (STANDARD SCI-TECH INC.) 11 October 2006<br>See the entire document. | 16-21 |
| A | KR 10-2002-0078126 A (TAEWOONG MEDICAL CO., LTD. et al.) 18 October 2002<br>See the entire document. | 16-21 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 19 MARCH 2018 (19.03.2018) | **19 MARCH 2018 (19.03.2018)** |

| Name and mailing address of the ISA/KR | Authorized officer |
|---|---|
| Korean Intellectual Property Office<br>Government Complex-Daejeon, 189 Seonsa-ro, Daejeon 302-701, Republic of Korea<br>Facsimile No. +82-42-481-8578 | Telephone No. |

Form PCT/ISA/210 (second sheet) (January 2015)

INTERNATIONAL SEARCH REPORT

International application No.
PCT/KR2017/012057

**Box No. II Observations where certain claims were found unsearchable (Continuation of item 2 of first sheet)**

This international search report has not been established in respect of certain claims under Article 17(2)(a) for the following reasons:

1. ☒ Claims Nos.: **1-15**
because they relate to subject matter not required to be searched by this Authority, namely:
Claims 1 to 15 pertain to a scientific or mathematical theory, and thus pertain to subject matter on which the International Searching Authority is not required to carry out an international search under the provisions of PCT Article 17(2)(a)(i) and PCT Rule 39.1(i).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

The invention of group 1: claims 16 to 21 pertain to a method for manufacturing a wire-mesh stent by selecting a wire-mesh movement type from a sandglass type or a radial movement type.
The invention of group 2: claims 22 and 23 pertain to a stent multi-deformable structure patterning method for making a wire-mesh by selecting a primitive n-gon.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☒ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

**INTERNATIONAL SEARCH REPORT**
Information on patent family members

International application No.
**PCT/KR2017/012057**

| Patent document cited in search report | Publication date | Patent family member | Publication date |
|---|---|---|---|
| KR 10-1382263 B1 | 07/04/2014 | KR 10-2013-0111740 A | 11/10/2013 |
| KR 10-2016-0110893 A | 22/09/2016 | AU 2016-229667 A1 | 15/09/2016 |
| | | CA 2972622 A1 | 15/09/2016 |
| | | CN 107249654 A | 13/10/2017 |
| | | EP 3269401 A1 | 17/01/2018 |
| | | JP 2018-502687 A | 01/02/2018 |
| | | US 2018-0008752 A1 | 11/01/2018 |
| | | WO 2016-144096 A1 | 15/09/2016 |
| KR 10-2013-0106115 A | 27/09/2013 | WO 2013-141441 A1 | 26/09/2013 |
| KR 10-0633020 B1 | 11/10/2006 | CN 101184449 A | 21/05/2008 |
| | | CN 101184449 B | 16/06/2010 |
| | | EP 1903984 A1 | 02/04/2008 |
| | | EP 1903984 B1 | 19/10/2016 |
| | | JP 2009-501049 A | 15/01/2009 |
| | | JP 4764925 B2 | 07/09/2011 |
| | | US 2008-0167709 A1 | 10/07/2008 |
| | | US 2011-0125250 A1 | 26/05/2011 |
| | | US 8210084 B2 | 03/07/2012 |
| | | WO 2007-011122 A1 | 25/01/2007 |
| KR 10-2002-0078126 A | 18/10/2002 | KR 10-0457629 B1 | 18/11/2004 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4655771 A
- KR 267019
- KR 1020080076586